# EUROPEAN PATENT APPLICATION

(11) **EP 3 017 826 A1**
(43) Date of publication of application: **11.05.2016**
(21) Application number: 15194612.6
(22) Date of filing: 24.03.2010
(51) Int. Cl.: A61K 39/02, A61K 39/09, A61K 39/095

(54) **COMBINATIONS OF MENINGOCOCCAL FACTOR H BINDING PROTEIN AND PNEUMOCOCCAL SACCHARIDE CONJUGATES**

(30) Priority: 24.03.2009 US 163005 P; 07.07.2009 US 270407 P
(62) Divisional of application: 10716044.2
(71) Applicant: Novartis AG, 4056 Basel (CH)
(72) Inventor: GUILIANI, Marzia, 53100 Siena (IT); RUGGIERO, Paolo, 53100 Siena (IT)
(74) Representative: Carpmaels & Ransford LLP

(57) **Abstract**

An immunogenic composition comprising: (i) a conjugated pneumococcal capsular saccharide; and (ii) a meningococcal factor H binding protein (fHBP) antigen, and not including meningococcal outer membrane vesicles, is useful for immunising a subject against bacterial meningitis.

## Description

### TECHNICAL FIELD

This invention is in the field of combination vaccines, in particular those containing both a conjugated pneumococcal capsular saccharide and a meningococcal fHBP antigen.

### BACKGROUND ART

*Neisseria meningitidis* (meningococcus) is a Gram-negative spherical bacterium. Current meningococcal vaccines are also based on capsular saccharides. These include monovalent serogroup C conjugate vaccines (MENJUGATE™, MENINGITEC™ and NEISVAC-C™) and 4-valent conjugate mixtures for serogroups A, C, W135 and Y (MENACTRA™). There is currently no useful vaccine authorised for general use against serogroup B ('MenB'). Current research efforts for making a MenB vaccine are focusing on outer membrane vesicles (*e*.*g*. MENZB™, HEXAMEN™, NONAMEN™) or on purified components from the outer membrane, such as lipooligosaccharide and outer membrane proteins.

*Streptococcus pneumoniae* (pneumococcus) is a Gram-positive spherical bacterium. Current pneumococcal vaccines are based on capsular saccharides. The authorised pediatric vaccines are (a) PREVNAR™, which is a 7-valent mixture of conjugated saccharides from serotypes 4, 6B, 9V, 14, 18C, 19F & 23F, (b) SYNFLORIX™, a 10-valent conjugate mixture which also covers serotypes 1, 5 and 7F, and (c) PREVNAR 13™, a 13-valent conjugate mixture which also covers serotypes 3, 6A & 19A. Other 9-valent, 10-valent, 11-valent and 13-valent conjugate combinations are also known. The same 7 serotypes as PREVNAR™ have also been conjugated to an outer membrane vesicle complex ('OMPC') from a serogroup B strain of meningococcus [1].

Reference 2 discloses a composition for immunising against both pneumococcus and MenB, formed by combining a 13-valent pneumococcal conjugate vaccine ('13vPnC', Wyeth) with a 9-valent MenB outer membrane vesicle vaccine (NONAMEN™, NVI). A similar combination product is discussed in reference 3.

There remains a need for further and improved combination vaccines for protecting against both serogroup B meningococcus and pneumococcus.

### DISCLOSURE OF THE INVENTION

Unlike the compositions disclosed in reference 2, which used a combination of three different engineered outer membrane vesicles, the meningococcal serogroup B antigen in compositions of the present invention is based on a small number of purified antigens. The aim is to avoid the presence of complex or undefined mixtures of MenB antigens (*e*.*g*. outer membrane vesicles, as used in references 1 and 2) in the composition. In particular, compositions of the invention include a purified meningococcal factor H binding protein (fHBP) antigen. It has been found that addition of pneumococcal conjugates to fHBP can enhance the anti-meningococcal response, and addition of fHBP to pneumococcal conjugates can enhance the anti-pneumococcus response.

Thus the invention provides an immunogenic composition comprising: (i) a conjugated pneumococcal capsular saccharide; and (ii) a meningococcal factor H binding protein (fHBP) antigen. The composition preferably does not include meningococcal outer membrane vesicles (including both naturally-occurring membrane vesicles that form spontaneously during bacterial growth and are released into culture medium, and artificial outer membrane vesicles that are formed *e*.*g*. by detergent treatment or sonication of meningococci).

A preferred composition includes: (i) an aluminium phosphate adjuvant; (ii) a conjugated pneumococcal capsular saccharide from each of at least pneumococcal serotypes 4, 6B, 9V, 14, 18C, 19F, and 23F, each of said saccharides being conjugated to a CRM197 carrier protein; and (iii) at least two different meningococcal factor H binding protein antigens, each of which is at least partially adsorbed to aluminium phosphate. A conjugated pneumococcal saccharide may also be adsorbed to aluminium phosphate. The composition may include sodium chloride and/or a buffer.

The invention also provides a process for preparing an immunogenic composition of the invention, comprising steps of: (i) mixing at least one conjugated pneumococcal capsular saccharide with an aluminium phosphate adjuvant to form a conjugate/adjuvant mixture; and (ii) mixing the conjugate/adjuvant mixture with at least one meningococcal factor H binding protein.

The invention also provides a process for preparing an immunogenic composition of the invention, comprising steps of: (i) mixing at least one conjugated pneumococcal capsular saccharide with at least one meningococcal factor H binding protein to form an antigen mixture; and (ii) mixing the antigen mixture with an aluminium phosphate adjuvant.

The invention also provides a process for preparing an immunogenic composition of the invention, comprising steps of: (i) mixing at least one meningococcal factor H binding protein with an aluminium phosphate adjuvant to form a protein/adjuvant mixture; and (ii) mixing the protein/adjuvant mixture with at least one conjugated pneumococcal capsular saccharide.

In a less preferred embodiment, the invention also provides a process for preparing an immunogenic composition of the invention, comprising steps of: (i) mixing at least one meningococcal factor H binding protein with an aluminium phosphate adjuvant to form a protein/adjuvant mixture; (ii) mixing at least one conjugated pneumococcal capsular saccharide with an aluminium phosphate adjuvant to form a conjugate/adjuvant mixture; and (iii) mixing the protein/adjuvant mixture and conjugate/adjuvant mixture.

Preferably a process of the invention does not include a step of mixing an aluminium hydroxide adjuvant with any of (i) a meningococcal factor H binding protein, (ii) an aluminium phosphate adjuvant, (iii) a conjugated pneumococcal capsular saccharide, (iv) a conjugate/adjuvant mixture, (v) an antigen mixture, or (vi) a protein/adjuvant mixture. Thus aluminium hydroxide is not added to be a component of the immunogenic composition.

### Conjugated pneumococcal capsular saccharide(s)

Compositions of the invention include at least one pneumococcal capsular saccharide. The capsular saccharide is conjugated to a carrier protein.

The invention can include capsular saccharide from one or more different pneumococcal serotypes. Where a composition includes saccharide antigens from more than one serotype, these are preferably prepared separately, conjugated separately, and then combined. Methods for purifying pneumococcal capsular saccharides are known in the art (*e.g*. see reference 4) and vaccines based on purified saccharides from 23 different serotypes have been known for many years. Improvements to these methods have also been described *e*.*g*. for serotype 3 as described in reference 5, or for serotypes 1, 4, 5, 6A, 6B, 7F and 19A as described in reference 6.

Pneumococcal capsular saccharide(s) will typically be selected from the following serotypes: 1,2,3, 4, 5, 6A, 6B, 7F, 8, 9N, 9V, 10A, 11A, 12F, 14, 15B, 17F, 18C, 19A, 19F, 20, 22F, 23F and/or 33F. Thus, in total, a composition may include a capsular saccharide from 1, 2, 3, 4, 5, 6, 7, 8, 9, 10, 11, 12, 13, 14, 15, 16, 17, 18, 19, 20, 21, 22, 23 or more different serotypes. Compositions which include at least serotype 6B saccharide are useful.

A useful combination of serotypes is a 7-valent combination *e*.*g*. including capsular saccharide from each of serotypes 4, 6B, 9V, 14, 18C, 19F, and 23F. Another useful combination is a 9-valent combination *e*.*g*. including capsular saccharide from each of serotypes 1, 4, 5, 6B, 9V, 14, 18C, 19F and 23F. Another useful combination is a 10-valent combination *e*.*g*. including capsular saccharide from each of serotypes 1, 4, 5, 6B, 7F, 9V, 14, 18C, 19F and 23F. An 11-valent combination may further include saccharide from serotype 3. A 12-valent combination may add to the 10-valent mixture: serotypes 6A and 19A; 6A and 22F; 19A and 22F; 6A and 15B; 19A and 15B; or 22F and 15B. A 13-valent combination may add to the 11-valent mixture: serotypes 19A and 22F; 8 and 12F; 8 and 15B; 8 and 19A; 8 and 22F; 12F and 15B; 12F and 19A; 12F and 22F; 15B and 19A; 15B and 22F; 6A and 19A, *etc.*

Thus a useful 13-valent combination includes capsular saccharide from serotypes 1, 3, 4, 5, 6A, 6B, 7F, 9V, 14, 18C, 19 (or 19A), 19F and 23F *e.g.* prepared as disclosed in references 7 to 10. One such combination includes serotype 6B saccharide at about 8µ/ml and the other 12 saccharides at concentrations of about 4µg/ml each. Another such combination includes serotype 6A and 6B saccharides at about 8µg/ml each and the other 11 saccharides at about 4µg/ml each.

Suitable carrier proteins for conjugates include bacterial toxins, such as diphtheria or tetanus toxins, or toxoids or mutants thereof. These are commonly used in conjugate vaccines. For example, the CRM 197 diphtheria toxin mutant is useful [11]. Other suitable carrier proteins include synthetic peptides [12,13], heat shock proteins [14,15], pertussis proteins [16,17], cytokines [18], lymphokines [18], hormones [18], growth factors [18], artificial proteins comprising multiple human CD4⁺ T cell epitopes from various pathogen-derived antigens [19] such as N19 [20], protein D from *H.influenzae* [21-23], pneumolysin [24] or its non-toxic derivatives [25], pneumococcal surface protein PspA [26], iron-uptake proteins [27], toxin A or B from *C.difficile* [28], recombinant *Pseudomonas aeruginosa* exoprotein A (rEPA) [29], *etc.* The OMPC used in reference 1 is excluded herein from possible carriers for pneumococcal saccharide because it is a meningococcal outer membrane vesicle.

Particularly useful carrier proteins for pneumococcal conjugate vaccines are CRM 197, tetanus toxoid, diphtheria toxoid and *H.influenzae* protein D. CRM197 is used in PREVNAR™. A 13-valent mixture may use CRM 197 as the carrier protein for each of the 13 conjugates, and CRM 197 may be present at about 55-60µg/ml.

Where a composition includes conjugates from more than one pneumococcal serotype, it is possible to use the same carrier protein for each separate conjugate, or to use different carrier proteins. In both cases, though, a mixture of different conjugates will usually be formed by preparing each serotype conjugate separately, and then mixing them to form a mixture of separate conjugates. Reference 30 describes potential advantages when using different carrier proteins in multivalent pneumococcal conjugate vaccines, but the PREVNAR™ product successfully uses the same carrier for each of seven different serotypes.

A carrier protein may be covalently conjugated to a pneumococcal saccharide directly or via a linker. Various linkers are known. For example, attachment may be via a carbonyl, which may be formed by reaction of a free hydroxyl group of a modified saccharide with CDI [31,32] followed by reaction with a protein to form a carbamate linkage. Carbodiimide condensation can be used [33]. An adipic acid linker can be used, which may be formed by coupling a free -NH₂ group (*e*.*g*. introduced to a saccharide by amination) with adipic acid (using, for example, diimide activation), and then coupling a protein to the resulting saccharide-adipic acid intermediate [34,35].Other linkers include β-propionamido [36], nitrophenyl-ethylamine [37], haloacyl halides [38], glycosidic linkages [39], 6-aminocaproic acid [40], N-succinimidyl-3-(2-pyridyldithio)-propionate (SPDP) [41], adipic acid dihydrazide ADH [42], C₄ to C₁₂ moieties [43], *etc.*

Conjugation via reductive amination can be used. The saccharide may first be oxidised with periodate to introduce an aldehyde group, which can then form a direct covalent linkage to a carrier protein via reductive amination *e*.*g*. to the ε-amino group of a lysine. If the saccharide includes multiple aldehyde groups per molecule then this linkage technique can lead to a cross-linked product, where multiple aldehydes react with multiple carrier amines. This cross-linking conjugation technique is particularly useful for at least pneumococcal serotypes 4, 6B, 9V, 14, 18C, 19F and 23F.

A pneumococcal saccharide may comprise a full-length intact saccharide as prepared from pneumococcus, and/or may comprise fragments of full-length saccharides *i.e.* the saccharides may be shorter than the native capsular saccharides seen in bacteria. The saccharides may thus be depolymerised, with depolymerisation occurring during or after saccharide purification but before conjugation. Depolymerisation reduces the chain length of the saccharides. Depolymerisation can be used in order to provide an optimum chain length for immunogenicity and/or to reduce chain length for physical manageability of the saccharides. Where more than one pneumococcal serotype is used then it is possible to use intact saccharides for each serotype, fragments for each serotype, or to use intact saccharides for some serotypes and fragments for other serotypes.

Where a composition includes saccharide from any of serotypes 4, 6B, 9V, 14, 19F and 23F, these saccharides are preferably intact. In contrast, where a composition includes saccharide from serotype 18C, this saccharide is preferably depolymerised.

A serotype 3 saccharide may also be depolymerised, For instance, a serotype 3 saccharide can be subjected to acid hydrolysis for depolymerisation [7] *e*.*g*. using acetic acid. The resulting fragments may then be oxidised for activation (*e*.*g*. periodate oxidation, maybe in the presence of bivalent cations *e.g.* with MgCl₂), conjugated to a carrier (*e.g.* CRM197) under reducing conditions (*e.g.* using sodium cyanoborohydride), and then (optionally) any unreacted aldehydes in the saccharide can be capped (*e.g.* using sodium borohydride) [7]. Conjugation may be performed on lyophilized material *e.g.* after co-lyophilizing activated saccharide and carrier.

A serotype 1 saccharide may be at least partially de-O-acetylated *e*.*g*. achieved by alkaline pH buffer treatment [8] such as by using a bicarbonate/carbonate buffer. Such (partially) de-O-acetylated saccharides can be oxidised for activation (*e.g.* periodate oxidation), conjugated to a carrier (*e.g.* CRM197) under reducing conditions (*e*.*g*. using sodium cyanoborohydride), and then (optionally) any unreacted aldehydes in the saccharide can be capped (*e*.*g*. using sodium borohydride) [8]. Conjugation may be performed on lyophilized material *e*.*g*. after co-lyophilizing activated saccharide and carrier.

A serotype 19A saccharide may be oxidised for activation (*e*.*g*. periodate oxidation), conjugated to a carrier (*e*.*g*. CRM197) in DMSO under reducing conditions, and then (optionally) any unreacted aldehydes in the saccharide can be capped (*e*.*g*. using sodium borohydride) [44]. Conjugation may be performed on lyophilized material *e*.*g*. after co-lyophilizing activated saccharide and carrier.

One or more pneumococcal capsular saccharide conjugates may be present in lyophilised form.

Pneumococcal conjugates can ideally elicit anticapsular antibodies that bind to the relevant saccharide *e.g.* elicit an anti-saccharide antibody level ≥0.20µg/mL [45]. The antibodies may be evaluated by enzyme immunoassay (EIA) and/or measurement of opsonophagocytic activity (OPA). The EIA method has been extensively validated and there is a link between antibody concentration and vaccine efficacy.

### Meningococcal factor H binding protein(s)

Compositions of the invention include at least one meningococcal factor H binding protein (fHBP).

The fHBP antigen has been characterised in detail. It has also been called protein '741' [SEQ IDs 2535 & 2536 in ref. 56], 'NMB1870', 'GNA1870' [refs. 46-48], 'P2086', 'LP2086' or 'ORF2086' [49-51]. It is naturally a lipoprotein and is expressed across all meningococcal serogroups. The structure of fHbp's C-terminal immunodominant domain ('fHbpC') has been determined by NMR [52]. This part of the protein forms an eight-stranded β-barrel, whose strands are connected by loops of variable lengths. The barrel is preceded by a short α-helix and by a flexible N-terminal tail.

The fHBP antigen falls into three distinct variants [53] and it has been found that serum raised against a given family is bactericidal within the same family, but is not active against strains which express one of the other two families *i.e.* there is intra-family cross-protection, but not inter-family cross-protection. Compositions of the invention can include a single fHBP variant, but advantageously include fHBP from two or three of the variants.

Where a composition comprises a single fHBP variant, it may include one of the following:
(a) a first polypeptide comprising a first amino acid sequence, where the first amino acid sequence comprises an amino acid sequence (i) having at least *a%* sequence identity to SEQ ID NO: 1 and/or (ii) consisting of a fragment of at least *x* contiguous amino acids from SEQ ID NO: 1;
(b) a second polypeptide, comprising a second amino acid sequence, where the second amino acid sequence comprises an amino acid sequence (i) having at least *b%* sequence identity to SEQ ID NO: 2 and/or (ii) consisting of a fragment of at least *y* contiguous amino acids from SEQ ID NO: 2;
(c) a third polypeptide, comprising a third amino acid sequence, where the third amino acid sequence comprises an amino acid sequence (i) having at least *c%* sequence identity to SEQ ID NO: 3 and/or (ii) consisting of a fragment of at least *z* contiguous amino acids from SEQ ID NO: 3.

Where a composition comprises two different meningococcal fHBP antigens, it may include a combination of: (i) a first and second polypeptide as defined above; (ii) a first and third polypeptide as defined above; or (iii) a second and third polypeptide as defined above. A combination of a first and third polypeptide is preferred.

Where a composition comprises two different meningococcal fHBP antigens, although these may share some sequences in common, the first, second and third polypeptides have different fHBP amino acid sequences.

A polypeptide comprising the first amino acid sequence will, when administered to a subject, elicit an antibody response comprising antibodies that bind to the wild-type meningococcus protein having nascent amino acid sequence SEQ ID NO: 60 (MC58). In some embodiments some or all of these antibodies do not bind to the wild-type meningococcus protein having nascent amino acid sequence SEQ ID NO: 61 or to the wild-type meningococcus protein having nascent amino acid sequence SEQ ID NO: 62.

A polypeptide comprising the second amino acid sequence will, when administered to a subject, elicit an antibody response comprising antibodies that bind to the wild-type meningococcus protein having nascent amino acid sequence SEQ ID NO: 61 (2996). In some embodiments some or all of these antibodies do not bind to the wild-type meningococcus protein having nascent amino acid sequence SEQ ID NO: 60 or to the wild-type meningococcus protein having nascent amino acid sequence SEQ ID NO: 62.

A polypeptide comprising the third amino acid sequence will, when administered to a subject, elicit an antibody response comprising antibodies that bind to the wild-type meningococcus protein having nascent amino acid sequence SEQ ID NO: 62 (M1239). In some embodiments some or all of these antibodies do not bind to the wild-type meningococcus protein having nascent amino acid sequence SEQ ID NO: 60 or to the wild-type meningococcus protein having nascent amino acid sequence SEQ ID NO: 61.

In some embodiments the fragment of at least x contiguous amino acids from SEQ ID NO: 1 is not also present within SEQ ID NO: 2 or within SEQ ID NO: 3. Similarly, the fragment of at least *y* contiguous amino acids from SEQ ID NO: 2 might not also be present within SEQ ID NO: 1 or within SEQ ID NO: 3. Similarly, the fragment of at least z contiguous amino acids from SEQ ID NO: 3 might not also be present within SEQ ID NO: 1 or within SEQ ID NO: 2. In some embodiments, when said fragment from one of SEQ ID NOs: 1 to 3 is aligned as a contiguous sequence against the other two SEQ ID NOs, the identity between the fragment and each of the other two SEQ ID NOs is less than 75% *e.g.* less than 70%, less than 65%, less than 60%, *etc.*

The value of *a* is at least 80 *e.g.* 82, 84, 86, 88, 90, 92, 94, 95, 96, 97, 98, 99 or more. The value of *b* is at least 80 *e.g.* 82, 84, 86, 88, 90, 92, 94, 95, 96, 97, 98, 99 or more. The value of *c* is at least 80 *e.g.* 82, 84, 86, 88, 90, 92, 94, 95, 96, 97, 98, 99 or more. The values of *a, b* and *c* may be the same or different. In some embodiments, *a b* and *c* are identical.

The value of x is at least 7 *e*.*g*. 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 120, 140, 160, 180, 200, 225, 250). The value of *y* is at least 7 *e*.*g*. 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 120, 140, 160, 180, 200, 225, 250). The value ofz is at least 7 *e*.*g*. 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 120, 140, 160, 180, 200, 225, 250). The values of *x, y* and *z* may be the same or different. In some embodiments, *x y* and z are identical.

Fragments preferably comprise an epitope from the respective SEQ ID NO: sequence. Other useful fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of the respective SEQ ID NO: while retaining at least one epitope thereof.

Amino acid sequences used with the invention may, compared to SEQ ID NOs: 1 2 or 3, include one or more (*e.g*. 1, 2, 3, 4, 5, 6, 7, 8, 9, 10, *etc*.) conservative amino acid replacements *i*.*e*. replacements of one amino acid with another which has a related side chain. Genetically-encoded amino acids are generally divided into four families: (1) acidic *i.e.* aspartate, glutamate; (2) basic *i.e.* lysine, arginine, histidine; (3) non-polar *i.e.* alanine, valine, leucine, isoleucine, proline, phenylalanine, methionine, tryptophan; and (4) uncharged polar *i.e.* glycine, asparagine, glutamine, cysteine, serine, threonine, tyrosine. Phenylalanine, tryptophan, and tyrosine are sometimes classified jointly as aromatic amino acids. In general, substitution of single amino acids within these families does not have a major effect on the biological activity. The polypeptides may have one or more (*e.g.* 1, 2, 3, 4, 5, 6, 7, 8, 9, 10, *etc*.) single amino acid deletions relative to a reference sequence. The polypeptides may also include one or more *(e.g.* 1, 2, 3, 4, 5, 6, 7, 8, 9, 10, *etc*.) insertions (*e*.*g*. each of 1, 2, 3, 4 or 5 amino acids) relative to a reference sequence.

A useful first amino acid sequence has at least 85% identity (*e*.*g*. >95% or 100%) to SEQ ID NO: 1. Another useful first amino acid sequence has at least 95% identity (*e*.*g*. >98% or 100%) to SEQ ID NO: 66. Another useful first amino acid sequence has at least 95% identity (*e*.*g*. >98% or 100%) to SEQ ID NO: 67.

A useful third amino acid sequence has at least 85% identity (*e*.*g*. >95% or 100%) to SEQ ID NO: 3. Another useful third amino acid sequence has at least 95% identity (*e*.*g*. >98% or 100%) to SEQ ID NO: 68.

Combinations comprising a mixture of first and third sequences based around SEQ ID NOs: 66 and 68 (or their close variants) are particularly useful. Another useful combination comprises a mixture of first and third sequences based around a mixture of SEQ ID NOs: 67 and 68 (or their close variants). Thus a composition may comprise a polypeptide comprising amino acid sequence SEQ ID NO: 63 and a polypeptide comprising amino acid sequence SEQ ID NO: 65.

In some embodiments fHBP polypeptide(s) are lipidated *e.g.* at a N-terminus cysteine. In other embodiments, however, fHBP polypeptide(s) are not lipidated. For lipidated fHBPs, lipids attached to cysteines will usually include palmitoyl residues *e*.*g*. as tripalmitoyl-S-glyceryl-cysteine (Pam3Cys), dipalmitoyl-S-glyceryl cysteine (Pam2Cys), N-acetyl (dipalmitoyl-S-glyceryl cysteine), *etc*. Examples of mature lipidated fHBP sequences are SEQ ID NO: 63 (including SEQ ID NO: 66), SEQ ID NO: 64 (including SEQ ID NO: 67), and SEQ ID NO: 65 (including SEQ ID NO: 68).

Administration of a fHBP will preferably elicit antibodies which can bind to a meningococcal polypeptide consisting of amino acid sequence SEQ ID NO: 1, 2 or 3. Advantageous fHBP antigens for use with the invention can elicit bactericidal anti-meningococcal antibodies after administration to a subject.

The total amount of a fHBP polypeptide will usually be between 1 and 500µg/dose *e*.*g*. between 60 and 200µg/dose or between 120 and 500µg/ml.

### Further antigen(s)

In addition to conjugated pneumococcal capsular saccharide(s) and meningococcal factor H binding protein(s), compositions of the invention can include further antigens from meningococcus, pneumococcus and/or further pathogen(s).

### Meningococcal polypeptide antigens

In addition to including meningococcal fHBP polypeptide antigen(s), a composition may include one or more further meningococcal polypeptide antigen(s). Thus a composition may include a polypeptide antigen selected from the group consisting of: 287, NadA, NspA, HmbR, NhhA, App, and/or Omp85. These antigens will usefully be present as purified polypeptides *e*.*g.* recombinant polypeptides. The antigen will preferably elicit bactericidal anti-meningococcal antibodies after administration to a subject. In some embodiments of the invention, immunogenic compositions either include only one meningococcal PorA serosubtype or, preferably, include no meningococcal PorA outer membrane protein.

A composition of the invention may include a 287 antigen. The 287 antigen was included in the published genome sequence for meningococcal serogroup B strain MC58 [54] as gene NMB2132 (GenBank accession number GI:7227388; SEQ ID NO: 9 herein). The sequences of 287 antigen from many strains have been published since then. For example, allelic forms of 287 can be seen in Figures 5 and 15 of reference 55, and in example 13 and figure 21 of reference 56 (SEQ IDs 3179 to 3184 therein). Various immunogenic fragments of the 287 antigen have also been reported. Preferred 287 antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 9; and/or (b) comprising a fragment of at least 'n' consecutive amino acids of SEQ ID NO: 9, wherein'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 9. The most useful 287 antigens of the invention can elicit antibodies which, after administration to a subject, can bind to a meningococcal polypeptide consisting of amino acid sequence SEQ ID NO: 9. Advantageous 287 antigens for use with the invention can elicit bactericidal anti-meningococcal antibodies after administration to a subject.

A composition of the invention may include a NadA antigen. The NadA antigen was included in the published genome sequence for meningococcal serogroup B strain MC58 [54] as gene NMB1994 (GenBank accession number GI:7227256; SEQ ID NO: 10 herein). The sequences of NadA antigen from many strains have been published since then, and the protein's activity as a Neisserial adhesin has been well documented. Various immunogenic fragments of NadA have also been reported. Preferred NadA antigens for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 10; and/or (b) comprising a fragment of at least 'n' consecutive amino acids of SEQ ID NO: 10, wherein 'n' is 7 or more (*e.g*. 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 10. The most useful NadA antigens of the invention can elicit antibodies which, after administration to a subject, can bind to a meningococcal polypeptide consisting of amino acid sequence SEQ ID NO: 10. Advantageous NadA antigens for use with the invention can elicit bactericidal anti-meningococcal antibodies after administration to a subject. SEQ ID NO: 6 is one such fragment.

A composition of the invention may include a NspA antigen. The NspA antigen was included in the published genome sequence for meningococcal serogroup B strain MC58 [54] as gene NMB0663 (GenBank accession number GI:7225888; SEQ ID NO: 11 herein). The antigen was previously known from references 57 & 58. The sequences of NspA antigen from many strains have been published since then. Various immunogenic fragments of NspA have also been reported. Preferred NspA antigens for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 11; and/or (b) comprising a fragment of at least 'n' consecutive amino acids of SEQ ID NO: 11, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 11. The most useful NspA antigens of the invention can elicit antibodies which, after administration to a subject, can bind to a meningococcal polypeptide consisting of amino acid sequence SEQ ID NO: 11. Advantageous NspA antigens for use with the invention can elicit bactericidal anti-meningococcal antibodies after administration to a subject.

Compositions of the invention may include a meningococcal HmbR antigen. The full-length HmbR sequence was included in the published genome sequence for meningococcal serogroup B strain MC58 [54] as gene NMB1668 (SEQ ID NO: 7 herein). Reference 59 reports a HmbR sequence from a different strain (SEQ ID NO: 8 herein). SEQ ID NOs: 7 and 8 differ in length by 1 amino acid and have 94.2% identity. The invention can use a polypeptide that comprises a full-length HmbR sequence, but it will often use a polypeptide that comprises a partial HmbR sequence. Thus in some embodiments a HmbR sequence used according to the invention may comprise an amino acid sequence having at least *i%* sequence identity to SEQ ID NO: 7, where the value of *i* is 50, 60, 70, 80, 90, 95, 99 or more. In other embodiments a HmbR sequence used according to the invention may comprise a fragment of at least *j* consecutive amino acids from SEQ ID NO: 7, where the value of *j* is 7, 8, 10, 12, 14, 16, 18, 20, 25, 30, 35, 40, 50, 60, 70, 80, 90, 100, 150, 200, 250 or more. In other embodiments a HmbR sequence used according to the invention may comprise an amino acid sequence (i) having at least *i%* sequence identity to SEQ ID NO: 7 and/or (ii) comprising a fragment of at least *j* consecutive amino acids from SEQ ID NO: 7. Preferred fragments of *j* amino acids comprise an epitope from SEQ ID NO: 7. Such epitopes will usually comprise amino acids that are located on the surface of HmbR. Useful epitopes include those with amino acids involved in HmbR's binding to haemoglobin, as antibodies that bind to these epitopes can block the ability of a bacterium to bind to host haemoglobin. The topology of HmbR, and its critical functional residues, were investigated in reference 60. The most useful HmbR antigens of the invention can elicit antibodies which, after administration to a subject, can bind to a meningococcal polypeptide consisting of amino acid sequence SEQ ID NO: 7. Advantageous HmbR antigens for use with the invention can elicit bactericidal anti-meningococcal antibodies after administration to a subject.

A composition of the invention may include a NhhA antigen. The NhhA antigen was included in the published genome sequence for meningococcal serogroup B strain MC58 [54] as gene NMB0992 (GenBank accession number GI:7226232; SEQ ID NO: 12 herein). The sequences of NhhA antigen from many strains have been published since *e.g.* refs 55 & 61, and various immunogenic fragments of NhhA have been reported. It is also known as Hsf. Preferred NhhA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 12; and/or (b) comprising a fragment of at least 'n' consecutive amino acids of SEQ ID NO: 12, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 12. The most useful NhhA antigens of the invention can elicit antibodies which, after administration to a subject, can bind to a meningococcal polypeptide consisting of amino acid sequence SEQ ID NO: 12. Advantageous NhhA antigens for use with the invention can elicit bactericidal anti-meningococcal antibodies after administration to a subject.

A composition of the invention may include an App antigen. The App antigen was included in the published genome sequence for meningococcal serogroup B strain MC58 [54] as gene NMB1985 (GenBank accession number GI:7227246; SEQ ID NO: 13 herein). The sequences of App antigen from many strains have been published since then. Various immunogenic fragments of App have also been reported. Preferred App antigens for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 13; and/or (b) comprising a fragment of at least 'n' consecutive amino acids of SEQ ID NO: 13, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 13. The most useful App antigens of the invention can elicit antibodies which, after administration to a subject, can bind to a meningococcal polypeptide consisting of amino acid sequence SEQ ID NO: 13. Advantageous App antigens for use with the invention can elicit bactericidal anti-meningococcal antibodies after administration to a subject.

A composition of the invention may include an Omp85 antigen. The Omp85 antigen was included in the published genome sequence for meningococcal serogroup B strain MC58 [54] as gene NMB0182 (GenBank accession number GI:7225401; SEQ ID NO: 14 herein). The sequences of Omp85 antigen from many strains have been published since then. Further information on Omp85 can be found in references 62 and 63. Various immunogenic fragments of Omp85 have also been reported. Preferred Omp85 antigens for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 14; and/or (b) comprising a fragment of at least 'n' consecutive amino acids of SEQ ID NO: 14, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 14. The most useful Omp85 antigens of the invention can elicit antibodies which, after administration to a subject, can bind to a meningococcal polypeptide consisting of amino acid sequence SEQ ID NO: 14. Advantageous Omp85 antigens for use with the invention can elicit bactericidal anti-meningococcal antibodies after administration to a subject.

### Meningococcal lipooligosaccharide

In addition to including meningococcal fHBP polypeptide antigen(s), a composition may include one or more meningococcal lipooligosaccharide (LOS) antigen(s). Meningococcal LOS is a glucosamine-based phospholipid that is found in the outer monolayer of the outer membrane of the bacterium. It includes a lipid A portion and a core oligosaccharide region, with the lipid A portion acting as a hydrophobic anchor in the membrane. Heterogeneity within the oligosaccharide core generates structural and antigenic diversity among different meningococcal strains, which has been used to subdivide the strains into 12 immunotypes (L1 to L12). The invention may use LOS from any immunotype *e.g*. from L1, L2, L3, L4, L5, L6, L7 and/or L8.

The L2 and L3 α-chains naturally include lacto-N-neotetraose (LNnT). Where the invention uses LOS from a L2 or L3 immunotype this LNnT may be absent. This absence can be achieved conveniently by using mutant strains that are engineered to disrupt their ability to synthesise the LNnT tetrasaccharide within the α-chain. It is known to achieve this goal by knockout of the enzymes that are responsible for the relevant biosynthetic additions [64,65]. For instance, knockout of the LgtB enzyme prevents addition of the terminal galactose of LNnT, as well as preventing downstream addition of the α-chain's terminal sialic acid. Knockout of the LgtA enzyme prevents addition of the N-acetyl-glucosamine of LNnT, and also the downstream additions. LgtA knockout may be accompanied by LgtC knockout. Similarly, knockout of the LgtE and/or GalE enzyme prevents addition of internal galactose, and knockout of LgtF prevents addition of glucose to the Hep^{I} residue. Any of these knockouts can be used, singly or in combination, to disrupt the LNnT tetrasaccharide in a L2, L3, L4, L7 or L9 immunotype strain. Knockout of at least LgtB is preferred, as this provides a LOS that retains useful immunogenicity while removing the LNnT epitope.

In addition to, or in place of, mutations to disrupt the LNnT epitope, a knockout of the *galE* gene also provides a useful modified LOS, and a lipid A fatty transferase gene may similarly be knocked out [66]. At least one primary O-linked fatty acid may be removed from LOS [67].LOS having a reduced number of secondary acyl chains per LOS molecule can also be used [68].The LOS will typically include at least the GlcNAc-Hep₂phosphoethanolamine-KDO₂-Lipid A structure [69]. The LOS may include a GlcNAcβ1-3Galβ1-4Glc trisaccharide while lacking the LNnT tetrasaccharide.

LOS may be included in compositions of the invention in various forms. It may be used in purified form on its own. It may be conjugated to a carrier protein. When LOS is conjugated, conjugation may be via a lipid A portion in the LOS or by any other suitable moiety *e*.*g*. its KDO residues. If the lipid A moiety of LOS is absent then such alternative linking is required. Conjugation techniques for LOS are known from *e.g.* references 67, 69, 70, 71, *etc.* Useful carrier proteins for these conjugates are discussed above *e*.*g*. bacterial toxins, such as diphtheria or tetanus toxins, or toxoids or mutants thereof.

The LOS may be from a strain (*e.g.* a genetically-engineered meningococcal strain) which has a fixed (*i.e.* not phase variable) LOS immunotype as described in reference 72. For example, L2 and L3 LOS immunotypes may be fixed. Such strains may have a rate of switching between immunotypes that is reduced by more than 2-fold (even >50_fold) relative to the original wild-type strain. Reference 72 discloses how this result can be achieved by modification of the *IgtA* and/or *IgtG* gene products.

LOS may be O-acetylated on a GlcNac residue attached to its Heptose II residue *e*.*g*. for L3 [73].

An immunogenic composition can include more than one type of LOS *e.g.* LOS from meningococcal immunotypes L2 and L3. For example, the LOS combinations disclosed in reference 74 may be used.

A LOS antigen can preferably elicit bactericidal anti-meningococcal antibodies after administration to a subject.

However, preferred compositions of the invention are free from meningococcal lipooligosaccharide.

### Meningococcal capsular saccharine antigen(s)

In addition to including meningococcal fHBP polypeptide antigen(s), a composition may include one or more meningococcal capsular saccharide conjugates. A composition of the invention may include one or more conjugates of capsular saccharides from 1, 2, 3, or 4 of meningococcal serogroups A, C, W135 and Y *e*.*g*. A+C, A+W135, A+Y, C+W135, C+Y, W135+Y, A+C+W135, A+C+Y, A+W135+Y, A+C+W135+Y, *etc*. Compositions including a serogroup C saccharide or including saccharides from all four of serogroups A, C, W135 and Y are ideal.

The capsular saccharide of serogroup A meningococcus is a homopolymer of (α1 →6)-linked *N*-acetyl-D-mannosamine-1-phosphate, with partial O-acetylation in the C3 and C4 positions. Acetylation at the C-3 position can be 70-95%. Conditions used to purify the saccharide can result in de-O-acetylation (*e*.*g*. under basic conditions), but it is useful to retain OAc at this C-3 position. In some embodiments, at least 50% *(e.g.* at least 60%, 70%, 80%, 90%, 95% or more) of the mannosamine residues in a serogroup A saccharides are O-acetylated at the C-3 position. Acetyl groups can be replaced with blocking groups to prevent hydrolysis [75], and such modified saccharides are still serogroup A saccharides within the meaning of the invention.

The serogroup C capsular saccharide is a homopolymer of (α 2→9)-linked sialic acid (*N*-acetyl neuraminic acid, or 'NeuNAc'). The saccharide structure is written as →9)-Neu *p* NAc 7/8 OAc-(α2→. Most serogroup C strains have O-acetyl groups at C-7 and/or C-8 of the sialic acid residues, but about 15% of clinical isolates lack these O-acetyl groups [76,77].The presence or absence of OAc groups generates unique epitopes, and the specificity of antibody binding to the saccharide may affect its bactericidal activity against O-acetylated (OAc+) and de-O-acetylated (OAc-) strains [78-80]. Serogroup C saccharides used with the invention may be prepared from either OAc+ or OAc- strains. Licensed MenC conjugate vaccines include both OAc- (NEISVAC-C™) and OAc+ (MENJUGATE™ & MENINGITEC™) saccharides. In some embodiments, strains for production of serogroup C conjugates are OAc+ strains, *e.g.* of serotype 16, serosubtype P1.7a,1, *etc..* Thus C:16:P1.7a,1 OAc+ strains may be used. OAc+ strains in serosubtype P1.1 are also useful, such as the C11 strain.

The serogroup W135 saccharide is a polymer of sialic acid-galactose disaccharide units. Like the serogroup C saccharide, it has variable O-acetylation, but at sialic acid 7 and 9 positions [81]. The structure is written as: →4)-D-Neu*p*5Ac(7/9OAc)-α-(2→6)-D-Gal-α-(1→.

The serogroup Y saccharide is similar to the serogroup W135 saccharide, except that the disaccharide repeating unit includes glucose instead of galactose. Like serogroup W135, it has variable O-acetylation at sialic acid 7 and 9 positions [81]. The serogroup Y structure is written as: →4)-D-Neu*p*5Ac(7/9OA*c)*-α-(2→6)-D-Glc-α-(1→.

The saccharides used according to the invention may be O-acetylated as described above (*e*.*g*. with the same O-acetylation pattern as seen in native capsular saccharides), or they may be partially or totally de-O-acetylated at one or more positions of the saccharide rings, or they may be hyper-O-acetylated relative to the native capsular saccharides.

The saccharide moieties in conjugates may comprise full-length saccharides as prepared from meningococci, and/or may comprise fragments of full-length saccharides *i*.*e*. the saccharides may be shorter than the native capsular saccharides seen in bacteria. The saccharides may thus be depolymerised, with depolymerisation occurring during or after saccharide purification but before conjugation. Depolymerisation reduces the chain length of the saccharides. One depolymerisation method involves the use of hydrogen peroxide. Hydrogen peroxide is added to a saccharide (*e*.*g*. to give a final H₂O₂ concentration of 1%), and the mixture is then incubated (*e*.*g*. at about 55°C) until a desired chain length reduction has been achieved. Another depolymerisation method involves acid hydrolysis. Other depolymerisation methods are known in the art. The saccharides used to prepare conjugates for use according to the invention may be obtainable by any of these depolymerisation methods. Depolymerisation can be used in order to provide an optimum chain length for immunogenicity and/or to reduce chain length for physical manageability of the saccharides. In some embodiments, saccharides have the following range of average degrees of polymerisation (Dp): A=10-20; C=12-22; W135=15-25; Y=15-25. In terms of molecular weight, rather than Dp, useful ranges are, for all serogroups: <100kDa; 5kDa-75kDa; 7kDa-50kDa; 8kDa-35kDa; 12kDa-25kDa; 15kDa-22kDa.

In some embodiments, the average molecular weight for saccharides from each of meningococcal serogroups A, C, W135 and Y may be more than 50kDa *e.g.* ≥75kDa, ≥100kDa, ≥110kDa, ≥120kDa, ≥130kDa, *etc.* [82], and even up to 1500kDa, in particular as determined by MALLS. For instance: a MenA saccharide may be in the range 50-500kDa *e.g*. 60-80kDa; a MenC saccharide may be in the range 100-210kDa; a MenW135 saccharide maybe in the range 60-190kDa *e.g*.120-140kDa; and/or a MenY saccharide may be in the range 60-190kDa *e.g*.150-160kDa.

The mass of meningococcal saccharide per serogroup in a composition will usually be between 1µg and 20µg *e*.*g*. between 2 and 10 µg per serogroup, or about 4µg or about 5µg or about 10µg. Where conjugates from more than one serogroup are included then they may be present at substantially equal masses *e*.*g*. the mass of each serogroup's saccharide is within +10% of each other. As an alternative to an equal ratio, a double mass of serogroup A saccharide may be used. Thus a vaccine may include MenA saccharide at 10µg and MenC, W135 and Y saccharides at 5µg each.

Useful carrier proteins and linkage chemistries are discussed above. Useful carriers include diphtheria toxoid, tetanus toxoid and CRM 197.

Conjugates with a saccharide:protein ratio (w/w) of between 1:5 (*i.e.* excess protein) and 5:1 (*i.e.* excess saccharide) may be used *e*.*g*. ratios between 1:2 and 5:1 and ratios between 1:1.25 and 1:2.5. As described in reference 83, different meningococcal serogroup conjugates in a mixture can have different saccharide:protein ratios *e*.*g*. one may have a ratio of between 1:2 & 1:5, whereas another has a ratio between 5:1 & 1:1.99.

As described in reference 84, a mixture can include one conjugate with direct saccharide/protein linkage and another conjugate with linkage via a linker. This arrangement applies particularly when using saccharide conjugates from different meningococcal serogroups *e*.*g*. MenA and MenC saccharides may be conjugated via a linker, whereas MenW135 and MenY saccharides may be conjugated directly to a carrier protein.

### Pneumococcal polypeptide antigen(s)

In addition to including conjugated pneumococcal capsular saccharide(s), a composition may include one or more pneumococcal polypeptide antigen(s). Thus a composition may include one or more of: (1) a spr0057 antigen; (2) a spr0286 antigen; (3) a spr0565 antigen; (4) a spr1098 antigen; (5) a spr1345 antigen; (6) a spr1416 antigen; (7) a spr1418 antigen; (8) a spr0867 antigen; (9) a spr1431 antigen; (10) a pneumolysin; (11) a spr2021 antigen; (12) a spr0096 antigen; (13) a spr1433 antigen; and/or (14) a spr1707 antigen.

A composition may include one or more of: (1) a PspA polypeptide; (2) a PsaA polypeptide; (3) a PspC polypeptide; (4) a LytA polypeptide; (5) a PhtA polypeptide; (6) a PhtA polypeptide; (7) a PhtA polypeptide; and/or (8) a PhtD polypeptide.

A composition may include a subunit of a pneumococcal pilus, such as RrgA, RrgB and/or RrgC.

A pneumococcal polypeptide antigen can preferably elicit protective antibodies after administration to a subject.

The original 'spr0057' sequence was annotated in reference 85 as 'Beta-N-acetyl-hexosaminidase precursor' (see GI:15902101). For reference purposes, the amino acid sequence of full length spr0057 as found in the R6 strain is given as SEQ ID NO: 18 herein. Preferred spr0057 polypeptides for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 18; and/or (b) comprising a fragment of at least 'n' consecutive amino acids of SEQ ID NO: 18, wherein 'n' is 7 or more (*e.g.* 8, 10, 12, 14, 16, 18, 20, 25, 30, 35, 40, 50, 60, 70, 80, 90, 100, 150, 200, 250 or more). These spr0057 proteins include variants of SEQ ID NO: 18. Preferred fragments of (b) comprise an epitope from SEQ ID NO: 18. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 18 while retaining at least one epitope of SEQ ID NO: 18. Other fragments omit one or more protein domains. One suitable fragment is SEQ ID NO: 32, which omits the natural leader peptide and sortase recognition sequences.

The original 'spr0286' sequence was annotated in reference 85 as 'Hyaluronate lyase precursor' (see GI:15902330). For reference purposes, the amino acid sequence of full length spr0286 as found in the R6 strain is given as SEQ ID NO: 19 herein. Preferred spr0286 polypeptides for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 19; and/or (b) comprising a fragment of at least 'n' consecutive amino acids of SEQ ID NO: 19, wherein 'n' is 7 or more (*e.g.* 8, 10, 12, 14, 16, 18, 20, 25, 30, 35, 40, 50, 60, 70, 80, 90, 100, 150, 200, 250 or more). These spr0286 proteins include variants of SEQ ID NO: 19. Preferred fragments of (b) comprise an epitope from SEQ ID NO: 19. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 19 while retaining at least one epitope of SEQ ID NO: 19. Other fragments omit one or more protein domains. One suitable fragment is SEQ ID NO: 33, which omits the natural leader peptide and sortase recognition sequences. Other suitable fragments are SEQ ID NOs: 34 and 35.

The original 'spr0565' sequence was annotated in reference 85 as 'beta-galactosidase precursor' (see GI:15902609). For reference purposes, the amino acid sequence of full length spr0565 as found in the R6 strain is given as SEQ ID NO: 20 herein. Preferred spr0565 polypeptides for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 20; and/or (b) comprising a fragment of at least 'n' consecutive amino acids of SEQ ID NO: 20, wherein 'n' is 7 or more (*e.g.* 8, 10, 12, 14, 16, 18, 20, 25, 30, 35, 40, 50, 60, 70, 80, 90, 100, 150, 200, 250 or more). These spr0565 proteins include variants of SEQ ID NO: 20 (*e.g.* SEQ ID NO: 66; see below). Preferred fragments of (b) comprise an epitope from SEQ ID NO: 20. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 20 while retaining at least one epitope of SEQ ID NO: 20. Other fragments omit one or more protein domains. One suitable fragment is SEQ ID NO: 36, which omits the natural leader peptide and sortase recognition sequences. Other suitable fragments are SEQ ID NOs: 37 and 38.

A variant form of spr0565 is SEQ ID NO: 39 herein. The use of this variant form for immunisation is reported in reference 86 (SEQ ID NO: 178 therein). Useful spr0565 polypeptides may comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 39; and/or (b) comprising a fragment of at least 'n' consecutive amino acids of SEQ ID NO: 39, wherein 'n' is 7 or more (*e.g.* 8, 10, 12, 14, 16, 18, 20, 25, 30, 35, 40, 50, 60, 70, 80, 90, 100, 150, 200, 250 or more). These polypeptides include variants of SEQ ID NO: 39. Preferred fragments of (b) comprise an epitope from SEQ ID NO: 39. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 39 while retaining at least one epitope of SEQ ID NO: 39. Other fragments omit one or more protein domains. Immunogenic fragments of SEQ ID NO: 39 are identified in table 1 of reference 86.

Because spr0565 is naturally a long polypeptide (>2000 aa) it can be more convenient to express fragments. Thus a suitable form of spr0565 for use with the invention may be less than 1500 amino acids long (*e.g.* <1400, <1300, <1200, <1100, *etc*.). Such short forms of spr0565 include 'spr0565A' (SEQ ID NO: 37) and 'spr0565B' (SEQ ID NO: 38).

The original 'spr1098' sequence was annotated in reference 85 as 'Sortase' (see GI:15903141). For reference purposes, the amino acid sequence of full length spr1098 as found in the R6 strain is given as SEQ ID NO: 21 herein. Preferred spr1098 polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 21; and/or (b) comprising a fragment of at least 'n' consecutive amino acids of SEQ ID NO: 21, wherein 'n' is 7 or more (*e.g.* 8, 10, 12, 14, 16, 18, 20, 25, 30, 35, 40, 50, 60, 70, 80, 90, 100, 150, 200, 250 or more). These spr1098 proteins include variants of SEQ ID NO: 21. Preferred fragments of (b) comprise an epitope from SEQ ID NO: 21. Other preferred fragments lack one or more amino acids (*e.g.* 1, 2, 3, 4, 5, 6, 7, 8, 9, 10, 15, 20, 25 or more) from the C-terminus and/or one or more amino acids (*e.g*. 1, 2, 3, 4, 5, 6, 7, 8, 9, 10, 15, 20, 25 or more) from the N-terminus of SEQ ID NO: 21 while retaining at least one epitope of SEQ ID NO: 21. Other fragments omit one or more protein domains. One suitable fragment is SEQ ID NO: 40, which omits the natural leader peptide sequence.

The original 'spr1345' sequence was annotated in reference 85 as 'hypothetical protein' (see GI:15903388). For reference purposes, the amino acid sequence of full length spr1345 as found in the R6 strain is given as SEQ ID NO: 22 herein. Preferred spr1345 polypeptides for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 22; and/or (b) comprising a fragment of at least 'n' consecutive amino acids of SEQ ID NO: 22, wherein 'n' is 7 or more (*e.g.* 8, 10, 12, 14, 16, 18, 20, 25, 30, 35, 40, 50, 60, 70, 80, 90, 100, 150, 200, 250 or more). These spr1345 proteins include variants of SEQ ID NO: 22. Preferred fragments of (b) comprise an epitope from SEQ ID NO: 22. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 22 while retaining at least one epitope of SEQ ID NO: 22. Other fragments omit one or more protein domains. One suitable fragment is SEQ ID NO: 41, which omits the natural leader peptide and sortase recognition sequences.

The original 'spr1416' sequence was annotated in reference 85 as 'hypothetical protein' (see GI:15903459). For reference purposes, the amino acid sequence of full length spr1416 as found in the R6 strain is given as SEQ ID NO: 23 herein. Preferred spr1416 polypeptides for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 23; and/or (b) comprising a fragment of at least 'n' consecutive amino acids of SEQ ID NO: 23, wherein 'n' is 7 or more (*e.g.* 8, 10, 12, 14, 16, 18, 20, 25, 30, 35, 40, 50, 60, 70, 80, 90, 100, 150, 200, 250 or more). These spr1416 proteins include variants of SEQ ID NO: 23. Preferred fragments of (b) comprise an epitope from SEQ ID NO: 23. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 23 while retaining at least one epitope of SEQ ID NO: 23. Other fragments omit one or more protein domains.

The original 'spr1418' sequence was annotated in reference 85 as 'hypothetical protein' (see GI:15903461). For reference purposes, the amino acid sequence of full length spr1418 as found in the R6 strain is given as SEQ ID NO: 24 herein. Preferred spr1418 polypeptides for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 24; and/or (b) comprising a fragment of at least 'n' consecutive amino acids of SEQ ID NO: 24, wherein 'n' is 7 or more (*e.g.* 8, 10, 12, 14, 16, 18, 20, 25, 30, 35, 40, 50, 60, 70, 80, 90, 100, 150, 200, 250 or more). These spr1418 proteins include variants of SEQ ID NO: 24. Preferred fragments of (b) comprise an epitope from SEQ ID NO: 24. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 24 while retaining at least one epitope of SEQ ID NO: 24. Other fragments omit one or more protein domains.

The original 'spr0867' sequence was annotated in reference 85 as 'Endo-beta-N-acetylglucosaminidase' (see GI:15902911). For reference purposes, the amino acid sequence of full length spr0867 as found in the R6 strain is given as SEQ ID NO: 25 herein. Preferred spr0867 polypeptides for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 25; and/or (b) comprising a fragment of at least 'n' consecutive amino acids of SEQ ID NO: 25, wherein 'n' is 7 or more (*e.g.* 8, 10, 12, 14, 16, 18, 20, 25, 30, 35, 40, 50, 60, 70, 80, 90, 100, 150, 200, 250 or more). These spr0867 proteins include variants of SEQ ID NO: 25. Preferred fragments of (b) comprise an epitope from SEQ ID NO: 25. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 25 while retaining at least one epitope of SEQ ID NO: 25. Other fragments omit one or more protein domains. One suitable fragment is SEQ ID NO: 42, which omits the natural leader peptide sequence.

The original 'spr1431' sequence was annotated in reference 85 as 1,4-beta-N-acetylmuramidase' (see GI:15903474). It is also known as 'LytC', and its use for immunisation is reported in reference 100. For reference purposes, the amino acid sequence of full length spr1431 as found in the R6 strain is given as SEQ ID NO: 26 herein. Preferred spr1431 polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 26; and/or (b) comprising a fragment of at least 'n' consecutive amino acids of SEQ ID NO: 26, wherein 'n' is 7 or more *(e.g.* 8, 10, 12, 14, 16, 18, 20, 25, 30, 35, 40, 50, 60, 70, 80, 90, 100, 150, 200, 250 or more). These spr1431 proteins include variants of SEQ ID NO: 26. Preferred fragments of (b) comprise an epitope from SEQ ID NO: 26. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 26 while retaining at least one epitope of SEQ ID NO: 26. Other fragments omit one or more protein domains. One suitable fragment is SEQ ID NO: 43, which omits the natural leader peptide sequence.

The amino acid sequence of full length pneumolysin as found in the R6 strain is given as SEQ ID NO: 27 herein. Preferred pneumolysin polypeptides for use with the invention comprise an amino acid sequence: (a) having 50% or more identity (*e*.*g*. 60%, 65%, 70%, 75%, 80%, 85%, 90%, 91 %, 92%, 93%, 94%, 95%, 96%, 97%, 98%, 99%, 99.5% or more) to SEQ ID NO: 27; and/or (b) comprising a fragment of at least 'n' consecutive amino acids of SEQ ID NO: 27, wherein 'n' is 7 or more (*e.g.* 8, 10, 12, 14, 16, 18, 20, 25, 30, 35, 40, 50, 60, 70, 80, 90, 100, 150, 200, 250 or more). These pneumolysin proteins include variants of SEQ ID NO: 27. Preferred fragments of (b) comprise an epitope from SEQ ID NO: 27. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 27 while retaining at least one epitope of SEQ ID NO: 27. Other fragments omit one or more protein domains. Mutant forms of pneumolysin for vaccination use are known in the art [25, 87-92], and these mutant forms may be used with the invention. Detoxification can be achieved by C-terminal truncation *(e.g.* see ref. 93) *e.g.* deleting 34 amino acids, 45 amino acids, 7 amino acids [94], *etc.* Further mutations, numbered according to SEQ ID NO: 27, include Pro325→Leu (*e.g*. SEQ ID NO: 44) and/or Trp433→Phe *(e.g.* SEQ ID NO: 45). These mutations may be combined with C-terminal truncations *e*.*g*. to combine a Pro325→Leu mutation with a 7-mer truncation (*e*.*g*. SEQ ID NO: 46).

The original 'spr2021' sequence was annotated in reference 85 as 'General stress protein GSP-781' (see GI:15904062). For reference purposes, the amino acid sequence of full length spr2021 as found in the R6 strain is given as SEQ ID NO: 28 herein. Preferred spr2021 polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 28; and/or (b) comprising a fragment of at least 'n' consecutive amino acids of SEQ ID NO: 28, wherein 'n' is 7 or more (*e.g.* 8, 10, 12, 14, 16, 18, 20, 25, 30, 35, 40, 50, 60, 70, 80, 90, 100, 150, 200, 250 or more). These spr2021 proteins include variants of SEQ ID NO: 28. Preferred fragments of (b) comprise an epitope from SEQ ID NO: 28. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 28 while retaining at least one epitope of SEQ ID NO: 28. Other fragments omit one or more protein domains. One suitable fragment is SEQ ID NO: 47, which omits the natural leader peptide sequence. Reference 86 annotates spr2021 as a secreted 45kDa protein with homology to GbpB and discloses its use as an immunogen (SEQ ID NO: 243 therein; SP2216). Immunogenic fragments of spr2021 are identified in table 1 of reference 86 (page 73). Another useful fragment of spr2021 is disclosed as SEQ ID NO: 1 of reference 95 (amino acids 28-278 of SEQ ID NO: 28 herein).

The original 'spr0096' sequence was annotated in reference 85 as 'hypothetical protein' (see GI:15902140). For reference purposes, the amino acid sequence of full length spr0096 as found in the R6 strain is given as SEQ ID NO: 29 herein. Preferred spr0096 polypeptides for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 29; and/or (b) comprising a fragment of at least 'n' consecutive amino acids of SEQ ID NO: 29, wherein 'n' is 7 or more (*e.g.* 8, 10, 12, 14, 16, 18, 20, 25, 30, 35, 40, 50, 60, 70, 80, 90, 100, 150, 200, 250 or more). These spr0096 proteins include variants of SEQ ID NO: 29 (*e.g.* SEQ ID NO: 40; see below). Preferred fragments of (b) comprise an epitope from SEQ ID NO: 29. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 29 while retaining at least one epitope of SEQ ID NO: 29. Other fragments omit one or more protein domains.

A variant form of spr0096, with an insert near its C-terminus relative to SEQ ID NO: 29, is SEQ ID NO: 48 herein. The use of this variant for immunisation is reported in reference 86 (SEQ ID NO: 150 therein), where it is annotated as a LysM domain protein. Thus a spr0096 for use with the invention may comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 48; and/or (b) comprising a fragment of at least 'n' consecutive amino acids of SEQ ID NO: 48, wherein 'n' is 7 or more (*e.g.* 8, 10, 12, 14, 16, 18, 20, 25, 30, 35, 40, 50, 60, 70, 80, 90, 100, 150, 200, 250 or more). These polypeptides include variants of SEQ ID NO: 48. Preferred fragments of (b) comprise an epitope from SEQ ID NO: 48. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 48 while retaining at least one epitope of SEQ ID NO: 48. Other fragments omit one or more protein domains. Immunogenic fragments of SEQID NO: 48 are identified in table 1 of reference 86.

A spr0096 polypeptide may be used in the form of a dimer *e.g.* a homodimer.

The original 'spr1433' sequence was annotated in reference 85 as 'hypothetical protein' (see GI:15903476). For reference purposes, the amino acid sequence of full length spr1433 as found in the R6 strain is given as SEQ ID NO: 30 herein. Preferred spr1433 polypeptides for use with the invention comprise an amino acid sequence: (a) having 50% or more identity *(e.g.* 60%, 65%, 70%, 75%, 80%, 85%, 90%, 91%, 92%, 93%, 94%, 95%, 96%, 97%, 98%, 99%, 99.5% or more) to SEQ ID NO: 30; and/or (b) comprising a fragment of at least 'n' consecutive amino acids of SEQ ID NO: 30, wherein 'n' is 7 or more (*e.g.* 8, 10, 12, 14, 16, 18, 20, 25, 30, 35, 40, 50, 60, 70, 80, 90, 100, 150, 200, 250 or more). These spr1433 proteins include variants of SEQ ID NO: 30. Preferred fragments of (b) comprise an epitope from SEQ ID NO: 30. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 30 while retaining at least one epitope of SEQ ID NO: 30. Other fragments omit one or more protein domains.

The original 'spr1707' sequence was annotated in reference 85 as 'ABC transporter substrate-binding protein - oligopeptide transport' (see GI:15903749). For reference purposes, the amino acid sequence of full length spr1707 as found in the R6 strain is given as SEQ ID NO: 31 herein. Preferred spr1707 polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 31; and/or (b) comprising a fragment of at least 'n' consecutive amino acids of SEQ ID NO: 31, wherein 'n' is 7 or more (*e.g.* 8, 10, 12, 14, 16, 18, 20, 25, 30, 35, 40, 50, 60, 70, 80, 90, 100, 150, 200, 250 or more). These spr1707 proteins include variants of SEQ ID NO: 31 (*e.g.* SEQ ID NO: 100; see below). Preferred fragments of (b) comprise an epitope from SEQ ID NO: 31. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 31 while retaining at least one epitope of SEQ ID NO: 31. Other fragments omit one or more protein domains.

A variant form of spr1707, differing from SEQ ID NO: 31 by 4 amino acids, is SEQ ID NO: 49 herein. The use of SEQ ID NO: 49for immunisation is reported in reference 86 (SEQ ID NO: 220 therein). Thus a spr1707 polypeptide for use with the invention may comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 49; and/or (b) comprising a fragment of at least 'n' consecutive amino acids of SEQ ID NO: 49, wherein 'n' is 7 or more (*e.g.* 8, 10, 12, 14, 16, 18, 20, 25, 30, 35, 40, 50, 60, 70, 80, 90, 100, 150, 200, 250 or more). These polypeptides include variants of SEQ ID NO: 49. Preferred fragments of (b) comprise an epitope from SEQ ID NO: 49. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 49 while retaining at least one epitope of SEQ ID NO: 49. Other fragments omit one or more protein domains. Immunogenic fragments of SEQ ID NO: 49 are identified in table 1 of reference 86.

PspA is the Pneumococcal surface protein A. For reference purposes, the amino acid sequence of full length PspA is SEQ ID NO: 50 herein. In the R6 genome PspA is spr0121 [85]. Preferred PspA polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 50; and/or (b) comprising a fragment of at least 'n' consecutive amino acids of SEQ ID NO: 50, wherein 'n' is 7 or more (*e.g.* 8, 10, 12, 14, 16, 18, 20, 25, 30, 35, 40, 50, 60, 70, 80, 90, 100, 150, 200, 250 or more). These PspA proteins include variants of SEQ ID NO: 50. Preferred fragments of (b) comprise an epitope from SEQ ID NO: 50. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 50 while retaining at least one epitope of SEQ ID NO: 50. Other fragments omit one or more protein domains. The use of PspA for immunisation is reported *inter alia* in reference 96. It can advantageously be administered in combination with PspC.

PsaA is the Pneumococcal surface adhesin. For reference purposes, the amino acid sequence of full length PsaA is SEQ ID NO: 51 herein. Preferred PsaA polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 51; and/or (b) comprising a fragment of at least 'n' consecutive amino acids of SEQ ID NO: 51, wherein 'n' is 7 or more (*e.g.* 8, 10, 12, 14, 16, 18, 20, 25, 30, 35, 40, 50, 60, 70, 80, 90, 100, 150, 200, 250 or more). These PsaA proteins include variants of SEQ ID NO: 51. Preferred fragments of (b) comprise an epitope from SEQ ID NO: 51. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 51 while retaining at least one epitope of SEQ ID NO: 51. Other fragments omit one or more protein domains. A useful fragment of PsaA is disclosed as SEQ ID NO: 3 in reference 95 (corresponding to amino acids 21-519 of SEQ ID NO: 51 herein). The use of PsaA for immunisation is reported in reference 97. It can be used in combination with PspA and/or PspC.

PspC is the pneumococcal surface protein C [98] and is also known as choline-binding protein A (CbpA). Its use for immunisation is reported in references 99 and 100. In the R6 strain it is spr1995 and, for reference, the amino acid sequence of full length spr1995 is SEQ ID NO: 52 herein. Preferred PspC polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 52; and/or (b) comprising a fragment of at least 'n' consecutive amino acids of SEQ ID NO: 52, wherein 'n' is 7 or more (*e.g.* 8, 10, 12, 14, 16, 18, 20, 25, 30, 35, 40, 50, 60, 70, 80, 90, 100, 150, 200, 250 or more). These spr1995 proteins include variants of SEQ ID NO: 52 (*e.g.* SEQ ID NO: 27; see below). Preferred fragments of (b) comprise an epitope from SEQ ID NO: 52. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 52 while retaining at least one epitope of SEQ ID NO: 52. Other fragments omit one or more protein domains.

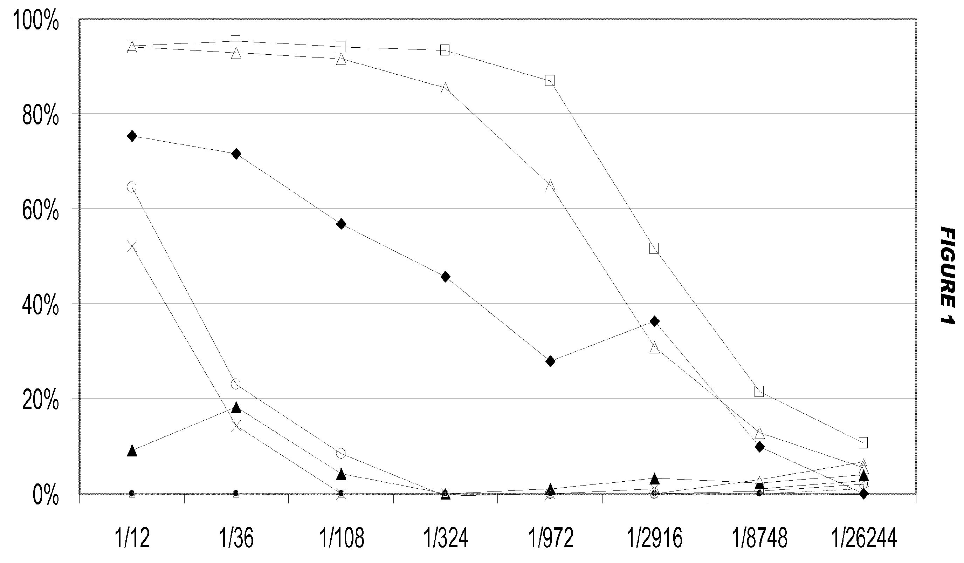

A variant of PspC is known as 'Hic'. It is similar to PspC, as shown in Figure 1 of reference 101, where it is reported to bind to factor H (fH). For reference purposes, the amino acid sequence of full length Hic is SEQ ID NO: 53 herein. A Hic protein may be used with the invention in addition to or in place of a PspC polypeptide. Preferred Hic polypeptides for use with the invention comprise an amino acid sequence: (a) having 50% or more identity (*e*.*g*. 60%, 65%, 70%, 75%, 80%, 85%, 90%, 91 %, 92%, 93%, 94%, 95%, 96%, 97%, 98%, 99%, 99.5% or more) to SEQ ID NO: 53; and/or (b) comprising a fragment of at least 'n' consecutive amino acids of SEQ ID NO: 53, wherein 'n' is 7 or more (*e.g.* 8, 10, 12, 14, 16, 18, 20, 25, 30, 35, 40, 50, 60, 70, 80, 90, 100, 150, 200, 250 or more). These Hic proteins include variants of SEQ ID NO: 53. Preferred fragments of (b) comprise an epitope from SEQ ID NO: 53. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 53 while retaining at least one epitope of SEQ ID NO: 53. Other fragments omit one or more protein domains.

PspC and/or Hic can advantageously be used in combination with PspA and/or PsaA.

LytA is the N-acetylmuramoyl-L-alanine amidase (autolysin). For reference purposes, the amino acid sequence of full length LytA is SEQ ID NO: 54 herein. In the R6 genome LytA is spr1754 [85]. Preferred LytA polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 54; and/or (b) comprising a fragment of at least'n' consecutive amino acids of SEQ ID NO: 54, wherein 'n' is 7 or more (*e.g.* 8, 10, 12, 14, 16, 18, 20, 25, 30, 35, 40, 50, 60, 70, 80, 90, 100, 150, 200, 250 or more). These LytA proteins include variants of SEQ ID NO: 54 (*e.g.* GI:18568354). Preferred fragments of (b) comprise an epitope from SEQ ID NO: 54. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 54 while retaining at least one epitope of SEQ ID NO: 54. Other fragments omit one or more protein domains. The use of LytA for immunisation is reported in reference 102, particularly in a form comprising the LytA choline binding domain fused to a heterologous promiscuous T helper epitope.

PhtA is the Pneumococcal histidine triad protein A. For reference purposes, the amino acid sequence of full length PhtA precursor is SEQ ID NO: 55 herein. In the R6 genome PhtA is spr1061 [85]. Preferred PhtA polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 55; and/or (b) comprising a fragment of at least'n' consecutive amino acids of SEQ ID NO: 55, wherein 'n' is 7 or more (*e.g.* 8, 10, 12, 14, 16, 18, 20, 25, 30, 35, 40, 50, 60, 70, 80, 90, 100, 150, 200, 250 or more). These PhtA proteins include variants of SEQ ID NO: 55. Preferred fragments of (b) comprise an epitope from SEQ ID NO: 55. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 55 while retaining at least one epitope of SEQ ID NO: 55. Other fragments omit one or more protein domains. The use of PhtA for immunisation is reported in references 103 and 104.

PhtB is the pneumococcal histidine triad protein B. For reference purposes, the amino acid sequence of full length PhtB precursor is SEQ ID NO: 56 herein. Xaa at residue 578 can be Lysine. Preferred PhtB polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 56; and/or (b) comprising a fragment of at least 'n' consecutive amino acids of SEQ ID NO: 56, wherein 'n' is 7 or more (*e.g.* 8, 10, 12, 14, 16, 18, 20, 25, 30, 35, 40, 50, 60, 70, 80, 90, 100, 150, 200, 250 or more). These PhtB proteins include variants of SEQ ID NO: 56. Preferred fragments of (b) comprise an epitope from SEQ ID NO: 56. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 56 while retaining at least one epitope of SEQ ID NO: 56. Other fragments omit one or more protein domains. The use of PhtB for immunisation is reported in references 103, 104 and 105.

PhtD is the Pneumococcal histidine triad protein D. For reference purposes, the amino acid sequence of full length PhtD precursor is SEQ ID NO: 57 herein. In the R6 genome PhtD is spr0907 [85]. Preferred PhtD polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 57; and/or (b) comprising a fragment of at least'n' consecutive amino acids of SEQ ID NO: 57, wherein 'n' is 7 or more (*e.g*. 8, 10, 12, 14, 16, 18, 20, 25, 30, 35, 40, 50, 60, 70, 80, 90, 100, 150, 200, 250 or more). These PhtD proteins include variants of SEQ ID NO: 57. Preferred fragments of (b) comprise an epitope from SEQ ID NO: 57. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 57 while retaining at least one epitope of SEQ ID NO: 57. Other fragments omit one or more protein domains. The use of PhtD for immunisation is reported in references 103, 104 and 106.

PhtE is the Pneumococcal histidine triad protein E. For reference purposes, the amino acid sequence of full length PhtE precursor is SEQ ID NO: 58 herein. In the R6 genome PhtE is spr0908 [85]. Preferred PhtE polypeptides for use with the invention comprise an amino acid sequence: (a) having 50% or more identity (*e*.*g*. 60%, 65%, 70%, 75%, 80%, 85%, 90%, 91%, 92%, 93%, 94%, 95%, 96%, 97%, 98%, 99%, 99.5% or more) to SEQ ID NO: 58; and/or (b) comprising a fragment of at least'n' consecutive amino acids of SEQ ID NO: 58, wherein 'n' is 7 or more (*e.g.* 8, 10, 12, 14, 16, 18, 20, 25, 30, 35, 40, 50, 60, 70, 80, 90, 100, 150, 200, 250 or more). These PhtE proteins include variants of SEQ ID NO: 58. Preferred fragments of (b) comprise an epitope from SEQ ID NO: 58. Other preferred fragments lack one or more amino acids *(e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 58 while retaining at least one epitope of SEQ ID NO: 58. Other fragments omit one or more protein domains. The use of PhtE for immunisation is reported in references 103 and 104.

### Further antigens from other pathogen(s)

In addition to conjugated pneumococcal capsular saccharide(s) and meningococcal factor H binding protein(s), compositions of the invention can include antigen(s) from further pathogen(s).

For example, the composition may comprise one or more of the following further antigen(s):
- an antigen from hepatitis B virus, such as the surface antigen HBsAg.
- an antigen from *Bordetella pertussis,* such as pertussis holotoxin (PT) and filamentous haemagglutinin (FHA) from *B.pertussis,* optionally also in combination with pertactin and/or agglutinogens 2 and 3.
- a diphtheria antigen, such as a diphtheria toxoid.
- a tetanus antigen, such as a tetanus toxoid.
- a saccharide antigen from *Haemophilus influenzae* B (Hib), typically conjugated.
- inactivated poliovirus antigen(s).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred.

### Hybrid polypeptides

A meningococcal factor H binding protein may be present in the composition as an individual separate polypeptide, or it maybe present as part of a 'hybrid' polypeptide *i.e.* where at least two (*e.g.* 2, 3, 4, 5, or more) antigens are expressed as a single polypeptide chain, as disclosed for meningococcal antigens in reference 107. This hybrid polypeptide approach can also be used for any additional meningococcal polypeptide(s) and/or pneumococcal polypeptides.

Hybrid polypeptides offer two main advantages: first, a polypeptide that may be unstable or poorly expressed on its own can be assisted by adding a suitable hybrid partner that overcomes the problem; second, commercial manufacture is simplified as only one expression and purification need be employed in order to produce two polypeptides which are both antigenically useful.

A hybrid polypeptide may comprise two or more meningococcal and/or pneumococcal polypeptide sequences as disclosed above. Hybrids consisting of amino acid sequences from two, three, four, five, six, seven, eight, nine, or ten antigens are useful. In particular, hybrids consisting of amino acid sequences from two, three, four, or five antigens are preferred, such as two or three antigens.

Hybrid polypeptides can be represented by the formula NH₂-A-{-X-L-}*ₙ*-B-COOH, wherein: X is an amino acid sequence of an alternative meningococcal or pneumococcal antigen, as described above; L is an optional linker amino acid sequence; A is an optional N-terminal amino acid sequence; B is an optional C-terminal amino acid sequence; *n* is an integer of 2 or more (*e.g.* 2, 3, 4, 5, 6, *etc*.). Usually *n* is 2 or 3.

In some embodiments at least one -X- moiety is a fHBP. In other embodiments, at least two -X- moieties are a fHBP *e*.*g*. different fHBP variants. In other embodiments a fHBP may be present as an individual polypeptide and at least one -X- moiety in a hybrid polypeptide is a non-fHBP meningococcal polypeptide and/or is a pneumococcal polypeptide.

If a -X- moiety has a leader peptide sequence in its wild-type form, this may be included or omitted in the hybrid protein. In some embodiments, the leader peptides will be deleted except for that of the -X-moiety located at the N-terminus of the hybrid protein *i.e.* the leader peptide of X₁ will be retained, but the leader peptides of X₂ ... Xₙ will be omitted. This is equivalent to deleting all leader peptides and using the leader peptide of X₁ as moiety -A-.

For each *n* instances of {-X-L-}, linker amino acid sequence -L- may be present or absent. For instance, when *n*=2 the hybrid may be NH₂-X₁-L₁-X₂-L₂-COOH, NH₂-X₁-X₂-COOH, NH₂-X₁-L₁-X₂-COOH, NH₂-X₁-X₂-L₂-COOH, *etc.* Linker amino acid sequence(s) -L- will typically be short (*e.g.* 20 or fewer amino acids *i.e.* 20, 19, 18, 17, 16, 15, 14, 13, 12, 11, 10, 9, 8, 7, 6, 5, 4, 3, 2, 1). Examples comprise short peptide sequences which facilitate cloning, poly-glycine linkers (*i.e*. comprising Gly*ₙ* where *n* = 2, 3, 4, 5, 6, 7, 8, 9, 10 or more), and histidine tags (*i.e.* His*ₙ* where *n* = 3, 4, 5, 6, 7, 8, 9, 10 or more). Other suitable linker amino acid sequences will be apparent to those skilled in the art. A useful linker is GSGGGG (SEQ ID NO: 15) or GSGSGGGG (SEQ ID NO:16), with the Gly-Ser dipeptide being formed from a *Bam*HI restriction site, thus aiding cloning and manipulation, and the (Gly)₄ tetrapeptide being a typical poly-glycine linker. Other suitable linkers, particularly for use as the final Lₙ are a Leu-Glu dipeptide or SEQ ID NO: 59.
- A- is an optional N-terminal amino acid sequence. This will typically be short *(e.g.* 40 or fewer amino acids *i.e.* 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct protein trafficking, or short peptide sequences which facilitate cloning or purification (*e.g*. histidine tags *i.e.* His*ₙ* where *n* = 3, 4, 5, 6, 7, 8, 9, 10 or more). Other suitable N-terminal amino acid sequences will be apparent to those skilled in the art. If X₁ lacks its own N-terminus methionine, -A- is preferably an oligopeptide (*e*.*g*. with 1, 2, 3, 4, 5, 6, 7 or 8 amino acids) which provides a N-terminus methionine *e*.*g*. Met-Ala-Ser, or a single Met residue.
- B- is an optional C-terminal amino acid sequence. This will typically be short (*e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include sequences to direct protein trafficking, short peptide sequences which facilitate cloning or purification (*e.g.* comprising histidine tags *i.e.* His*ₙ* where *n* = 3, 4, 5, 6, 7, 8, 9, 10 or more, such as SEQ ID NO: 17), or sequences which enhance protein stability. Other suitable C-terminal amino acid sequences will be apparent to those skilled in the art.

A particularly useful combination of meningococcal polypeptide antigens, including a fHBP antigen, is disclosed in references 107 and 108, and a composition of the invention may thus include a conjugated pneumococcal capsular saccharide, a fHBP, and 1, 2, 3 or 4 of: (1) a 'NadA' protein; (2) a '936' protein; (3) a '953' protein; and (4) a '287' protein. For instance, a composition may include a conjugated pneumococcal capsular saccharide and: (i) a first polypeptide having amino acid sequence SEQ ID NO: 4; (ii) a second polypeptide having amino acid sequence SEQ ID NO: 5; and (iii) a third polypeptide having amino acid sequence SEQ ID NO: 6.

### Adjuvant(s)

Compositions of the invention may include an immunological adjuvant. Thus, for example, they may include an aluminium salt adjuvant or an oil-in-water emulsion (*e*.*g*. a squalene-in-water emulsion). Other adjuvants may also be used.

Suitable aluminium salts include hydroxides (*e.g.* oxyhydroxides), phosphates (*e.g.* hydroxyphosphates, orthophosphates), (*e*.*g*. see chapters 8 & 9 of ref. 109), or mixtures thereof. The salts can take any suitable form (*e.g.* gel, crystalline, amorphous, *etc*.). The concentration of Al⁺⁺⁺ in a composition for administration to a patient is preferably less than 5mg/ml *e.g.* ≤4 mg/ml, ≤3 mg/ml, ≤2 mg/ml, ≤1 mg/ml, *etc.* A preferred range is between 0.3 and 1mg/ml. A maximum of 0.85mg/dose is preferred.

A preferred aluminium salt adjuvant for use with the invention is an aluminium phosphate. This adjuvant is compatible with both fHBP and the PREVNAR™ and SYNFLORIX™ pneumococcal conjugate vaccines. The adjuvants known as "aluminium phosphate" are typically aluminium hydroxyphosphates, often also containing a small amount of sulfate (*i*.*e*. aluminium hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (*e*.*g*. when heated to 200°C) indicates the presence of structural hydroxyls.

The P/Al molar ratio of an aluminium phosphate adjuvant will generally be between 0.3 and 1.2, preferably between 0.8 and 1.2, or between 0.85 and 1.0, and more preferably about 0.9. A P/Al molar ratio of at least 0.5 can provide an adjuvant with better aging properties.

The aluminium phosphate adjuvant will generally be amorphous *(i.e.* amorphous to X-rays). It will generally be particulate (*e*.*g*. plate-like morphology as seen in transmission electron micrographs). Typical diameters of the plates are 10-100nm, and these form aggregates sized 0.5-20µm (*e*.*g*. about 1-10µm). Adsorptive capacities of between 0.7-1.5 mg protein per mg Al⁺⁺⁺ at pH 7.4 have been reported for aluminium phosphate adjuvants.

A typical adjuvant is amorphous aluminium phosphate with P/Al molar ratio between 0.84 and 0.92, and this adjuvant may be included at 0.6mg Al³⁺/ml.

The concentration of Al⁺⁺⁺ in a composition for administration to a patient is preferably less than 5mg/ml *e.g.* ≤4 mg/ml, ≤3 mg/ml, ≤2 mg/ml, ≤1 mg/ml, *etc*. A preferred range is between 0.2 and 1mg/ml. A maximum Al⁺⁺⁺ concentration of 0.85mg/dose is preferred.

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g.* about 5.7.

The adjuvants known as "aluminium hydroxide" are typically aluminium oxyhydroxide salts, which are usually at least partially crystalline. Aluminium oxyhydroxide, which can be represented by the formula AIO(OH), can be distinguished from other aluminium compounds by infrared (IR) spectroscopy, in particular by the presence of an adsorption band at 1070cm⁻¹ and a strong shoulder at 3090-3100cm⁻¹ [chapter 9 of ref. 109]. Aluminium hydroxide adjuvants generally have a PZC of about 11.4.

In one embodiment of the invention, an adjuvant component includes a mixture of both an aluminium hydroxide and an aluminium phosphate. In this case there may be more aluminium phosphate than hydroxide *e.g.* a weight ratio of at least 2:1 *e.g.* ≥5:1, ≥6:1, ≥7:1, ≥8:1, ≥9:1, *etc.* Most preferably, however, an adjuvant component does not include aluminium hydroxide. Thus a composition may include aluminium hydroxyphosphate but no aluminium oxyhydroxides.

In preferred compositions of the invention, fHBP is adsorbed to an aluminium phosphate adjuvant. At least 50% of total fHBP in the composition may be adsorbed *e.g.* >50%, >60%, >70%, >80%, >90%, >95% or substantially 100%. The proportion of adsorbed fHBP can be controlled by altering salt concentration and/or pH during formulation *e*.*g*. in general, a higher NaCl concentration can decrease fHBP's adsorption. To facilitate stable adsorption of fHBP three useful techniques may be used: (i) adsorption may take place at a pH which is equal to or below the adjuvant's PZC; (ii) a fHBP and adjuvant are selected such that the fHBP's pI and the adjuvant's PZC are both within the range of 5.0 to 7.0; and (iii) if the fHBP has an isoelectric point above the adjuvant's PZC then a buffer is added to bring the pH to within 1.2 pH units of the PZC.

Suspensions of aluminium salt(s) used to prepare compositions of the invention may contain a buffer (*e.g.* a phosphate or a histidine or a Tris buffer), but this is not always necessary. The suspensions are preferably sterile and pyrogen-free. A suspension may include free aqueous phosphate ions *e*.*g*. present at a concentration between 1.0 and 20 mM, preferably between 5 and 15 mM, and more preferably about 10 mM. The suspensions may also comprise sodium chloride.

As an alternative to aluminium salt(s), various oil-in-water emulsion adjuvants are known. These typically include at least one oil and at least one surfactant, with the oil(s) and surfactant(s) being biodegradable (metabolisable) and biocompatible. The oil droplets in the emulsion are generally less than 5 µm in diameter, and may even have a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e*.*g*. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols. Mixtures of oils can be used.

Where a composition includes a tocopherol, any of the α, β, γ, δ, ε or ξ tocopherols can be used, but α-tocopherols are preferred. The tocopherol can take several forms *e*.*g*. different salts and/or isomers. Salts include organic salts, such as succinate, acetate, nicotinate, *etc.* D-α-tocopherol and DL-α-tocopherol can both be used. A preferred α-tocopherol is DL-α-tocopherol, and the preferred salt of this tocopherol is the succinate.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate), Span 85 (sorbitan trioleate), lecithin and Triton X-100.

Mixtures of surfactants can be used *e*.*g*. Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth-9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
- A sub-micron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [110-112], as described in more detail in Chapter 10 of ref. 109 and chapter 12 of ref. 113. The MF59 emulsion may include citrate ions *e*.*g*. 10mM sodium citrate buffer.
- An emulsion of squalene, a tocopherol, and polysorbate 80 (Tween 80). The emulsion may include phosphate buffered saline. It may also include Span 85 (*e*.*g*. at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. Squalene and Tween 80 may be present volume ratio of about 5:2 or at a weight ratio of about 11:5. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e*.*g*. with an average diameter of between 100 and 250nm, preferably about 180nm. The emulsion may also include a 3-de-O-acylated monophosphoryl lipid A (3d-MPL). Another useful emulsion of this type may comprise, per human dose, 0.5-10 mg squalene, 0.5-11 mg tocopherol, and 0.1-4 mg polysorbate 80 [114].
- An emulsion of squalene, a tocopherol, and a Triton detergent (*e*.*g*. Triton X-100). The emulsion may also include a 3d-MPL. The emulsion may contain a phosphate buffer.
- An emulsion comprising a polysorbate (*e.g.* polysorbate 80), a Triton detergent *(e.g.* Triton X-100) and a tocopherol (*e*.*g*. an α-tocopherol succinate). The emulsion may include these three components at a mass ratio of about 75:11:10 (*e*.*g*. 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL. The aqueous phase may contain a phosphate buffer.
- An emulsion of squalane, polysorbate 80 and poloxamer 401 ("Pluronic™L121"). The emulsion can be formulated in phosphate buffered saline, pH 7.4. This emulsion is a useful delivery vehicle for muramyl dipeptides, and has been used with threonyl-MDP in the "SAF-1" adjuvant [115] (0.05-1% Thr-MDP, 5% squalane, 2.5% Pluronic L121 and 0.2% polysorbate 80). It can also be used without the Thr-MDP, as in the "AF" adjuvant [116] (5% squalane, 1.25% Pluronic L121 and 0.2% polysorbate 80). Microfluidisation is preferred.
- An emulsion comprising squalene, an aqueous solvent, a polyoxyethylene alkyl ether hydrophilic nonionic surfactant (*e*.*g*. polyoxyethylene (12) cetostearyl ether) and a hydrophobic nonionic surfactant (*e*.*g*. a sorbitan ester or mannide ester, such as sorbitan monoleate or 'Span 80'). The emulsion is preferably thermoreversible and/or has at least 90% of the oil droplets (by volume) with a size less than 200 nm [117]. The emulsion may also include one or more of: alditol; a cryoprotective agent (*e.g.* a sugar, such as dodecylmaltoside and/or sucrose); and/or an alkylpolyglycoside. Such emulsions may be lyophilized.
- An emulsion having from 0.5-50% of an oil, 0.1-10% of a phospholipid, and 0.05-5% of a non-ionic surfactant. As described in reference 118, preferred phospholipid components are phosphatidylcholine, phosphatidylethanolamine, phosphatidylserine, phosphatidylinositol, phosphatidylglycerol, phosphatidic acid, sphingomyelin and cardiolipin. Sub-micron droplet sizes are advantageous.
- A sub-micron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 119, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion comprising a mineral oil, a non-ionic lipophilic ethoxylated fatty alcohol, and a non-ionic hydrophilic surfactant (*e*.*g*. an ethoxylated fatty alcohol and/or polyoxyethylene-polyoxypropylene block copolymer) [120].
- An emulsion comprising a mineral oil, a non-ionic hydrophilic ethoxylated fatty alcohol, and a non-ionic lipophilic surfactant (*e*.*g*. an ethoxylated fatty alcohol and/or polyoxyethylene-polyoxypropylene block copolymer) [120].
- An emulsion in which a saponin (*e*.*g*. QuilA or QS21) and a sterol (*e.g.* a cholesterol) are associated as helical micelles [121].

Oil-in-water emulsions can be used as adjuvants on their own, or as carriers for further immunostimulatory compounds *e*.*g*. immunostimulatory oligonucleotides, 3d-MPL, *etc.*

### Pharmaceutical compositions

The invention is concerned with pharmaceutical compositions for administration to a patient. These will typically include a pharmaceutically acceptable carrier. A thorough discussion of pharmaceutically acceptable carriers is available in reference 122.

Effective dosage volumes can be routinely established, but a typical human dose of the composition has a volume of about 0.5ml *e.g.* for intramuscular injection. This is the dosage volume for the PREVNAR™ product, the RIVM OMV-based vaccine and MeNZB™. These dosage volumes are typical for intramuscular injection, but similar doses may be used for other delivery routes *e*.*g*. an intranasal OMV-based vaccine for atomisation may have a volume of about 100µl or about 130µl per spray, with four sprays administered to give a total dose of about 0.5ml.

The pH of a composition of the invention is usually between 6 and 8, and more preferably between 6.5 and 7.5 (*e*.*g*. about 7). Compositions may include a buffer *e.g.* a Tris buffer, a citrate buffer, phosphate buffer, a sodium succinate buffer, or a histidine buffer.

The composition may be sterile and/or pyrogen-free. Compositions of the invention may be isotonic with respect to humans.

Compositions of the invention for administration to patients are immunogenic, and are more preferably vaccine compositions. Vaccines according to the invention may either be prophylactic (*i*.*e*. to prevent infection) or therapeutic (*i*.*e*. to treat infection), but will typically be prophylactic. Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e*.*g*. non-human primate, primate, *etc*.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. The antigen content of compositions of the invention will generally be expressed in terms of the amount of protein per dose.

Meningococci and pneumococci affect various areas of the body and so the compositions of the invention may be prepared in various liquid forms. For example, the compositions may be prepared as injectables, either as solutions or suspensions. The composition may be prepared for pulmonary administration *e*.*g*. by an inhaler, using a fine spray. The composition may be prepared for nasal, aural or ocular administration *e*.*g*. as spray or drops. Injectables for intramuscular administration are typical.

Compositions of the invention may include an antimicrobial, particularly when packaged in multiple dose format. Antimicrobials such as thiomersal and 2-phenoxyethanol are commonly found in vaccines, but it is preferred to use either a mercury-free preservative or no preservative at all.

Compositions of the invention may comprise detergent *e.g.* a Tween (polysorbate), such as Tween 80. Detergents are generally present at low levels *e.g.* <0.01%.

Compositions of the invention may include sodium salts (*e*.*g*. sodium chloride) to give tonicity. A concentration of 10±2 mg/ml NaCl is typical *e*.*g*. about 9 mg/ml.

### Methods of treatment

The invention also provides a method for raising an immune response in a mammal, comprising administering a composition of the invention to the mammal. The immune response is preferably protective against both meningococcus and pneumococcus (for at least the meningococcal serogroups and pneumococcal serotypes represented in the composition) and preferably involves antibodies. The method may raise a booster response in a patient that has already been primed.

The mammal is preferably a human. Where the vaccine is for prophylactic use, the human is preferably a child *(e.g.* a toddler or infant) or a teenager; where the vaccine is for therapeutic use, the human is preferably an adult. A vaccine intended for children may also be administered to adults *e.g.* to assess safety, dosage, immunogenicity, *etc.*

The invention also provides compositions of the invention for use as a medicament. The medicament is preferably used, as described above, to raise an immune response in a mammal (*i*.*e*. it is an immunogenic composition) and is more preferably a vaccine.

The invention also provides the use of: (i) at least one conjugated pneumococcal capsular saccharide; and (ii) a meningococcal factor H binding protein (fHBP) antigen, in the manufacture of a medicament for raising an immune response, as described above, in a mammal.

These uses and methods are preferably for the prevention and/or treatment of a disease caused by *N.meningitidis* and/or *S.pneumoniae e.g.* bacterial (or, more specifically, meningococcal and/or pneumococcal) meningitis, or septicemia.

One way of checking efficacy of therapeutic treatment involves monitoring meningococcal and/or pneumococcal infection after administration of the composition of the invention. One way of checking efficacy of prophylactic treatment involves monitoring immune responses against antigens after administration of the composition. Immunogenicity of compositions of the invention can be determined by administering them to test subjects (*e*.*g*. children 12-16 months age, or animal models [123]) and then determining standard parameters including serum bactericidal antibodies (SBA) and ELISA titres (GMT) for meningococcus. These immune responses will generally be determined around 4 weeks after administration of the composition, and compared to values determined before administration of the composition. A SBA increase of at least 4-fold or 8-fold is preferred. Where more than one dose of the composition is administered, more than one post-administration determination may be made.

Compositions of the invention will generally be administered directly to a patient. Direct delivery may be accomplished by parenteral injection (*e*.*g*. subcutaneously, intraperitoneally, intravenously, intramuscularly, or to the interstitial space of a tissue), or by any other suitable route. The invention may be used to elicit systemic and/or mucosal immunity. Intramuscular administration to the thigh or the upper arm is preferred. Injection maybe via a needle *(e.g.* a hypodermic needle), but needle-free injection may alternatively be used. A typical intramuscular dose is 0.5 ml.

Dosage treatment can be a single dose schedule or a multiple dose schedule. Multiple doses may be used in a primary immunisation schedule and/or in a booster immunisation schedule. A primary dose schedule may be followed by a booster dose schedule. Suitable timing between priming doses (*e*.*g*. between 4-16 weeks), and between priming and boosting, can be routinely determined. Multiple doses *(e.g.* 2 or 3 doses) are typical for establishing immunity against both pneumococcus and meningococcus.

In some embodiments of the invention, the pneumococcal and meningococcal antigens may be co-administered but separately *i.e.* the two antigens may be for simultaneous, separate or sequential administration. Typically, however, the two antigens will be admixed for simultaneous combined administration.

### General

The practice of the present invention will employ, unless otherwise indicated, conventional methods of chemistry, biochemistry, molecular biology, immunology and pharmacology, within the skill of the art. Such techniques are explained fully in the literature. See, *e.g*., references 124-130, *etc.*

The term "comprising" encompasses "including" as well as "consisting" *e*.*g*. a composition "comprising" X may consist exclusively of X or may include something additional *e*.*g*. X + Y.

The term "about" in relation to a numerical value x is optional and means, for example, x±10%.

Where the invention concerns an "epitope", this epitope may be a B-cell epitope and/or a T-cell epitope, but will usually be a B-cell epitope. Such epitopes can be identified empirically (*e*.*g*. using PEPSCAN [131,132] or similar methods), or they can be predicted (*e*.*g*. using the Jameson-Wolf antigenic index [133], matrix-based approaches [134], MAPITOPE [135], TEPITOPE [136,137], neural networks [138], OptiMer & EpiMer [139,140], ADEPT [141], Tsites [142], hydrophilicity [143], antigenic index [144] or the methods disclosed in references 145-149, *etc*.). Epitopes are the parts of an antigen that are recognised by and bind to the antigen binding sites of antibodies or T-cell receptors, and they may also be referred to as "antigenic determinants".

Where the invention uses a "purified" antigen, this antigen is separated from its naturally occurring environment. For example, the antigen will be substantially free from other meningococcal components, other than from any other purified antigens that are present. A mixture of purified antigens will typically be prepared by purifying each antigen separately and then re-combining them, even if the two antigens are naturally present in admixture.

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of ref. 150. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in ref. 151.

The word "substantially" does not exclude "completely" *e*.*g*. a composition which is "substantially free" from Y may be completely free from Y. Where necessary, the word "substantially" may be omitted from the definition of the invention.

### BRIEF DESCRIPTION OF DRAWINGS

Figure 1 shows results of an opsonophagocytic activity assay against 6B Finland pneumococcus. The graph shows OPA killing % against serum dilution. Symbols are explained below for mouse groups 1 to 7, except for the filled diamonds (◆) which show data from a positive control anti-6B serum and filled triangles (▲) which show data using pre-immunisation serum. Some lines are not visible because they run along the X-axis (*i.e.* 0% activity).

### MODES FOR CARRYING OUT THE INVENTION

In seeking a vaccine for protecting against both serogroup B meningococcus and pneumococcus, meningococcal fHBP antigen (strain MC58; 50µg/ml) was combined with a 7-valent pneumococcal capsular saccharide conjugate mixture (serotypes 4, 9V, 14, 18C, 19F and 23F at 4µg/ml; 6B at 8µg/ml). Compositions were intraperitoneally administered to seven groups of CD1 mice (8 mice per group) on a two-dose schedule (days 0 and 21). An aluminium phosphate adjuvant was used. None of the compositions included meningococcal outer membrane vesicles.

Five different compositions (A to E) were administered to mice:

| | **fHBP** | **PCV7** | **Adjuvant** | **pH** | **Dosage volume** |
|---|---|---|---|---|---|
| **A** | - | + | 100µg | 6.01 | 100 µl |
| **B** | + | - | - | 7.05 | 200 µl |
| **C** | + | - | 100µg | 6.94 | 200 µl |
| **D** | + | + | 100µg | 6.93 | 200 µl |
| **E** | - | - | 100µg | - | 200 µl |

Seven groups of mice (1 to 7) were used and they received compositions A to E as follows:

| | **Day 0** | **Day 21** | **Symbol in** **Figure 1** |
|---|---|---|---|
| **1** | A | - | X |
| **2** | A | A | Δ |
| **3** | B | B | ■ |
| **4** | C | C | ◊ |
| **5** | D | B | ○ |
| **6** | D | D | □ |
| **7** | E | E | ● |

Blood was taken at days 16 and 35 for evaluation of immune responses. Pneumococcal immunogenicity was assessed by an opsonophagocytosis assay and by the antibody titer against the saccharides. Meningococcal immunogenicity was assessed by serum bactericidal assay against two different strains (MC58 and H44/76).

Meningococcal SBA titers were as follows:

| | **MC58** | | **H44/76** | |
|---|---|---|---|---|
| | **Day 16** | **Day 35** | **Day 16** | **Day 35** |
| **1** | - | <16 | - | <16 |
| **2** | - | <16 | - | <16 |
| **3** | <16 | 2048 | <16 | 4096 |
| **4** | <16 | 2048 | <16 | 2048 |
| **5** | 16 | 4096 | 64 | 8192 |
| **6** | <16 | 1024 | <16 | 1024 |
| **7** | - | <16 | - | <16 |

Titers were acceptable in all of groups 3 to 6, but the highest titers after 35 days were seen in group 5 for both strains. These data indicate that the addition of pneumococcal conjugates to fHBP can enhance the anti-fHBP response.

OPA activity was assessed against the 6B Finland strain of pneumococcus using the UAB-MOPA method of reference 152 with baby rabbit complement. Results are shown in Figure 1. The best response is in group 6 (□) and then group 2 (Δ). The difference between groups 2 and 6 was that the immunising composition for group 6 included meningococcal fHBP in addition to the pneumococcal conjugates. Thus these data indicate that the addition of fHBP to pneumococcal conjugates can enhance the anti-pneumococcus response, at least against serotype 6B.

Sera were also assessed against the TIGR4 strain. OPA responses were similar in groups 2 and 6. Sera were also tested against pneumococcus serotype 35B, which is not covered by the 7-valent conjugates. As expected, the sera were not very effective in the OPA assay.

The OPA assay results can be expressed as an OPA titer, which is the reciprocal serum dilution yielding 50% bacterial killing. If the 50% of killing threshold lies between two dilutions, the titer is expressed as a range. By this measure, titers against the serotype 6B and 4 strains were as follows after the day 21 immunization:

| | **6B Finland** | **TIGR4** |
|---|---|---|
| **1** | 12 | 108-324 |
| **2** | 972-2916 | 2916-8748 |
| **3** | <12 | - |
| **4** | <12 | - |
| **5** | 12-36 | 324-972 |
| **6** | 2916 | 2916-8748 |
| **7** | <12 | <12 |

Overall, therefore, these data show that conjugated pneumococcal capsular saccharides can enhance the immunogenicity of meningococcal fHBP, and *vice versa*.

In separate experiments, mice were immunised with (i) 10µg of outer membrane vesicles prepared from strain MC58 of serogroup B meningococcus, (ii) 0.4µg of 7-valent pneumococcal capsular saccharide conjugate mixture, and (iii) a mixture of (i) and (ii). All compositions included aluminium hydroxide adjuvant. IgG titres (GMT) against the vesicles and against the serotype 14 saccharide were measured by Luminex assay. On day 34 (after immunization on days 1 and 21) titres were as follows in the three groups, measured in relative units (RLU/ml):

| | **(i)** | **(ii)** | **(iii)** |
|---|---|---|---|
| **Anti-OMV** | 0.14 | 5.61 | 2.65 |
| **Anti-CP14** | 76.1 | 1.7 | 81.7 |

Although these data are incomplete and not fully conclusive, the decrease in anti-OMV responses in group (iii) compared to group (ii) suggests that the advantageous interaction between pneumococcal saccharides and fHBP is not universal to all meningococcal antigens.

It will be understood that the invention has been described by way of example only and modifications may be made whilst remaining within the scope and spirit of the invention.

### REFERENCES

[1] Kilpi et al. (2003) Clin Infect Dis 37:1155-64.
[2] van den Dobbelsteen et al. (2007) Vaccine 25:2491-6.
[3] Bos et al. (2006) Pharmacoeconomics 24:141-53.
[4] WHO Technical Report Series No. 927, 2005. Pages 64-98.
[5] US-2008/0102498.
[6] US-2006/0228381.
[7] US-2007/0231340.
[8] US-2007/0184072.
[9] US-2006/0228380.
[10] WO2008/143709.
[11] *Research Disclosure,* 453077 (Jan 2002)
[12] EP-A-0378881.
[13] EP-A-0427347.
[14] WO93/17712
[15] WO94/03208.
[16] WO98/58668.
[17] EP-A-0471177.
[18] WO91/01146
[19] Falugi et al. (2001) Eur J Immunol 31:3816-3824.
[20] Baraldo et al. (2004) Infect Immun 72(8):4884-7.
[21] EP-A-0594610.
[22] Ruan et al. (1990) J Immunol 145:3379-3384.
[23] WO00/56360.
[24] Kuo et al. (1995) Infect Immun 63:2706-13.
[25] Michon et al. (1998) Vaccine. 16:1732-41.
[26] WO02/091998.
[27] WO01/72337
[28] WO00/61761.
[29] WO0O/338
[30] WO2007/071707
[31] Bethell G.S. et al., J. Biol. Chem., 1979, 254, 2572-4
[32] Hearn M.T.W., J. Chromatogr., 1981, 218, 509-18
[33] WO2007/000343.
[34] Mol. Immol., 1985, 22, 907-919
[35] EP-A-0208375
[36] WO00/10599
[37] Gever et al., Med. Microbiol. Immunol, 165 : 171-288 (1979).
[38] US patent 4,057,685.
[39] US patents 4,673,574; 4,761,283; 4,808,700.
[40] US patent 4,459,286.
[41] US patent 5,204,098
[42] US patent 4,965,338
[43] US patent 4,663,160.
[44] US-2007/0184071.
[45] Jodar et al. (2003) Vaccine 21:3265-72.
[46] Masignani et al. (2003) J Exp Med 197:789-799.
[47] Welsch et al. (2004) J Immunol 172:5605-15.
[48] Hou et al. (2005) J Infect Dis 192(4):580-90.
[49] WO03/063766.
[50] Fletcher et al. (2004) Infect Immun 72:2088-2100.
[51] Zhu et al. (2005) Infect Immun 73(10):6838-45.
[52] Cantini et al. (2006) J. Biol. Chem. 281:7220-7227
[53] WO2004/048404
[54] Tettelin et al. (2000) Science 287:1809-1815.
[55] WO00/66741.
[56] WO99/57280
[57] Martin et al. (1997) J Exp Med 185(7):1173-83.
[58] WO96/29412.
[59] US-5,698,438.
[60] Perkins-Balding et al. (2003) Microbiology 149:3423-35.
[61] WO01/55182.
[62] WO01/38350.
[63] WO00/23595.
[64] Ram et al. (2003) J Biol Chem 278:50853-62.
[65] WO2004/014417.
[66] WO98/53851
[67] US-6531131
[68] WO00/26384.
[69] US-6645503
[70] WO03/070282.
[71] WO94/08021
[72] WO2004/015099.
[73] WO2007/1443.
[74] WO2007/144317.
[75] WO03/080678.
[76] Glode et al. (1979) J Infect Dis 139:52-56
[77] WO94/05325; US patent 5,425,946.
[78] Arakere & Frasch (1991) Infect. Immun. 59:4349-4356.
[79] Michon et al. (2000) Dev. Biol. 103:151-160.
[80] Rubinstein & Stein (1998) J. Immunol. 141:4357-4362.
[81] WO2005/033148
[82] WO2007/000314.
[83] WO2007/000341.
[84] WO2007/000342.
[85] Hoskins et al. (2001) J.Bacteriol. 183:5709-5717.
[86] WO2004/092209.
[87] Kirkham et al. (2006) Infect Immun. 74(1):586-93.
[88] WO2005/108580.
[89] Berry et al. (1999) Infect Immun 67(2):981-5.
[90] US-6716432.
[91] WO90/06951.
[92] WO99/03884.
[93] Baba et al. (2002) Infect Immun 70: 107-113.
[94] US-7217791
[95] WO2008/061953.
[96] Briles et al. (2000) J Infect Dis 182:1694-1701.
[97] Talkington et al. (1996) Microb Pathog. 21(1):17-22.
[98] WO99/53940.
[99] WO02/22168.
[100] WO02/22167.
[101] WO02/08426.
[102] WO2003/104272.
[103] WO00/37105.
[104] Adamou et al. (2001) Infect Immun. 69(2):949-58.
[105] Ogunniyi et al. (2007) Infect Immun. 75(1):350-7.
[106] WO98/18930.
[107] Giuliani et al. (2006) PNAS USA 103:10834-9.
[108] WO2004/032958.
[109] Vaccine Design... (1995) eds. Powell & Newman. ISBN: 030644867X. Plenum.
[110] WO90/14837.
[111] Podda & Del Giudice (2003) Expert Rev Vaccines 2:197-203.
[112] Podda (2001) Vaccine 19: 2673-2680.
[113] Vaccine Adjuvants: Preparation Methods and Research Protocols (Volume 42 of Methods in Molecular Medicine series). ISBN: 1-59259-083-7. Ed. O'Hagan.
[114] WO2008/043774.
[115] Allison & Byars (1992) Res Immunol 143:519-25.
[116] Hariharan et al. (1995) Cancer Res 55:3486-9.
[117] US-2007/0014805.
[118] WO95/11700.
[119] US patent 6,080,725.
[120] WO2006/113373.
[121] WO2005/097181.
[122] Gennaro (2000) Remington: The Science and Practice of Pharmacy. 20th edition, ISBN: 0683306472.
[123] WO01/30390.
[124] Methods In Enzymology (S. Colowick and N. Kaplan, eds., Academic Press, Inc.)
[125] Handbook of Experimental Immunology, Vols. I-IV (D.M. Weir and C.C. Blackwell, eds, 1986, Blackwell Scientific Publications)
[126] Sambrook et al. (2001) Molecular Cloning: A Laboratory Manual, 3rd edition (Cold Spring Harbor Laboratory Press).
*[127]* Handbook of Surface and Colloidal Chemistry (Birdi, K.S. ed., CRC Press, 1997)
[128] Ausubel et al. (eds) (2002) Short protocols in molecular biology, 5th edition (Current Protocols).
[129] Molecular Biology Techniques: An Intensive Laboratory Course, (Ream et al., eds., 1998, Academic Press)
[130] PCR (Introduction to Biotechniques Series), 2nd ed. (Newton & Graham eds., 1997, Springer Verlag)
[131] Geysen et al. (1984) PNAS USA 81:3998-4002.
[132] Carter (1994) Methods Mol Biol 36:207-23.
[133] Jameson, BA et al. 1988, CABIOS 4(1):181-186.
[134] Raddrizzani & Hammer (2000) Brief Bioinform 1(2):179-89.
[135] Bublil et al. (2007) Proteins 68(1):294-304.
[136] De Lalla et al. (1999) J. Immunol. 163:1725-29.
[137] Kwok et al. (2001) Trends Immunol 22:583-88.
[138] Brusic et al. (1998) Bioinformatics 14(2):121-30
[139] Meister et al. (1995) Vaccine 13(6):581-91.
[140] Roberts et al. (1996) AIDSRes Hum Retroviruses 12(7):593-610.
[141] Maksyutov & Zagrebelnaya (1993) Comput Appl Biosci 9(3):291-7.
[142] Feller & de la Cruz (1991) Nature 349(6311):720-1.
[143] Hopp (1993) Peptide Research 6:183-190.
[144] Welling et al. (1985) FEBS Lett. 188:215-218.
[145] Davenport et al. (1995) Immunogenetics 42:392-297.
[146] Tsurui & Takahashi (2007) J Pharmacol Sci. 105(4):299-316.
[147] Tong et al. (2007) Brief Bioinform. 8(2):96-108.
[148] Schirle et al. (2001) J Immunol Methods. 257(1-2):1-16.
[149] Chen et al. (2007) Amino Acids 33(3):423-8.
[150] Current Protocols in Molecular Biology (F.M. Ausubel et al., eds., 1987) Supplement 30
[151] Smith & Waterman (1981) Adv. Appl. Math. 2: 482-489.
[152] Nahm & Burton (2008) http://www.vaccine.uab.edu/MOPA4%20Protocol.pdf

## Claims

1. An immunogenic composition comprising: (i) a conjugated pneumococcal capsular saccharide; and (ii) a meningococcal factor H binding protein (fHBP) antigen, but not including meningococcal outer membrane vesicles.

2. The composition of claim 1, including capsular saccharide from two or more different pneumococcal serotypes; for instance, wherein capsular saccharides are selected from the following pneumococcal serotypes: 1, 2, 3, 4, 5, 6A, 6B, 7F, 8, 9N, 9V, 10A, 11A, 12F, 14, 15B, 17F, 18C, 19A, 19F, 20, 22F, 23F and 33F.

3. The composition of claim 2, comprising (a) capsular saccharide from each of serotypes 4, 6B, 9V, 14, 18C, 19F, and 23F or (b) capsular saccharide from each of serotypes 1, 3, 4, 5, 6A, 6B, 7F, 9V, 14, 18C, 19, 19F and 23F.

4. The composition of claim 2 or claim 3, wherein each serotype's saccharide is separately conjugated to a carrier protein selected from the group consisting of CRM 197, tetanus toxoid, diphtheria toxoid and *H. influenzae* protein D, and wherein the carrier protein for different serotypes may be the same or different.

5. The composition of any preceding claim, wherein
(I) the composition includes a single fHBP polypeptide comprising: (a) an amino acid sequence having at least 85% sequence identity to SEQ ID NO: 1 and/or comprising an amino acid sequence consisting of a fragment of at least 7 contiguous amino acids from SEQ ID NO: 1; or (b) an amino acid sequence having at least 85% sequence identity to SEQ ID NO: 2 and/or comprising an amino acid sequence consisting of a fragment of at least 7 contiguous amino acids from SEQ ID NO: 2; or (c) an amino acid sequence having at least 85% sequence identity to SEQ ID NO: 3 and/or comprising an amino acid sequence consisting of a fragment of at least 7 contiguous amino acids from SEQ ID NO: 3; or
(II) the composition includes two different fHBP polypeptides: (a) a first polypeptide, comprising an amino acid sequence having at least 85% sequence identity to SEQ ID NO: 1 and/or comprising an amino acid sequence consisting of a fragment of at least 7 contiguous amino acids from SEQ ID NO: 1; (b) a second polypeptide, comprising an amino acid sequence having at least 85% sequence identity to SEQ ID NO: 3 and/or comprising an amino acid sequence consisting of a fragment of at least 7 contiguous amino acids from SEQ ID NO: 3; or
(III) the composition includes two different fHBP polypeptides: (a) a first polypeptide, comprising an amino acid sequence having at least 85% sequence identity to SEQ ID NO: 1 and/or comprising an amino acid sequence consisting of a fragment of at least 7 contiguous amino acids from SEQ ID NO: 1; and (b) a second polypeptide, comprising an amino acid sequence having at least 85% sequence identity to SEQ ID NO: 2 and/or comprising an amino acid sequence consisting of a fragment of at least 7 contiguous amino acids from SEQ ID NO: 2; or
(IV) the composition includes three different fHBP polypeptides: (a) a first polypeptide, comprising an amino acid sequence having at least 85% sequence identity to SEQ ID NO: 1 and/or comprising an amino acid sequence consisting of a fragment of at least 7 contiguous amino acids from SEQ ID NO: 1; (b) a second polypeptide, comprising an amino acid sequence having at least 85% sequence identity to SEQ ID NO: 2 and/or comprising an amino acid sequence consisting of a fragment of at least 7 contiguous amino acids from SEQ ID NO: 2; and (c) a third polypeptide, comprising an amino acid sequence having at least 85% sequence identity to SEQ ID NO: 3 and/or comprising an amino acid sequence consisting of a fragment of at least 7 contiguous amino acids from SEQ ID NO: 3.

6. The composition of any preceding claim, including a polypeptide comprising an amino acid sequence having at least 93% sequence identity to SEQ ID NO: 3 and/or comprising an amino acid sequence consisting of a fragment of at least 40 contiguous amino acids from SEQ ID NO: 3

7. The composition of any preceding claim, comprising an aluminium phosphate adjuvant; for instance, comprising: (i) an aluminium phosphate adjuvant; (ii) a conjugated pneumococcal capsular saccharide from each of pneumococcal serotypes 4, 6B, 9V, 14, 18C, 19F, and 23F, each of said saccharides being conjugated to a CRM197 carrier protein; and (iii) at least two different meningococcal factor H binding protein antigens, each of which is at least partially adsorbed to an aluminium phosphate adjuvant.

8. The composition of claim 7, wherein (I) at least one of the conjugated pneumococcal saccharides is adsorbed to an aluminium phosphate adjuvant; and/or (II) the composition includes sodium chloride and/or a buffer.

9. The composition of any preceding claim, wherein: (I) a fHBP polypeptide therein is lipidated at a N-terminus cysteine; (II) the total amount of fHBP polypeptide(s) in the composition is less than 600µg; (III) the total amount of fHBP polypeptide(s) in the composition is less than 200µg; (IV) the total amount of fHBP polypeptide(s) in the composition is less than 60µg; (V) the composition further includes one or more conjugate(s) of capsular saccharides from 1, 2, 3, or 4 of meningococcal serogroups A, C, W135 and Y *e*.*g*. including a conjugate of a serogroup C meningococcal capsular saccharide; (VI) the composition includes an aluminium hydroxyphosphate adjuvant; and/or (VII) the composition is free from aluminium hydroxide.

10. The composition of any one of claims 1 to 9, for use as a medicament *e*.*g*. for use in a method for raising an immune response in a mammal by administering the composition to the mammal.

11. A process for preparing an immunogenic composition, comprising steps of:
(A) (i) mixing at least one conjugated pneumococcal capsular saccharide with an aluminium phosphate adjuvant to form a conjugate/adjuvant mixture; and (ii) mixing the conjugate/adjuvant mixture with at least one meningococcal factor H binding protein; or
(B) (i) mixing at least one conjugated pneumococcal capsular saccharide with at least one meningococcal factor H binding protein to form an antigen mixture; and (ii) mixing the antigen mixture with an aluminium phosphate adjuvant; or
(C) (i) mixing at least one meningococcal factor H binding protein with an aluminium phosphate adjuvant to form a protein/adjuvant mixture; and (ii) mixing the protein/adjuvant mixture with at least one conjugated pneumococcal capsular saccharide; or
(D) (i) mixing at least one meningococcal factor H binding protein with an aluminium phosphate adjuvant to form a protein/adjuvant mixture; (ii) mixing at least one conjugated pneumococcal capsular saccharide with an aluminium phosphate adjuvant to form a conjugate/adjuvant mixture; and (iii) mixing the protein/adjuvant mixture and conjugate/adjuvant mixture.

12. The process of claim 11, which does not include a step of mixing an aluminium hydroxide adjuvant with any of (i) a meningococcal factor H binding protein, (ii) an aluminium phosphate adjuvant, (iii) a conjugated pneumococcal capsular saccharide; (iv) a conjugate/adjuvant mixture; (v) an antigen mixture; or (vi) a protein/adjuvant mixture.


SEQUENCE LISTING

<110>   NOVARTIS AG

<120>   COMBINATIONS OF MENINGOCOCCAL FACTOR H BINDING PROTEIN AND
PNEUMOCOCCAL
        SACCHARIDE CONJUGATES

<130>   P064883EP

<150>   EP 10716044.2
<151>   2010-03-24

<150>   PCT/IB2010/000734
<151>   2010-03-24

<150>   USSN 61/163,005
<151>   2009-03-24

<150>   USSN 61/270,407
<151>   2009-07-07

<160>   68

<170>   SeqWin2010, version 1.0

<210>   1
<211>   248
<212>   PRT
<213>   Neisseria meningitidis

<400>   1
Val Ala Ala Asp Ile Gly Ala Gly Leu Ala Asp Ala Leu Thr Ala Pro
1               5                   10                  15

Leu Asp His Lys Asp Lys Gly Leu Gln Ser Leu Thr Leu Asp Gln Ser
            20                  25                  30

Val Arg Lys Asn Glu Lys Leu Lys Leu Ala Ala Gln Gly Ala Glu Lys
        35                  40                  45

Thr Tyr Gly Asn Gly Asp Ser Leu Asn Thr Gly Lys Leu Lys Asn Asp
    50                  55                  60

Lys Val Ser Arg Phe Asp Phe Ile Arg Gln Ile Glu Val Asp Gly Gln
65                  70                  75                  80

Leu Ile Thr Leu Glu Ser Gly Glu Phe Gln Val Tyr Lys Gln Ser His
                85                  90                  95

Ser Ala Leu Thr Ala Phe Gln Thr Glu Gln Ile Gln Asp Ser Glu His
            100                 105                 110

Ser Gly Lys Met Val Ala Lys Arg Gln Phe Arg Ile Gly Asp Ile Ala
        115                 120                 125

Gly Glu His Thr Ser Phe Asp Lys Leu Pro Glu Gly Gly Arg Ala Thr
    130                 135                 140

Tyr Arg Gly Thr Ala Phe Gly Ser Asp Asp Ala Gly Gly Lys Leu Thr
145                 150                 155                 160

Tyr Thr Ile Asp Phe Ala Ala Lys Gln Gly Asn Gly Lys Ile Glu His

                165                 170                 175

Leu Lys Ser Pro Glu Leu Asn Val Asp Leu Ala Ala Ala Asp Ile Lys
            180                 185                 190

Pro Asp Gly Lys Arg His Ala Val Ile Ser Gly Ser Val Leu Tyr Asn
        195                 200                 205

Gln Ala Glu Lys Gly Ser Tyr Ser Leu Gly Ile Phe Gly Gly Lys Ala
    210                 215                 220

Gln Glu Val Ala Gly Ser Ala Glu Val Lys Thr Val Asn Gly Ile Arg
225                 230                 235                 240

His Ile Gly Leu Ala Ala Lys Gln
                245

<210> 2
<211> 247
<212> PRT
<213> Neisseria meningitidis

<400> 2
Val Ala Ala Asp Ile Gly Ala Gly Leu Ala Asp Ala Leu Thr Ala Pro
1               5                   10                  15

Leu Asp His Lys Asp Lys Ser Leu Gln Ser Leu Thr Leu Asp Gln Ser
            20                  25                  30

Val Arg Lys Asn Glu Lys Leu Lys Leu Ala Ala Gln Gly Ala Glu Lys
        35                  40                  45

Thr Tyr Gly Asn Gly Asp Ser Leu Asn Thr Gly Lys Leu Lys Asn Asp
    50                  55                  60

Lys Val Ser Arg Phe Asp Phe Ile Arg Gln Ile Glu Val Asp Gly Gln
65                  70                  75                  80

Leu Ile Thr Leu Glu Ser Gly Glu Phe Gln Ile Tyr Lys Gln Asp His
                85                  90                  95

Ser Ala Val Val Ala Leu Gln Ile Glu Lys Ile Asn Asn Pro Asp Lys
            100                 105                 110

Ile Asp Ser Leu Ile Asn Gln Arg Ser Phe Leu Val Ser Gly Leu Gly
        115                 120                 125

Gly Glu His Thr Ala Phe Asn Gln Leu Pro Asp Gly Lys Ala Glu Tyr
    130                 135                 140

His Gly Lys Ala Phe Ser Ser Asp Asp Ala Gly Gly Lys Leu Thr Tyr
145                 150                 155                 160

Thr Ile Asp Phe Ala Ala Lys Gln Gly His Gly Lys Ile Glu His Leu
                165                 170                 175

Lys Thr Pro Glu Gln Asn Val Glu Leu Ala Ala Ala Glu Leu Lys Ala
            180                 185                 190

Asp Glu Lys Ser His Ala Val Ile Leu Gly Asp Thr Arg Tyr Gly Ser
        195                 200                 205

Glu Glu Lys Gly Thr Tyr His Leu Ala Leu Phe Gly Asp Arg Ala Gln

210                 215                 220

Glu Ile Ala Gly Ser Ala Thr Val Lys Ile Gly Glu Lys Val His Glu
225                 230                 235                 240

Ile Gly Ile Ala Gly Lys Gln
                245

<210>   3
<211>   250
<212>   PRT
<213>   Neisseria meningitidis

<400>   3
Val Ala Ala Asp Ile Gly Thr Gly Leu Ala Asp Ala Leu Thr Ala Pro
1               5                   10                  15

Leu Asp His Lys Asp Lys Gly Leu Lys Ser Leu Thr Leu Glu Asp Ser
            20                  25                  30

Ile Pro Gln Asn Gly Thr Leu Thr Leu Ser Ala Gln Gly Ala Glu Lys
        35                  40                  45

Thr Phe Lys Ala Gly Asp Lys Asp Asn Ser Leu Asn Thr Gly Lys Leu
    50                  55                  60

Lys Asn Asp Lys Ile Ser Arg Phe Asp Phe Val Gln Lys Ile Glu Val
65                  70                  75                  80

Asp Gly Gln Thr Ile Thr Leu Ala Ser Gly Glu Phe Gln Ile Tyr Lys
                85                  90                  95

Gln Asn His Ser Ala Val Val Ala Leu Gln Ile Glu Lys Ile Asn Asn
            100                 105                 110

Pro Asp Lys Thr Asp Ser Leu Ile Asn Gln Arg Ser Phe Leu Val Ser
        115                 120                 125

Gly Leu Gly Gly Glu His Thr Ala Phe Asn Gln Leu Pro Gly Gly Lys
    130                 135                 140

Ala Glu Tyr His Gly Lys Ala Phe Ser Ser Asp Asp Pro Asn Gly Arg
145                 150                 155                 160

Leu His Tyr Ser Ile Asp Phe Thr Lys Lys Gln Gly Tyr Gly Arg Ile
                165                 170                 175

Glu His Leu Lys Thr Leu Glu Gln Asn Val Glu Leu Ala Ala Ala Glu
            180                 185                 190

Leu Lys Ala Asp Glu Lys Ser His Ala Val Ile Leu Gly Asp Thr Arg
        195                 200                 205

Tyr Gly Ser Glu Glu Lys Gly Thr Tyr His Leu Ala Leu Phe Gly Asp
    210                 215                 220

Arg Ala Gln Glu Ile Ala Gly Ser Ala Thr Val Lys Ile Gly Glu Lys
225                 230                 235                 240

Val His Glu Ile Gly Ile Ala Gly Lys Gln
                245                 250

<210>   4

<211>   644
<212>   PRT
<213>   Neisseria meningitidis

<400>   4
Met Ala Ser Pro Asp Val Lys Ser Ala Asp Thr Leu Ser Lys Pro Ala
1               5                   10                  15

Ala Pro Val Val Ser Glu Lys Glu Thr Glu Ala Lys Glu Asp Ala Pro
            20                  25                  30

Gln Ala Gly Ser Gln Gly Gln Gly Ala Pro Ser Ala Gln Gly Gly Gln
        35                  40                  45

Asp Met Ala Ala Val Ser Glu Glu Asn Thr Gly Asn Gly Gly Ala Ala
    50                  55                  60

Ala Thr Asp Lys Pro Lys Asn Glu Asp Glu Gly Ala Gln Asn Asp Met
65                  70                  75                  80

Pro Gln Asn Ala Ala Asp Thr Asp Ser Leu Thr Pro Asn His Thr Pro
                85                  90                  95

Ala Ser Asn Met Pro Ala Gly Asn Met Glu Asn Gln Ala Pro Asp Ala
            100                 105                 110

Gly Glu Ser Glu Gln Pro Ala Asn Gln Pro Asp Met Ala Asn Thr Ala
        115                 120                 125

Asp Gly Met Gln Gly Asp Asp Pro Ser Ala Gly Gly Glu Asn Ala Gly
    130                 135                 140

Asn Thr Ala Ala Gln Gly Thr Asn Gln Ala Glu Asn Asn Gln Thr Ala
145                 150                 155                 160

Gly Ser Gln Asn Pro Ala Ser Ser Thr Asn Pro Ser Ala Thr Asn Ser
                165                 170                 175

Gly Gly Asp Phe Gly Arg Thr Asn Val Gly Asn Ser Val Val Ile Asp
            180                 185                 190

Gly Pro Ser Gln Asn Ile Thr Leu Thr His Cys Lys Gly Asp Ser Cys
        195                 200                 205

Ser Gly Asn Asn Phe Leu Asp Glu Glu Val Gln Leu Lys Ser Glu Phe
    210                 215                 220

Glu Lys Leu Ser Asp Ala Asp Lys Ile Ser Asn Tyr Lys Lys Asp Gly
225                 230                 235                 240

Lys Asn Asp Gly Lys Asn Asp Lys Phe Val Gly Leu Val Ala Asp Ser
                245                 250                 255

Val Gln Met Lys Gly Ile Asn Gln Tyr Ile Ile Phe Tyr Lys Pro Lys
            260                 265                 270

Pro Thr Ser Phe Ala Arg Phe Arg Arg Ser Ala Arg Ser Arg Arg Ser
        275                 280                 285

Leu Pro Ala Glu Met Pro Leu Ile Pro Val Asn Gln Ala Asp Thr Leu
    290                 295                 300

Ile Val Asp Gly Glu Ala Val Ser Leu Thr Gly His Ser Gly Asn Ile

305                 310                 315                 320

Phe Ala Pro Glu Gly Asn Tyr Arg Tyr Leu Thr Tyr Gly Ala Glu Lys
                325                 330                 335

Leu Pro Gly Gly Ser Tyr Ala Leu Arg Val Gln Gly Glu Pro Ser Lys
            340                 345                 350

Gly Glu Met Leu Ala Gly Thr Ala Val Tyr Asn Gly Glu Val Leu His
        355                 360                 365

Phe His Thr Glu Asn Gly Arg Pro Ser Pro Ser Arg Gly Arg Phe Ala
    370                 375                 380

Ala Lys Val Asp Phe Gly Ser Lys Ser Val Asp Gly Ile Ile Asp Ser
385                 390                 395                 400

Gly Asp Gly Leu His Met Gly Thr Gln Lys Phe Lys Ala Ala Ile Asp
                405                 410                 415

Gly Asn Gly Phe Lys Gly Thr Trp Thr Glu Asn Gly Gly Gly Asp Val
            420                 425                 430

Ser Gly Lys Phe Tyr Gly Pro Ala Gly Glu Glu Val Ala Gly Lys Tyr
        435                 440                 445

Ser Tyr Arg Pro Thr Asp Ala Glu Lys Gly Gly Phe Gly Val Phe Ala
    450                 455                 460

Gly Lys Lys Glu Gln Asp Gly Ser Gly Gly Gly Gly Ala Thr Tyr Lys
465                 470                 475                 480

Val Asp Glu Tyr His Ala Asn Ala Arg Phe Ala Ile Asp His Phe Asn
                485                 490                 495

Thr Ser Thr Asn Val Gly Gly Phe Tyr Gly Leu Thr Gly Ser Val Glu
            500                 505                 510

Phe Asp Gln Ala Lys Arg Asp Gly Lys Ile Asp Ile Thr Ile Pro Val
        515                 520                 525

Ala Asn Leu Gln Ser Gly Ser Gln His Phe Thr Asp His Leu Lys Ser
    530                 535                 540

Ala Asp Ile Phe Asp Ala Ala Gln Tyr Pro Asp Ile Arg Phe Val Ser
545                 550                 555                 560

Thr Lys Phe Asn Phe Asn Gly Lys Lys Leu Val Ser Val Asp Gly Asn
                565                 570                 575

Leu Thr Met His Gly Lys Thr Ala Pro Val Lys Leu Lys Ala Glu Lys
            580                 585                 590

Phe Asn Cys Tyr Gln Ser Pro Met Ala Lys Thr Glu Val Cys Gly Gly
        595                 600                 605

Asp Phe Ser Thr Thr Ile Asp Arg Thr Lys Trp Gly Val Asp Tyr Leu
    610                 615                 620

Val Asn Val Gly Met Thr Lys Ser Val Arg Ile Asp Ile Gln Ile Glu
625                 630                 635                 640

Ala Ala Lys Gln

<210>   5
<211>   434
<212>   PRT
<213>   Neisseria meningitidis

<400>   5
Met Val Ser Ala Val Ile Gly Ser Ala Ala Val Gly Ala Lys Ser Ala
1               5                   10                  15

Val Asp Arg Arg Thr Thr Gly Ala Gln Thr Asp Asp Asn Val Met Ala
            20                  25                  30

Leu Arg Ile Glu Thr Thr Ala Arg Ser Tyr Leu Arg Gln Asn Asn Gln
        35                  40                  45

Thr Lys Gly Tyr Thr Pro Gln Ile Ser Val Val Gly Tyr Asp Arg His
    50                  55                  60

Leu Leu Leu Leu Gly Gln Val Ala Thr Glu Gly Glu Lys Gln Phe Val
65                  70                  75                  80

Gly Gln Ile Ala Arg Ser Glu Gln Ala Ala Glu Gly Val Tyr Asn Tyr
                85                  90                  95

Ile Thr Val Ala Ser Leu Pro Arg Thr Ala Gly Asp Ile Ala Gly Asp
            100                 105                 110

Thr Trp Asn Thr Ser Lys Val Arg Ala Thr Leu Leu Gly Ile Ser Pro
        115                 120                 125

Ala Thr Arg Ala Arg Val Lys Ile Val Thr Tyr Gly Asn Val Thr Tyr
    130                 135                 140

Val Met Gly Ile Leu Thr Pro Glu Glu Gln Ala Gln Ile Thr Gln Lys
145                 150                 155                 160

Val Ser Thr Thr Val Gly Val Gln Lys Val Ile Thr Leu Tyr Gln Asn
                165                 170                 175

Tyr Val Gln Arg Gly Ser Gly Gly Gly Gly Val Ala Ala Asp Ile Gly
            180                 185                 190

Ala Gly Leu Ala Asp Ala Leu Thr Ala Pro Leu Asp His Lys Asp Lys
        195                 200                 205

Gly Leu Gln Ser Leu Thr Leu Asp Gln Ser Val Arg Lys Asn Glu Lys
    210                 215                 220

Leu Lys Leu Ala Ala Gln Gly Ala Glu Lys Thr Tyr Gly Asn Gly Asp
225                 230                 235                 240

Ser Leu Asn Thr Gly Lys Leu Lys Asn Asp Lys Val Ser Arg Phe Asp
                245                 250                 255

Phe Ile Arg Gln Ile Glu Val Asp Gly Gln Leu Ile Thr Leu Glu Ser
            260                 265                 270

Gly Glu Phe Gln Val Tyr Lys Gln Ser His Ser Ala Leu Thr Ala Phe
        275                 280                 285

Gln Thr Glu Gln Ile Gln Asp Ser Glu His Ser Gly Lys Met Val Ala

290                 295                 300

Lys Arg Gln Phe Arg Ile Gly Asp Ile Ala Gly Glu His Thr Ser Phe
305                 310                 315                 320

Asp Lys Leu Pro Glu Gly Gly Arg Ala Thr Tyr Arg Gly Thr Ala Phe
                325                 330                 335

Gly Ser Asp Asp Ala Gly Gly Lys Leu Thr Tyr Thr Ile Asp Phe Ala
            340                 345                 350

Ala Lys Gln Gly Asn Gly Lys Ile Glu His Leu Lys Ser Pro Glu Leu
        355                 360                 365

Asn Val Asp Leu Ala Ala Ala Asp Ile Lys Pro Asp Gly Lys Arg His
    370                 375                 380

Ala Val Ile Ser Gly Ser Val Leu Tyr Asn Gln Ala Glu Lys Gly Ser
385                 390                 395                 400

Tyr Ser Leu Gly Ile Phe Gly Gly Lys Ala Gln Glu Val Ala Gly Ser
                405                 410                 415

Ala Glu Val Lys Thr Val Asn Gly Ile Arg His Ile Gly Leu Ala Ala
            420                 425                 430

Lys Gln


<210>   6
<211>   327
<212>   PRT
<213>   Neisseria meningitidis

<400>   6
Ala Thr Asn Asp Asp Asp Val Lys Lys Ala Ala Thr Val Ala Ile Ala
1               5                   10                  15

Ala Ala Tyr Asn Asn Gly Gln Glu Ile Asn Gly Phe Lys Ala Gly Glu
            20                  25                  30

Thr Ile Tyr Asp Ile Asp Glu Asp Gly Thr Ile Thr Lys Lys Asp Ala
        35                  40                  45

Thr Ala Ala Asp Val Glu Ala Asp Asp Phe Lys Gly Leu Gly Leu Lys
    50                  55                  60

Lys Val Val Thr Asn Leu Thr Lys Thr Val Asn Glu Asn Lys Gln Asn
65                  70                  75                  80

Val Asp Ala Lys Val Lys Ala Ala Glu Ser Glu Ile Glu Lys Leu Thr
                85                  90                  95

Thr Lys Leu Ala Asp Thr Asp Ala Ala Leu Ala Asp Thr Asp Ala Ala
            100                 105                 110

Leu Asp Ala Thr Thr Asn Ala Leu Asn Lys Leu Gly Glu Asn Ile Thr
        115                 120                 125

Thr Phe Ala Glu Glu Thr Lys Thr Asn Ile Val Lys Ile Asp Glu Lys
    130                 135                 140

Leu Glu Ala Val Ala Asp Thr Val Asp Lys His Ala Glu Ala Phe Asn

145                 150                 155                 160

Asp Ile Ala Asp Ser Leu Asp Glu Thr Asn Thr Lys Ala Asp Glu Ala
                165                 170                 175

Val Lys Thr Ala Asn Glu Ala Lys Gln Thr Ala Glu Glu Thr Lys Gln
            180                 185                 190

Asn Val Asp Ala Lys Val Lys Ala Ala Glu Thr Ala Ala Gly Lys Ala
        195                 200                 205

Glu Ala Ala Ala Gly Thr Ala Asn Thr Ala Ala Asp Lys Ala Glu Ala
    210                 215                 220

Val Ala Ala Lys Val Thr Asp Ile Lys Ala Asp Ile Ala Thr Asn Lys
225                 230                 235                 240

Asp Asn Ile Ala Lys Lys Ala Asn Ser Ala Asp Val Tyr Thr Arg Glu
                245                 250                 255

Glu Ser Asp Ser Lys Phe Val Arg Ile Asp Gly Leu Asn Ala Thr Thr
            260                 265                 270

Glu Lys Leu Asp Thr Arg Leu Ala Ser Ala Glu Lys Ser Ile Ala Asp
        275                 280                 285

His Asp Thr Arg Leu Asn Gly Leu Asp Lys Thr Val Ser Asp Leu Arg
    290                 295                 300

Lys Glu Thr Arg Gln Gly Leu Ala Glu Gln Ala Ala Leu Ser Gly Leu
305                 310                 315                 320

Phe Gln Pro Tyr Asn Val Gly
                325

<210>   7
<211>   792
<212>   PRT
<213>   Neisseria meningitidis

<400>   7
Met Lys Pro Leu Gln Met Leu Pro Ile Ala Ala Leu Val Gly Ser Ile
1               5                   10                  15

Phe Gly Asn Pro Val Leu Ala Ala Asp Glu Ala Ala Thr Glu Thr Thr
            20                  25                  30

Pro Val Lys Ala Glu Ile Lys Ala Val Arg Val Lys Gly Gln Arg Asn
        35                  40                  45

Ala Pro Ala Ala Val Glu Arg Val Asn Leu Asn Arg Ile Lys Gln Glu
    50                  55                  60

Met Ile Arg Asp Asn Lys Asp Leu Val Arg Tyr Ser Thr Asp Val Gly
65                  70                  75                  80

Leu Ser Asp Ser Gly Arg His Gln Lys Gly Phe Ala Val Arg Gly Val
                85                  90                  95

Glu Gly Asn Arg Val Gly Val Ser Ile Asp Gly Val Asn Leu Pro Asp
            100                 105                 110

Ser Glu Glu Asn Ser Leu Tyr Ala Arg Tyr Gly Asn Phe Asn Ser Ser

115                 120                 125

Arg Leu Ser Ile Asp Pro Glu Leu Val Arg Asn Ile Glu Ile Val Lys
    130                 135                 140

Gly Ala Asp Ser Phe Asn Thr Gly Ser Gly Ala Leu Gly Gly Gly Val
145                 150                 155                 160

Asn Tyr Gln Thr Leu Gln Gly Arg Asp Leu Leu Leu Asp Asp Arg Gln
                165                 170                 175

Phe Gly Val Met Met Lys Asn Gly Tyr Ser Thr Arg Asn Arg Glu Trp
            180                 185                 190

Thr Asn Thr Leu Gly Phe Gly Val Ser Asn Asp Arg Val Asp Ala Ala
        195                 200                 205

Leu Leu Tyr Ser Gln Arg Arg Gly His Glu Thr Glu Ser Ala Gly Asn
    210                 215                 220

Arg Gly Tyr Ala Val Glu Gly Glu Gly Ser Gly Ala Asn Ile Arg Gly
225                 230                 235                 240

Ser Ala Arg Gly Ile Pro Asp Ser Ser Lys His Lys Tyr Asn His His
                245                 250                 255

Ala Leu Gly Lys Ile Ala Tyr Gln Ile Asn Asp Asn His Arg Ile Gly
            260                 265                 270

Ala Ser Leu Asn Gly Gln Gln Gly His Asn Tyr Thr Val Glu Glu Ser
        275                 280                 285

Tyr Asn Leu Thr Ala Ser Ser Trp Arg Glu Ala Asp Asp Val Asn Arg
    290                 295                 300

Arg Arg Asn Ala Asn Leu Phe Tyr Glu Trp Met Pro Asp Ser Asn Trp
305                 310                 315                 320

Leu Ser Ser Leu Lys Ala Asp Phe Asp Tyr Gln Lys Thr Lys Val Ala
                325                 330                 335

Ala Val Asn Asn Lys Gly Ser Phe Pro Met Asp Tyr Ser Thr Trp Thr
            340                 345                 350

Arg Asn Tyr Asn Gln Lys Asp Leu Asp Glu Ile Tyr Asn Arg Ser Met
        355                 360                 365

Asp Thr Arg Phe Lys Arg Phe Thr Leu Arg Leu Asp Ser His Pro Leu
    370                 375                 380

Gln Leu Gly Gly Gly Arg His Arg Leu Ser Phe Lys Thr Phe Val Ser
385                 390                 395                 400

Arg Arg Asp Phe Glu Asn Leu Asn Arg Asp Asp Tyr Tyr Phe Ser Gly
                405                 410                 415

Arg Val Val Arg Thr Thr Ser Ser Ile Gln His Pro Val Lys Thr Thr
            420                 425                 430

Asn Tyr Gly Phe Ser Leu Ser Asp Gln Ile Gln Trp Asn Asp Val Phe
        435                 440                 445

Ser Ser Arg Ala Gly Ile Arg Tyr Asp His Thr Lys Met Thr Pro Gln

450                 455                 460

Glu Leu Asn Ala Glu Cys His Ala Cys Asp Lys Thr Pro Pro Ala Ala
465                 470                 475                 480

Asn Thr Tyr Lys Gly Trp Ser Gly Phe Val Gly Leu Ala Ala Gln Leu
                485                 490                 495

Asn Gln Ala Trp Arg Val Gly Tyr Asp Ile Thr Ser Gly Tyr Arg Val
            500                 505                 510

Pro Asn Ala Ser Glu Val Tyr Phe Thr Tyr Asn His Gly Ser Gly Asn
        515                 520                 525

Trp Leu Pro Asn Pro Asn Leu Lys Ala Glu Arg Ser Thr Thr His Thr
    530                 535                 540

Leu Ser Leu Gln Gly Arg Ser Glu Lys Gly Met Leu Asp Ala Asn Leu
545                 550                 555                 560

Tyr Gln Ser Asn Tyr Arg Asn Phe Leu Ser Glu Glu Gln Lys Leu Thr
                565                 570                 575

Thr Ser Gly Thr Pro Gly Cys Thr Glu Glu Asn Ala Tyr Tyr Gly Ile
            580                 585                 590

Cys Ser Asp Pro Tyr Lys Glu Lys Leu Asp Trp Gln Met Lys Asn Ile
        595                 600                 605

Asp Lys Ala Arg Ile Arg Gly Ile Glu Leu Thr Gly Arg Leu Asn Val
    610                 615                 620

Asp Lys Val Ala Ser Phe Val Pro Glu Gly Trp Lys Leu Phe Gly Ser
625                 630                 635                 640

Leu Gly Tyr Ala Lys Ser Lys Leu Ser Gly Asp Asn Ser Leu Leu Ser
                645                 650                 655

Thr Gln Pro Leu Lys Val Ile Ala Gly Ile Asp Tyr Glu Ser Pro Ser
            660                 665                 670

Glu Lys Trp Gly Val Phe Ser Arg Leu Thr Tyr Leu Gly Ala Lys Lys
        675                 680                 685

Val Lys Asp Ala Gln Tyr Thr Val Tyr Glu Asn Lys Gly Trp Gly Thr
    690                 695                 700

Pro Leu Gln Lys Lys Val Lys Asp Tyr Pro Trp Leu Asn Lys Ser Ala
705                 710                 715                 720

Tyr Val Phe Asp Met Tyr Gly Phe Tyr Lys Pro Ala Lys Asn Leu Thr
                725                 730                 735

Leu Arg Ala Gly Val Tyr Asn Leu Phe Asn Arg Lys Tyr Thr Thr Trp
            740                 745                 750

Asp Ser Leu Arg Gly Leu Tyr Ser Tyr Ser Thr Thr Asn Ala Val Asp
        755                 760                 765

Arg Asp Gly Lys Gly Leu Asp Arg Tyr Arg Ala Pro Gly Arg Asn Tyr
    770                 775                 780

Ala Val Ser Leu Glu Trp Lys Phe

785                 790

<210>   8
<211>   793
<212>   PRT
<213>   Neisseria meningitidis

<400>   8
Met Lys Pro Leu Gln Met Leu Pro Ile Ala Ala Leu Val Gly Ser Ile
1               5                   10                  15

Phe Gly Asn Pro Val Phe Ala Ala Asp Glu Ala Ala Thr Glu Thr Thr
            20                  25                  30

Pro Val Lys Ala Glu Val Lys Ala Val Arg Val Lys Gly Gln Arg Asn
        35                  40                  45

Ala Pro Ala Ala Val Glu Arg Val Asn Leu Asn Arg Ile Lys Gln Glu
    50                  55                  60

Met Ile Arg Asp Asn Lys Asp Leu Val Arg Tyr Ser Thr Asp Val Gly
65                  70                  75                  80

Leu Ser Asp Ser Gly Arg His Gln Lys Gly Phe Ala Val Arg Gly Val
                85                  90                  95

Glu Gly Asn Arg Val Gly Val Ser Ile Asp Gly Val Asn Leu Pro Asp
            100                 105                 110

Ser Glu Glu Asn Ser Leu Tyr Ala Arg Tyr Gly Asn Phe Asn Ser Ser
        115                 120                 125

Arg Leu Ser Ile Asp Pro Glu Leu Val Arg Asn Ile Asp Ile Val Lys
    130                 135                 140

Gly Ala Asp Ser Phe Asn Thr Gly Ser Gly Ala Leu Gly Gly Gly Val
145                 150                 155                 160

Asn Tyr Gln Thr Leu Gln Gly Arg Asp Leu Leu Leu Pro Glu Arg Gln
                165                 170                 175

Phe Gly Val Met Met Lys Asn Gly Tyr Ser Thr Arg Asn Arg Glu Trp
            180                 185                 190

Thr Asn Thr Leu Gly Phe Gly Val Ser Asn Asp Arg Val Asp Ala Ala
        195                 200                 205

Leu Leu Tyr Ser Gln Arg Arg Gly His Glu Thr Glu Ser Ala Gly Lys
    210                 215                 220

Arg Gly Tyr Pro Val Glu Gly Ala Gly Ser Gly Ala Asn Ile Arg Gly
225                 230                 235                 240

Ser Ala Arg Gly Ile Pro Asp Pro Ser Gln His Lys Tyr Asn His His
                245                 250                 255

Ala Leu Gly Lys Ile Ala Tyr Gln Ile Asn Asp Asn His Arg Ile Gly
            260                 265                 270

Ala Ser Leu Asn Gly Gln Gln Gly His Asn Tyr Thr Val Glu Glu Ser
        275                 280                 285

Tyr Asn Leu Leu Ala Ser Tyr Trp Arg Glu Ala Asp Asp Val Asn Arg

290                 295                 300

Arg Arg Asn Thr Asn Leu Phe Tyr Glu Trp Thr Pro Glu Ser Asp Arg
305                 310                 315                 320

Leu Ser Met Val Lys Ala Asp Val Asp Tyr Gln Lys Thr Lys Val Ser
                325                 330                 335

Ala Val Asn Tyr Lys Gly Ser Phe Pro Ile Glu Asp Ser Ser Thr Leu
            340                 345                 350

Thr Arg Asn Tyr Asn Gln Lys Asp Leu Asp Glu Ile Tyr Asn Arg Ser
        355                 360                 365

Met Asp Thr Arg Phe Lys Arg Ile Thr Leu Arg Leu Asp Ser His Pro
    370                 375                 380

Leu Gln Leu Gly Gly Gly Arg His Arg Leu Ser Phe Lys Thr Phe Ala
385                 390                 395                 400

Ser Arg Arg Asp Phe Glu Asn Leu Asn Arg Asp Asp Tyr Tyr Phe Ser
                405                 410                 415

Gly Arg Val Val Arg Thr Thr Ser Ser Ile Gln His Pro Val Lys Thr
            420                 425                 430

Thr Asn Tyr Gly Phe Ser Leu Ser Asp Gln Ile Gln Trp Asn Asp Val
        435                 440                 445

Phe Ser Ser Arg Ala Gly Ile Arg Tyr Asp His Thr Lys Met Thr Pro
    450                 455                 460

Gln Glu Leu Asn Ala Glu Cys His Ala Cys Asp Lys Thr Pro Pro Ala
465                 470                 475                 480

Ala Asn Thr Tyr Lys Gly Trp Ser Gly Phe Val Gly Leu Ala Ala Gln
                485                 490                 495

Leu Asn Gln Ala Trp Arg Val Gly Tyr Asp Ile Thr Ser Gly Tyr Arg
            500                 505                 510

Val Pro Asn Ala Ser Glu Val Tyr Phe Thr Tyr Asn His Gly Ser Gly
        515                 520                 525

Asn Trp Leu Pro Asn Pro Asn Leu Lys Ala Glu Arg Thr Thr Thr His
    530                 535                 540

Thr Leu Ser Leu Gln Gly Arg Ser Glu Lys Gly Thr Leu Asp Ala Asn
545                 550                 555                 560

Leu Tyr Gln Ser Asn Tyr Arg Asn Phe Leu Ser Glu Glu Gln Lys Leu
                565                 570                 575

Thr Thr Ser Gly Asp Val Ser Cys Thr Gln Met Asn Tyr Tyr Tyr Gly
            580                 585                 590

Met Cys Ser Asn Pro Tyr Ser Glu Lys Leu Glu Trp Gln Met Gln Asn
        595                 600                 605

Ile Asp Lys Ala Arg Ile Arg Gly Ile Glu Leu Thr Gly Arg Leu Asn
    610                 615                 620

Val Asp Lys Val Ala Ser Phe Val Pro Glu Gly Trp Lys Leu Phe Gly

625                 630                 635                 640

Ser Leu Gly Tyr Ala Lys Ser Lys Leu Ser Gly Asp Asn Ser Leu Leu
                645                 650                 655

Ser Thr Gln Pro Leu Lys Val Ile Ala Gly Ile Asp Tyr Glu Ser Pro
            660                 665                 670

Ser Glu Lys Trp Gly Val Phe Ser Arg Leu Thr Tyr Leu Gly Ala Lys
        675                 680                 685

Lys Val Lys Asp Ala Gln Tyr Thr Val Tyr Glu Asn Lys Gly Trp Gly
    690                 695                 700

Thr Pro Leu Gln Lys Lys Val Lys Asp Tyr Pro Trp Leu Asn Lys Ser
705                 710                 715                 720

Ala Tyr Val Phe Asp Met Tyr Gly Phe Tyr Lys Pro Val Lys Asn Leu
                725                 730                 735

Thr Leu Arg Ala Gly Val Tyr Asn Val Phe Asn Arg Lys Tyr Thr Thr
            740                 745                 750

Trp Asp Ser Leu Arg Gly Leu Tyr Ser Tyr Ser Thr Thr Asn Ser Val
        755                 760                 765

Asp Arg Asp Gly Lys Gly Leu Asp Arg Tyr Arg Ala Pro Ser Arg Asn
    770                 775                 780

Tyr Ala Val Ser Leu Glu Trp Lys Phe
785                 790

<210>   9
<211>   488
<212>   PRT
<213>   Neisseria meningitidis

<400>   9
Met Phe Lys Arg Ser Val Ile Ala Met Ala Cys Ile Phe Ala Leu Ser
1               5                   10                  15

Ala Cys Gly Gly Gly Gly Gly Gly Ser Pro Asp Val Lys Ser Ala Asp
            20                  25                  30

Thr Leu Ser Lys Pro Ala Ala Pro Val Val Ser Glu Lys Glu Thr Glu
        35                  40                  45

Ala Lys Glu Asp Ala Pro Gln Ala Gly Ser Gln Gly Gln Gly Ala Pro
    50                  55                  60

Ser Ala Gln Gly Ser Gln Asp Met Ala Ala Val Ser Glu Glu Asn Thr
65                  70                  75                  80

Gly Asn Gly Gly Ala Val Thr Ala Asp Asn Pro Lys Asn Glu Asp Glu
                85                  90                  95

Val Ala Gln Asn Asp Met Pro Gln Asn Ala Ala Gly Thr Asp Ser Ser
            100                 105                 110

Thr Pro Asn His Thr Pro Asp Pro Asn Met Leu Ala Gly Asn Met Glu
        115                 120                 125

Asn Gln Ala Thr Asp Ala Gly Glu Ser Ser Gln Pro Ala Asn Gln Pro

130                 135                 140

Asp Met Ala Asn Ala Ala Asp Gly Met Gln Gly Asp Asp Pro Ser Ala
145                 150                 155                 160

Gly Gly Gln Asn Ala Gly Asn Thr Ala Ala Gln Gly Ala Asn Gln Ala
                165                 170                 175

Gly Asn Asn Gln Ala Ala Gly Ser Ser Asp Pro Ile Pro Ala Ser Asn
            180                 185                 190

Pro Ala Pro Ala Asn Gly Gly Ser Asn Phe Gly Arg Val Asp Leu Ala
        195                 200                 205

Asn Gly Val Leu Ile Asp Gly Pro Ser Gln Asn Ile Thr Leu Thr His
    210                 215                 220

Cys Lys Gly Asp Ser Cys Ser Gly Asn Asn Phe Leu Asp Glu Glu Val
225                 230                 235                 240

Gln Leu Lys Ser Glu Phe Glu Lys Leu Ser Asp Ala Asp Lys Ile Ser
                245                 250                 255

Asn Tyr Lys Lys Asp Gly Lys Asn Asp Lys Phe Val Gly Leu Val Ala
            260                 265                 270

Asp Ser Val Gln Met Lys Gly Ile Asn Gln Tyr Ile Ile Phe Tyr Lys
        275                 280                 285

Pro Lys Pro Thr Ser Phe Ala Arg Phe Arg Arg Ser Ala Arg Ser Arg
    290                 295                 300

Arg Ser Leu Pro Ala Glu Met Pro Leu Ile Pro Val Asn Gln Ala Asp
305                 310                 315                 320

Thr Leu Ile Val Asp Gly Glu Ala Val Ser Leu Thr Gly His Ser Gly
                325                 330                 335

Asn Ile Phe Ala Pro Glu Gly Asn Tyr Arg Tyr Leu Thr Tyr Gly Ala
            340                 345                 350

Glu Lys Leu Pro Gly Gly Ser Tyr Ala Leu Arg Val Gln Gly Glu Pro
        355                 360                 365

Ala Lys Gly Glu Met Leu Ala Gly Ala Ala Val Tyr Asn Gly Glu Val
    370                 375                 380

Leu His Phe His Thr Glu Asn Gly Arg Pro Tyr Pro Thr Arg Gly Arg
385                 390                 395                 400

Phe Ala Ala Lys Val Asp Phe Gly Ser Lys Ser Val Asp Gly Ile Ile
                405                 410                 415

Asp Ser Gly Asp Asp Leu His Met Gly Thr Gln Lys Phe Lys Ala Ala
            420                 425                 430

Ile Asp Gly Asn Gly Phe Lys Gly Thr Trp Thr Glu Asn Gly Ser Gly
        435                 440                 445

Asp Val Ser Gly Lys Phe Tyr Gly Pro Ala Gly Glu Glu Val Ala Gly
    450                 455                 460

Lys Tyr Ser Tyr Arg Pro Thr Asp Ala Glu Lys Gly Gly Phe Gly Val

465                 470                 475                 480

Phe Ala Gly Lys Lys Glu Gln Asp
                485

<210>   10
<211>   364
<212>   PRT
<213>   Neisseria meningitidis

<400>   10
Met Ser Met Lys His Phe Pro Ser Lys Val Leu Thr Thr Ala Ile Leu
1               5                   10                  15

Ala Thr Phe Cys Ser Gly Ala Leu Ala Ala Thr Ser Asp Asp Asp Val
            20                  25                  30

Lys Lys Ala Ala Thr Val Ala Ile Val Ala Ala Tyr Asn Asn Gly Gln
        35                  40                  45

Glu Ile Asn Gly Phe Lys Ala Gly Glu Thr Ile Tyr Asp Ile Gly Glu
    50                  55                  60

Asp Gly Thr Ile Thr Gln Lys Asp Ala Thr Ala Ala Asp Val Glu Ala
65                  70                  75                  80

Asp Asp Phe Lys Gly Leu Gly Leu Lys Lys Val Val Thr Asn Leu Thr
                85                  90                  95

Lys Thr Val Asn Glu Asn Lys Gln Asn Val Asp Ala Lys Val Lys Ala
            100                 105                 110

Ala Glu Ser Glu Ile Glu Lys Leu Thr Thr Lys Leu Ala Asp Thr Asp
        115                 120                 125

Ala Ala Leu Ala Asp Thr Asp Ala Ala Leu Asp Glu Thr Thr Asn Ala
    130                 135                 140

Leu Asn Lys Leu Gly Glu Asn Ile Thr Thr Phe Ala Glu Glu Thr Lys
145                 150                 155                 160

Thr Asn Ile Val Lys Ile Asp Glu Lys Leu Glu Ala Val Ala Asp Thr
                165                 170                 175

Val Asp Lys His Ala Glu Ala Phe Asn Asp Ile Ala Asp Ser Leu Asp
            180                 185                 190

Glu Thr Asn Thr Lys Ala Asp Glu Ala Val Lys Thr Ala Asn Glu Ala
        195                 200                 205

Lys Gln Thr Ala Glu Glu Thr Lys Gln Asn Val Asp Ala Lys Val Lys
    210                 215                 220

Ala Ala Glu Thr Ala Ala Gly Lys Ala Glu Ala Ala Ala Gly Thr Ala
225                 230                 235                 240

Asn Thr Ala Ala Asp Lys Ala Glu Ala Val Ala Ala Lys Val Thr Asp
                245                 250                 255

Ile Lys Ala Asp Ile Ala Thr Asn Lys Ala Asp Ile Ala Lys Asn Ser
            260                 265                 270

Ala Arg Ile Asp Ser Leu Asp Lys Asn Val Ala Asn Leu Arg Lys Glu

275                 280                 285

Thr Arg Gln Gly Leu Ala Glu Gln Ala Ala Leu Ser Gly Leu Phe Gln
    290                 295                 300

Pro Tyr Asn Val Gly Arg Phe Asn Val Thr Ala Ala Val Gly Gly Tyr
305                 310                 315                 320

Lys Ser Glu Ser Ala Val Ala Ile Gly Thr Gly Phe Arg Phe Thr Glu
                325                 330                 335

Asn Phe Ala Ala Lys Ala Gly Val Ala Val Gly Thr Ser Ser Gly Ser
            340                 345                 350

Ser Ala Ala Tyr His Val Gly Val Asn Tyr Glu Trp
        355                 360

<210>   11
<211>   174
<212>   PRT
<213>   Neisseria meningitidis

<400>   11
Met Lys Lys Ala Leu Ala Thr Leu Ile Ala Leu Ala Leu Pro Ala Ala
1               5                   10                  15

Ala Leu Ala Glu Gly Ala Ser Gly Phe Tyr Val Gln Ala Asp Ala Ala
            20                  25                  30

His Ala Lys Ala Ser Ser Ser Leu Gly Ser Ala Lys Gly Phe Ser Pro
        35                  40                  45

Arg Ile Ser Ala Gly Tyr Arg Ile Asn Asp Leu Arg Phe Ala Val Asp
    50                  55                  60

Tyr Thr Arg Tyr Lys Asn Tyr Lys Ala Pro Ser Thr Asp Phe Lys Leu
65                  70                  75                  80

Tyr Ser Ile Gly Ala Ser Ala Ile Tyr Asp Phe Asp Thr Gln Ser Pro
                85                  90                  95

Val Lys Pro Tyr Leu Gly Ala Arg Leu Ser Leu Asn Arg Ala Ser Val
            100                 105                 110

Asp Leu Gly Gly Ser Asp Ser Phe Ser Gln Thr Ser Ile Gly Leu Gly
        115                 120                 125

Val Leu Thr Gly Val Ser Tyr Ala Val Thr Pro Asn Val Asp Leu Asp
    130                 135                 140

Ala Gly Tyr Arg Tyr Asn Tyr Ile Gly Lys Val Asn Thr Val Lys Asn
145                 150                 155                 160

Val Arg Ser Gly Glu Leu Ser Ala Gly Val Arg Val Lys Phe
                165                 170

<210>   12
<211>   591
<212>   PRT
<213>   Neisseria meningitidis

<400>   12
Met Asn Lys Ile Tyr Arg Ile Ile Trp Asn Ser Ala Leu Asn Ala Trp

1               5                   10                  15

Val Val Val Ser Glu Leu Thr Arg Asn His Thr Lys Arg Ala Ser Ala
            20                  25                  30

Thr Val Lys Thr Ala Val Leu Ala Thr Leu Leu Phe Ala Thr Val Gln
        35                  40                  45

Ala Ser Ala Asn Asn Glu Glu Gln Glu Glu Asp Leu Tyr Leu Asp Pro
    50                  55                  60

Val Gln Arg Thr Val Ala Val Leu Ile Val Asn Ser Asp Lys Glu Gly
65                  70                  75                  80

Thr Gly Glu Lys Glu Lys Val Glu Glu Asn Ser Asp Trp Ala Val Tyr
                85                  90                  95

Phe Asn Glu Lys Gly Val Leu Thr Ala Arg Glu Ile Thr Leu Lys Ala
            100                 105                 110

Gly Asp Asn Leu Lys Ile Lys Gln Asn Gly Thr Asn Phe Thr Tyr Ser
        115                 120                 125

Leu Lys Lys Asp Leu Thr Asp Leu Thr Ser Val Gly Thr Glu Lys Leu
    130                 135                 140

Ser Phe Ser Ala Asn Gly Asn Lys Val Asn Ile Thr Ser Asp Thr Lys
145                 150                 155                 160

Gly Leu Asn Phe Ala Lys Glu Thr Ala Gly Thr Asn Gly Asp Thr Thr
                165                 170                 175

Val His Leu Asn Gly Ile Gly Ser Thr Leu Thr Asp Thr Leu Leu Asn
            180                 185                 190

Thr Gly Ala Thr Thr Asn Val Thr Asn Asp Asn Val Thr Asp Asp Glu
        195                 200                 205

Lys Lys Arg Ala Ala Ser Val Lys Asp Val Leu Asn Ala Gly Trp Asn
    210                 215                 220

Ile Lys Gly Val Lys Pro Gly Thr Thr Ala Ser Asp Asn Val Asp Phe
225                 230                 235                 240

Val Arg Thr Tyr Asp Thr Val Glu Phe Leu Ser Ala Asp Thr Lys Thr
                245                 250                 255

Thr Thr Val Asn Val Glu Ser Lys Asp Asn Gly Lys Lys Thr Glu Val
            260                 265                 270

Lys Ile Gly Ala Lys Thr Ser Val Ile Lys Glu Lys Asp Gly Lys Leu
        275                 280                 285

Val Thr Gly Lys Asp Lys Gly Glu Asn Gly Ser Ser Thr Asp Glu Gly
    290                 295                 300

Glu Gly Leu Val Thr Ala Lys Glu Val Ile Asp Ala Val Asn Lys Ala
305                 310                 315                 320

Gly Trp Arg Met Lys Thr Thr Thr Ala Asn Gly Gln Thr Gly Gln Ala
                325                 330                 335

Asp Lys Phe Glu Thr Val Thr Ser Gly Thr Asn Val Thr Phe Ala Ser

340                 345                 350

Gly Lys Gly Thr Thr Ala Thr Val Ser Lys Asp Asp Gln Gly Asn Ile
        355                 360                 365

Thr Val Met Tyr Asp Val Asn Val Gly Asp Ala Leu Asn Val Asn Gln
    370                 375                 380

Leu Gln Asn Ser Gly Trp Asn Leu Asp Ser Lys Ala Val Ala Gly Ser
385                 390                 395                 400

Ser Gly Lys Val Ile Ser Gly Asn Val Ser Pro Ser Lys Gly Lys Met
                405                 410                 415

Asp Glu Thr Val Asn Ile Asn Ala Gly Asn Asn Ile Glu Ile Thr Arg
            420                 425                 430

Asn Gly Lys Asn Ile Asp Ile Ala Thr Ser Met Thr Pro Gln Phe Ser
        435                 440                 445

Ser Val Ser Leu Gly Ala Gly Ala Asp Ala Pro Thr Leu Ser Val Asp
    450                 455                 460

Gly Asp Ala Leu Asn Val Gly Ser Lys Lys Asp Asn Lys Pro Val Arg
465                 470                 475                 480

Ile Thr Asn Val Ala Pro Gly Val Lys Glu Gly Asp Val Thr Asn Val
                485                 490                 495

Ala Gln Leu Lys Gly Val Ala Gln Asn Leu Asn Asn Arg Ile Asp Asn
            500                 505                 510

Val Asp Gly Asn Ala Arg Ala Gly Ile Ala Gln Ala Ile Ala Thr Ala
        515                 520                 525

Gly Leu Val Gln Ala Tyr Leu Pro Gly Lys Ser Met Met Ala Ile Gly
    530                 535                 540

Gly Gly Thr Tyr Arg Gly Glu Ala Gly Tyr Ala Ile Gly Tyr Ser Ser
545                 550                 555                 560

Ile Ser Asp Gly Gly Asn Trp Ile Ile Lys Gly Thr Ala Ser Gly Asn
                565                 570                 575

Ser Arg Gly His Phe Gly Ala Ser Ala Ser Val Gly Tyr Gln Trp
            580                 585                 590

<210>   13
<211>   1457
<212>   PRT
<213>   Neisseria meningitidis

<400>   13
Met Lys Thr Thr Asp Lys Arg Thr Thr Glu Thr His Arg Lys Ala Pro
1               5                   10                  15

Lys Thr Gly Arg Ile Arg Phe Ser Pro Ala Tyr Leu Ala Ile Cys Leu
            20                  25                  30

Ser Phe Gly Ile Leu Pro Gln Ala Trp Ala Gly His Thr Tyr Phe Gly
        35                  40                  45

Ile Asn Tyr Gln Tyr Tyr Arg Asp Phe Ala Glu Asn Lys Gly Lys Phe

50                  55                  60

Ala Val Gly Ala Lys Asp Ile Glu Val Tyr Asn Lys Lys Gly Glu Leu
65                  70                  75                  80

Val Gly Lys Ser Met Thr Lys Ala Pro Met Ile Asp Phe Ser Val Val
                85                  90                  95

Ser Arg Asn Gly Val Ala Ala Leu Val Gly Asp Gln Tyr Ile Val Ser
            100                 105                 110

Val Ala His Asn Gly Gly Tyr Asn Asn Val Asp Phe Gly Ala Glu Gly
        115                 120                 125

Arg Asn Pro Asp Gln His Arg Phe Thr Tyr Lys Ile Val Lys Arg Asn
    130                 135                 140

Asn Tyr Lys Ala Gly Thr Lys Gly His Pro Tyr Gly Gly Asp Tyr His
145                 150                 155                 160

Met Pro Arg Leu His Lys Phe Val Thr Asp Ala Glu Pro Val Glu Met
                165                 170                 175

Thr Ser Tyr Met Asp Gly Arg Lys Tyr Ile Asp Gln Asn Asn Tyr Pro
            180                 185                 190

Asp Arg Val Arg Ile Gly Ala Gly Arg Gln Tyr Trp Arg Ser Asp Glu
        195                 200                 205

Asp Glu Pro Asn Asn Arg Glu Ser Ser Tyr His Ile Ala Ser Ala Tyr
    210                 215                 220

Ser Trp Leu Val Gly Gly Asn Thr Phe Ala Gln Asn Gly Ser Gly Gly
225                 230                 235                 240

Gly Thr Val Asn Leu Gly Ser Glu Lys Ile Lys His Ser Pro Tyr Gly
                245                 250                 255

Phe Leu Pro Thr Gly Gly Ser Phe Gly Asp Ser Gly Ser Pro Met Phe
            260                 265                 270

Ile Tyr Asp Ala Gln Lys Gln Lys Trp Leu Ile Asn Gly Val Leu Gln
        275                 280                 285

Thr Gly Asn Pro Tyr Ile Gly Lys Ser Asn Gly Phe Gln Leu Val Arg
    290                 295                 300

Lys Asp Trp Phe Tyr Asp Glu Ile Phe Ala Gly Asp Thr His Ser Val
305                 310                 315                 320

Phe Tyr Glu Pro Arg Gln Asn Gly Lys Tyr Ser Phe Asn Asp Asp Asn
                325                 330                 335

Asn Gly Thr Gly Lys Ile Asn Ala Lys His Glu His Asn Ser Leu Pro
            340                 345                 350

Asn Arg Leu Lys Thr Arg Thr Val Gln Leu Phe Asn Val Ser Leu Ser
        355                 360                 365

Glu Thr Ala Arg Glu Pro Val Tyr His Ala Ala Gly Gly Val Asn Ser
    370                 375                 380

Tyr Arg Pro Arg Leu Asn Asn Gly Glu Asn Ile Ser Phe Ile Asp Glu

385                 390                 395                 400

Gly Lys Gly Glu Leu Ile Leu Thr Ser Asn Ile Asn Gln Gly Ala Gly
                405                 410                 415

Gly Leu Tyr Phe Gln Gly Asp Phe Thr Val Ser Pro Glu Asn Asn Glu
            420                 425                 430

Thr Trp Gln Gly Ala Gly Val His Ile Ser Glu Asp Ser Thr Val Thr
        435                 440                 445

Trp Lys Val Asn Gly Val Ala Asn Asp Arg Leu Ser Lys Ile Gly Lys
    450                 455                 460

Gly Thr Leu His Val Gln Ala Lys Gly Glu Asn Gln Gly Ser Ile Ser
465                 470                 475                 480

Val Gly Asp Gly Thr Val Ile Leu Asp Gln Gln Ala Asp Asp Lys Gly
                485                 490                 495

Lys Lys Gln Ala Phe Ser Glu Ile Gly Leu Val Ser Gly Arg Gly Thr
            500                 505                 510

Val Gln Leu Asn Ala Asp Asn Gln Phe Asn Pro Asp Lys Leu Tyr Phe
        515                 520                 525

Gly Phe Arg Gly Gly Arg Leu Asp Leu Asn Gly His Ser Leu Ser Phe
    530                 535                 540

His Arg Ile Gln Asn Thr Asp Glu Gly Ala Met Ile Val Asn His Asn
545                 550                 555                 560

Gln Asp Lys Glu Ser Thr Val Thr Ile Thr Gly Asn Lys Asp Ile Ala
                565                 570                 575

Thr Thr Gly Asn Asn Asn Ser Leu Asp Ser Lys Lys Glu Ile Ala Tyr
            580                 585                 590

Asn Gly Trp Phe Gly Glu Lys Asp Thr Thr Lys Thr Asn Gly Arg Leu
        595                 600                 605

Asn Leu Val Tyr Gln Pro Ala Ala Glu Asp Arg Thr Leu Leu Leu Ser
    610                 615                 620

Gly Gly Thr Asn Leu Asn Gly Asn Ile Thr Gln Thr Asn Gly Lys Leu
625                 630                 635                 640

Phe Phe Ser Gly Arg Pro Thr Pro His Ala Tyr Asn His Leu Asn Asp
                645                 650                 655

His Trp Ser Gln Lys Glu Gly Ile Pro Arg Gly Glu Ile Val Trp Asp
            660                 665                 670

Asn Asp Trp Ile Asn Arg Thr Phe Lys Ala Glu Asn Phe Gln Ile Lys
        675                 680                 685

Gly Gly Gln Ala Val Val Ser Arg Asn Val Ala Lys Val Lys Gly Asp
    690                 695                 700

Trp His Leu Ser Asn His Ala Gln Ala Val Phe Gly Val Ala Pro His
705                 710                 715                 720

Gln Ser His Thr Ile Cys Thr Arg Ser Asp Trp Thr Gly Leu Thr Asn

725                 730                 735

Cys Val Glu Lys Thr Ile Thr Asp Asp Lys Val Ile Ala Ser Leu Thr
            740                 745                 750

Lys Thr Asp Ile Ser Gly Asn Val Asp Leu Ala Asp His Ala His Leu
        755                 760                 765

Asn Leu Thr Gly Leu Ala Thr Leu Asn Gly Asn Leu Ser Ala Asn Gly
    770                 775                 780

Asp Thr Arg Tyr Thr Val Ser His Asn Ala Thr Gln Asn Gly Asn Leu
785                 790                 795                 800

Ser Leu Val Gly Asn Ala Gln Ala Thr Phe Asn Gln Ala Thr Leu Asn
                805                 810                 815

Gly Asn Thr Ser Ala Ser Gly Asn Ala Ser Phe Asn Leu Ser Asp His
            820                 825                 830

Ala Val Gln Asn Gly Ser Leu Thr Leu Ser Gly Asn Ala Lys Ala Asn
        835                 840                 845

Val Ser His Ser Ala Leu Asn Gly Asn Val Ser Leu Ala Asp Lys Ala
    850                 855                 860

Val Phe His Phe Glu Ser Ser Arg Phe Thr Gly Gln Ile Ser Gly Gly
865                 870                 875                 880

Lys Asp Thr Ala Leu His Leu Lys Asp Ser Glu Trp Thr Leu Pro Ser
                885                 890                 895

Gly Thr Glu Leu Gly Asn Leu Asn Leu Asp Asn Ala Thr Ile Thr Leu
            900                 905                 910

Asn Ser Ala Tyr Arg His Asp Ala Ala Gly Ala Gln Thr Gly Ser Ala
        915                 920                 925

Thr Asp Ala Pro Arg Arg Arg Ser Arg Arg Ser Arg Arg Ser Leu Leu
    930                 935                 940

Ser Val Thr Pro Pro Thr Ser Val Glu Ser Arg Phe Asn Thr Leu Thr
945                 950                 955                 960

Val Asn Gly Lys Leu Asn Gly Gln Gly Thr Phe Arg Phe Met Ser Glu
                965                 970                 975

Leu Phe Gly Tyr Arg Ser Asp Lys Leu Lys Leu Ala Glu Ser Ser Glu
            980                 985                 990

Gly Thr Tyr Thr Leu Ala Val Asn Asn Thr Gly Asn Glu Pro Ala Ser
        995                 1000                1005

Leu Glu Gln Leu Thr Val Val Glu Gly Lys Asp Asn Lys Pro Leu Ser
    1010                1015                1020

Glu Asn Leu Asn Phe Thr Leu Gln Asn Glu His Val Asp Ala Gly Ala
1025                1030                1035                1040

Trp Arg Tyr Gln Leu Ile Arg Lys Asp Gly Glu Phe Arg Leu His Asn
                1045                1050                1055

Pro Val Lys Glu Gln Glu Leu Ser Asp Lys Leu Gly Lys Ala Glu Ala

1060                1065                1070

Lys Lys Gln Ala Glu Lys Asp Asn Ala Gln Ser Leu Asp Ala Leu Ile
        1075                1080                1085

Ala Ala Gly Arg Asp Ala Val Glu Lys Thr Glu Ser Val Ala Glu Pro
    1090                1095                1100

Ala Arg Gln Ala Gly Gly Glu Asn Val Gly Ile Met Gln Ala Glu Glu
1105                1110                1115                1120

Glu Lys Lys Arg Val Gln Ala Asp Lys Asp Thr Ala Leu Ala Lys Gln
                1125                1130                1135

Arg Glu Ala Glu Thr Arg Pro Ala Thr Thr Ala Phe Pro Arg Ala Arg
            1140                1145                1150

Arg Ala Arg Arg Asp Leu Pro Gln Leu Gln Pro Gln Pro Gln Pro Gln
        1155                1160                1165

Pro Gln Arg Asp Leu Ile Ser Arg Tyr Ala Asn Ser Gly Leu Ser Glu
    1170                1175                1180

Phe Ser Ala Thr Leu Asn Ser Val Phe Ala Val Gln Asp Glu Leu Asp
1185                1190                1195                1200

Arg Val Phe Ala Glu Asp Arg Arg Asn Ala Val Trp Thr Ser Gly Ile
                1205                1210                1215

Arg Asp Thr Lys His Tyr Arg Ser Gln Asp Phe Arg Ala Tyr Arg Gln
            1220                1225                1230

Gln Thr Asp Leu Arg Gln Ile Gly Met Gln Lys Asn Leu Gly Ser Gly
        1235                1240                1245

Arg Val Gly Ile Leu Phe Ser His Asn Arg Thr Glu Asn Thr Phe Asp
    1250                1255                1260

Asp Gly Ile Gly Asn Ser Ala Arg Leu Ala His Gly Ala Val Phe Gly
1265                1270                1275                1280

Gln Tyr Gly Ile Asp Arg Phe Tyr Ile Gly Ile Ser Ala Gly Ala Gly
                1285                1290                1295

Phe Ser Ser Gly Ser Leu Ser Asp Gly Ile Gly Gly Lys Ile Arg Arg
            1300                1305                1310

Arg Val Leu His Tyr Gly Ile Gln Ala Arg Tyr Arg Ala Gly Phe Gly
        1315                1320                1325

Gly Phe Gly Ile Glu Pro His Ile Gly Ala Thr Arg Tyr Phe Val Gln
    1330                1335                1340

Lys Ala Asp Tyr Arg Tyr Glu Asn Val Asn Ile Ala Thr Pro Gly Leu
1345                1350                1355                1360

Ala Phe Asn Arg Tyr Arg Ala Gly Ile Lys Ala Asp Tyr Ser Phe Lys
                1365                1370                1375

Pro Ala Gln His Ile Ser Ile Thr Pro Tyr Leu Ser Leu Ser Tyr Thr
            1380                1385                1390

Asp Ala Ala Ser Gly Lys Val Arg Thr Arg Val Asn Thr Ala Val Leu

1395                1400                1405

Ala Gln Asp Phe Gly Lys Thr Arg Ser Ala Glu Trp Gly Val Asn Ala
    1410                1415                1420

Glu Ile Lys Gly Phe Thr Leu Ser Leu His Ala Ala Ala Ala Lys Gly
1425                1430                1435                1440

Pro Gln Leu Glu Ala Gln His Ser Ala Gly Ile Lys Leu Gly Tyr Arg
                1445                1450                1455

Trp


<210>   14
<211>   797
<212>   PRT
<213>   Neisseria meningitidis

<400>   14
Met Lys Leu Lys Gln Ile Ala Ser Ala Leu Met Met Leu Gly Ile Ser
1               5                   10                  15

Pro Leu Ala Leu Ala Asp Phe Thr Ile Gln Asp Ile Arg Val Glu Gly
            20                  25                  30

Leu Gln Arg Thr Glu Pro Ser Thr Val Phe Asn Tyr Leu Pro Val Lys
        35                  40                  45

Val Gly Asp Thr Tyr Asn Asp Thr His Gly Ser Ala Ile Ile Lys Ser
    50                  55                  60

Leu Tyr Ala Thr Gly Phe Phe Asp Asp Val Arg Val Glu Thr Ala Asp
65                  70                  75                  80

Gly Gln Leu Leu Leu Thr Val Ile Glu Arg Pro Thr Ile Gly Ser Leu
                85                  90                  95

Asn Ile Thr Gly Ala Lys Met Leu Gln Asn Asp Ala Ile Lys Lys Asn
            100                 105                 110

Leu Glu Ser Phe Gly Leu Ala Gln Ser Gln Tyr Phe Asn Gln Ala Thr
        115                 120                 125

Leu Asn Gln Ala Val Ala Gly Leu Lys Glu Glu Tyr Leu Gly Arg Gly
    130                 135                 140

Lys Leu Asn Ile Gln Ile Thr Pro Lys Val Thr Lys Leu Ala Arg Asn
145                 150                 155                 160

Arg Val Asp Ile Asp Ile Thr Ile Asp Glu Gly Lys Ser Ala Lys Ile
                165                 170                 175

Thr Asp Ile Glu Phe Glu Gly Asn Gln Val Tyr Ser Asp Arg Lys Leu
            180                 185                 190

Met Arg Gln Met Ser Leu Thr Glu Gly Gly Ile Trp Thr Trp Leu Thr
        195                 200                 205

Arg Ser Asn Gln Phe Asn Glu Gln Lys Phe Ala Gln Asp Met Glu Lys
    210                 215                 220

Val Thr Asp Phe Tyr Gln Asn Asn Gly Tyr Phe Asp Phe Arg Ile Leu

225                 230                 235                 240

Asp Thr Asp Ile Gln Thr Asn Glu Asp Lys Thr Lys Gln Thr Ile Lys
                245                 250                 255

Ile Thr Val His Glu Gly Gly Arg Phe Arg Trp Gly Lys Val Ser Ile
            260                 265                 270

Glu Gly Asp Thr Asn Glu Val Pro Lys Ala Glu Leu Glu Lys Leu Leu
        275                 280                 285

Thr Met Lys Pro Gly Lys Trp Tyr Glu Arg Gln Gln Met Thr Ala Val
    290                 295                 300

Leu Gly Glu Ile Gln Asn Arg Met Gly Ser Ala Gly Tyr Ala Tyr Ser
305                 310                 315                 320

Glu Ile Ser Val Gln Pro Leu Pro Asn Ala Glu Thr Lys Thr Val Asp
                325                 330                 335

Phe Val Leu His Ile Glu Pro Gly Arg Lys Ile Tyr Val Asn Glu Ile
            340                 345                 350

His Ile Thr Gly Asn Asn Lys Thr Arg Asp Glu Val Val Arg Arg Glu
        355                 360                 365

Leu Arg Gln Met Glu Ser Ala Pro Tyr Asp Thr Ser Lys Leu Gln Arg
    370                 375                 380

Ser Lys Glu Arg Val Glu Leu Leu Gly Tyr Phe Asp Asn Val Gln Phe
385                 390                 395                 400

Asp Ala Val Pro Leu Ala Gly Thr Pro Asp Lys Val Asp Leu Asn Met
                405                 410                 415

Ser Leu Thr Glu Arg Ser Thr Gly Ser Leu Asp Leu Ser Ala Gly Trp
            420                 425                 430

Val Gln Asp Thr Gly Leu Val Met Ser Ala Gly Val Ser Gln Asp Asn
        435                 440                 445

Leu Phe Gly Thr Gly Lys Ser Ala Ala Leu Arg Ala Ser Arg Ser Lys
    450                 455                 460

Thr Thr Leu Asn Gly Ser Leu Ser Phe Thr Asp Pro Tyr Phe Thr Ala
465                 470                 475                 480

Asp Gly Val Ser Leu Gly Tyr Asp Val Tyr Gly Lys Ala Phe Asp Pro
                485                 490                 495

Arg Lys Ala Ser Thr Ser Ile Lys Gln Tyr Lys Thr Thr Thr Ala Gly
            500                 505                 510

Ala Gly Ile Arg Met Ser Val Pro Val Thr Glu Tyr Asp Arg Val Asn
        515                 520                 525

Phe Gly Leu Val Ala Glu His Leu Thr Val Asn Thr Tyr Asn Lys Ala
    530                 535                 540

Pro Lys His Tyr Ala Asp Phe Ile Lys Lys Tyr Gly Lys Thr Asp Gly
545                 550                 555                 560

Thr Asp Gly Ser Phe Lys Gly Trp Leu Tyr Lys Gly Thr Val Gly Trp

                        565                         570                         575

Gly Arg Asn Lys Thr Asp Ser Ala Leu Trp Pro Thr Arg Gly Tyr Leu
                580                 585                 590

Thr Gly Val Asn Ala Glu Ile Ala Leu Pro Gly Ser Lys Leu Gln Tyr
            595                 600                 605

Tyr Ser Ala Thr His Asn Gln Thr Trp Phe Phe Pro Leu Ser Lys Thr
    610                 615                 620

Phe Thr Leu Met Leu Gly Gly Glu Val Gly Ile Ala Gly Gly Tyr Gly
625                 630                 635                 640

Arg Thr Lys Glu Ile Pro Phe Phe Glu Asn Phe Tyr Gly Gly Gly Leu
                645                 650                 655

Gly Ser Val Arg Gly Tyr Glu Ser Gly Thr Leu Gly Pro Lys Val Tyr
                660                 665                 670

Asp Glu Tyr Gly Glu Lys Ile Ser Tyr Gly Gly Asn Lys Lys Ala Asn
            675                 680                 685

Val Ser Ala Glu Leu Leu Phe Pro Met Pro Gly Ala Lys Asp Ala Arg
    690                 695                 700

Thr Val Arg Leu Ser Leu Phe Ala Asp Ala Gly Ser Val Trp Asp Gly
705                 710                 715                 720

Lys Thr Tyr Asp Asp Asn Ser Ser Ser Ala Thr Gly Gly Arg Val Gln
                725                 730                 735

Asn Ile Tyr Gly Ala Gly Asn Thr His Lys Ser Thr Phe Thr Asn Glu
                740                 745                 750

Leu Arg Tyr Ser Ala Gly Gly Ala Val Thr Trp Leu Ser Pro Leu Gly
            755                 760                 765

Pro Met Lys Phe Ser Tyr Ala Tyr Pro Leu Lys Lys Lys Pro Glu Asp
    770                 775                 780

Glu Ile Gln Arg Phe Gln Phe Gln Leu Gly Thr Thr Phe
785                 790                 795

<210>   15
<211>   6
<212>   PRT
<213>   Artificial Sequence

<220>
<223>   Linker

<400>   15
Gly Ser Gly Gly Gly Gly
1               5

<210>   16
<211>   8
<212>   PRT
<213>   Artificial Sequence

<220>
<223>   Linker

<400>   16
Gly Ser Gly Ser Gly Gly Gly Gly
1               5

<210>   17
<211>   6
<212>   PRT
<213>   Artificial Sequence

<220>
<223>   Hexa-histidine tag

<400>   17
His His His His His His
1               5

<210>   18
<211>   1312
<212>   PRT
<213>   Streptococcus pneumoniae

<400>   18
Met Lys His Glu Lys Gln Gln Arg Phe Ser Ile Arg Lys Tyr Ala Val
1               5                   10                  15

Gly Ala Ala Ser Val Leu Ile Gly Phe Ala Phe Gln Ala Gln Thr Val
            20                  25                  30

Ala Ala Asp Gly Val Thr Pro Thr Thr Thr Glu Asn Gln Pro Thr Ile
        35                  40                  45

His Thr Val Ser Asp Ser Pro Gln Ser Ser Glu Asn Arg Thr Glu Glu
    50                  55                  60

Thr Pro Lys Ala Glu Leu Gln Pro Glu Ala Pro Lys Thr Val Glu Thr
65                  70                  75                  80

Glu Thr Pro Ala Thr Asp Lys Val Ala Ser Leu Pro Lys Thr Glu Glu
                85                  90                  95

Lys Pro Gln Glu Glu Val Ser Ser Thr Pro Ser Asp Lys Ala Glu Val
            100                 105                 110

Val Thr Pro Thr Ser Ala Glu Lys Glu Thr Ala Asn Lys Lys Glu Glu
        115                 120                 125

Glu Ala Ser Pro Lys Lys Glu Glu Ala Lys Glu Val Asp Ser Lys Glu
    130                 135                 140

Ser Asn Thr Asp Lys Thr Asp Lys Asp Lys Pro Ala Lys Lys Asp Glu
145                 150                 155                 160

Ala Lys Ala Glu Ala Asp Lys Pro Glu Thr Glu Thr Gly Lys Glu Arg
                165                 170                 175

Ala Ala Thr Val Asn Glu Lys Leu Ala Lys Lys Lys Ile Val Ser Ile
            180                 185                 190

Asp Ala Gly Arg Lys Tyr Phe Ser Pro Glu Gln Leu Lys Glu Ile Ile
        195                 200                 205

Asp Lys Ala Lys His Tyr Gly Tyr Thr Asp Leu His Leu Leu Val Gly

210                 215                 220

Asn Asp Gly Leu Arg Phe Met Leu Asp Asp Met Ser Ile Thr Ala Asn
225                 230                 235                 240

Gly Lys Thr Tyr Ala Ser Asp Asp Val Lys Arg Ala Ile Glu Lys Gly
                245                 250                 255

Thr Asn Asp Tyr Tyr Asn Asp Pro Asn Gly Asn His Leu Thr Glu Ser
            260                 265                 270

Gln Met Thr Asp Leu Ile Asn Tyr Ala Lys Asp Lys Gly Ile Gly Leu
        275                 280                 285

Ile Pro Thr Val Asn Ser Pro Gly His Met Asp Ala Ile Leu Asn Ala
    290                 295                 300

Met Lys Glu Leu Gly Ile Gln Asn Pro Asn Phe Ser Tyr Phe Gly Lys
305                 310                 315                 320

Lys Ser Ala Arg Thr Val Asp Leu Asp Asn Glu Gln Ala Val Ala Phe
                325                 330                 335

Thr Lys Ala Leu Ile Asp Lys Tyr Ala Ala Tyr Phe Ala Lys Lys Thr
            340                 345                 350

Glu Ile Phe Asn Ile Gly Leu Asp Glu Tyr Ala Asn Asp Ala Thr Asp
        355                 360                 365

Ala Lys Gly Trp Ser Val Leu Gln Ala Asp Lys Tyr Tyr Pro Asn Glu
    370                 375                 380

Gly Tyr Pro Val Lys Gly Tyr Glu Lys Phe Ile Ala Tyr Ala Asn Asp
385                 390                 395                 400

Leu Ala Arg Ile Val Lys Ser His Gly Leu Lys Pro Met Ala Phe Asn
                405                 410                 415

Asp Gly Ile Tyr Tyr Asn Ser Asp Thr Ser Phe Gly Ser Phe Asp Lys
            420                 425                 430

Asp Ile Ile Val Ser Met Trp Thr Gly Gly Trp Gly Gly Tyr Asp Val
        435                 440                 445

Ala Ser Ser Lys Leu Leu Ala Glu Lys Gly His Gln Ile Leu Asn Thr
    450                 455                 460

Asn Asp Ala Trp Cys Tyr Val Leu Gly Arg Asn Ala Asp Gly Gln Gly
465                 470                 475                 480

Trp Tyr Asn Leu Asp Gln Gly Leu Asn Gly Ile Lys Asn Thr Pro Ile
                485                 490                 495

Thr Ser Val Pro Lys Thr Glu Gly Ala Asp Ile Pro Ile Ile Gly Gly
            500                 505                 510

Met Val Ala Ala Trp Ala Asp Thr Pro Ser Ala Arg Tyr Ser Pro Ser
        515                 520                 525

His Leu Phe Lys Leu Met Arg His Phe Ala Asn Ala Asn Ala Glu Tyr
    530                 535                 540

Phe Ala Ala Asp Tyr Glu Ser Ala Glu Gln Ala Leu Asn Glu Val Pro

545                 550                 555                 560

Lys Asp Leu Asn Arg Tyr Thr Ala Glu Ser Val Ala Ala Val Lys Glu
                565                 570                 575

Ala Glu Lys Ala Ile Arg Ser Leu Asp Ser Asn Leu Ser Arg Ala Gln
            580                 585                 590

Gln Asp Thr Ile Asp Gln Ala Ile Ala Lys Leu Gln Glu Thr Val Asn
        595                 600                 605

Asn Leu Thr Leu Thr Pro Glu Ala Gln Lys Glu Glu Glu Ala Lys Arg
    610                 615                 620

Glu Val Glu Lys Leu Ala Lys Asn Lys Val Ile Ser Ile Asp Ala Gly
625                 630                 635                 640

Arg Lys Tyr Phe Thr Leu Asp Gln Leu Lys Arg Ile Val Asp Lys Ala
                645                 650                 655

Ser Glu Leu Gly Tyr Ser Asp Val His Leu Leu Leu Gly Asn Asp Gly
            660                 665                 670

Leu Arg Phe Leu Leu Asn Asp Met Thr Ile Thr Ala Asn Gly Lys Thr
        675                 680                 685

Tyr Ala Ser Asp Asp Val Lys Lys Ala Ile Ile Glu Gly Thr Lys Ala
    690                 695                 700

Tyr Tyr Asp Asp Pro Asn Gly Thr Ala Leu Thr Gln Ala Glu Val Thr
705                 710                 715                 720

Glu Leu Ile Glu Tyr Ala Lys Ser Lys Asp Ile Gly Leu Ile Pro Ala
                725                 730                 735

Ile Asn Ser Pro Gly His Met Asp Ala Met Leu Val Ala Met Glu Lys
            740                 745                 750

Leu Gly Ile Lys Asn Pro Gln Ala His Phe Asp Lys Val Ser Lys Thr
        755                 760                 765

Thr Met Asp Leu Lys Asn Glu Glu Ala Met Asn Phe Val Lys Ala Leu
    770                 775                 780

Ile Gly Lys Tyr Met Asp Phe Phe Ala Gly Lys Thr Lys Ile Phe Asn
785                 790                 795                 800

Phe Gly Thr Asp Glu Tyr Ala Asn Asp Ala Thr Ser Ala Gln Gly Trp
                805                 810                 815

Tyr Tyr Leu Lys Trp Tyr Gln Leu Tyr Gly Lys Phe Ala Glu Tyr Ala
            820                 825                 830

Asn Thr Leu Ala Ala Met Ala Lys Glu Arg Gly Leu Gln Pro Met Ala
        835                 840                 845

Phe Asn Asp Gly Phe Tyr Tyr Glu Asp Lys Asp Asp Val Gln Phe Asp
    850                 855                 860

Lys Asp Val Leu Ile Ser Tyr Trp Ser Lys Gly Trp Trp Gly Tyr Asn
865                 870                 875                 880

Leu Ala Ser Pro Gln Tyr Leu Ala Ser Lys Gly Tyr Lys Phe Leu Asn

885                 890                 895

Thr Asn Gly Asp Trp Tyr Tyr Val Ile Gly Asn His Lys Gln Asp Glu
            900                 905                 910

Ala Tyr Pro Leu Ser Lys Ala Val Glu Asn Ser Gly Lys Val Pro Phe
        915                 920                 925

Asn Gln Leu Ala Ser Thr Lys Tyr Pro Glu Val Asp Leu Pro Thr Val
    930                 935                 940

Gly Ser Met Leu Ser Ile Trp Ala Asp Arg Pro Ser Ala Glu Tyr Lys
945                 950                 955                 960

Glu Glu Glu Ile Phe Glu Leu Met Thr Ala Phe Ala Asp His Asn Lys
                965                 970                 975

Asp Tyr Phe Arg Ala Asn Tyr Asn Ala Leu Arg Glu Glu Leu Ala Lys
            980                 985                 990

Ile Pro Thr Asn Leu Glu Gly Tyr Ser Lys Glu Ser Leu Glu Ala Leu
        995                 1000                1005

Asp Ala Ala Lys Thr Ala Leu Asn Tyr Asn Leu Asn Arg Asn Lys Gln
    1010                1015                1020

Ala Glu Leu Asp Thr Leu Val Ala Asn Leu Lys Ala Ala Leu Gln Gly
1025                1030                1035                1040

Leu Lys Pro Ala Ala Thr His Ser Gly Ser Leu Asp Glu Asn Glu Val
                1045                1050                1055

Ala Ala Asn Val Glu Thr Arg Pro Glu Leu Ile Thr Arg Thr Glu Glu
            1060                1065                1070

Ile Pro Phe Glu Val Ile Lys Lys Glu Asn Pro Asn Leu Pro Ala Gly
        1075                1080                1085

Gln Glu Asn Ile Ile Thr Ala Gly Val Lys Gly Glu Arg Thr His Tyr
    1090                1095                1100

Ile Ser Val Leu Thr Glu Asn Gly Lys Thr Thr Glu Thr Val Leu Asp
1105                1110                1115                1120

Ser Gln Val Thr Lys Glu Val Ile Asn Gln Val Val Glu Val Gly Ser
                1125                1130                1135

Pro Val Thr His Lys Gly Asp Glu Ser Gly Leu Ala Pro Thr Thr Glu
            1140                1145                1150

Val Lys Pro Arg Leu Asp Ile Gln Glu Glu Glu Ile Pro Phe Thr Thr
        1155                1160                1165

Val Thr Arg Glu Asn Pro Leu Leu Leu Lys Gly Lys Thr Gln Val Ile
    1170                1175                1180

Thr Lys Gly Val Asn Gly His Arg Ser Asn Phe Tyr Ser Val Ser Thr
1185                1190                1195                1200

Ser Ala Asp Gly Lys Glu Val Lys Thr Leu Val Asn Ser Val Val Ala
                1205                1210                1215

Gln Glu Ala Val Thr Gln Ile Val Glu Val Gly Thr Met Val Thr His

1220                    1225                    1230

Val Gly Asp Glu Asn Gly Gln Ala Ala Ile Ala Glu Glu Lys Pro Lys
        1235                    1240                    1245

Leu Glu Ile Pro Ser Gln Pro Ala Pro Ser Thr Ala Pro Ala Glu Glu
    1250                    1255                    1260

Ser Lys Ala Leu Pro Gln Asp Pro Ala Pro Val Val Thr Glu Lys Lys
1265                    1270                    1275                    1280

Leu Pro Glu Thr Gly Thr His Asp Ser Ala Glu Leu Val Val Ala Gly
                1285                    1290                    1295

Leu Met Ser Thr Leu Ala Ala Tyr Gly Leu Thr Lys Arg Lys Glu Asp
            1300                    1305                    1310

<210>   19
<211>   1078
<212>   PRT
<213>   Streptococcus pneumoniae

<400>   19
Met Ile Leu Gln Tyr Val Tyr Trp Ser Val Tyr Met Gln Thr Lys Thr
1               5                   10                  15

Lys Lys Leu Ile Val Ser Leu Ser Ser Leu Val Leu Ser Gly Phe Leu
            20                  25                  30

Leu Asn His Tyr Met Thr Val Gly Ala Glu Glu Thr Thr Thr Asn Thr
        35                  40                  45

Ile Gln Gln Ser Gln Lys Glu Val Gln Tyr Gln Gln Arg Asp Thr Lys
    50                  55                  60

Asn Leu Val Glu Asn Gly Asp Phe Gly Gln Thr Glu Asp Gly Ser Ser
65                  70                  75                  80

Pro Trp Thr Gly Ser Lys Ala Gln Gly Trp Ser Ala Trp Val Asp Gln
                85                  90                  95

Lys Asn Ser Ser Ala Asp Ala Ser Thr Arg Val Ile Glu Ala Lys Asp
            100                 105                 110

Gly Ala Ile Thr Ile Ser Ser Pro Glu Lys Leu Arg Ala Ala Val His
        115                 120                 125

Arg Met Val Pro Ile Glu Ala Lys Lys Lys Tyr Lys Leu Arg Phe Lys
    130                 135                 140

Ile Lys Thr Asp Asn Lys Val Gly Ile Ala Lys Val Arg Ile Ile Glu
145                 150                 155                 160

Glu Ser Gly Lys Asp Lys Arg Leu Trp Asn Ser Ala Thr Thr Ser Gly
                165                 170                 175

Thr Lys Asp Trp Gln Thr Ile Glu Ala Asp Tyr Ser Pro Thr Leu Asp
            180                 185                 190

Val Asp Lys Ile Lys Leu Glu Leu Phe Tyr Glu Thr Gly Thr Gly Thr
        195                 200                 205

Val Ser Phe Lys Asp Ile Glu Leu Val Glu Val Ala Asp Gln Pro Ser

210                 215                 220

Glu Asp Ser Gln Thr Asp Lys Gln Leu Glu Glu Lys Ile Asp Leu Pro
225                 230                 235                 240

Ile Gly Lys Lys His Val Phe Ser Leu Ala Asp Tyr Thr Tyr Lys Val
                245                 250                 255

Glu Asn Pro Asp Val Ala Ser Val Lys Asn Gly Ile Leu Glu Pro Leu
            260                 265                 270

Lys Glu Gly Thr Thr Asn Val Ile Val Ser Lys Asp Gly Lys Glu Val
        275                 280                 285

Lys Lys Ile Pro Leu Lys Ile Leu Ala Ser Val Lys Asp Thr Tyr Thr
    290                 295                 300

Asp Arg Leu Asp Asp Trp Asn Gly Ile Ile Ala Gly Asn Gln Tyr Tyr
305                 310                 315                 320

Asp Ser Lys Asn Glu Gln Met Ala Lys Leu Asn Gln Glu Leu Glu Gly
                325                 330                 335

Lys Val Ala Asp Ser Leu Ser Ser Ile Ser Ser Gln Ala Asp Arg Ile
            340                 345                 350

Tyr Leu Trp Glu Lys Phe Ser Asn Tyr Lys Thr Ser Ala Asn Leu Thr
        355                 360                 365

Ala Thr Tyr Arg Lys Leu Glu Glu Met Ala Lys Gln Val Thr Asn Pro
    370                 375                 380

Ser Ser Arg Tyr Tyr Gln Asp Glu Thr Val Val Arg Thr Val Arg Asp
385                 390                 395                 400

Ser Met Glu Trp Met His Lys His Val Tyr Asn Ser Glu Lys Ser Ile
                405                 410                 415

Val Gly Asn Trp Trp Asp Tyr Glu Ile Gly Thr Pro Arg Ala Ile Asn
            420                 425                 430

Asn Thr Leu Ser Leu Met Lys Glu Tyr Phe Ser Asp Glu Glu Ile Lys
        435                 440                 445

Lys Tyr Thr Asp Val Ile Glu Lys Phe Val Pro Asp Pro Glu His Phe
    450                 455                 460

Arg Lys Thr Thr Asp Asn Pro Phe Lys Ala Leu Gly Gly Asn Leu Val
465                 470                 475                 480

Asp Met Gly Arg Val Lys Val Ile Ala Gly Leu Leu Arg Lys Asp Asp
                485                 490                 495

Gln Glu Ile Ser Ser Thr Ile Arg Ser Ile Glu Gln Val Phe Lys Leu
            500                 505                 510

Val Asp Gln Gly Glu Gly Phe Tyr Gln Asp Gly Ser Tyr Ile Asp His
        515                 520                 525

Thr Asn Val Ala Tyr Thr Gly Ala Tyr Gly Asn Val Leu Ile Asp Gly
    530                 535                 540

Leu Ser Gln Leu Leu Pro Val Ile Gln Lys Thr Lys Asn Pro Ile Asp

545                 550                 555                 560

Lys Asp Lys Met Gln Thr Met Tyr His Trp Ile Asp Lys Ser Phe Ala
                565                 570                 575

Pro Leu Leu Val Asn Gly Glu Leu Met Asp Met Ser Arg Gly Arg Ser
            580                 585                 590

Ile Ser Arg Ala Asn Ser Glu Gly His Val Ala Ala Val Glu Val Leu
        595                 600                 605

Arg Gly Ile His Arg Ile Ala Asp Met Ser Glu Gly Glu Thr Lys Gln
    610                 615                 620

Arg Leu Gln Ser Leu Val Lys Thr Ile Val Gln Ser Asp Ser Tyr Tyr
625                 630                 635                 640

Asp Val Phe Lys Asn Leu Lys Thr Tyr Lys Asp Ile Ser Leu Met Gln
                645                 650                 655

Ser Leu Leu Ser Asp Ala Gly Val Ala Ser Val Pro Arg Thr Ser Tyr
            660                 665                 670

Leu Ser Ala Phe Asn Lys Met Asp Lys Thr Ala Met Tyr Asn Ala Glu
        675                 680                 685

Lys Gly Phe Gly Phe Gly Leu Ser Leu Phe Ser Ser Arg Thr Leu Asn
    690                 695                 700

Tyr Glu His Met Asn Lys Glu Asn Lys Arg Gly Trp Tyr Thr Ser Asp
705                 710                 715                 720

Gly Met Phe Tyr Leu Tyr Asn Gly Asp Leu Ser His Tyr Ser Asp Gly
                725                 730                 735

Tyr Trp Pro Thr Val Asn Pro Tyr Lys Met Pro Gly Thr Thr Glu Thr
            740                 745                 750

Asp Ala Lys Arg Ala Asp Ser Asp Thr Gly Lys Val Leu Pro Ser Ala
        755                 760                 765

Phe Val Gly Thr Ser Lys Leu Asp Asp Ala Asn Ala Thr Ala Thr Met
    770                 775                 780

Asp Phe Thr Asn Trp Asn Gln Thr Leu Thr Ala His Lys Ser Trp Phe
785                 790                 795                 800

Met Leu Lys Asp Lys Ile Ala Phe Leu Gly Ser Asn Ile Gln Asn Thr
                805                 810                 815

Ser Thr Asp Thr Ala Ala Thr Thr Ile Asp Gln Arg Lys Leu Glu Ser
            820                 825                 830

Ser Asn Pro Tyr Lys Val Tyr Val Asn Asp Lys Glu Ala Ser Leu Thr
        835                 840                 845

Glu Gln Glu Lys Asp Tyr Pro Glu Thr Gln Ser Val Phe Leu Glu Ser
    850                 855                 860

Ser Asp Ser Lys Lys Asn Ile Gly Tyr Phe Phe Phe Lys Lys Ser Ser
865                 870                 875                 880

Ile Ser Met Ser Lys Ala Leu Gln Lys Gly Ala Trp Lys Asp Ile Asn

885                 890                 895

Glu Gly Gln Ser Asp Lys Glu Val Glu Asn Glu Phe Leu Thr Ile Ser
            900                 905                 910

Gln Ala His Lys Gln Asn Gly Asp Ser Tyr Gly Tyr Met Leu Ile Pro
        915                 920                 925

Asn Val Asp Arg Ala Thr Phe Asn Gln Met Ile Lys Glu Leu Glu Ser
    930                 935                 940

Ser Leu Ile Glu Asn Asn Glu Thr Leu Gln Ser Val Tyr Asp Ala Lys
945                 950                 955                 960

Gln Gly Val Trp Gly Ile Val Lys Tyr Asp Asp Ser Val Ser Thr Ile
                965                 970                 975

Ser Asn Gln Phe Gln Val Leu Lys Arg Gly Val Tyr Thr Ile Arg Lys
            980                 985                 990

Glu Gly Asp Glu Tyr Lys Ile Ala Tyr Tyr Asn Pro Glu Thr Gln Glu
        995                 1000                1005

Ser Ala Pro Asp Gln Glu Val Phe Lys Lys Leu Glu Gln Ala Ala Gln
    1010                1015                1020

Pro Gln Val Gln Asn Ser Lys Glu Lys Glu Lys Ser Glu Glu Glu Lys
1025                1030                1035                1040

Asn His Ser Asp Gln Lys Asn Leu Pro Gln Thr Gly Glu Gly Gln Ser
                1045                1050                1055

Ile Leu Ala Ser Leu Gly Phe Leu Leu Leu Gly Ala Phe Tyr Leu Phe
            1060                1065                1070

Arg Arg Gly Lys Asn Asn
        1075

<210>   20
<211>   2228
<212>   PRT
<213>   Streptococcus pneumoniae

<400>   20
Met Gly Lys Gly His Trp Asn Arg Lys Arg Val Tyr Ser Ile Arg Lys
1               5                   10                  15

Phe Ala Val Gly Ala Cys Ser Val Met Ile Gly Thr Cys Ala Val Leu
            20                  25                  30

Leu Gly Gly Asn Ile Ala Gly Glu Ser Val Val Tyr Ala Asp Glu Thr
        35                  40                  45

Leu Ile Thr His Thr Ala Glu Lys Pro Lys Glu Glu Lys Met Ile Val
    50                  55                  60

Glu Glu Lys Ala Asp Lys Ala Leu Glu Thr Lys Asn Val Val Glu Arg
65                  70                  75                  80

Thr Glu Gln Ser Glu Pro Ser Ser Thr Glu Ala Ile Ala Ser Glu Lys
                85                  90                  95

Lys Glu Asp Glu Ala Val Thr Pro Lys Glu Glu Lys Val Ser Ala Lys

            100                 105                 110

Pro Glu Glu Lys Ala Pro Arg Ile Glu Ser Gln Ala Ser Ser Gln Glu
        115                 120                 125

Lys Pro Leu Lys Glu Asp Ala Lys Ala Val Thr Asn Glu Glu Val Asn
    130                 135                 140

Gln Met Ile Glu Asn Arg Lys Val Asp Phe Asn Gln Asn Trp Tyr Phe
145                 150                 155                 160

Lys Leu Asn Ala Asn Ser Lys Glu Ala Ile Lys Pro Asp Ala Asp Val
                165                 170                 175

Ser Thr Trp Lys Lys Leu Asp Leu Pro Tyr Asp Trp Ser Ile Phe Asn
            180                 185                 190

Asp Phe Asp His Glu Ser Pro Ala Gln Asn Glu Gly Gly Gln Leu Asn
        195                 200                 205

Gly Gly Glu Ala Trp Tyr Arg Lys Thr Phe Lys Leu Asp Glu Lys Asp
    210                 215                 220

Leu Lys Lys Asn Val Arg Leu Thr Phe Asp Gly Val Tyr Met Asp Ser
225                 230                 235                 240

Gln Val Tyr Val Asn Gly Gln Leu Val Gly His Tyr Pro Asn Gly Tyr
                245                 250                 255

Asn Gln Phe Ser Tyr Asp Ile Thr Lys Tyr Leu Tyr Lys Asp Gly Arg
            260                 265                 270

Glu Asn Val Ile Ala Val His Ala Val Asn Lys Gln Pro Ser Ser Arg
        275                 280                 285

Trp Tyr Ser Gly Ser Gly Ile Tyr Arg Asp Val Thr Leu Gln Val Thr
    290                 295                 300

Asp Lys Val His Val Glu Lys Asn Gly Thr Thr Ile Leu Thr Pro Lys
305                 310                 315                 320

Leu Glu Glu Gln Gln His Gly Lys Val Glu Thr His Val Thr Ser Lys
                325                 330                 335

Ile Val Asn Thr Asp Asp Lys Asp His Glu Leu Val Ala Glu Tyr Gln
            340                 345                 350

Ile Val Glu Arg Gly Gly His Ala Val Thr Gly Leu Val Arg Thr Ala
        355                 360                 365

Ser Arg Thr Leu Lys Ala His Glu Ser Thr Ser Leu Asp Ala Ile Leu
    370                 375                 380

Glu Val Glu Arg Pro Lys Leu Trp Thr Val Leu Asn Asp Lys Pro Ala
385                 390                 395                 400

Leu Tyr Glu Leu Ile Thr Arg Val Tyr Arg Asp Gly Gln Leu Val Asp
                405                 410                 415

Ala Lys Lys Asp Leu Phe Gly Tyr Arg Tyr Tyr His Trp Thr Pro Asn
            420                 425                 430

Glu Gly Phe Ser Leu Asn Gly Glu Arg Ile Lys Phe His Gly Val Ser

435                 440                 445

Leu His His Asp His Gly Ala Leu Gly Ala Glu Glu Asn Tyr Lys Ala
    450                 455                 460

Glu Tyr Arg Arg Leu Lys Gln Met Lys Glu Met Gly Val Asn Ser Ile
465                 470                 475                 480

Arg Thr Thr His Asn Pro Ala Ser Glu Gln Thr Leu Gln Ile Ala Ala
                485                 490                 495

Glu Leu Gly Leu Leu Val Gln Glu Glu Ala Phe Asp Thr Trp Tyr Gly
            500                 505                 510

Gly Lys Lys Pro Tyr Asp Tyr Gly Arg Phe Phe Glu Lys Asp Ala Thr
        515                 520                 525

His Pro Glu Ala Arg Lys Gly Glu Lys Trp Ser Asp Phe Asp Leu Arg
    530                 535                 540

Thr Met Val Glu Arg Gly Lys Asn Asn Pro Ala Ile Phe Met Trp Ser
545                 550                 555                 560

Ile Gly Asn Glu Ile Gly Glu Ala Asn Gly Asp Ala His Ser Leu Ala
                565                 570                 575

Thr Val Lys Arg Leu Val Lys Val Ile Lys Asp Val Asp Lys Thr Arg
            580                 585                 590

Tyr Val Thr Met Gly Ala Asp Lys Phe Arg Phe Gly Asn Gly Ser Gly
        595                 600                 605

Gly His Glu Lys Ile Ala Asp Glu Leu Asp Ala Val Gly Phe Asn Tyr
    610                 615                 620

Ser Glu Asp Asn Tyr Lys Ala Leu Arg Ala Lys His Pro Lys Trp Leu
625                 630                 635                 640

Ile Tyr Gly Ser Glu Thr Ser Ser Ala Thr Arg Thr Arg Gly Ser Tyr
                645                 650                 655

Tyr Arg Pro Glu Arg Glu Leu Lys His Ser Asn Gly Pro Glu Arg Asn
            660                 665                 670

Tyr Glu Gln Ser Asp Tyr Gly Asn Asp Arg Val Gly Trp Gly Lys Thr
        675                 680                 685

Ala Thr Ala Ser Trp Thr Phe Asp Arg Asp Asn Ala Gly Tyr Ala Gly
    690                 695                 700

Gln Phe Ile Trp Thr Gly Thr Asp Tyr Ile Gly Glu Pro Thr Pro Trp
705                 710                 715                 720

His Asn Gln Asn Gln Thr Pro Val Lys Ser Ser Tyr Phe Gly Ile Val
                725                 730                 735

Asp Thr Ala Gly Ile Pro Lys His Asp Phe Tyr Leu Tyr Gln Ser Gln
            740                 745                 750

Trp Val Ser Val Lys Lys Lys Pro Met Val His Leu Leu Pro His Trp
        755                 760                 765

Asn Trp Glu Asn Lys Glu Leu Ala Ser Lys Val Ala Asp Ser Glu Gly

770                 775                 780

Lys Ile Pro Val Arg Ala Tyr Ser Asn Ala Ser Ser Val Glu Leu Phe
785                 790                 795                 800

Leu Asn Gly Lys Ser Leu Gly Leu Lys Thr Phe Asn Lys Lys Gln Thr
                805                 810                 815

Ser Asp Gly Arg Thr Tyr Gln Glu Gly Ala Asn Ala Asn Glu Leu Tyr
            820                 825                 830

Leu Glu Trp Lys Val Ala Tyr Gln Pro Gly Thr Leu Glu Ala Ile Ala
        835                 840                 845

Arg Asp Glu Ser Gly Lys Glu Ile Ala Arg Asp Lys Ile Thr Thr Ala
    850                 855                 860

Gly Lys Pro Ala Ala Val Arg Leu Ile Lys Glu Asp His Ala Ile Ala
865                 870                 875                 880

Ala Asp Gly Lys Asp Leu Thr Tyr Ile Tyr Tyr Glu Ile Val Asp Ser
                885                 890                 895

Gln Gly Asn Val Val Pro Thr Ala Asn Asn Leu Val Arg Phe Gln Leu
            900                 905                 910

His Gly Gln Gly Gln Leu Val Gly Val Asp Asn Gly Glu Gln Ala Ser
        915                 920                 925

Arg Glu Arg Tyr Lys Ala Gln Ala Asp Gly Ser Trp Ile Arg Lys Ala
    930                 935                 940

Phe Asn Gly Lys Gly Val Ala Ile Val Lys Ser Thr Glu Gln Ala Gly
945                 950                 955                 960

Lys Phe Thr Leu Thr Ala His Ser Asp Leu Leu Lys Ser Asn Gln Val
                965                 970                 975

Thr Val Phe Thr Gly Lys Lys Glu Gly Gln Glu Lys Thr Val Leu Gly
            980                 985                 990

Thr Glu Val Pro Lys Val Gln Thr Ile Ile Gly Glu Ala Pro Glu Met
        995                 1000                1005

Pro Thr Thr Val Pro Phe Val Tyr Ser Asp Gly Ser Arg Ala Glu Arg
    1010                1015                1020

Pro Val Thr Trp Ser Leu Val Asp Val Ser Lys Pro Gly Ile Val Thr
1025                1030                1035                1040

Val Lys Gly Met Ala Asp Gly Arg Glu Val Glu Ala Arg Val Glu Val
                1045                1050                1055

Ile Ala Leu Lys Ser Glu Leu Pro Val Val Lys Arg Ile Ala Pro Asn
            1060                1065                1070

Thr Asn Leu Asn Ser Val Asp Lys Ser Val Ser Tyr Val Leu Thr Asp
        1075                1080                1085

Gly Ser Val Gln Glu Tyr Glu Val Asp Lys Trp Glu Ile Ala Glu Glu
    1090                1095                1100

Asp Lys Ala Lys Leu Ala Ile Pro Gly Ser Arg Ile Gln Ala Thr Gly

1105                1110                1115                1120

Tyr Leu Glu Gly Gln Pro Ile His Ala Thr Leu Val Val Glu Glu Gly
                1125                1130                1135

Asn Pro Ala Ala Pro Val Val Pro Thr Val Thr Val Gly Gly Glu Ala
            1140                1145                1150

Val Thr Gly Leu Thr Ser Arg Gln Pro Met Gln Tyr Arg Thr Leu Ser
        1155                1160                1165

Tyr Gly Ala Gln Leu Pro Glu Val Thr Ala Ser Ala Glu Asn Ala Asp
    1170                1175                1180

Val Thr Val Leu Gln Ala Ser Ala Ala Asn Gly Met Arg Ala Ser Ile
1185                1190                1195                1200

Phe Ile Gln Pro Lys Asp Gly Gly Pro Leu Gln Thr Tyr Ala Ile Gln
                1205                1210                1215

Phe Leu Glu Glu Ala Pro Lys Ile Ala His Leu Ser Leu Gln Val Glu
            1220                1225                1230

Lys Ala Asp Ser Leu Lys Glu Asp Gln Thr Val Lys Leu Ser Val Arg
        1235                1240                1245

Ala His Tyr Gln Asp Gly Thr Gln Ala Val Leu Pro Ala Asp Lys Val
    1250                1255                1260

Thr Phe Ser Thr Ser Gly Glu Gly Glu Val Ala Ile Arg Lys Gly Met
1265                1270                1275                1280

Leu Glu Leu His Lys Pro Gly Ala Val Thr Leu Asn Ala Glu Tyr Glu
                1285                1290                1295

Gly Ala Lys Gly Gln Val Glu Leu Thr Ile Gln Ala Asn Thr Glu Lys
            1300                1305                1310

Lys Ile Ala Gln Ser Ile Arg Pro Val Asn Val Val Thr Asp Leu His
        1315                1320                1325

Gln Glu Pro Ser Leu Pro Ala Thr Val Thr Val Glu Tyr Asp Lys Gly
    1330                1335                1340

Phe Pro Lys Thr His Lys Val Thr Trp Gln Ala Ile Pro Lys Glu Lys
1345                1350                1355                1360

Leu Asp Ser Tyr Gln Ile Phe Glu Val Leu Gly Lys Val Glu Gly Ile
                1365                1370                1375

Asp Leu Glu Ala Arg Ala Lys Val Ser Val Glu Gly Ile Val Ser Val
            1380                1385                1390

Glu Glu Val Ser Val Thr Thr Pro Ile Ala Glu Ala Pro Gln Leu Pro
        1395                1400                1405

Glu Ser Val Arg Thr Tyr Asp Ser Asn Gly His Val Ser Ser Ala Lys
    1410                1415                1420

Val Ala Trp Asp Ala Ile Arg Pro Glu Gln Tyr Ala Lys Glu Gly Val
1425                1430                1435                1440

Phe Thr Val Asn Gly Arg Leu Glu Gly Thr Gln Leu Thr Thr Lys Leu

1445                1450                1455

His Val Arg Val Ser Ala Gln Thr Glu Gln Gly Ala Asn Ile Ser Asp
        1460                1465                1470

Gln Trp Thr Gly Ser Glu Leu Pro Leu Ala Phe Ala Ser Asp Ser Asn
    1475                1480                1485

Pro Ser Asp Pro Val Ser Asn Val Asn Asp Lys Leu Ile Ser Tyr Asn
    1490                1495                1500

Asn Gln Pro Ala Asn Arg Trp Thr Asn Trp Asn Arg Ser Asn Pro Glu
1505                1510                1515                1520

Ala Ser Val Gly Val Leu Phe Gly Asp Ser Gly Ile Leu Ser Lys Arg
                1525                1530                1535

Ser Val Asp Asn Leu Ser Val Gly Phe His Glu Asp His Gly Val Gly
        1540                1545                1550

Ala Pro Lys Ser Tyr Val Ile Glu Tyr Tyr Val Gly Lys Thr Val Pro
    1555                1560                1565

Thr Ala Pro Lys Asn Pro Ser Phe Val Gly Asn Glu Asp His Val Phe
    1570                1575                1580

Asn Asp Ser Ala Asn Trp Lys Pro Val Thr Asn Leu Lys Ala Pro Ala
1585                1590                1595                1600

Gln Leu Lys Ala Gly Glu Met Asn His Phe Ser Phe Asp Lys Val Glu
                1605                1610                1615

Thr Tyr Ala Ile Arg Ile Arg Met Val Lys Ala Asp Asn Lys Arg Gly
        1620                1625                1630

Thr Ser Ile Thr Glu Val Gln Ile Phe Ala Lys Gln Val Ala Ala Ala
    1635                1640                1645

Lys Gln Gly Gln Thr Arg Ile Gln Val Asp Gly Lys Asp Leu Ala Asn
    1650                1655                1660

Phe Asn Pro Asp Leu Thr Asp Tyr Tyr Leu Glu Ser Val Asp Gly Lys
1665                1670                1675                1680

Val Pro Ala Val Thr Ala Asn Val Ser Asn Asn Gly Leu Ala Thr Val
                1685                1690                1695

Val Pro Ser Val Arg Glu Gly Glu Pro Val Arg Val Ile Ala Lys Ala
        1700                1705                1710

Glu Asn Gly Asp Ile Leu Gly Glu Tyr Arg Leu His Phe Thr Lys Asp
    1715                1720                1725

Lys Asn Leu Leu Ser His Lys Pro Val Ala Ala Val Lys Gln Ala Arg
    1730                1735                1740

Leu Leu Gln Val Gly Gln Ala Leu Glu Leu Pro Thr Lys Val Pro Val
1745                1750                1755                1760

Tyr Phe Thr Gly Lys Asp Gly Tyr Glu Thr Lys Asp Leu Thr Val Glu
                1765                1770                1775

Trp Glu Glu Val Pro Ala Glu Asn Leu Thr Lys Ala Gly Gln Phe Thr

1780                1785                1790

Val Arg Gly Arg Val Leu Gly Ser Asn Leu Val Ala Glu Val Thr Val
        1795                1800                1805

Arg Val Thr Asp Lys Leu Gly Glu Thr Leu Ser Asp Asn Pro Asn Tyr
    1810                1815                1820

Asp Glu Asn Ser Asn Gln Ala Phe Ala Ser Ala Thr Asn Asp Ile Asp
1825                1830                1835                1840

Lys Asn Ser His Asp Arg Val Asp Tyr Leu Asn Asp Gly Asp His Ser
                1845                1850                1855

Glu Asn Arg Arg Trp Thr Asn Trp Ser Pro Thr Pro Ser Ser Asn Pro
            1860                1865                1870

Glu Val Ser Ala Gly Val Ile Phe Arg Glu Asn Gly Lys Ile Val Glu
        1875                1880                1885

Arg Thr Val Ala Gln Ala Lys Leu His Phe Phe Ala Asp Ser Gly Thr
    1890                1895                1900

Asp Ala Pro Ser Lys Leu Val Leu Glu Arg Tyr Val Gly Pro Gly Phe
1905                1910                1915                1920

Glu Val Pro Thr Tyr Tyr Ser Asn Tyr Gln Ala Tyr Glu Ser Gly His
                1925                1930                1935

Pro Phe Asn Asn Pro Glu Asn Trp Glu Ala Val Pro Tyr Arg Ala Asp
            1940                1945                1950

Lys Asp Ile Ala Ala Gly Asp Glu Ile Asn Val Thr Phe Lys Ala Val
        1955                1960                1965

Lys Ala Lys Val Met Arg Trp Arg Met Glu Arg Lys Ala Asp Lys Ser
    1970                1975                1980

Gly Val Ala Met Ile Glu Met Thr Phe Leu Ala Pro Ser Glu Leu Pro
1985                1990                1995                2000

Gln Glu Ser Thr Gln Ser Lys Ile Leu Val Asp Gly Lys Glu Leu Ala
                2005                2010                2015

Asp Phe Ala Glu Asn Arg Gln Asp Tyr Gln Ile Thr Tyr Lys Gly Gln
            2020                2025                2030

Arg Pro Lys Val Ser Val Glu Glu Asn Asn Gln Val Ala Ser Thr Val
        2035                2040                2045

Val Asp Ser Gly Glu Asp Ser Leu Pro Val Leu Val Arg Leu Val Ser
    2050                2055                2060

Glu Ser Gly Lys Gln Val Lys Glu Tyr Arg Ile Gln Leu Thr Lys Glu
2065                2070                2075                2080

Lys Pro Val Ser Ala Val Gln Glu Asp Leu Pro Lys Leu Glu Phe Val
                2085                2090                2095

Glu Lys Asp Leu Ala Tyr Lys Thr Val Glu Lys Lys Asp Ser Thr Leu
            2100                2105                2110

Tyr Leu Gly Glu Thr Arg Val Glu Gln Glu Gly Lys Val Gly Lys Glu

2115                2120                2125

Arg Ile Phe Thr Val Ile Asn Pro Asp Gly Ser Lys Glu Glu Lys Leu
    2130                2135                2140

Arg Glu Val Val Glu Val Pro Thr Asp Arg Ile Val Leu Val Gly Thr
2145                2150                2155                2160

Lys Pro Val Ala Gln Glu Ala Lys Lys Pro Gln Val Ser Glu Lys Ala
                2165                2170                2175

Asp Thr Lys Pro Ile Asp Ser Ser Glu Ala Asp Gln Thr Asn Lys Ala
            2180                2185                2190

Gln Leu Pro Asn Thr Gly Ser Ala Ala Ser Gln Ala Ala Val Ala Ala
        2195                2200                2205

Gly Leu Ala Leu Leu Gly Leu Ser Ala Gly Leu Val Val Thr Lys Gly
    2210                2215                2220

Lys Lys Glu Asp
2225

<210>   21
<211>   247
<212>   PRT
<213>   Streptococcus pneumoniae

<400>   21
Met Ser Gln Lys Asn Asn Lys Lys Lys Asn Lys Arg Lys Asn Leu Leu
1               5                   10                  15

Thr Asn Ile Leu Ala Gly Phe Leu Ile Leu Leu Ser Leu Ala Leu Ile
            20                  25                  30

Phe Asn Thr Gln Ile Arg Asn Ile Phe Ile Val Trp Asn Thr Asn Lys
        35                  40                  45

Tyr Gln Val Ser Gln Val Ser Lys Glu Lys Leu Glu Glu Asn Gln Asp
    50                  55                  60

Thr Glu Gly Asn Phe Asp Phe Asp Ser Val Lys Ala Ile Ser Ser Glu
65                  70                  75                  80

Ala Val Leu Thr Ser Gln Trp Asp Ala Gln Lys Leu Pro Val Ile Gly
                85                  90                  95

Gly Ile Ala Ile Pro Glu Leu Glu Met Asn Leu Pro Ile Phe Lys Gly
            100                 105                 110

Leu Asp Asn Val Asn Leu Phe Tyr Gly Ala Gly Thr Met Lys Arg Glu
        115                 120                 125

Gln Val Met Gly Glu Gly Asn Tyr Ser Leu Ala Ser His His Ile Phe
    130                 135                 140

Gly Val Asp Asn Ala Asn Lys Met Leu Phe Ser Pro Leu Asp Asn Ala
145                 150                 155                 160

Lys Asn Gly Met Lys Ile Tyr Leu Thr Asp Lys Asn Lys Val Tyr Thr
                165                 170                 175

Tyr Glu Ile Arg Glu Val Lys Arg Val Thr Pro Asp Arg Val Asp Glu

180                 185                 190

Val Asp Asp Arg Asp Gly Val Asn Glu Ile Thr Leu Val Thr Cys Glu
        195                 200                 205

Asp Leu Ala Ala Thr Glu Arg Ile Ile Val Lys Gly Asp Leu Lys Glu
    210                 215                 220

Thr Lys Asp Tyr Ser Gln Thr Ser Asp Glu Ile Leu Thr Ala Phe Asn
225                 230                 235                 240

Gln Pro Tyr Lys Gln Phe Tyr
                245

<210>   22
<211>   202
<212>   PRT
<213>   Streptococcus pneumoniae

<400>   22
Met Val Pro Lys Thr Ala Thr Ser Thr Glu Thr Lys Thr Ile Thr Arg
1               5                   10                  15

Ile Ile His Tyr Val Asp Lys Val Thr Asn Gln Asn Val Lys Glu Asp
            20                  25                  30

Val Val Gln Pro Val Thr Leu Ser Arg Thr Lys Thr Glu Asn Lys Val
        35                  40                  45

Thr Gly Val Val Thr Tyr Gly Glu Trp Thr Thr Gly Asn Trp Asp Glu
    50                  55                  60

Val Ile Ser Gly Lys Ile Asp Lys Tyr Lys Asp Pro Asp Ile Pro Thr
65                  70                  75                  80

Val Glu Ser Gln Glu Val Thr Ser Asp Ser Ser Asp Lys Glu Ile Thr
                85                  90                  95

Val Arg Tyr Asp Arg Leu Ser Thr Pro Glu Lys Pro Ile Pro Gln Pro
            100                 105                 110

Asn Pro Glu His Pro Ser Val Pro Thr Pro Asn Pro Glu Leu Pro Asn
        115                 120                 125

Gln Glu Thr Pro Thr Pro Asp Lys Pro Thr Pro Glu Pro Gly Thr Pro
    130                 135                 140

Lys Thr Glu Thr Pro Val Asn Pro Asp Pro Glu Val Pro Thr Tyr Glu
145                 150                 155                 160

Thr Gly Lys Arg Glu Glu Leu Pro Asn Thr Gly Thr Glu Ala Asn Ala
                165                 170                 175

Thr Leu Ala Ser Ala Gly Ile Met Thr Leu Leu Ala Gly Leu Gly Leu
            180                 185                 190

Gly Phe Phe Lys Lys Lys Glu Asp Glu Lys
        195                 200

<210>   23
<211>   128
<212>   PRT
<213>   Streptococcus pneumoniae

<400>   23
Met Glu Lys Asp Met Asn Leu Lys Arg Glu Gln Glu Phe Val Ser Gln
1               5                   10                  15

Tyr His Phe Asp Ala Arg Asn Phe Glu Trp Glu Asn Glu Asn Gly Ala
            20                  25                  30

Pro Glu Thr Lys Val Asp Val Asn Phe Gln Leu Leu Gln His Asp Gln
        35                  40                  45

Glu Asn Gln Val Thr Ser Leu Ile Val Ile Leu Ser Phe Met Ile Val
    50                  55                  60

Phe Asp Lys Phe Val Ile Ser Gly Thr Ile Ser Gln Val Asn His Ile
65                  70                  75                  80

Asp Gly Arg Ile Val Asn Glu Pro Asn Glu Leu Asn Gln Glu Glu Val
                85                  90                  95

Glu Thr Leu Ala Arg Pro Cys Leu Asn Met Leu Asn Arg Leu Thr Tyr
            100                 105                 110

Glu Val Thr Glu Ile Ala Leu Asp Leu Pro Gly Ile Asn Leu Glu Phe
        115                 120                 125

<210>   24
<211>   259
<212>   PRT
<213>   Streptococcus pneumoniae

<400>   24
Met Lys Lys Asn Ser Leu Tyr Ile Ile Ser Ser Leu Phe Phe Ala Cys
1               5                   10                  15

Val Leu Phe Val Tyr Ala Thr Ala Thr Asn Phe Gln Asn Ser Thr Ser
            20                  25                  30

Ala Arg Gln Val Lys Thr Glu Thr Tyr Thr Asn Thr Val Thr Asn Val
        35                  40                  45

Pro Ile Asp Ile Arg Tyr Asn Ser Asp Lys Tyr Phe Ile Ser Gly Phe
    50                  55                  60

Ala Ser Glu Val Ser Val Val Leu Thr Gly Ala Asn Arg Leu Ser Leu
65                  70                  75                  80

Ala Ser Glu Met Gln Glu Ser Thr Arg Lys Phe Lys Val Thr Ala Asp
                85                  90                  95

Leu Thr Asp Ala Gly Val Gly Thr Ile Glu Val Pro Leu Ser Ile Glu
            100                 105                 110

Asp Leu Pro Asn Gly Leu Thr Ala Val Ala Thr Pro Gln Lys Ile Thr
        115                 120                 125

Val Lys Ile Gly Lys Lys Ala Gln Lys Asp Lys Val Lys Ile Val Pro
    130                 135                 140

Glu Ile Asp Pro Ser Gln Ile Asp Ser Arg Val Gln Ile Glu Asn Val
145                 150                 155                 160

Met Val Ser Asp Lys Glu Val Ser Ile Thr Ser Asp Gln Glu Thr Leu

165                 170                 175

Asp Arg Ile Asp Lys Ile Ile Ala Val Leu Pro Thr Ser Glu Arg Ile
            180                 185                 190

Thr Gly Asn Tyr Ser Gly Ser Val Pro Leu Gln Ala Ile Asp Arg Asn
        195                 200                 205

Gly Val Val Leu Pro Ala Val Ile Thr Pro Phe Asp Thr Ile Met Lys
    210                 215                 220

Val Thr Thr Lys Pro Val Ala Pro Ser Ser Ser Thr Ser Asn Ser Ser
225                 230                 235                 240

Thr Ser Ser Ser Ser Glu Thr Ser Ser Ser Thr Lys Ala Thr Ser Ser
                245                 250                 255

Lys Thr Asn

<210>   25
<211>   721
<212>   PRT
<213>   Streptococcus pneumoniae

<400>   25
Met Asn Leu Gly Glu Phe Trp Tyr Asn Lys Ile Asn Lys Asn Arg Gly
1               5                   10                  15

Arg Arg Leu Met Lys Lys Val Arg Phe Ile Phe Leu Ala Leu Leu Phe
            20                  25                  30

Phe Leu Ala Ser Pro Glu Gly Ala Met Ala Ser Asp Gly Thr Trp Gln
        35                  40                  45

Gly Lys Gln Tyr Leu Lys Glu Asp Gly Ser Gln Ala Ala Asn Glu Trp
    50                  55                  60

Val Phe Asp Thr His Tyr Gln Ser Trp Phe Tyr Ile Lys Ala Asp Ala
65                  70                  75                  80

Asn Tyr Ala Glu Asn Glu Trp Leu Lys Gln Gly Asp Asp Tyr Phe Tyr
                85                  90                  95

Leu Lys Ser Gly Gly Tyr Met Ala Lys Ser Glu Trp Val Glu Asp Lys
            100                 105                 110

Gly Ala Phe Tyr Tyr Leu Asp Gln Asp Gly Lys Met Lys Arg Asn Ala
        115                 120                 125

Trp Val Gly Thr Ser Tyr Val Gly Ala Thr Gly Ala Lys Val Ile Glu
    130                 135                 140

Asp Trp Val Tyr Asp Ser Gln Tyr Asp Ala Trp Phe Tyr Ile Lys Ala
145                 150                 155                 160

Asp Gly Gln His Ala Glu Lys Glu Trp Leu Gln Ile Lys Gly Lys Asp
                165                 170                 175

Tyr Tyr Phe Lys Ser Gly Gly Tyr Leu Leu Thr Ser Gln Trp Ile Asn
            180                 185                 190

Gln Ala Tyr Val Asn Ala Ser Gly Ala Lys Val Gln Gln Gly Trp Leu

195                 200                 205

Phe Asp Lys Gln Tyr Gln Ser Trp Phe Tyr Ile Lys Glu Asn Gly Asn
    210                 215                 220

Tyr Ala Asp Lys Glu Trp Ile Phe Glu Asn Gly His Tyr Tyr Tyr Leu
225                 230                 235                 240

Lys Ser Gly Gly Tyr Met Ala Ala Asn Glu Trp Ile Trp Asp Lys Glu
                245                 250                 255

Ser Trp Phe Tyr Leu Lys Phe Asp Gly Lys Ile Ala Glu Lys Glu Trp
            260                 265                 270

Val Tyr Asp Ser His Ser Gln Ala Trp Tyr Tyr Phe Lys Ser Gly Gly
        275                 280                 285

Tyr Met Ala Ala Asn Glu Trp Ile Trp Asp Lys Glu Ser Trp Phe Tyr
    290                 295                 300

Leu Lys Phe Asp Gly Lys Met Ala Glu Lys Glu Trp Val Tyr Asp Ser
305                 310                 315                 320

His Ser Gln Ala Trp Tyr Tyr Phe Lys Ser Gly Gly Tyr Met Thr Ala
                325                 330                 335

Asn Glu Trp Ile Trp Asp Lys Glu Ser Trp Phe Tyr Leu Lys Ser Asp
            340                 345                 350

Gly Lys Ile Ala Glu Lys Glu Trp Val Tyr Asp Ser His Ser Gln Ala
        355                 360                 365

Trp Tyr Tyr Phe Lys Ser Gly Gly Tyr Met Thr Ala Asn Glu Trp Ile
    370                 375                 380

Trp Asp Lys Glu Ser Trp Phe Tyr Leu Lys Ser Asp Gly Lys Met Ala
385                 390                 395                 400

Glu Lys Glu Trp Val Tyr Asp Ser His Ser Gln Ala Trp Tyr Tyr Phe
                405                 410                 415

Lys Ser Gly Gly Tyr Met Ala Lys Asn Glu Thr Val Asp Gly Tyr Gln
            420                 425                 430

Leu Gly Ser Asp Gly Lys Trp Leu Gly Gly Lys Ala Thr Asn Lys Asn
        435                 440                 445

Ala Ala Tyr Tyr Gln Val Val Pro Val Thr Ala Asn Val Tyr Asp Ser
    450                 455                 460

Asp Gly Glu Lys Leu Ser Tyr Ile Ser Gln Gly Ser Val Val Trp Leu
465                 470                 475                 480

Asp Lys Asp Arg Lys Ser Asp Asp Lys Arg Leu Ala Ile Thr Ile Ser
                485                 490                 495

Gly Leu Ser Gly Tyr Met Lys Thr Glu Asp Leu Gln Ala Leu Asp Ala
            500                 505                 510

Ser Lys Asp Phe Ile Pro Tyr Tyr Glu Ser Asp Gly His Arg Phe Tyr
        515                 520                 525

His Tyr Val Ala Gln Asn Ala Ser Ile Pro Val Ala Ser His Leu Ser

530                 535                 540

Asp Met Glu Val Gly Lys Lys Tyr Tyr Ser Ala Asp Gly Leu His Phe
545                 550                 555                 560

Asp Gly Phe Lys Leu Glu Asn Pro Phe Leu Phe Lys Asp Leu Thr Glu
                565                 570                 575

Ala Thr Asn Tyr Ser Ala Glu Glu Leu Asp Lys Val Phe Ser Leu Leu
            580                 585                 590

Asn Ile Asn Asn Ser Leu Leu Glu Asn Lys Gly Ala Thr Phe Lys Glu
        595                 600                 605

Ala Glu Glu His Tyr His Ile Asn Ala Leu Tyr Leu Leu Ala His Ser
    610                 615                 620

Ala Leu Glu Ser Asn Trp Gly Arg Ser Lys Ile Ala Lys Asp Lys Asn
625                 630                 635                 640

Asn Phe Phe Gly Ile Thr Ala Tyr Asp Thr Thr Pro Tyr Leu Ser Ala
                645                 650                 655

Lys Thr Phe Asp Asp Val Asp Lys Gly Ile Leu Gly Ala Thr Lys Trp
            660                 665                 670

Ile Lys Glu Asn Tyr Ile Asp Arg Gly Arg Thr Phe Leu Gly Asn Lys
        675                 680                 685

Ala Ser Gly Met Asn Val Glu Tyr Ala Ser Asp Pro Tyr Trp Gly Glu
    690                 695                 700

Lys Ile Ala Ser Val Met Met Lys Ile Asn Glu Lys Leu Gly Gly Lys
705                 710                 715                 720

Asp


<210>  26
<211>  501
<212>  PRT
<213>  Streptococcus pneumoniae

<400>  26
Met Ser Val Thr Phe Phe Ile Gly Glu Glu Arg Leu Lys Ile Lys Ile
1               5                   10                  15

Gly Leu Ala Ser Ile Cys Leu Leu Gly Leu Ala Thr Ser His Val Ala
            20                  25                  30

Ala Asn Glu Thr Glu Val Ala Lys Thr Ser Gln Asp Thr Thr Thr Ala
        35                  40                  45

Ser Ser Ser Ser Glu Gln Asn Gln Ser Ser Asn Lys Thr Gln Thr Ser
    50                  55                  60

Ala Glu Val Gln Thr Asn Ala Ala Ala Tyr Trp Asp Gly Asp Tyr Tyr
65                  70                  75                  80

Val Lys Asp Asp Gly Ser Lys Ala Gln Ser Glu Trp Ile Phe Asp Asn
                85                  90                  95

Tyr Tyr Lys Ala Trp Phe Tyr Ile Asn Ser Asp Gly Arg Tyr Ser Gln

            100                 105                 110

Asn Glu Trp His Gly Asn Tyr Tyr Leu Lys Ser Gly Gly Tyr Met Ala
        115                 120                 125

Gln Asn Glu Trp Ile Tyr Asp Ser Asn Tyr Lys Ser Trp Phe Tyr Leu
    130                 135                 140

Lys Ser Asp Gly Ala Tyr Ala His Gln Glu Trp Gln Leu Ile Gly Asn
145                 150                 155                 160

Lys Trp Tyr Tyr Phe Lys Lys Trp Gly Tyr Met Ala Lys Ser Gln Trp
                165                 170                 175

Gln Gly Ser Tyr Phe Leu Asn Gly Gln Gly Ala Met Ile Gln Asn Glu
            180                 185                 190

Trp Leu Tyr Asp Pro Ala Tyr Ser Ala Tyr Phe Tyr Leu Lys Ser Asp
        195                 200                 205

Gly Thr Tyr Ala Asn Gln Glu Trp Gln Lys Val Gly Gly Lys Trp Tyr
    210                 215                 220

Tyr Phe Lys Lys Trp Gly Tyr Met Ala Arg Asn Glu Trp Gln Gly Asn
225                 230                 235                 240

Tyr Tyr Leu Thr Gly Ser Gly Ala Met Ala Thr Asp Glu Val Ile Met
                245                 250                 255

Asp Gly Ala Arg Tyr Ile Phe Ala Ala Ser Gly Glu Leu Lys Glu Lys
            260                 265                 270

Lys Asp Leu Asn Val Gly Trp Val His Arg Asp Gly Lys Arg Tyr Phe
        275                 280                 285

Phe Asn Asn Arg Glu Glu Gln Val Gly Thr Glu His Ala Lys Lys Ile
    290                 295                 300

Ile Asp Ile Ser Glu His Asn Gly Arg Ile Asn Asp Trp Lys Lys Val
305                 310                 315                 320

Ile Asp Glu Asn Lys Val Asp Gly Val Ile Val Arg Leu Gly Tyr Ser
                325                 330                 335

Gly Lys Glu Asp Lys Glu Leu Ala His Asn Ile Lys Glu Leu Asn Arg
            340                 345                 350

Leu Gly Ile Pro Tyr Gly Val Tyr Leu Tyr Thr Tyr Ala Glu Asn Glu
        355                 360                 365

Thr Asp Ala Glu Asn Asp Ala Lys Gln Thr Ile Glu Leu Ile Lys Lys
    370                 375                 380

Tyr Asn Met Asn Leu Ser Tyr Pro Ile Tyr Tyr Asp Val Glu Asn Trp
385                 390                 395                 400

Glu Tyr Val Asn Lys Ser Lys Arg Ala Pro Ser Asp Thr Asp Thr Trp
                405                 410                 415

Val Lys Ile Ile Asn Lys Tyr Met Asp Thr Met Lys Gln Ala Gly Tyr
            420                 425                 430

Gln Asn Val Tyr Val Tyr Ser Tyr Arg Ser Leu Leu Gln Thr Arg Leu

435                 440                 445

Lys His Pro Asp Ile Leu Lys His Val Asn Trp Val Ala Ala Tyr Thr
    450                 455                 460

Asn Ala Leu Glu Trp Glu Asn Pro Tyr Tyr Ser Gly Glu Lys Gly Trp
465                 470                 475                 480

Gln Tyr Thr Ser Ser Glu Tyr Met Lys Gly Ile Gln Gly Arg Val Asp
                485                 490                 495

Val Ser Val Trp Tyr
            500

<210>   27
<211>   471
<212>   PRT
<213>   Streptococcus pneumoniae

<400>   27
Met Ala Asn Lys Ala Val Asn Asp Phe Ile Leu Ala Met Asn Tyr Asp
1               5                   10                  15

Lys Lys Lys Leu Leu Thr His Gln Gly Glu Ser Ile Glu Asn Arg Phe
            20                  25                  30

Ile Lys Glu Gly Asn Gln Leu Pro Asp Glu Phe Val Val Ile Glu Arg
        35                  40                  45

Lys Lys Arg Ser Leu Ser Thr Asn Thr Ser Asp Ile Ser Val Thr Ala
    50                  55                  60

Thr Asn Asp Ser Arg Leu Tyr Pro Gly Ala Leu Leu Val Val Asp Glu
65                  70                  75                  80

Thr Leu Leu Glu Asn Asn Pro Thr Leu Leu Ala Val Asp Arg Ala Pro
                85                  90                  95

Met Thr Tyr Ser Ile Asp Leu Pro Gly Leu Ala Ser Ser Asp Ser Phe
            100                 105                 110

Leu Gln Val Glu Asp Pro Ser Asn Ser Ser Val Arg Gly Ala Val Asn
        115                 120                 125

Asp Leu Leu Ala Lys Trp His Gln Asp Tyr Gly Gln Val Asn Asn Val
    130                 135                 140

Pro Ala Arg Met Gln Tyr Glu Lys Ile Thr Ala His Ser Met Glu Gln
145                 150                 155                 160

Leu Lys Val Lys Phe Gly Ser Asp Phe Glu Lys Thr Gly Asn Ser Leu
                165                 170                 175

Asp Ile Asp Phe Asn Ser Val His Ser Gly Glu Lys Gln Ile Gln Ile
            180                 185                 190

Val Asn Phe Lys Gln Ile Tyr Tyr Thr Val Ser Val Asp Ala Val Lys
        195                 200                 205

Asn Pro Gly Asp Val Phe Gln Asp Thr Val Thr Val Glu Asp Leu Lys
    210                 215                 220

Gln Arg Gly Ile Ser Ala Glu Arg Pro Leu Val Tyr Ile Ser Ser Val

225                 230                 235                 240

Ala Tyr Gly Arg Gln Val Tyr Leu Lys Leu Glu Thr Thr Ser Lys Ser
                245                 250                 255

Asp Glu Val Glu Ala Ala Phe Glu Ala Leu Ile Lys Gly Val Lys Val
            260                 265                 270

Ala Pro Gln Thr Glu Trp Lys Gln Ile Leu Asp Asn Thr Glu Val Lys
        275                 280                 285

Ala Val Ile Leu Gly Gly Asp Pro Ser Ser Gly Ala Arg Val Val Thr
    290                 295                 300

Gly Lys Val Asp Met Val Glu Asp Leu Ile Gln Glu Gly Ser Arg Phe
305                 310                 315                 320

Thr Ala Asp His Pro Gly Leu Pro Ile Ser Tyr Thr Thr Ser Phe Leu
                325                 330                 335

Arg Asp Asn Val Val Ala Thr Phe Gln Asn Ser Thr Asp Tyr Val Glu
            340                 345                 350

Thr Lys Val Thr Ala Tyr Arg Asn Gly Asp Leu Leu Leu Asp His Ser
        355                 360                 365

Gly Ala Tyr Val Ala Gln Tyr Tyr Ile Thr Trp Asp Glu Leu Ser Tyr
    370                 375                 380

Asp His Gln Gly Lys Glu Val Leu Thr Pro Lys Ala Trp Asp Arg Asn
385                 390                 395                 400

Gly Gln Asp Leu Thr Ala His Phe Thr Thr Ser Ile Pro Leu Lys Gly
                405                 410                 415

Asn Val Arg Asn Leu Ser Val Lys Ile Arg Glu Cys Thr Gly Leu Ala
            420                 425                 430

Trp Glu Trp Trp Arg Thr Val Tyr Glu Lys Thr Asp Leu Pro Leu Val
        435                 440                 445

Arg Lys Arg Thr Ile Ser Ile Trp Gly Thr Thr Leu Tyr Pro Gln Val
    450                 455                 460

Glu Asp Lys Val Glu Asn Asp
465                 470

<210>   28
<211>   392
<212>   PRT
<213>   Streptococcus pneumoniae

<400>   28
Met Lys Lys Lys Ile Leu Ala Ser Leu Leu Leu Ser Thr Val Met Val
1               5                   10                  15

Ser Gln Val Ala Val Leu Thr Thr Ala His Ala Glu Thr Thr Asp Asp
            20                  25                  30

Lys Ile Ala Ala Gln Asp Asn Lys Ile Ser Asn Leu Thr Ala Gln Gln
        35                  40                  45

Gln Glu Ala Gln Lys Gln Val Asp Gln Ile Gln Glu Gln Val Ser Ala

50                  55                  60

Ile Gln Ala Glu Gln Ser Asn Leu Gln Ala Glu Asn Asp Arg Leu Gln
65                  70                  75                  80

Ala Glu Ser Lys Lys Leu Glu Gly Glu Ile Thr Glu Leu Ser Lys Asn
                85                  90                  95

Ile Val Ser Arg Asn Gln Ser Leu Glu Lys Gln Ala Arg Ser Ala Gln
            100                 105                 110

Thr Asn Gly Ala Val Thr Ser Tyr Ile Asn Thr Ile Val Asn Ser Lys
        115                 120                 125

Ser Ile Thr Glu Ala Ile Ser Arg Val Ala Ala Met Ser Glu Ile Val
    130                 135                 140

Ser Ala Asn Asn Lys Met Leu Glu Gln Gln Lys Ala Asp Lys Lys Ala
145                 150                 155                 160

Ile Ser Glu Lys Gln Val Ala Asn Asn Asp Ala Ile Asn Thr Val Ile
                165                 170                 175

Ala Asn Gln Gln Lys Leu Ala Asp Asp Ala Gln Ala Leu Thr Thr Lys
            180                 185                 190

Gln Ala Glu Leu Lys Ala Ala Glu Leu Ser Leu Ala Ala Glu Lys Ala
        195                 200                 205

Thr Ala Glu Gly Glu Lys Ala Ser Leu Leu Glu Gln Lys Ala Ala Ala
    210                 215                 220

Glu Ala Glu Ala Arg Ala Ala Ala Val Ala Glu Ala Ala Tyr Lys Glu
225                 230                 235                 240

Lys Arg Ala Ser Gln Gln Gln Ser Val Leu Ala Ser Ala Asn Thr Asn
                245                 250                 255

Leu Thr Ala Gln Val Gln Ala Val Ser Glu Ser Ala Ala Ala Pro Val
            260                 265                 270

Arg Ala Lys Val Arg Pro Thr Tyr Ser Thr Asn Ala Ser Ser Tyr Pro
        275                 280                 285

Ile Gly Glu Cys Thr Trp Gly Val Lys Thr Leu Ala Pro Trp Ala Gly
    290                 295                 300

Asp Tyr Trp Gly Asn Gly Ala Gln Trp Ala Thr Ser Ala Ala Ala Ala
305                 310                 315                 320

Gly Phe Arg Thr Gly Ser Thr Pro Gln Val Gly Ala Ile Ala Cys Trp
                325                 330                 335

Asn Asp Gly Gly Tyr Gly His Val Ala Val Val Thr Ala Val Glu Ser
            340                 345                 350

Thr Thr Arg Ile Gln Val Ser Glu Ser Asn Tyr Ala Gly Asn Arg Thr
        355                 360                 365

Ile Gly Asn His Arg Gly Trp Phe Asn Pro Thr Thr Thr Ser Glu Gly
    370                 375                 380

Phe Val Thr Tyr Ile Tyr Ala Asp

385                 390

<210>   29
<211>   167
<212>   PRT
<213>   Streptococcus pneumoniae

<400>   29
Met Lys Ser Ile Thr Lys Lys Ile Lys Ala Thr Leu Ala Gly Val Ala
1               5                   10                  15

Ala Leu Phe Ala Val Phe Ala Pro Ser Phe Val Ser Ala Gln Glu Ser
            20                  25                  30

Ser Thr Tyr Thr Val Lys Glu Gly Asp Thr Leu Ser Glu Ile Ala Glu
        35                  40                  45

Thr His Asn Thr Thr Val Glu Lys Leu Ala Glu Asn Asn His Ile Asp
    50                  55                  60

Asn Ile His Leu Ile Tyr Val Asp Gln Glu Leu Val Ile Asp Gly Pro
65                  70                  75                  80

Val Ala Pro Val Ala Thr Pro Ala Pro Ala Thr Tyr Ala Ala Pro Ala
                85                  90                  95

Ala Gln Asp Glu Thr Val Ser Ala Pro Val Ala Glu Thr Pro Val Val
            100                 105                 110

Ser Glu Thr Val Val Ser Thr Val Ser Gly Ser Glu Ala Glu Ala Lys
        115                 120                 125

Glu Trp Ile Ala Gln Lys Glu Ser Gly Gly Ser Tyr Thr Ala Thr Asn
    130                 135                 140

Gly Arg Tyr Ile Gly Arg Tyr Gly Ser Trp Thr Ala Ala Lys Asn Phe
145                 150                 155                 160

Trp Leu Asn Asn Gly Trp Tyr
                165

<210>   30
<211>   225
<212>   PRT
<213>   Streptococcus pneumoniae

<400>   30
Met Thr Tyr Leu Asp Ala Phe Lys Ser Gly Thr Leu Val Leu Pro Ser
1               5                   10                  15

Ala Leu Leu Leu His Phe Lys Glu Leu Phe Pro Ser Ser Asp Asp Phe
            20                  25                  30

Leu Val Trp Gln Phe Phe Tyr Leu Gln Asn Thr Thr Gly Leu Glu Glu
        35                  40                  45

Met Ser Pro Ser Gln Ile Ala Glu Arg Ile Gly Lys Glu Ile Ser Asp
    50                  55                  60

Val Asn Gln Ser Ile Ser Asn Leu Thr Glu Arg Gly Leu Leu Gln Tyr
65                  70                  75                  80

Arg Thr Ile Glu Leu Asn Gly Glu Ile Glu Leu Leu Phe Asp Ala Ser

85                  90                  95

Leu Ala Leu Glu Arg Leu Asp Asp Leu Phe Gly Ala Val His Ser Ser
            100                 105                 110

Ser Asp Gln Leu Thr Ser Gln Asn Gln Leu Lys Asp Leu Val Glu Thr
        115                 120                 125

Phe Gln Gln Glu Leu Gly Arg Leu Leu Thr Pro Phe Glu Ile Glu Asp
    130                 135                 140

Leu Thr Lys Thr Leu Lys Glu Asp Gly Thr Ser Ala Asp Leu Ile Lys
145                 150                 155                 160

Glu Ala Leu Arg Glu Ala Val Leu Asn Gly Lys Pro Asn Trp Lys Tyr
                165                 170                 175

Ile Gln Ala Ile Leu Arg Asn Trp Arg His Glu Gly Ile Lys Ser Val
            180                 185                 190

Ala Gln Ile Glu Ala Lys Arg Ala Glu Arg Glu Ala Ser Asn Pro Gln
        195                 200                 205

Leu Thr Gln Val Ser Ala Asp Phe Arg Asn Ala Met Asp Leu Trp Lys
    210                 215                 220

Asp
225

<210>   31
<211>   659
<212>   PRT
<213>   Streptococcus pneumoniae

<400>   31
Met Lys Lys Asn Arg Val Phe Ala Thr Ala Gly Leu Val Leu Leu Ala
1               5                   10                  15

Ala Gly Val Leu Ala Ala Cys Ser Ser Ser Lys Ser Ser Asp Ser Ser
            20                  25                  30

Ala Pro Lys Ala Tyr Gly Tyr Val Tyr Thr Ala Asp Pro Glu Thr Leu
        35                  40                  45

Asp Tyr Leu Ile Ser Arg Lys Asn Ser Thr Thr Val Val Thr Ser Asn
    50                  55                  60

Gly Ile Asp Gly Leu Phe Thr Asn Asp Asn Tyr Gly Asn Leu Ala Pro
65                  70                  75                  80

Ala Val Ala Glu Asp Trp Glu Val Ser Lys Asp Gly Leu Thr Tyr Thr
                85                  90                  95

Tyr Lys Ile Arg Lys Gly Val Lys Trp Phe Thr Ser Asp Gly Glu Glu
            100                 105                 110

Tyr Ala Glu Val Thr Ala Lys Asp Phe Val Asn Gly Leu Lys His Ala
        115                 120                 125

Ala Asp Lys Lys Ser Glu Ala Met Tyr Leu Ala Glu Asn Ser Val Lys
    130                 135                 140

Gly Leu Ala Asp Tyr Leu Ser Gly Thr Ser Thr Asp Phe Ser Thr Val

145                 150                 155                 160

Gly Val Lys Ala Val Asp Asp Tyr Thr Leu Gln Tyr Thr Leu Asn Gln
                165                 170                 175

Pro Glu Pro Phe Trp Asn Ser Lys Leu Thr Tyr Ser Ile Phe Trp Pro
            180                 185                 190

Leu Asn Glu Glu Phe Glu Thr Ser Lys Gly Ser Asp Phe Ala Lys Pro
        195                 200                 205

Thr Asp Pro Thr Ser Leu Leu Tyr Asn Gly Pro Phe Leu Leu Lys Gly
    210                 215                 220

Leu Thr Ala Lys Ser Ser Val Glu Phe Val Lys Asn Glu Gln Tyr Trp
225                 230                 235                 240

Asp Lys Glu Asn Val His Leu Asp Thr Ile Asn Leu Ala Tyr Tyr Asp
                245                 250                 255

Gly Ser Asp Gln Glu Ser Leu Glu Arg Asn Phe Thr Ser Gly Ala Tyr
            260                 265                 270

Ser Tyr Ala Arg Leu Tyr Pro Thr Ser Ser Asn Tyr Ser Lys Val Ala
        275                 280                 285

Glu Glu Tyr Lys Asp Asn Ile Tyr Tyr Thr Gln Ser Gly Ser Gly Ile
    290                 295                 300

Ala Gly Leu Gly Val Asn Ile Asp Arg Gln Ser Tyr Asn Tyr Thr Ser
305                 310                 315                 320

Lys Thr Thr Asp Ser Glu Lys Val Ala Thr Lys Lys Ala Leu Leu Asn
                325                 330                 335

Lys Asp Phe Arg Gln Ala Leu Asn Phe Ala Leu Asp Arg Ser Ala Tyr
            340                 345                 350

Ser Ala Gln Ile Asn Gly Lys Asp Gly Ala Ala Leu Ala Val Arg Asn
        355                 360                 365

Leu Phe Val Lys Pro Asp Phe Val Ser Ala Gly Glu Lys Thr Phe Gly
    370                 375                 380

Asp Leu Val Ala Ala Gln Leu Pro Ala Tyr Gly Asp Glu Trp Lys Gly
385                 390                 395                 400

Val Asn Leu Ala Asp Gly Gln Asp Gly Leu Phe Asn Ala Asp Lys Ala
                405                 410                 415

Lys Ala Glu Phe Ala Lys Ala Lys Lys Ala Leu Glu Ala Asp Gly Val
            420                 425                 430

Gln Phe Pro Ile His Leu Asp Val Pro Val Asp Gln Ala Ser Lys Asn
        435                 440                 445

Tyr Ile Ser Arg Ile Gln Ser Phe Lys Gln Ser Val Glu Thr Val Leu
    450                 455                 460

Gly Val Glu Asn Val Val Val Asp Ile Gln Gln Met Thr Ser Asp Glu
465                 470                 475                 480

Phe Leu Asn Ile Thr Tyr Tyr Ala Ala Asn Ala Ser Ser Glu Asp Trp

485                 490                 495

Asp Val Ser Gly Gly Val Ser Trp Gly Pro Asp Tyr Gln Asp Pro Ser
            500                 505                 510

Thr Tyr Leu Asp Ile Leu Lys Thr Thr Ser Ser Glu Thr Thr Lys Thr
        515                 520                 525

Tyr Leu Gly Phe Asp Asn Pro Asn Ser Pro Ser Val Val Gln Val Gly
    530                 535                 540

Leu Lys Glu Tyr Asp Lys Leu Val Asp Glu Ala Ala Lys Glu Thr Ser
545                 550                 555                 560

Asp Leu Asn Val Arg Tyr Glu Lys Tyr Ala Ala Ala Gln Ala Trp Leu
                565                 570                 575

Thr Asp Ser Ser Leu Phe Ile Pro Ala Met Ala Ser Ser Gly Ala Ala
            580                 585                 590

Pro Val Leu Ser Arg Ile Val Pro Phe Thr Gly Ala Ser Ala Gln Thr
        595                 600                 605

Gly Ser Lys Gly Ser Asp Val Tyr Phe Lys Tyr Leu Lys Leu Gln Asp
    610                 615                 620

Lys Ala Val Thr Lys Glu Glu Tyr Glu Lys Ala Arg Glu Lys Trp Leu
625                 630                 635                 640

Lys Glu Lys Ala Glu Ser Asn Glu Lys Ala Gln Lys Glu Leu Ala Ser
                645                 650                 655

His Val Lys

<210>   32
<211>   1247
<212>   PRT
<213>   Streptococcus pneumoniae

<400>   32
Ala Asp Gly Val Thr Pro Thr Thr Thr Glu Asn Gln Pro Thr Ile His
1               5                   10                  15

Thr Val Ser Asp Ser Pro Gln Ser Ser Glu Asn Arg Thr Glu Glu Thr
            20                  25                  30

Pro Lys Ala Glu Leu Gln Pro Glu Ala Pro Lys Thr Val Glu Thr Glu
        35                  40                  45

Thr Pro Ala Thr Asp Lys Val Ala Ser Leu Pro Lys Thr Glu Glu Lys
    50                  55                  60

Pro Gln Glu Glu Val Ser Ser Thr Pro Ser Asp Lys Ala Glu Val Val
65                  70                  75                  80

Thr Pro Thr Ser Ala Glu Lys Glu Thr Ala Asn Lys Lys Glu Glu Glu
                85                  90                  95

Ala Ser Pro Lys Lys Glu Glu Ala Lys Glu Val Asp Ser Lys Glu Ser
            100                 105                 110

Asn Thr Asp Lys Thr Asp Lys Asp Lys Pro Ala Lys Lys Asp Glu Ala

        115                 120                 125

Lys Ala Glu Ala Asp Lys Pro Glu Thr Glu Thr Gly Lys Glu Arg Ala
    130                 135                 140

Ala Thr Val Asn Glu Lys Leu Ala Lys Lys Lys Ile Val Ser Ile Asp
145                 150                 155                 160

Ala Gly Arg Lys Tyr Phe Ser Pro Glu Gln Leu Lys Glu Ile Ile Asp
                165                 170                 175

Lys Ala Lys His Tyr Gly Tyr Thr Asp Leu His Leu Leu Val Gly Asn
            180                 185                 190

Asp Gly Leu Arg Phe Met Leu Asp Asp Met Ser Ile Thr Ala Asn Gly
        195                 200                 205

Lys Thr Tyr Ala Ser Asp Asp Val Lys Arg Ala Ile Glu Lys Gly Thr
    210                 215                 220

Asn Asp Tyr Tyr Asn Asp Pro Asn Gly Asn His Leu Thr Glu Ser Gln
225                 230                 235                 240

Met Thr Asp Leu Ile Asn Tyr Ala Lys Asp Lys Gly Ile Gly Leu Ile
                245                 250                 255

Pro Thr Val Asn Ser Pro Gly His Met Asp Ala Ile Leu Asn Ala Met
            260                 265                 270

Lys Glu Leu Gly Ile Gln Asn Pro Asn Phe Ser Tyr Phe Gly Lys Lys
        275                 280                 285

Ser Ala Arg Thr Val Asp Leu Asp Asn Glu Gln Ala Val Ala Phe Thr
    290                 295                 300

Lys Ala Leu Ile Asp Lys Tyr Ala Ala Tyr Phe Ala Lys Lys Thr Glu
305                 310                 315                 320

Ile Phe Asn Ile Gly Leu Asp Glu Tyr Ala Asn Asp Ala Thr Asp Ala
                325                 330                 335

Lys Gly Trp Ser Val Leu Gln Ala Asp Lys Tyr Tyr Pro Asn Glu Gly
            340                 345                 350

Tyr Pro Val Lys Gly Tyr Glu Lys Phe Ile Ala Tyr Ala Asn Asp Leu
        355                 360                 365

Ala Arg Ile Val Lys Ser His Gly Leu Lys Pro Met Ala Phe Asn Asp
    370                 375                 380

Gly Ile Tyr Tyr Asn Ser Asp Thr Ser Phe Gly Ser Phe Asp Lys Asp
385                 390                 395                 400

Ile Ile Val Ser Met Trp Thr Gly Gly Trp Gly Gly Tyr Asp Val Ala
                405                 410                 415

Ser Ser Lys Leu Leu Ala Glu Lys Gly His Gln Ile Leu Asn Thr Asn
            420                 425                 430

Asp Ala Trp Cys Tyr Val Leu Gly Arg Asn Ala Asp Gly Gln Gly Trp
        435                 440                 445

Tyr Asn Leu Asp Gln Gly Leu Asn Gly Ile Lys Asn Thr Pro Ile Thr

450                 455                 460

Ser Val Pro Lys Thr Glu Gly Ala Asp Ile Pro Ile Ile Gly Gly Met
465                 470                 475                 480

Val Ala Ala Trp Ala Asp Thr Pro Ser Ala Arg Tyr Ser Pro Ser His
                485                 490                 495

Leu Phe Lys Leu Met Arg His Phe Ala Asn Ala Asn Ala Glu Tyr Phe
            500                 505                 510

Ala Ala Asp Tyr Glu Ser Ala Glu Gln Ala Leu Asn Glu Val Pro Lys
        515                 520                 525

Asp Leu Asn Arg Tyr Thr Ala Glu Ser Val Ala Ala Val Lys Glu Ala
    530                 535                 540

Glu Lys Ala Ile Arg Ser Leu Asp Ser Asn Leu Ser Arg Ala Gln Gln
545                 550                 555                 560

Asp Thr Ile Asp Gln Ala Ile Ala Lys Leu Gln Glu Thr Val Asn Asn
                565                 570                 575

Leu Thr Leu Thr Pro Glu Ala Gln Lys Glu Glu Glu Ala Lys Arg Glu
            580                 585                 590

Val Glu Lys Leu Ala Lys Asn Lys Val Ile Ser Ile Asp Ala Gly Arg
        595                 600                 605

Lys Tyr Phe Thr Leu Asp Gln Leu Lys Arg Ile Val Asp Lys Ala Ser
    610                 615                 620

Glu Leu Gly Tyr Ser Asp Val His Leu Leu Leu Gly Asn Asp Gly Leu
625                 630                 635                 640

Arg Phe Leu Leu Asn Asp Met Thr Ile Thr Ala Asn Gly Lys Thr Tyr
                645                 650                 655

Ala Ser Asp Asp Val Lys Lys Ala Ile Ile Glu Gly Thr Lys Ala Tyr
            660                 665                 670

Tyr Asp Asp Pro Asn Gly Thr Ala Leu Thr Gln Ala Glu Val Thr Glu
        675                 680                 685

Leu Ile Glu Tyr Ala Lys Ser Lys Asp Ile Gly Leu Ile Pro Ala Ile
    690                 695                 700

Asn Ser Pro Gly His Met Asp Ala Met Leu Val Ala Met Glu Lys Leu
705                 710                 715                 720

Gly Ile Lys Asn Pro Gln Ala His Phe Asp Lys Val Ser Lys Thr Thr
                725                 730                 735

Met Asp Leu Lys Asn Glu Glu Ala Met Asn Phe Val Lys Ala Leu Ile
            740                 745                 750

Gly Lys Tyr Met Asp Phe Phe Ala Gly Lys Thr Lys Ile Phe Asn Phe
        755                 760                 765

Gly Thr Asp Glu Tyr Ala Asn Asp Ala Thr Ser Ala Gln Gly Trp Tyr
    770                 775                 780

Tyr Leu Lys Trp Tyr Gln Leu Tyr Gly Lys Phe Ala Glu Tyr Ala Asn

785                 790                 795                 800

Thr Leu Ala Ala Met Ala Lys Glu Arg Gly Leu Gln Pro Met Ala Phe
                805                 810                 815

Asn Asp Gly Phe Tyr Tyr Glu Asp Lys Asp Asp Val Gln Phe Asp Lys
            820                 825                 830

Asp Val Leu Ile Ser Tyr Trp Ser Lys Gly Trp Trp Gly Tyr Asn Leu
        835                 840                 845

Ala Ser Pro Gln Tyr Leu Ala Ser Lys Gly Tyr Lys Phe Leu Asn Thr
    850                 855                 860

Asn Gly Asp Trp Tyr Tyr Val Ile Gly Asn His Lys Gln Asp Glu Ala
865                 870                 875                 880

Tyr Pro Leu Ser Lys Ala Val Glu Asn Ser Gly Lys Val Pro Phe Asn
                885                 890                 895

Gln Leu Ala Ser Thr Lys Tyr Pro Glu Val Asp Leu Pro Thr Val Gly
            900                 905                 910

Ser Met Leu Ser Ile Trp Ala Asp Arg Pro Ser Ala Glu Tyr Lys Glu
        915                 920                 925

Glu Glu Ile Phe Glu Leu Met Thr Ala Phe Ala Asp His Asn Lys Asp
    930                 935                 940

Tyr Phe Arg Ala Asn Tyr Asn Ala Leu Arg Glu Glu Leu Ala Lys Ile
945                 950                 955                 960

Pro Thr Asn Leu Glu Gly Tyr Ser Lys Glu Ser Leu Glu Ala Leu Asp
                965                 970                 975

Ala Ala Lys Thr Ala Leu Asn Tyr Asn Leu Asn Arg Asn Lys Gln Ala
            980                 985                 990

Glu Leu Asp Thr Leu Val Ala Asn Leu Lys Ala Ala Leu Gln Gly Leu
        995                 1000                1005

Lys Pro Ala Ala Thr His Ser Gly Ser Leu Asp Glu Asn Glu Val Ala
    1010                1015                1020

Ala Asn Val Glu Thr Arg Pro Glu Leu Ile Thr Arg Thr Glu Glu Ile
1025                1030                1035                1040

Pro Phe Glu Val Ile Lys Lys Glu Asn Pro Asn Leu Pro Ala Gly Gln
                1045                1050                1055

Glu Asn Ile Ile Thr Ala Gly Val Lys Gly Glu Arg Thr His Tyr Ile
            1060                1065                1070

Ser Val Leu Thr Glu Asn Gly Lys Thr Thr Glu Thr Val Leu Asp Ser
        1075                1080                1085

Gln Val Thr Lys Glu Val Ile Asn Gln Val Val Glu Val Gly Ser Pro
    1090                1095                1100

Val Thr His Lys Gly Asp Glu Ser Gly Leu Ala Pro Thr Thr Glu Val
1105                1110                1115                1120

Lys Pro Arg Leu Asp Ile Gln Glu Glu Glu Ile Pro Phe Thr Thr Val

1125                1130                1135

Thr Arg Glu Asn Pro Leu Leu Leu Lys Gly Lys Thr Gln Val Ile Thr
        1140                1145                1150

Lys Gly Val Asn Gly His Arg Ser Asn Phe Tyr Ser Val Ser Thr Ser
    1155                1160                1165

Ala Asp Gly Lys Glu Val Lys Thr Leu Val Asn Ser Val Val Ala Gln
    1170                1175                1180

Glu Ala Val Thr Gln Ile Val Glu Val Gly Thr Met Val Thr His Val
1185                1190                1195                1200

Gly Asp Glu Asn Gly Gln Ala Ala Ile Ala Glu Glu Lys Pro Lys Leu
                1205                1210                1215

Glu Ile Pro Ser Gln Pro Ala Pro Ser Thr Ala Pro Ala Glu Glu Ser
        1220                1225                1230

Lys Ala Leu Pro Gln Asp Pro Ala Pro Val Val Thr Glu Lys Lys
    1235                1240                1245

<210>   33
<211>   1008
<212>   PRT
<213>   Streptococcus pneumoniae

<400>   33
Gly Ala Glu Glu Thr Thr Thr Asn Thr Ile Gln Gln Ser Gln Lys Glu
1               5                   10                  15

Val Gln Tyr Gln Gln Arg Asp Thr Lys Asn Leu Val Glu Asn Gly Asp
            20                  25                  30

Phe Gly Gln Thr Glu Asp Gly Ser Ser Pro Trp Thr Gly Ser Lys Ala
        35                  40                  45

Gln Gly Trp Ser Ala Trp Val Asp Gln Lys Asn Ser Ser Ala Asp Ala
    50                  55                  60

Ser Thr Arg Val Ile Glu Ala Lys Asp Gly Ala Ile Thr Ile Ser Ser
65                  70                  75                  80

Pro Glu Lys Leu Arg Ala Ala Val His Arg Met Val Pro Ile Glu Ala
                85                  90                  95

Lys Lys Lys Tyr Lys Leu Arg Phe Lys Ile Lys Thr Asp Asn Lys Val
            100                 105                 110

Gly Ile Ala Lys Val Arg Ile Ile Glu Glu Ser Gly Lys Asp Lys Arg
        115                 120                 125

Leu Trp Asn Ser Ala Thr Thr Ser Gly Thr Lys Asp Trp Gln Thr Ile
    130                 135                 140

Glu Ala Asp Tyr Ser Pro Thr Leu Asp Val Asp Lys Ile Lys Leu Glu
145                 150                 155                 160

Leu Phe Tyr Glu Thr Gly Thr Gly Thr Val Ser Phe Lys Asp Ile Glu
                165                 170                 175

Leu Val Glu Val Ala Asp Gln Pro Ser Glu Asp Ser Gln Thr Asp Lys

180                 185                 190

Gln Leu Glu Glu Lys Ile Asp Leu Pro Ile Gly Lys Lys His Val Phe
        195                 200                 205

Ser Leu Ala Asp Tyr Thr Tyr Lys Val Glu Asn Pro Asp Val Ala Ser
    210                 215                 220

Val Lys Asn Gly Ile Leu Glu Pro Leu Lys Glu Gly Thr Thr Asn Val
225                 230                 235                 240

Ile Val Ser Lys Asp Gly Lys Glu Val Lys Lys Ile Pro Leu Lys Ile
                245                 250                 255

Leu Ala Ser Val Lys Asp Thr Tyr Thr Asp Arg Leu Asp Asp Trp Asn
            260                 265                 270

Gly Ile Ile Ala Gly Asn Gln Tyr Tyr Asp Ser Lys Asn Glu Gln Met
        275                 280                 285

Ala Lys Leu Asn Gln Glu Leu Glu Gly Lys Val Ala Asp Ser Leu Ser
    290                 295                 300

Ser Ile Ser Ser Gln Ala Asp Arg Ile Tyr Leu Trp Glu Lys Phe Ser
305                 310                 315                 320

Asn Tyr Lys Thr Ser Ala Asn Leu Thr Ala Thr Tyr Arg Lys Leu Glu
                325                 330                 335

Glu Met Ala Lys Gln Val Thr Asn Pro Ser Ser Arg Tyr Tyr Gln Asp
            340                 345                 350

Glu Thr Val Val Arg Thr Val Arg Asp Ser Met Glu Trp Met His Lys
        355                 360                 365

His Val Tyr Asn Ser Glu Lys Ser Ile Val Gly Asn Trp Trp Asp Tyr
    370                 375                 380

Glu Ile Gly Thr Pro Arg Ala Ile Asn Asn Thr Leu Ser Leu Met Lys
385                 390                 395                 400

Glu Tyr Phe Ser Asp Glu Glu Ile Lys Lys Tyr Thr Asp Val Ile Glu
                405                 410                 415

Lys Phe Val Pro Asp Pro Glu His Phe Arg Lys Thr Thr Asp Asn Pro
            420                 425                 430

Phe Lys Ala Leu Gly Gly Asn Leu Val Asp Met Gly Arg Val Lys Val
        435                 440                 445

Ile Ala Gly Leu Leu Arg Lys Asp Asp Gln Glu Ile Ser Ser Thr Ile
    450                 455                 460

Arg Ser Ile Glu Gln Val Phe Lys Leu Val Asp Gln Gly Glu Gly Phe
465                 470                 475                 480

Tyr Gln Asp Gly Ser Tyr Ile Asp His Thr Asn Val Ala Tyr Thr Gly
                485                 490                 495

Ala Tyr Gly Asn Val Leu Ile Asp Gly Leu Ser Gln Leu Leu Pro Val
            500                 505                 510

Ile Gln Lys Thr Lys Asn Pro Ile Asp Lys Asp Lys Met Gln Thr Met

515                 520                 525

Tyr His Trp Ile Asp Lys Ser Phe Ala Pro Leu Leu Val Asn Gly Glu
    530                 535                 540

Leu Met Asp Met Ser Arg Gly Arg Ser Ile Ser Arg Ala Asn Ser Glu
545                 550                 555                 560

Gly His Val Ala Ala Val Glu Val Leu Arg Gly Ile His Arg Ile Ala
                565                 570                 575

Asp Met Ser Glu Gly Glu Thr Lys Gln Arg Leu Gln Ser Leu Val Lys
            580                 585                 590

Thr Ile Val Gln Ser Asp Ser Tyr Tyr Asp Val Phe Lys Asn Leu Lys
        595                 600                 605

Thr Tyr Lys Asp Ile Ser Leu Met Gln Ser Leu Leu Ser Asp Ala Gly
    610                 615                 620

Val Ala Ser Val Pro Arg Thr Ser Tyr Leu Ser Ala Phe Asn Lys Met
625                 630                 635                 640

Asp Lys Thr Ala Met Tyr Asn Ala Glu Lys Gly Phe Gly Phe Gly Leu
                645                 650                 655

Ser Leu Phe Ser Ser Arg Thr Leu Asn Tyr Glu His Met Asn Lys Glu
            660                 665                 670

Asn Lys Arg Gly Trp Tyr Thr Ser Asp Gly Met Phe Tyr Leu Tyr Asn
        675                 680                 685

Gly Asp Leu Ser His Tyr Ser Asp Gly Tyr Trp Pro Thr Val Asn Pro
    690                 695                 700

Tyr Lys Met Pro Gly Thr Thr Glu Thr Asp Ala Lys Arg Ala Asp Ser
705                 710                 715                 720

Asp Thr Gly Lys Val Leu Pro Ser Ala Phe Val Gly Thr Ser Lys Leu
                725                 730                 735

Asp Asp Ala Asn Ala Thr Ala Thr Met Asp Phe Thr Asn Trp Asn Gln
            740                 745                 750

Thr Leu Thr Ala His Lys Ser Trp Phe Met Leu Lys Asp Lys Ile Ala
        755                 760                 765

Phe Leu Gly Ser Asn Ile Gln Asn Thr Ser Thr Asp Thr Ala Ala Thr
    770                 775                 780

Thr Ile Asp Gln Arg Lys Leu Glu Ser Ser Asn Pro Tyr Lys Val Tyr
785                 790                 795                 800

Val Asn Asp Lys Glu Ala Ser Leu Thr Glu Gln Glu Lys Asp Tyr Pro
                805                 810                 815

Glu Thr Gln Ser Val Phe Leu Glu Ser Ser Asp Ser Lys Lys Asn Ile
            820                 825                 830

Gly Tyr Phe Phe Phe Lys Lys Ser Ser Ile Ser Met Ser Lys Ala Leu
        835                 840                 845

Gln Lys Gly Ala Trp Lys Asp Ile Asn Glu Gly Gln Ser Asp Lys Glu

850                 855                 860

Val Glu Asn Glu Phe Leu Thr Ile Ser Gln Ala His Lys Gln Asn Gly
865                 870                 875                 880

Asp Ser Tyr Gly Tyr Met Leu Ile Pro Asn Val Asp Arg Ala Thr Phe
                885                 890                 895

Asn Gln Met Ile Lys Glu Leu Glu Ser Ser Leu Ile Glu Asn Asn Glu
            900                 905                 910

Thr Leu Gln Ser Val Tyr Asp Ala Lys Gln Gly Val Trp Gly Ile Val
        915                 920                 925

Lys Tyr Asp Asp Ser Val Ser Thr Ile Ser Asn Gln Phe Gln Val Leu
    930                 935                 940

Lys Arg Gly Val Tyr Thr Ile Arg Lys Glu Gly Asp Glu Tyr Lys Ile
945                 950                 955                 960

Ala Tyr Tyr Asn Pro Glu Thr Gln Glu Ser Ala Pro Asp Gln Glu Val
                965                 970                 975

Phe Lys Lys Leu Glu Gln Ala Ala Gln Pro Gln Val Gln Asn Ser Lys
            980                 985                 990

Glu Lys Glu Lys Ser Glu Glu Glu Lys Asn His Ser Asp Gln Lys Asn
        995                 1000                1005

<210>   34
<211>   628
<212>   PRT
<213>   Streptococcus pneumoniae

<400>   34
Gly Ala Glu Glu Thr Thr Thr Asn Thr Ile Gln Gln Ser Gln Lys Glu
1               5                   10                  15

Val Gln Tyr Gln Gln Arg Asp Thr Lys Asn Leu Val Glu Asn Gly Asp
            20                  25                  30

Phe Gly Gln Thr Glu Asp Gly Ser Ser Pro Trp Thr Gly Ser Lys Ala
        35                  40                  45

Gln Gly Trp Ser Ala Trp Val Asp Gln Lys Asn Ser Ser Ala Asp Ala
    50                  55                  60

Ser Thr Arg Val Ile Glu Ala Lys Asp Gly Ala Ile Thr Ile Ser Ser
65                  70                  75                  80

Pro Glu Lys Leu Arg Ala Ala Val His Arg Met Val Pro Ile Glu Ala
                85                  90                  95

Lys Lys Lys Tyr Lys Leu Arg Phe Lys Ile Lys Thr Asp Asn Lys Val
            100                 105                 110

Gly Ile Ala Lys Val Arg Ile Ile Glu Glu Ser Gly Lys Asp Lys Arg
        115                 120                 125

Leu Trp Asn Ser Ala Thr Thr Ser Gly Thr Lys Asp Trp Gln Thr Ile
    130                 135                 140

Glu Ala Asp Tyr Ser Pro Thr Leu Asp Val Asp Lys Ile Lys Leu Glu

145                 150                 155                 160

Leu Phe Tyr Glu Thr Gly Thr Gly Thr Val Ser Phe Lys Asp Ile Glu
                165                 170                 175

Leu Val Glu Val Ala Asp Gln Pro Ser Glu Asp Ser Gln Thr Asp Lys
            180                 185                 190

Gln Leu Glu Glu Lys Ile Asp Leu Pro Ile Gly Lys Lys His Val Phe
        195                 200                 205

Ser Leu Ala Asp Tyr Thr Tyr Lys Val Glu Asn Pro Asp Val Ala Ser
    210                 215                 220

Val Lys Asn Gly Ile Leu Glu Pro Leu Lys Glu Gly Thr Thr Asn Val
225                 230                 235                 240

Ile Val Ser Lys Asp Gly Lys Glu Val Lys Lys Ile Pro Leu Lys Ile
                245                 250                 255

Leu Ala Ser Val Lys Asp Thr Tyr Thr Asp Arg Leu Asp Asp Trp Asn
            260                 265                 270

Gly Ile Ile Ala Gly Asn Gln Tyr Tyr Asp Ser Lys Asn Glu Gln Met
        275                 280                 285

Ala Lys Leu Asn Gln Glu Leu Glu Gly Lys Val Ala Asp Ser Leu Ser
    290                 295                 300

Ser Ile Ser Ser Gln Ala Asp Arg Ile Tyr Leu Trp Glu Lys Phe Ser
305                 310                 315                 320

Asn Tyr Lys Thr Ser Ala Asn Leu Thr Ala Thr Tyr Arg Lys Leu Glu
                325                 330                 335

Glu Met Ala Lys Gln Val Thr Asn Pro Ser Ser Arg Tyr Tyr Gln Asp
            340                 345                 350

Glu Thr Val Val Arg Thr Val Arg Asp Ser Met Glu Trp Met His Lys
        355                 360                 365

His Val Tyr Asn Ser Glu Lys Ser Ile Val Gly Asn Trp Trp Asp Tyr
    370                 375                 380

Glu Ile Gly Thr Pro Arg Ala Ile Asn Asn Thr Leu Ser Leu Met Lys
385                 390                 395                 400

Glu Tyr Phe Ser Asp Glu Glu Ile Lys Lys Tyr Thr Asp Val Ile Glu
                405                 410                 415

Lys Phe Val Pro Asp Pro Glu His Phe Arg Lys Thr Thr Asp Asn Pro
            420                 425                 430

Phe Lys Ala Leu Gly Gly Asn Leu Val Asp Met Gly Arg Val Lys Val
        435                 440                 445

Ile Ala Gly Leu Leu Arg Lys Asp Asp Gln Glu Ile Ser Ser Thr Ile
    450                 455                 460

Arg Ser Ile Glu Gln Val Phe Lys Leu Val Asp Gln Gly Glu Gly Phe
465                 470                 475                 480

Tyr Gln Asp Gly Ser Tyr Ile Asp His Thr Asn Val Ala Tyr Thr Gly

485                 490                 495

Ala Tyr Gly Asn Val Leu Ile Asp Gly Leu Ser Gln Leu Leu Pro Val
            500                 505                 510

Ile Gln Lys Thr Lys Asn Pro Ile Asp Lys Asp Lys Met Gln Thr Met
        515                 520                 525

Tyr His Trp Ile Asp Lys Ser Phe Ala Pro Leu Leu Val Asn Gly Glu
    530                 535                 540

Leu Met Asp Met Ser Arg Gly Arg Ser Ile Ser Arg Ala Asn Ser Glu
545                 550                 555                 560

Gly His Val Ala Ala Val Glu Val Leu Arg Gly Ile His Arg Ile Ala
                565                 570                 575

Asp Met Ser Glu Gly Glu Thr Lys Gln Arg Leu Gln Ser Leu Val Lys
            580                 585                 590

Thr Ile Val Gln Ser Asp Ser Tyr Tyr Asp Val Phe Lys Asn Leu Lys
        595                 600                 605

Thr Tyr Lys Asp Ile Ser Leu Met Gln Ser Leu Leu Ser Asp Ala Gly
    610                 615                 620

Val Ala Ser Val
625

<210>   35
<211>   452
<212>   PRT
<213>   Streptococcus pneumoniae

<400>   35
Ala Asn Ser Glu Gly His Val Ala Ala Val Glu Val Leu Arg Gly Ile
1               5                   10                  15

His Arg Ile Ala Asp Met Ser Glu Gly Glu Thr Lys Gln Arg Leu Gln
            20                  25                  30

Ser Leu Val Lys Thr Ile Val Gln Ser Asp Ser Tyr Tyr Asp Val Phe
        35                  40                  45

Lys Asn Leu Lys Thr Tyr Lys Asp Ile Ser Leu Met Gln Ser Leu Leu
    50                  55                  60

Ser Asp Ala Gly Val Ala Ser Val Pro Arg Thr Ser Tyr Leu Ser Ala
65                  70                  75                  80

Phe Asn Lys Met Asp Lys Thr Ala Met Tyr Asn Ala Glu Lys Gly Phe
                85                  90                  95

Gly Phe Gly Leu Ser Leu Phe Ser Ser Arg Thr Leu Asn Tyr Glu His
            100                 105                 110

Met Asn Lys Glu Asn Lys Arg Gly Trp Tyr Thr Ser Asp Gly Met Phe
        115                 120                 125

Tyr Leu Tyr Asn Gly Asp Leu Ser His Tyr Ser Asp Gly Tyr Trp Pro
    130                 135                 140

Thr Val Asn Pro Tyr Lys Met Pro Gly Thr Thr Glu Thr Asp Ala Lys

145                 150                 155                 160

Arg Ala Asp Ser Asp Thr Gly Lys Val Leu Pro Ser Ala Phe Val Gly
                165                 170                 175

Thr Ser Lys Leu Asp Asp Ala Asn Ala Thr Ala Thr Met Asp Phe Thr
            180                 185                 190

Asn Trp Asn Gln Thr Leu Thr Ala His Lys Ser Trp Phe Met Leu Lys
        195                 200                 205

Asp Lys Ile Ala Phe Leu Gly Ser Asn Ile Gln Asn Thr Ser Thr Asp
    210                 215                 220

Thr Ala Ala Thr Thr Ile Asp Gln Arg Lys Leu Glu Ser Ser Asn Pro
225                 230                 235                 240

Tyr Lys Val Tyr Val Asn Asp Lys Glu Ala Ser Leu Thr Glu Gln Glu
                245                 250                 255

Lys Asp Tyr Pro Glu Thr Gln Ser Val Phe Leu Glu Ser Ser Asp Ser
            260                 265                 270

Lys Lys Asn Ile Gly Tyr Phe Phe Phe Lys Lys Ser Ser Ile Ser Met
        275                 280                 285

Ser Lys Ala Leu Gln Lys Gly Ala Trp Lys Asp Ile Asn Glu Gly Gln
    290                 295                 300

Ser Asp Lys Glu Val Glu Asn Glu Phe Leu Thr Ile Ser Gln Ala His
305                 310                 315                 320

Lys Gln Asn Gly Asp Ser Tyr Gly Tyr Met Leu Ile Pro Asn Val Asp
                325                 330                 335

Arg Ala Thr Phe Asn Gln Met Ile Lys Glu Leu Glu Ser Ser Leu Ile
            340                 345                 350

Glu Asn Asn Glu Thr Leu Gln Ser Val Tyr Asp Ala Lys Gln Gly Val
        355                 360                 365

Trp Gly Ile Val Lys Tyr Asp Asp Ser Val Ser Thr Ile Ser Asn Gln
    370                 375                 380

Phe Gln Val Leu Lys Arg Gly Val Tyr Thr Ile Arg Lys Glu Gly Asp
385                 390                 395                 400

Glu Tyr Lys Ile Ala Tyr Tyr Asn Pro Glu Thr Gln Glu Ser Ala Pro
                405                 410                 415

Asp Gln Glu Val Phe Lys Lys Leu Glu Gln Ala Ala Gln Pro Gln Val
            420                 425                 430

Gln Asn Ser Lys Glu Lys Glu Lys Ser Glu Glu Glu Lys Asn His Ser
        435                 440                 445

Asp Gln Lys Asn
    450

<210>   36
<211>   2148
<212>   PRT
<213>   Streptococcus pneumoniae

<400>   36
Asp Glu Thr Leu Ile Thr His Thr Ala Glu Lys Pro Lys Glu Glu Lys
1               5                   10                  15

Met Ile Val Glu Glu Lys Ala Asp Lys Ala Leu Glu Thr Lys Asn Val
            20                  25                  30

Val Glu Arg Thr Glu Gln Ser Glu Pro Ser Ser Thr Glu Ala Ile Ala
        35                  40                  45

Ser Glu Lys Lys Glu Asp Glu Ala Val Thr Pro Lys Glu Glu Lys Val
    50                  55                  60

Ser Ala Lys Pro Glu Glu Lys Ala Pro Arg Ile Glu Ser Gln Ala Ser
65                  70                  75                  80

Ser Gln Glu Lys Pro Leu Lys Glu Asp Ala Lys Ala Val Thr Asn Glu
                85                  90                  95

Glu Val Asn Gln Met Ile Glu Asn Arg Lys Val Asp Phe Asn Gln Asn
            100                 105                 110

Trp Tyr Phe Lys Leu Asn Ala Asn Ser Lys Glu Ala Ile Lys Pro Asp
        115                 120                 125

Ala Asp Val Ser Thr Trp Lys Lys Leu Asp Leu Pro Tyr Asp Trp Ser
    130                 135                 140

Ile Phe Asn Asp Phe Asp His Glu Ser Pro Ala Gln Asn Glu Gly Gly
145                 150                 155                 160

Gln Leu Asn Gly Gly Glu Ala Trp Tyr Arg Lys Thr Phe Lys Leu Asp
                165                 170                 175

Glu Lys Asp Leu Lys Lys Asn Val Arg Leu Thr Phe Asp Gly Val Tyr
            180                 185                 190

Met Asp Ser Gln Val Tyr Val Asn Gly Gln Leu Val Gly His Tyr Pro
        195                 200                 205

Asn Gly Tyr Asn Gln Phe Ser Tyr Asp Ile Thr Lys Tyr Leu Tyr Lys
    210                 215                 220

Asp Gly Arg Glu Asn Val Ile Ala Val His Ala Val Asn Lys Gln Pro
225                 230                 235                 240

Ser Ser Arg Trp Tyr Ser Gly Ser Gly Ile Tyr Arg Asp Val Thr Leu
                245                 250                 255

Gln Val Thr Asp Lys Val His Val Glu Lys Asn Gly Thr Thr Ile Leu
            260                 265                 270

Thr Pro Lys Leu Glu Glu Gln Gln His Gly Lys Val Glu Thr His Val
        275                 280                 285

Thr Ser Lys Ile Val Asn Thr Asp Asp Lys Asp His Glu Leu Val Ala
    290                 295                 300

Glu Tyr Gln Ile Val Glu Arg Gly Gly His Ala Val Thr Gly Leu Val
305                 310                 315                 320

Arg Thr Ala Ser Arg Thr Leu Lys Ala His Glu Ser Thr Ser Leu Asp

325                 330                 335

Ala Ile Leu Glu Val Glu Arg Pro Lys Leu Trp Thr Val Leu Asn Asp
            340                 345                 350

Lys Pro Ala Leu Tyr Glu Leu Ile Thr Arg Val Tyr Arg Asp Gly Gln
        355                 360                 365

Leu Val Asp Ala Lys Lys Asp Leu Phe Gly Tyr Arg Tyr Tyr His Trp
    370                 375                 380

Thr Pro Asn Glu Gly Phe Ser Leu Asn Gly Glu Arg Ile Lys Phe His
385                 390                 395                 400

Gly Val Ser Leu His His Asp His Gly Ala Leu Gly Ala Glu Glu Asn
                405                 410                 415

Tyr Lys Ala Glu Tyr Arg Arg Leu Lys Gln Met Lys Glu Met Gly Val
            420                 425                 430

Asn Ser Ile Arg Thr Thr His Asn Pro Ala Ser Glu Gln Thr Leu Gln
        435                 440                 445

Ile Ala Ala Glu Leu Gly Leu Leu Val Gln Glu Glu Ala Phe Asp Thr
    450                 455                 460

Trp Tyr Gly Gly Lys Lys Pro Tyr Asp Tyr Gly Arg Phe Phe Glu Lys
465                 470                 475                 480

Asp Ala Thr His Pro Glu Ala Arg Lys Gly Glu Lys Trp Ser Asp Phe
                485                 490                 495

Asp Leu Arg Thr Met Val Glu Arg Gly Lys Asn Asn Pro Ala Ile Phe
            500                 505                 510

Met Trp Ser Ile Gly Asn Glu Ile Gly Glu Ala Asn Gly Asp Ala His
        515                 520                 525

Ser Leu Ala Thr Val Lys Arg Leu Val Lys Val Ile Lys Asp Val Asp
    530                 535                 540

Lys Thr Arg Tyr Val Thr Met Gly Ala Asp Lys Phe Arg Phe Gly Asn
545                 550                 555                 560

Gly Ser Gly Gly His Glu Lys Ile Ala Asp Glu Leu Asp Ala Val Gly
                565                 570                 575

Phe Asn Tyr Ser Glu Asp Asn Tyr Lys Ala Leu Arg Ala Lys His Pro
            580                 585                 590

Lys Trp Leu Ile Tyr Gly Ser Glu Thr Ser Ser Ala Thr Arg Thr Arg
        595                 600                 605

Gly Ser Tyr Tyr Arg Pro Glu Arg Glu Leu Lys His Ser Asn Gly Pro
    610                 615                 620

Glu Arg Asn Tyr Glu Gln Ser Asp Tyr Gly Asn Asp Arg Val Gly Trp
625                 630                 635                 640

Gly Lys Thr Ala Thr Ala Ser Trp Thr Phe Asp Arg Asp Asn Ala Gly
                645                 650                 655

Tyr Ala Gly Gln Phe Ile Trp Thr Gly Thr Asp Tyr Ile Gly Glu Pro

660                 665                 670

Thr Pro Trp His Asn Gln Asn Gln Thr Pro Val Lys Ser Ser Tyr Phe
        675                 680                 685

Gly Ile Val Asp Thr Ala Gly Ile Pro Lys His Asp Phe Tyr Leu Tyr
    690                 695                 700

Gln Ser Gln Trp Val Ser Val Lys Lys Lys Pro Met Val His Leu Leu
705                 710                 715                 720

Pro His Trp Asn Trp Glu Asn Lys Glu Leu Ala Ser Lys Val Ala Asp
                725                 730                 735

Ser Glu Gly Lys Ile Pro Val Arg Ala Tyr Ser Asn Ala Ser Ser Val
            740                 745                 750

Glu Leu Phe Leu Asn Gly Lys Ser Leu Gly Leu Lys Thr Phe Asn Lys
        755                 760                 765

Lys Gln Thr Ser Asp Gly Arg Thr Tyr Gln Glu Gly Ala Asn Ala Asn
    770                 775                 780

Glu Leu Tyr Leu Glu Trp Lys Val Ala Tyr Gln Pro Gly Thr Leu Glu
785                 790                 795                 800

Ala Ile Ala Arg Asp Glu Ser Gly Lys Glu Ile Ala Arg Asp Lys Ile
                805                 810                 815

Thr Thr Ala Gly Lys Pro Ala Ala Val Arg Leu Ile Lys Glu Asp His
            820                 825                 830

Ala Ile Ala Ala Asp Gly Lys Asp Leu Thr Tyr Ile Tyr Tyr Glu Ile
        835                 840                 845

Val Asp Ser Gln Gly Asn Val Val Pro Thr Ala Asn Asn Leu Val Arg
    850                 855                 860

Phe Gln Leu His Gly Gln Gly Gln Leu Val Gly Val Asp Asn Gly Glu
865                 870                 875                 880

Gln Ala Ser Arg Glu Arg Tyr Lys Ala Gln Ala Asp Gly Ser Trp Ile
                885                 890                 895

Arg Lys Ala Phe Asn Gly Lys Gly Val Ala Ile Val Lys Ser Thr Glu
            900                 905                 910

Gln Ala Gly Lys Phe Thr Leu Thr Ala His Ser Asp Leu Leu Lys Ser
        915                 920                 925

Asn Gln Val Thr Val Phe Thr Gly Lys Lys Glu Gly Gln Glu Lys Thr
    930                 935                 940

Val Leu Gly Thr Glu Val Pro Lys Val Gln Thr Ile Ile Gly Glu Ala
945                 950                 955                 960

Pro Glu Met Pro Thr Thr Val Pro Phe Val Tyr Ser Asp Gly Ser Arg
                965                 970                 975

Ala Glu Arg Pro Val Thr Trp Ser Leu Val Asp Val Ser Lys Pro Gly
            980                 985                 990

Ile Val Thr Val Lys Gly Met Ala Asp Gly Arg Glu Val Glu Ala Arg

995                 1000                1005

Val Glu Val Ile Ala Leu Lys Ser Glu Leu Pro Val Val Lys Arg Ile
    1010                1015                1020

Ala Pro Asn Thr Asn Leu Asn Ser Val Asp Lys Ser Val Ser Tyr Val
1025                1030                1035                1040

Leu Thr Asp Gly Ser Val Gln Glu Tyr Glu Val Asp Lys Trp Glu Ile
                1045                1050                1055

Ala Glu Glu Asp Lys Ala Lys Leu Ala Ile Pro Gly Ser Arg Ile Gln
            1060                1065                1070

Ala Thr Gly Tyr Leu Glu Gly Gln Pro Ile His Ala Thr Leu Val Val
        1075                1080                1085

Glu Glu Gly Asn Pro Ala Ala Pro Val Val Pro Thr Val Thr Val Gly
    1090                1095                1100

Gly Glu Ala Val Thr Gly Leu Thr Ser Arg Gln Pro Met Gln Tyr Arg
1105                1110                1115                1120

Thr Leu Ser Tyr Gly Ala Gln Leu Pro Glu Val Thr Ala Ser Ala Glu
                1125                1130                1135

Asn Ala Asp Val Thr Val Leu Gln Ala Ser Ala Ala Asn Gly Met Arg
            1140                1145                1150

Ala Ser Ile Phe Ile Gln Pro Lys Asp Gly Gly Pro Leu Gln Thr Tyr
        1155                1160                1165

Ala Ile Gln Phe Leu Glu Glu Ala Pro Lys Ile Ala His Leu Ser Leu
    1170                1175                1180

Gln Val Glu Lys Ala Asp Ser Leu Lys Glu Asp Gln Thr Val Lys Leu
1185                1190                1195                1200

Ser Val Arg Ala His Tyr Gln Asp Gly Thr Gln Ala Val Leu Pro Ala
                1205                1210                1215

Asp Lys Val Thr Phe Ser Thr Ser Gly Glu Gly Glu Val Ala Ile Arg
            1220                1225                1230

Lys Gly Met Leu Glu Leu His Lys Pro Gly Ala Val Thr Leu Asn Ala
        1235                1240                1245

Glu Tyr Glu Gly Ala Lys Gly Gln Val Glu Leu Thr Ile Gln Ala Asn
    1250                1255                1260

Thr Glu Lys Lys Ile Ala Gln Ser Ile Arg Pro Val Asn Val Val Thr
1265                1270                1275                1280

Asp Leu His Gln Glu Pro Ser Leu Pro Ala Thr Val Thr Val Glu Tyr
                1285                1290                1295

Asp Lys Gly Phe Pro Lys Thr His Lys Val Thr Trp Gln Ala Ile Pro
            1300                1305                1310

Lys Glu Lys Leu Asp Ser Tyr Gln Ile Phe Glu Val Leu Gly Lys Val
        1315                1320                1325

Glu Gly Ile Asp Leu Glu Ala Arg Ala Lys Val Ser Val Glu Gly Ile

1330                1335                1340

Val Ser Val Glu Glu Val Ser Val Thr Thr Pro Ile Ala Glu Ala Pro
1345                1350                1355                1360

Gln Leu Pro Glu Ser Val Arg Thr Tyr Asp Ser Asn Gly His Val Ser
                1365                1370                1375

Ser Ala Lys Val Ala Trp Asp Ala Ile Arg Pro Glu Gln Tyr Ala Lys
            1380                1385                1390

Glu Gly Val Phe Thr Val Asn Gly Arg Leu Glu Gly Thr Gln Leu Thr
        1395                1400                1405

Thr Lys Leu His Val Arg Val Ser Ala Gln Thr Glu Gln Gly Ala Asn
    1410                1415                1420

Ile Ser Asp Gln Trp Thr Gly Ser Glu Leu Pro Leu Ala Phe Ala Ser
1425                1430                1435                1440

Asp Ser Asn Pro Ser Asp Pro Val Ser Asn Val Asn Asp Lys Leu Ile
                1445                1450                1455

Ser Tyr Asn Asn Gln Pro Ala Asn Arg Trp Thr Asn Trp Asn Arg Ser
            1460                1465                1470

Asn Pro Glu Ala Ser Val Gly Val Leu Phe Gly Asp Ser Gly Ile Leu
        1475                1480                1485

Ser Lys Arg Ser Val Asp Asn Leu Ser Val Gly Phe His Glu Asp His
    1490                1495                1500

Gly Val Gly Ala Pro Lys Ser Tyr Val Ile Glu Tyr Tyr Val Gly Lys
1505                1510                1515                1520

Thr Val Pro Thr Ala Pro Lys Asn Pro Ser Phe Val Gly Asn Glu Asp
                1525                1530                1535

His Val Phe Asn Asp Ser Ala Asn Trp Lys Pro Val Thr Asn Leu Lys
            1540                1545                1550

Ala Pro Ala Gln Leu Lys Ala Gly Glu Met Asn His Phe Ser Phe Asp
        1555                1560                1565

Lys Val Glu Thr Tyr Ala Ile Arg Ile Arg Met Val Lys Ala Asp Asn
    1570                1575                1580

Lys Arg Gly Thr Ser Ile Thr Glu Val Gln Ile Phe Ala Lys Gln Val
1585                1590                1595                1600

Ala Ala Ala Lys Gln Gly Gln Thr Arg Ile Gln Val Asp Gly Lys Asp
                1605                1610                1615

Leu Ala Asn Phe Asn Pro Asp Leu Thr Asp Tyr Tyr Leu Glu Ser Val
            1620                1625                1630

Asp Gly Lys Val Pro Ala Val Thr Ala Asn Val Ser Asn Asn Gly Leu
        1635                1640                1645

Ala Thr Val Val Pro Ser Val Arg Glu Gly Glu Pro Val Arg Val Ile
    1650                1655                1660

Ala Lys Ala Glu Asn Gly Asp Ile Leu Gly Glu Tyr Arg Leu His Phe

1665                1670                1675                1680

Thr Lys Asp Lys Asn Leu Leu Ser His Lys Pro Val Ala Ala Val Lys
                1685                1690                1695

Gln Ala Arg Leu Leu Gln Val Gly Gln Ala Leu Glu Leu Pro Thr Lys
            1700                1705                1710

Val Pro Val Tyr Phe Thr Gly Lys Asp Gly Tyr Glu Thr Lys Asp Leu
        1715                1720                1725

Thr Val Glu Trp Glu Glu Val Pro Ala Glu Asn Leu Thr Lys Ala Gly
    1730                1735                1740

Gln Phe Thr Val Arg Gly Arg Val Leu Gly Ser Asn Leu Val Ala Glu
1745                1750                1755                1760

Val Thr Val Arg Val Thr Asp Lys Leu Gly Glu Thr Leu Ser Asp Asn
                1765                1770                1775

Pro Asn Tyr Asp Glu Asn Ser Asn Gln Ala Phe Ala Ser Ala Thr Asn
            1780                1785                1790

Asp Ile Asp Lys Asn Ser His Asp Arg Val Asp Tyr Leu Asn Asp Gly
        1795                1800                1805

Asp His Ser Glu Asn Arg Arg Trp Thr Asn Trp Ser Pro Thr Pro Ser
    1810                1815                1820

Ser Asn Pro Glu Val Ser Ala Gly Val Ile Phe Arg Glu Asn Gly Lys
1825                1830                1835                1840

Ile Val Glu Arg Thr Val Ala Gln Ala Lys Leu His Phe Phe Ala Asp
                1845                1850                1855

Ser Gly Thr Asp Ala Pro Ser Lys Leu Val Leu Glu Arg Tyr Val Gly
            1860                1865                1870

Pro Gly Phe Glu Val Pro Thr Tyr Tyr Ser Asn Tyr Gln Ala Tyr Glu
        1875                1880                1885

Ser Gly His Pro Phe Asn Asn Pro Glu Asn Trp Glu Ala Val Pro Tyr
    1890                1895                1900

Arg Ala Asp Lys Asp Ile Ala Ala Gly Asp Glu Ile Asn Val Thr Phe
1905                1910                1915                1920

Lys Ala Val Lys Ala Lys Val Met Arg Trp Arg Met Glu Arg Lys Ala
                1925                1930                1935

Asp Lys Ser Gly Val Ala Met Ile Glu Met Thr Phe Leu Ala Pro Ser
            1940                1945                1950

Glu Leu Pro Gln Glu Ser Thr Gln Ser Lys Ile Leu Val Asp Gly Lys
        1955                1960                1965

Glu Leu Ala Asp Phe Ala Glu Asn Arg Gln Asp Tyr Gln Ile Thr Tyr
    1970                1975                1980

Lys Gly Gln Arg Pro Lys Val Ser Val Glu Glu Asn Asn Gln Val Ala
1985                1990                1995                2000

Ser Thr Val Val Asp Ser Gly Glu Asp Ser Leu Pro Val Leu Val Arg

                2005                2010                2015

Leu Val Ser Glu Ser Gly Lys Gln Val Lys Glu Tyr Arg Ile Gln Leu
            2020                2025                2030

Thr Lys Glu Lys Pro Val Ser Ala Val Gln Glu Asp Leu Pro Lys Leu
        2035                2040                2045

Glu Phe Val Glu Lys Asp Leu Ala Tyr Lys Thr Val Glu Lys Lys Asp
    2050                2055                2060

Ser Thr Leu Tyr Leu Gly Glu Thr Arg Val Glu Gln Glu Gly Lys Val
2065                2070                2075                2080

Gly Lys Glu Arg Ile Phe Thr Val Ile Asn Pro Asp Gly Ser Lys Glu
                2085                2090                2095

Glu Lys Leu Arg Glu Val Val Glu Val Pro Thr Asp Arg Ile Val Leu
            2100                2105                2110

Val Gly Thr Lys Pro Val Ala Gln Glu Ala Lys Lys Pro Gln Val Ser
        2115                2120                2125

Glu Lys Ala Asp Thr Lys Pro Ile Asp Ser Ser Glu Ala Asp Gln Thr
    2130                2135                2140

Asn Lys Ala Gln
2145

<210> 37
<211> 1063
<212> PRT
<213> Streptococcus pneumoniae

<400> 37
Asp Glu Thr Leu Ile Thr His Thr Ala Glu Lys Pro Lys Glu Glu Lys
1               5                   10                  15

Met Ile Val Glu Glu Lys Ala Asp Lys Ala Leu Glu Thr Lys Asn Val
            20                  25                  30

Val Glu Arg Thr Glu Gln Ser Glu Pro Ser Ser Thr Glu Ala Ile Ala
        35                  40                  45

Ser Glu Lys Lys Glu Asp Glu Ala Val Thr Pro Lys Glu Glu Lys Val
    50                  55                  60

Ser Ala Lys Pro Glu Glu Lys Ala Pro Arg Ile Glu Ser Gln Ala Ser
65                  70                  75                  80

Ser Gln Glu Lys Pro Leu Lys Glu Asp Ala Lys Ala Val Thr Asn Glu
                85                  90                  95

Glu Val Asn Gln Met Ile Glu Asn Arg Lys Val Asp Phe Asn Gln Asn
            100                 105                 110

Trp Tyr Phe Lys Leu Asn Ala Asn Ser Lys Glu Ala Ile Lys Pro Asp
        115                 120                 125

Ala Asp Val Ser Thr Trp Lys Lys Leu Asp Leu Pro Tyr Asp Trp Ser
    130                 135                 140

Ile Phe Asn Asp Phe Asp His Glu Ser Pro Ala Gln Asn Glu Gly Gly

145                 150                 155                 160

Gln Leu Asn Gly Gly Glu Ala Trp Tyr Arg Lys Thr Phe Lys Leu Asp
                165                 170                 175

Glu Lys Asp Leu Lys Lys Asn Val Arg Leu Thr Phe Asp Gly Val Tyr
            180                 185                 190

Met Asp Ser Gln Val Tyr Val Asn Gly Gln Leu Val Gly His Tyr Pro
        195                 200                 205

Asn Gly Tyr Asn Gln Phe Ser Tyr Asp Ile Thr Lys Tyr Leu Tyr Lys
    210                 215                 220

Asp Gly Arg Glu Asn Val Ile Ala Val His Ala Val Asn Lys Gln Pro
225                 230                 235                 240

Ser Ser Arg Trp Tyr Ser Gly Ser Gly Ile Tyr Arg Asp Val Thr Leu
                245                 250                 255

Gln Val Thr Asp Lys Val His Val Glu Lys Asn Gly Thr Thr Ile Leu
            260                 265                 270

Thr Pro Lys Leu Glu Glu Gln Gln His Gly Lys Val Glu Thr His Val
        275                 280                 285

Thr Ser Lys Ile Val Asn Thr Asp Asp Lys Asp His Glu Leu Val Ala
    290                 295                 300

Glu Tyr Gln Ile Val Glu Arg Gly Gly His Ala Val Thr Gly Leu Val
305                 310                 315                 320

Arg Thr Ala Ser Arg Thr Leu Lys Ala His Glu Ser Thr Ser Leu Asp
                325                 330                 335

Ala Ile Leu Glu Val Glu Arg Pro Lys Leu Trp Thr Val Leu Asn Asp
            340                 345                 350

Lys Pro Ala Leu Tyr Glu Leu Ile Thr Arg Val Tyr Arg Asp Gly Gln
        355                 360                 365

Leu Val Asp Ala Lys Lys Asp Leu Phe Gly Tyr Arg Tyr Tyr His Trp
    370                 375                 380

Thr Pro Asn Glu Gly Phe Ser Leu Asn Gly Glu Arg Ile Lys Phe His
385                 390                 395                 400

Gly Val Ser Leu His His Asp His Gly Ala Leu Gly Ala Glu Glu Asn
                405                 410                 415

Tyr Lys Ala Glu Tyr Arg Arg Leu Lys Gln Met Lys Glu Met Gly Val
            420                 425                 430

Asn Ser Ile Arg Thr Thr His Asn Pro Ala Ser Glu Gln Thr Leu Gln
        435                 440                 445

Ile Ala Ala Glu Leu Gly Leu Leu Val Gln Glu Glu Ala Phe Asp Thr
    450                 455                 460

Trp Tyr Gly Gly Lys Lys Pro Tyr Asp Tyr Gly Arg Phe Phe Glu Lys
465                 470                 475                 480

Asp Ala Thr His Pro Glu Ala Arg Lys Gly Glu Lys Trp Ser Asp Phe

                485                 490                 495

Asp Leu Arg Thr Met Val Glu Arg Gly Lys Asn Asn Pro Ala Ile Phe
            500                 505                 510

Met Trp Ser Ile Gly Asn Glu Ile Gly Glu Ala Asn Gly Asp Ala His
        515                 520                 525

Ser Leu Ala Thr Val Lys Arg Leu Val Lys Val Ile Lys Asp Val Asp
    530                 535                 540

Lys Thr Arg Tyr Val Thr Met Gly Ala Asp Lys Phe Arg Phe Gly Asn
545                 550                 555                 560

Gly Ser Gly Gly His Glu Lys Ile Ala Asp Glu Leu Asp Ala Val Gly
                565                 570                 575

Phe Asn Tyr Ser Glu Asp Asn Tyr Lys Ala Leu Arg Ala Lys His Pro
            580                 585                 590

Lys Trp Leu Ile Tyr Gly Ser Glu Thr Ser Ser Ala Thr Arg Thr Arg
        595                 600                 605

Gly Ser Tyr Tyr Arg Pro Glu Arg Glu Leu Lys His Ser Asn Gly Pro
    610                 615                 620

Glu Arg Asn Tyr Glu Gln Ser Asp Tyr Gly Asn Asp Arg Val Gly Trp
625                 630                 635                 640

Gly Lys Thr Ala Thr Ala Ser Trp Thr Phe Asp Arg Asp Asn Ala Gly
                645                 650                 655

Tyr Ala Gly Gln Phe Ile Trp Thr Gly Thr Asp Tyr Ile Gly Glu Pro
            660                 665                 670

Thr Pro Trp His Asn Gln Asn Gln Thr Pro Val Lys Ser Ser Tyr Phe
        675                 680                 685

Gly Ile Val Asp Thr Ala Gly Ile Pro Lys His Asp Phe Tyr Leu Tyr
    690                 695                 700

Gln Ser Gln Trp Val Ser Val Lys Lys Lys Pro Met Val His Leu Leu
705                 710                 715                 720

Pro His Trp Asn Trp Glu Asn Lys Glu Leu Ala Ser Lys Val Ala Asp
                725                 730                 735

Ser Glu Gly Lys Ile Pro Val Arg Ala Tyr Ser Asn Ala Ser Ser Val
            740                 745                 750

Glu Leu Phe Leu Asn Gly Lys Ser Leu Gly Leu Lys Thr Phe Asn Lys
        755                 760                 765

Lys Gln Thr Ser Asp Gly Arg Thr Tyr Gln Glu Gly Ala Asn Ala Asn
    770                 775                 780

Glu Leu Tyr Leu Glu Trp Lys Val Ala Tyr Gln Pro Gly Thr Leu Glu
785                 790                 795                 800

Ala Ile Ala Arg Asp Glu Ser Gly Lys Glu Ile Ala Arg Asp Lys Ile
                805                 810                 815

Thr Thr Ala Gly Lys Pro Ala Ala Val Arg Leu Ile Lys Glu Asp His

820                 825                 830

Ala Ile Ala Ala Asp Gly Lys Asp Leu Thr Tyr Ile Tyr Tyr Glu Ile
        835                 840                 845

Val Asp Ser Gln Gly Asn Val Val Pro Thr Ala Asn Asn Leu Val Arg
    850                 855                 860

Phe Gln Leu His Gly Gln Gly Gln Leu Val Gly Val Asp Asn Gly Glu
865                 870                 875                 880

Gln Ala Ser Arg Glu Arg Tyr Lys Ala Gln Ala Asp Gly Ser Trp Ile
                885                 890                 895

Arg Lys Ala Phe Asn Gly Lys Gly Val Ala Ile Val Lys Ser Thr Glu
            900                 905                 910

Gln Ala Gly Lys Phe Thr Leu Thr Ala His Ser Asp Leu Leu Lys Ser
        915                 920                 925

Asn Gln Val Thr Val Phe Thr Gly Lys Lys Glu Gly Gln Glu Lys Thr
    930                 935                 940

Val Leu Gly Thr Glu Val Pro Lys Val Gln Thr Ile Ile Gly Glu Ala
945                 950                 955                 960

Pro Glu Met Pro Thr Thr Val Pro Phe Val Tyr Ser Asp Gly Ser Arg
                965                 970                 975

Ala Glu Arg Pro Val Thr Trp Ser Leu Val Asp Val Ser Lys Pro Gly
            980                 985                 990

Ile Val Thr Val Lys Gly Met Ala Asp Gly Arg Glu Val Glu Ala Arg
        995                 1000                1005

Val Glu Val Ile Ala Leu Lys Ser Glu Leu Pro Val Val Lys Arg Ile
    1010                1015                1020

Ala Pro Asn Thr Asn Leu Asn Ser Val Asp Lys Ser Val Ser Tyr Val
1025                1030                1035                1040

Leu Thr Asp Gly Ser Val Gln Glu Tyr Glu Val Asp Lys Trp Glu Ile
                1045                1050                1055

Ala Glu Glu Asp Lys Ala Lys
            1060

<210>   38
<211>   1093
<212>   PRT
<213>   Streptococcus pneumoniae

<400>   38
Ile Ala Glu Glu Asp Lys Ala Lys Leu Ala Ile Pro Gly Ser Arg Ile
1               5                   10                  15

Gln Ala Thr Gly Tyr Leu Glu Gly Gln Pro Ile His Ala Thr Leu Val
            20                  25                  30

Val Glu Glu Gly Asn Pro Ala Ala Pro Val Val Pro Thr Val Thr Val
        35                  40                  45

Gly Gly Glu Ala Val Thr Gly Leu Thr Ser Arg Gln Pro Met Gln Tyr

50                  55                  60

Arg Thr Leu Ser Tyr Gly Ala Gln Leu Pro Glu Val Thr Ala Ser Ala
65                  70                  75                  80

Glu Asn Ala Asp Val Thr Val Leu Gln Ala Ser Ala Ala Asn Gly Met
                85                  90                  95

Arg Ala Ser Ile Phe Ile Gln Pro Lys Asp Gly Gly Pro Leu Gln Thr
            100                 105                 110

Tyr Ala Ile Gln Phe Leu Glu Glu Ala Pro Lys Ile Ala His Leu Ser
        115                 120                 125

Leu Gln Val Glu Lys Ala Asp Ser Leu Lys Glu Asp Gln Thr Val Lys
    130                 135                 140

Leu Ser Val Arg Ala His Tyr Gln Asp Gly Thr Gln Ala Val Leu Pro
145                 150                 155                 160

Ala Asp Lys Val Thr Phe Ser Thr Ser Gly Glu Gly Glu Val Ala Ile
                165                 170                 175

Arg Lys Gly Met Leu Glu Leu His Lys Pro Gly Ala Val Thr Leu Asn
            180                 185                 190

Ala Glu Tyr Glu Gly Ala Lys Gly Gln Val Glu Leu Thr Ile Gln Ala
        195                 200                 205

Asn Thr Glu Lys Lys Ile Ala Gln Ser Ile Arg Pro Val Asn Val Val
    210                 215                 220

Thr Asp Leu His Gln Glu Pro Ser Leu Pro Ala Thr Val Thr Val Glu
225                 230                 235                 240

Tyr Asp Lys Gly Phe Pro Lys Thr His Lys Val Thr Trp Gln Ala Ile
                245                 250                 255

Pro Lys Glu Lys Leu Asp Ser Tyr Gln Ile Phe Glu Val Leu Gly Lys
            260                 265                 270

Val Glu Gly Ile Asp Leu Glu Ala Arg Ala Lys Val Ser Val Glu Gly
        275                 280                 285

Ile Val Ser Val Glu Glu Val Ser Val Thr Thr Pro Ile Ala Glu Ala
    290                 295                 300

Pro Gln Leu Pro Glu Ser Val Arg Thr Tyr Asp Ser Asn Gly His Val
305                 310                 315                 320

Ser Ser Ala Lys Val Ala Trp Asp Ala Ile Arg Pro Glu Gln Tyr Ala
                325                 330                 335

Lys Glu Gly Val Phe Thr Val Asn Gly Arg Leu Glu Gly Thr Gln Leu
            340                 345                 350

Thr Thr Lys Leu His Val Arg Val Ser Ala Gln Thr Glu Gln Gly Ala
        355                 360                 365

Asn Ile Ser Asp Gln Trp Thr Gly Ser Glu Leu Pro Leu Ala Phe Ala
    370                 375                 380

Ser Asp Ser Asn Pro Ser Asp Pro Val Ser Asn Val Asn Asp Lys Leu

385                 390                 395                 400

Ile Ser Tyr Asn Asn Gln Pro Ala Asn Arg Trp Thr Asn Trp Asn Arg
                405                 410                 415

Ser Asn Pro Glu Ala Ser Val Gly Val Leu Phe Gly Asp Ser Gly Ile
            420                 425                 430

Leu Ser Lys Arg Ser Val Asp Asn Leu Ser Val Gly Phe His Glu Asp
        435                 440                 445

His Gly Val Gly Ala Pro Lys Ser Tyr Val Ile Glu Tyr Tyr Val Gly
    450                 455                 460

Lys Thr Val Pro Thr Ala Pro Lys Asn Pro Ser Phe Val Gly Asn Glu
465                 470                 475                 480

Asp His Val Phe Asn Asp Ser Ala Asn Trp Lys Pro Val Thr Asn Leu
                485                 490                 495

Lys Ala Pro Ala Gln Leu Lys Ala Gly Glu Met Asn His Phe Ser Phe
            500                 505                 510

Asp Lys Val Glu Thr Tyr Ala Ile Arg Ile Arg Met Val Lys Ala Asp
        515                 520                 525

Asn Lys Arg Gly Thr Ser Ile Thr Glu Val Gln Ile Phe Ala Lys Gln
    530                 535                 540

Val Ala Ala Ala Lys Gln Gly Gln Thr Arg Ile Gln Val Asp Gly Lys
545                 550                 555                 560

Asp Leu Ala Asn Phe Asn Pro Asp Leu Thr Asp Tyr Tyr Leu Glu Ser
                565                 570                 575

Val Asp Gly Lys Val Pro Ala Val Thr Ala Asn Val Ser Asn Asn Gly
            580                 585                 590

Leu Ala Thr Val Val Pro Ser Val Arg Glu Gly Glu Pro Val Arg Val
        595                 600                 605

Ile Ala Lys Ala Glu Asn Gly Asp Ile Leu Gly Glu Tyr Arg Leu His
    610                 615                 620

Phe Thr Lys Asp Lys Asn Leu Leu Ser His Lys Pro Val Ala Ala Val
625                 630                 635                 640

Lys Gln Ala Arg Leu Leu Gln Val Gly Gln Ala Leu Glu Leu Pro Thr
                645                 650                 655

Lys Val Pro Val Tyr Phe Thr Gly Lys Asp Gly Tyr Glu Thr Lys Asp
            660                 665                 670

Leu Thr Val Glu Trp Glu Glu Val Pro Ala Glu Asn Leu Thr Lys Ala
        675                 680                 685

Gly Gln Phe Thr Val Arg Gly Arg Val Leu Gly Ser Asn Leu Val Ala
    690                 695                 700

Glu Val Thr Val Arg Val Thr Asp Lys Leu Gly Glu Thr Leu Ser Asp
705                 710                 715                 720

Asn Pro Asn Tyr Asp Glu Asn Ser Asn Gln Ala Phe Ala Ser Ala Thr

                725                 730                 735

Asn Asp Ile Asp Lys Asn Ser His Asp Arg Val Asp Tyr Leu Asn Asp
            740                 745                 750

Gly Asp His Ser Glu Asn Arg Arg Trp Thr Asn Trp Ser Pro Thr Pro
        755                 760                 765

Ser Ser Asn Pro Glu Val Ser Ala Gly Val Ile Phe Arg Glu Asn Gly
    770                 775                 780

Lys Ile Val Glu Arg Thr Val Ala Gln Ala Lys Leu His Phe Phe Ala
785                 790                 795                 800

Asp Ser Gly Thr Asp Ala Pro Ser Lys Leu Val Leu Glu Arg Tyr Val
                805                 810                 815

Gly Pro Gly Phe Glu Val Pro Thr Tyr Tyr Ser Asn Tyr Gln Ala Tyr
            820                 825                 830

Glu Ser Gly His Pro Phe Asn Asn Pro Glu Asn Trp Glu Ala Val Pro
        835                 840                 845

Tyr Arg Ala Asp Lys Asp Ile Ala Ala Gly Asp Glu Ile Asn Val Thr
    850                 855                 860

Phe Lys Ala Val Lys Ala Lys Val Met Arg Trp Arg Met Glu Arg Lys
865                 870                 875                 880

Ala Asp Lys Ser Gly Val Ala Met Ile Glu Met Thr Phe Leu Ala Pro
                885                 890                 895

Ser Glu Leu Pro Gln Glu Ser Thr Gln Ser Lys Ile Leu Val Asp Gly
            900                 905                 910

Lys Glu Leu Ala Asp Phe Ala Glu Asn Arg Gln Asp Tyr Gln Ile Thr
        915                 920                 925

Tyr Lys Gly Gln Arg Pro Lys Val Ser Val Glu Glu Asn Asn Gln Val
    930                 935                 940

Ala Ser Thr Val Val Asp Ser Gly Glu Asp Ser Leu Pro Val Leu Val
945                 950                 955                 960

Arg Leu Val Ser Glu Ser Gly Lys Gln Val Lys Glu Tyr Arg Ile Gln
                965                 970                 975

Leu Thr Lys Glu Lys Pro Val Ser Ala Val Gln Glu Asp Leu Pro Lys
            980                 985                 990

Leu Glu Phe Val Glu Lys Asp Leu Ala Tyr Lys Thr Val Glu Lys Lys
        995                 1000                1005

Asp Ser Thr Leu Tyr Leu Gly Glu Thr Arg Val Glu Gln Glu Gly Lys
    1010                1015                1020

Val Gly Lys Glu Arg Ile Phe Thr Val Ile Asn Pro Asp Gly Ser Lys
1025                1030                1035                1040

Glu Glu Lys Leu Arg Glu Val Val Glu Val Pro Thr Asp Arg Ile Val
                1045                1050                1055

Leu Val Gly Thr Lys Pro Val Ala Gln Glu Ala Lys Lys Pro Gln Val

1060                1065                1070

Ser Glu Lys Ala Asp Thr Lys Pro Ile Asp Ser Ser Glu Ala Asp Gln
        1075                1080                1085

Thr Asn Lys Ala Gln
    1090

<210>   39
<211>   2233
<212>   PRT
<213>   Streptococcus pneumoniae

<400>   39
Met Gly Lys Gly His Trp Asn Arg Lys Arg Val Tyr Ser Ile Arg Lys
1               5                   10                  15

Phe Ala Val Gly Ala Cys Ser Val Met Ile Gly Thr Cys Ala Val Leu
            20                  25                  30

Leu Gly Gly Asn Ile Ala Gly Glu Ser Val Val Tyr Ala Asp Glu Thr
        35                  40                  45

Leu Ile Thr His Thr Ala Glu Lys Pro Lys Glu Glu Lys Met Ile Val
    50                  55                  60

Glu Glu Lys Ala Asp Lys Ala Leu Glu Thr Lys Asn Ile Val Glu Arg
65                  70                  75                  80

Thr Glu Gln Ser Glu Pro Ser Ser Thr Glu Ala Ile Ala Ser Glu Lys
                85                  90                  95

Lys Glu Asp Glu Ala Val Thr Pro Lys Glu Glu Lys Val Ser Ala Lys
            100                 105                 110

Pro Glu Glu Lys Ala Pro Arg Ile Glu Ser Gln Ala Ser Asn Gln Glu
        115                 120                 125

Lys Pro Leu Lys Glu Asp Ala Lys Ala Val Thr Asn Glu Glu Val Asn
    130                 135                 140

Gln Met Ile Glu Asp Arg Lys Val Asp Phe Asn Gln Asn Trp Tyr Phe
145                 150                 155                 160

Lys Leu Asn Ala Asn Ser Lys Glu Ala Ile Lys Pro Asp Ala Asp Val
                165                 170                 175

Ser Thr Trp Lys Lys Leu Asp Leu Pro Tyr Asp Trp Ser Ile Phe Asn
            180                 185                 190

Asp Phe Asp His Glu Ser Pro Ala Gln Asn Glu Gly Gly Gln Leu Asn
        195                 200                 205

Gly Gly Glu Ala Trp Tyr Arg Lys Thr Phe Lys Leu Asp Glu Lys Asp
    210                 215                 220

Leu Lys Lys Asn Val Arg Leu Thr Phe Asp Gly Val Tyr Met Asp Ser
225                 230                 235                 240

Gln Val Tyr Val Asn Gly Gln Leu Val Gly His Tyr Pro Asn Gly Tyr
                245                 250                 255

Asn Gln Phe Ser Tyr Asp Ile Thr Lys Tyr Leu Gln Lys Asp Gly Arg

260                 265                 270

Glu Asn Val Ile Ala Val His Ala Val Asn Lys Gln Pro Ser Ser Arg
        275                 280                 285

Trp Tyr Ser Gly Ser Gly Ile Tyr Arg Asp Val Thr Leu Gln Val Thr
    290                 295                 300

Asp Lys Val His Val Glu Lys Asn Gly Thr Thr Ile Leu Thr Pro Lys
305                 310                 315                 320

Leu Glu Glu Gln Gln His Gly Lys Val Glu Thr His Val Thr Ser Lys
                325                 330                 335

Ile Val Asn Thr Asp Asp Lys Asp His Glu Leu Val Ala Glu Tyr Gln
            340                 345                 350

Ile Val Glu Arg Gly Gly His Ala Val Thr Gly Leu Val Arg Thr Ala
        355                 360                 365

Ser Arg Thr Leu Lys Ala His Glu Ser Thr Ser Leu Asp Ala Ile Leu
    370                 375                 380

Glu Val Glu Arg Pro Lys Leu Trp Thr Val Leu Asn Asp Lys Pro Ala
385                 390                 395                 400

Leu Tyr Glu Leu Ile Thr Arg Val Tyr Arg Asp Gly Gln Leu Val Asp
                405                 410                 415

Ala Lys Lys Asp Leu Phe Gly Tyr Arg Tyr Tyr His Trp Thr Pro Asn
            420                 425                 430

Glu Gly Phe Ser Leu Asn Gly Glu Arg Ile Lys Phe His Gly Val Ser
        435                 440                 445

Leu His His Asp His Gly Ala Leu Gly Ala Glu Glu Asn Tyr Lys Ala
    450                 455                 460

Glu Tyr Arg Arg Leu Lys Gln Met Lys Glu Met Gly Val Asn Ser Ile
465                 470                 475                 480

Arg Thr Thr His Asn Pro Ala Ser Glu Gln Thr Leu Gln Ile Ala Ala
                485                 490                 495

Glu Leu Gly Leu Leu Val Gln Glu Glu Ala Phe Asp Thr Trp Tyr Gly
            500                 505                 510

Gly Lys Lys Pro Tyr Asp Tyr Gly Arg Phe Phe Glu Lys Asp Ala Thr
        515                 520                 525

His Pro Glu Ala Arg Lys Gly Glu Lys Trp Ser Asp Phe Asp Leu Arg
    530                 535                 540

Thr Met Val Glu Arg Gly Lys Asn Asn Pro Ala Ile Phe Met Trp Ser
545                 550                 555                 560

Ile Gly Asn Glu Ile Gly Glu Ala Asn Gly Asp Ala His Ser Leu Ala
                565                 570                 575

Thr Val Lys Arg Leu Val Lys Val Ile Lys Asp Val Asp Lys Thr Arg
            580                 585                 590

Tyr Val Thr Met Gly Ala Asp Lys Phe Arg Phe Gly Asn Gly Ser Gly

595                 600                 605

Gly His Glu Lys Ile Ala Asp Glu Leu Asp Ala Val Gly Phe Asn Tyr
    610                 615                 620

Ser Glu Asp Asn Tyr Lys Ala Leu Arg Ala Lys His Pro Lys Trp Leu
625                 630                 635                 640

Ile Tyr Gly Ser Glu Thr Ser Ser Ala Thr Arg Thr Arg Gly Ser Tyr
                645                 650                 655

Tyr Arg Pro Glu Arg Glu Leu Lys His Ser Asn Gly Pro Glu Arg Asn
            660                 665                 670

Tyr Glu Gln Ser Asp Tyr Gly Asn Asp Arg Val Gly Trp Gly Lys Thr
        675                 680                 685

Ala Thr Ala Ser Trp Thr Phe Asp Arg Asp Asn Ala Gly Tyr Ala Gly
    690                 695                 700

Gln Phe Ile Trp Thr Gly Thr Asp Tyr Ile Gly Glu Pro Thr Pro Trp
705                 710                 715                 720

His Asn Gln Asn Gln Thr Pro Val Lys Ser Ser Tyr Phe Gly Ile Val
                725                 730                 735

Asp Thr Ala Gly Ile Pro Lys His Asp Phe Tyr Leu Tyr Gln Ser Gln
            740                 745                 750

Trp Val Ser Val Lys Lys Lys Pro Met Val His Leu Leu Pro His Trp
        755                 760                 765

Asn Trp Glu Asn Lys Glu Leu Ala Ser Lys Val Ala Asp Ser Glu Gly
    770                 775                 780

Lys Ile Pro Val Arg Ala Tyr Ser Asn Ala Ser Ser Val Glu Leu Phe
785                 790                 795                 800

Leu Asn Gly Lys Ser Leu Gly Leu Lys Thr Phe Asn Lys Lys Gln Thr
                805                 810                 815

Ser Asp Gly Arg Thr Tyr Gln Glu Gly Ala Asn Ala Asn Glu Leu Tyr
            820                 825                 830

Leu Glu Trp Lys Val Ala Tyr Gln Pro Gly Thr Leu Glu Ala Ile Ala
        835                 840                 845

Arg Asp Glu Ser Gly Lys Glu Ile Ala Arg Asp Lys Ile Thr Thr Ala
    850                 855                 860

Gly Lys Pro Ala Ala Val Arg Leu Ile Lys Glu Asp His Ala Ile Ala
865                 870                 875                 880

Ala Asp Gly Lys Asp Leu Thr Tyr Ile Tyr Tyr Glu Ile Val Asp Ser
                885                 890                 895

Gln Gly Asn Val Val Pro Thr Ala Asn Asn Leu Val Arg Phe Gln Leu
            900                 905                 910

His Gly Gln Gly Gln Leu Val Gly Val Asp Asn Gly Glu Gln Ala Ser
        915                 920                 925

Arg Glu Arg Tyr Lys Ala Gln Ala Asp Gly Ser Trp Ile Arg Lys Ala

930                 935                 940

Phe Asn Gly Lys Gly Val Ala Ile Val Lys Ser Thr Glu Gln Ala Gly
945                 950                 955                 960

Lys Phe Thr Leu Thr Ala His Ser Asp Leu Leu Lys Ser Asn Gln Val
                965                 970                 975

Thr Val Phe Thr Gly Lys Lys Glu Gly Gln Glu Lys Thr Val Leu Gly
            980                 985                 990

Thr Glu Val Pro Lys Val Gln Thr Ile Ile Gly Glu Ala Pro Glu Met
        995                 1000                1005

Pro Thr Thr Val Pro Phe Val Tyr Ser Asp Gly Ser Arg Ala Glu Arg
    1010                1015                1020

Pro Val Thr Trp Ser Ser Val Asp Val Ser Lys Pro Gly Ile Val Thr
1025                1030                1035                1040

Val Lys Gly Met Ala Asp Gly Arg Glu Val Glu Ala Arg Val Glu Val
                1045                1050                1055

Ile Ala Leu Lys Ser Glu Leu Pro Val Val Lys Arg Ile Ala Pro Asn
            1060                1065                1070

Thr Asp Leu Asn Ser Val Asp Lys Ser Val Ser Tyr Val Leu Ile Asp
        1075                1080                1085

Gly Ser Val Glu Glu Tyr Glu Val Asp Lys Trp Glu Ile Ala Glu Glu
    1090                1095                1100

Asp Lys Ala Lys Leu Ala Ile Pro Gly Ser Arg Ile Gln Ala Thr Gly
1105                1110                1115                1120

Tyr Leu Glu Gly Gln Pro Ile His Ala Thr Leu Val Val Glu Glu Gly
                1125                1130                1135

Asn Pro Ala Ala Pro Ala Val Pro Thr Val Thr Val Gly Gly Glu Ala
            1140                1145                1150

Val Thr Gly Leu Thr Ser Gln Lys Pro Met Gln Tyr Arg Thr Leu Ala
        1155                1160                1165

Tyr Gly Ala Lys Leu Pro Glu Val Thr Ala Ser Ala Lys Asn Ala Ala
    1170                1175                1180

Val Thr Val Leu Gln Ala Ser Ala Ala Asn Gly Met Arg Ala Ser Ile
1185                1190                1195                1200

Phe Ile Gln Pro Lys Asp Gly Gly Pro Leu Gln Thr Tyr Ala Ile Gln
                1205                1210                1215

Phe Leu Glu Glu Ala Pro Lys Ile Ala His Leu Ser Leu Gln Val Glu
            1220                1225                1230

Lys Ala Asp Ser Leu Lys Glu Asp Gln Thr Val Lys Leu Ser Val Arg
        1235                1240                1245

Ala His Tyr Gln Asp Gly Thr Gln Ala Val Leu Pro Ala Asp Lys Val
    1250                1255                1260

Thr Phe Ser Thr Ser Gly Glu Gly Glu Val Ala Ile Arg Lys Gly Met

1265                1270                1275                1280

Leu Glu Leu His Lys Pro Gly Ala Val Thr Leu Asn Ala Glu Tyr Glu
                1285                1290                1295

Gly Ala Lys Asp Gln Val Glu Leu Thr Ile Gln Ala Asn Thr Glu Lys
            1300                1305                1310

Lys Ile Ala Gln Ser Ile Arg Pro Val Asn Val Val Thr Asp Leu His
        1315                1320                1325

Gln Glu Pro Ser Leu Pro Ala Thr Val Thr Val Glu Tyr Asp Lys Gly
    1330                1335                1340

Phe Pro Lys Thr His Lys Val Thr Trp Gln Ala Ile Pro Lys Glu Lys
1345                1350                1355                1360

Leu Asp Ser Tyr Gln Thr Phe Glu Val Leu Gly Lys Val Glu Gly Ile
                1365                1370                1375

Asp Leu Glu Ala Arg Ala Lys Val Ser Val Glu Gly Ile Val Ser Val
            1380                1385                1390

Glu Glu Val Ser Val Thr Thr Pro Ile Ala Glu Ala Pro Gln Leu Pro
        1395                1400                1405

Glu Ser Val Arg Thr Tyr Asp Ser Asn Gly His Val Ser Ser Ala Lys
    1410                1415                1420

Val Ala Trp Asp Ala Ile Arg Pro Glu Gln Tyr Ala Lys Glu Gly Val
1425                1430                1435                1440

Phe Thr Val Asn Gly Arg Leu Glu Gly Thr Gln Leu Thr Thr Lys Leu
                1445                1450                1455

His Val Arg Val Ser Ala Gln Thr Glu Gln Gly Ala Asn Ile Ser Asp
            1460                1465                1470

Gln Trp Thr Gly Ser Glu Leu Pro Leu Ala Phe Ala Ser Asp Ser Asn
        1475                1480                1485

Pro Ser Asp Pro Val Ser Asn Val Asn Asp Lys Leu Ile Ser Tyr Asn
    1490                1495                1500

Asn Gln Pro Ala Asn Arg Trp Thr Asn Trp Asn Arg Thr Asn Pro Glu
1505                1510                1515                1520

Ala Ser Val Gly Val Leu Phe Gly Asp Ser Gly Ile Leu Ser Lys Arg
                1525                1530                1535

Ser Val Asp Asn Leu Ser Val Gly Phe His Glu Asp His Gly Val Gly
            1540                1545                1550

Val Pro Lys Ser Tyr Val Ile Glu Tyr Tyr Val Gly Lys Thr Val Pro
        1555                1560                1565

Thr Ala Pro Lys Asn Pro Ser Phe Val Gly Asn Glu Asp His Val Phe
    1570                1575                1580

Asn Asp Ser Ala Asn Trp Lys Pro Val Thr Asn Leu Lys Ala Pro Ala
1585                1590                1595                1600

Gln Leu Lys Ala Gly Glu Met Asn His Phe Ser Phe Asp Lys Val Glu

1605                1610                1615

Thr Tyr Ala Val Arg Ile Arg Met Val Lys Ala Asp Asn Lys Arg Gly
        1620                1625                1630

Thr Ser Ile Thr Glu Val Gln Ile Phe Ala Lys Gln Val Ala Ala Ala
    1635                1640                1645

Lys Gln Gly Gln Thr Arg Ile Gln Val Asp Gly Lys Asp Leu Ala Asn
    1650                1655                1660

Phe Asn Pro Asp Leu Thr Asp Tyr Tyr Leu Glu Ser Val Asp Gly Lys
1665                1670                1675                1680

Val Pro Ala Val Thr Ala Ser Val Ser Asn Asn Gly Leu Ala Thr Val
                1685                1690                1695

Val Pro Ser Val Arg Glu Gly Glu Pro Val Arg Val Ile Ala Lys Ala
        1700                1705                1710

Glu Asn Gly Asp Ile Leu Gly Glu Tyr Arg Leu His Phe Thr Lys Asp
    1715                1720                1725

Lys Ser Leu Leu Ser His Lys Pro Val Ala Ala Val Lys Gln Ala Arg
    1730                1735                1740

Leu Leu Gln Val Gly Gln Ala Leu Glu Leu Pro Thr Lys Val Pro Val
1745                1750                1755                1760

Tyr Phe Thr Gly Lys Asp Gly Tyr Glu Thr Lys Asp Leu Thr Val Glu
                1765                1770                1775

Trp Glu Glu Val Pro Ala Glu Asn Leu Thr Lys Ala Gly Gln Phe Thr
        1780                1785                1790

Val Arg Gly Arg Val Leu Gly Ser Asn Leu Val Ala Glu Ile Thr Val
    1795                1800                1805

Arg Val Thr Asp Lys Leu Gly Glu Thr Leu Ser Asp Asn Pro Asn Tyr
    1810                1815                1820

Asp Glu Asn Ser Asn Gln Ala Phe Ala Ser Ala Thr Asn Asp Ile Asp
1825                1830                1835                1840

Lys Asn Ser His Asp Arg Val Asp Tyr Leu Asn Asp Gly Asp His Ser
                1845                1850                1855

Glu Asn Arg Arg Trp Thr Asn Trp Ser Pro Thr Pro Ser Ser Asn Pro
        1860                1865                1870

Glu Val Ser Ala Gly Val Ile Phe Arg Glu Asn Gly Lys Ile Val Glu
    1875                1880                1885

Arg Thr Val Thr Gln Gly Lys Val Gln Phe Phe Ala Asp Ser Gly Thr
    1890                1895                1900

Asp Ala Pro Ser Lys Leu Val Leu Glu Arg Tyr Val Gly Pro Glu Phe
1905                1910                1915                1920

Glu Val Pro Thr Tyr Tyr Ser Asn Tyr Gln Ala Tyr Asp Ala Asp His
                1925                1930                1935

Pro Phe Asn Asn Pro Glu Asn Trp Glu Ala Val Pro Tyr Arg Ala Asp

1940                1945                1950

Lys Asp Ile Ala Ala Gly Asp Glu Ile Asn Val Thr Phe Lys Ala Ile
        1955                1960                1965

Lys Ala Lys Ala Met Arg Trp Arg Met Glu Arg Lys Ala Asp Lys Ser
    1970                1975                1980

Gly Val Ala Met Ile Glu Met Thr Phe Leu Ala Pro Ser Glu Leu Pro
1985                1990                1995                2000

Gln Glu Ser Thr Gln Ser Lys Ile Leu Val Asp Gly Lys Glu Leu Ala
                2005                2010                2015

Asp Phe Ala Glu Asn Arg Gln Asp Tyr Gln Ile Thr Tyr Lys Gly Gln
            2020                2025                2030

Arg Pro Lys Val Ser Val Glu Glu Asn Asn Gln Val Ala Ser Thr Val
        2035                2040                2045

Val Asp Ser Gly Glu Asp Ser Phe Pro Val Leu Val Arg Leu Val Ser
    2050                2055                2060

Glu Ser Gly Lys Gln Val Lys Glu Tyr Arg Ile His Leu Thr Lys Glu
2065                2070                2075                2080

Lys Pro Val Ser Glu Lys Thr Val Ala Ala Val Gln Glu Asp Leu Pro
                2085                2090                2095

Lys Ile Glu Phe Val Glu Lys Asp Leu Ala Tyr Lys Thr Val Glu Lys
            2100                2105                2110

Lys Asp Ser Thr Leu Tyr Leu Gly Glu Thr Arg Val Glu Gln Glu Gly
        2115                2120                2125

Lys Val Gly Lys Glu Arg Ile Phe Thr Ala Ile Asn Pro Asp Gly Ser
    2130                2135                2140

Lys Glu Glu Lys Leu Arg Glu Val Val Glu Val Pro Thr Asp Arg Ile
2145                2150                2155                2160

Val Leu Val Gly Thr Lys Pro Val Ala Gln Glu Ala Lys Lys Pro Gln
                2165                2170                2175

Val Ser Glu Lys Ala Asp Thr Lys Pro Ile Asp Ser Ser Glu Ala Ser
            2180                2185                2190

Gln Thr Asn Lys Ala Gln Leu Pro Ser Thr Gly Ser Ala Ala Ser Gln
        2195                2200                2205

Ala Ala Val Ala Ala Gly Leu Thr Leu Leu Gly Leu Ser Ala Gly Leu
    2210                2215                2220

Val Val Thr Lys Gly Lys Lys Glu Asp
2225                2230

<210>  40
<211>  218
<212>  PRT
<213>  Streptococcus pneumoniae

<400>  40
Ala Leu Ile Phe Asn Thr Gln Ile Arg Asn Ile Phe Ile Val Trp Asn

1               5                   10                  15

Thr Asn Lys Tyr Gln Val Ser Gln Val Ser Lys Glu Lys Leu Glu Glu
            20                  25                  30

Asn Gln Asp Thr Glu Gly Asn Phe Asp Phe Asp Ser Val Lys Ala Ile
        35                  40                  45

Ser Ser Glu Ala Val Leu Thr Ser Gln Trp Asp Ala Gln Lys Leu Pro
    50                  55                  60

Val Ile Gly Gly Ile Ala Ile Pro Glu Leu Glu Met Asn Leu Pro Ile
65                  70                  75                  80

Phe Lys Gly Leu Asp Asn Val Asn Leu Phe Tyr Gly Ala Gly Thr Met
                85                  90                  95

Lys Arg Glu Gln Val Met Gly Glu Gly Asn Tyr Ser Leu Ala Ser His
            100                 105                 110

His Ile Phe Gly Val Asp Asn Ala Asn Lys Met Leu Phe Ser Pro Leu
        115                 120                 125

Asp Asn Ala Lys Asn Gly Met Lys Ile Tyr Leu Thr Asp Lys Asn Lys
    130                 135                 140

Val Tyr Thr Tyr Glu Ile Arg Glu Val Lys Arg Val Thr Pro Asp Arg
145                 150                 155                 160

Val Asp Glu Val Asp Asp Arg Asp Gly Val Asn Glu Ile Thr Leu Val
                165                 170                 175

Thr Cys Glu Asp Leu Ala Ala Thr Glu Arg Ile Ile Val Lys Gly Asp
            180                 185                 190

Leu Lys Glu Thr Lys Asp Tyr Ser Gln Thr Ser Asp Glu Ile Leu Thr
        195                 200                 205

Ala Phe Asn Gln Pro Tyr Lys Gln Phe Tyr
    210                 215

<210>   41
<211>   165
<212>   PRT
<213>   Streptococcus pneumoniae

<400>   41
Val Pro Lys Thr Ala Thr Ser Thr Glu Thr Lys Thr Ile Thr Arg Ile
1               5                   10                  15

Ile His Tyr Val Asp Lys Val Thr Asn Gln Asn Val Lys Glu Asp Val
            20                  25                  30

Val Gln Pro Val Thr Leu Ser Arg Thr Lys Thr Glu Asn Lys Val Thr
        35                  40                  45

Gly Val Val Thr Tyr Gly Glu Trp Thr Thr Gly Asn Trp Asp Glu Val
    50                  55                  60

Ile Ser Gly Lys Ile Asp Lys Tyr Lys Asp Pro Asp Ile Pro Thr Val
65                  70                  75                  80

Glu Ser Gln Glu Val Thr Ser Asp Ser Ser Asp Lys Glu Ile Thr Val

85                  90                  95

Arg Tyr Asp Arg Leu Ser Thr Pro Glu Lys Pro Ile Pro Gln Pro Asn
            100                 105                 110

Pro Glu His Pro Ser Val Pro Thr Pro Asn Pro Glu Leu Pro Asn Gln
        115                 120                 125

Glu Thr Pro Thr Pro Asp Lys Pro Thr Pro Glu Pro Gly Thr Pro Lys
    130                 135                 140

Thr Glu Thr Pro Val Asn Pro Asp Pro Glu Val Pro Thr Tyr Glu Thr
145                 150                 155                 160

Gly Lys Arg Glu Glu
                165

<210>   42
<211>   680
<212>   PRT
<213>   Streptococcus pneumoniae

<400>   42
Ala Ser Asp Gly Thr Trp Gln Gly Lys Gln Tyr Leu Lys Glu Asp Gly
1               5                   10                  15

Ser Gln Ala Ala Asn Glu Trp Val Phe Asp Thr His Tyr Gln Ser Trp
            20                  25                  30

Phe Tyr Ile Lys Ala Asp Ala Asn Tyr Ala Glu Asn Glu Trp Leu Lys
        35                  40                  45

Gln Gly Asp Asp Tyr Phe Tyr Leu Lys Ser Gly Gly Tyr Met Ala Lys
    50                  55                  60

Ser Glu Trp Val Glu Asp Lys Gly Ala Phe Tyr Tyr Leu Asp Gln Asp
65                  70                  75                  80

Gly Lys Met Lys Arg Asn Ala Trp Val Gly Thr Ser Tyr Val Gly Ala
                85                  90                  95

Thr Gly Ala Lys Val Ile Glu Asp Trp Val Tyr Asp Ser Gln Tyr Asp
            100                 105                 110

Ala Trp Phe Tyr Ile Lys Ala Asp Gly Gln His Ala Glu Lys Glu Trp
        115                 120                 125

Leu Gln Ile Lys Gly Lys Asp Tyr Tyr Phe Lys Ser Gly Gly Tyr Leu
    130                 135                 140

Leu Thr Ser Gln Trp Ile Asn Gln Ala Tyr Val Asn Ala Ser Gly Ala
145                 150                 155                 160

Lys Val Gln Gln Gly Trp Leu Phe Asp Lys Gln Tyr Gln Ser Trp Phe
                165                 170                 175

Tyr Ile Lys Glu Asn Gly Asn Tyr Ala Asp Lys Glu Trp Ile Phe Glu
            180                 185                 190

Asn Gly His Tyr Tyr Tyr Leu Lys Ser Gly Gly Tyr Met Ala Ala Asn
        195                 200                 205

Glu Trp Ile Trp Asp Lys Glu Ser Trp Phe Tyr Leu Lys Phe Asp Gly

210                 215                 220

Lys Ile Ala Glu Lys Glu Trp Val Tyr Asp Ser His Ser Gln Ala Trp
225                 230                 235                 240

Tyr Tyr Phe Lys Ser Gly Gly Tyr Met Ala Ala Asn Glu Trp Ile Trp
                245                 250                 255

Asp Lys Glu Ser Trp Phe Tyr Leu Lys Phe Asp Gly Lys Met Ala Glu
            260                 265                 270

Lys Glu Trp Val Tyr Asp Ser His Ser Gln Ala Trp Tyr Tyr Phe Lys
        275                 280                 285

Ser Gly Gly Tyr Met Thr Ala Asn Glu Trp Ile Trp Asp Lys Glu Ser
    290                 295                 300

Trp Phe Tyr Leu Lys Ser Asp Gly Lys Ile Ala Glu Lys Glu Trp Val
305                 310                 315                 320

Tyr Asp Ser His Ser Gln Ala Trp Tyr Tyr Phe Lys Ser Gly Gly Tyr
                325                 330                 335

Met Thr Ala Asn Glu Trp Ile Trp Asp Lys Glu Ser Trp Phe Tyr Leu
            340                 345                 350

Lys Ser Asp Gly Lys Met Ala Glu Lys Glu Trp Val Tyr Asp Ser His
        355                 360                 365

Ser Gln Ala Trp Tyr Tyr Phe Lys Ser Gly Gly Tyr Met Ala Lys Asn
    370                 375                 380

Glu Thr Val Asp Gly Tyr Gln Leu Gly Ser Asp Gly Lys Trp Leu Gly
385                 390                 395                 400

Gly Lys Ala Thr Asn Lys Asn Ala Ala Tyr Tyr Gln Val Val Pro Val
                405                 410                 415

Thr Ala Asn Val Tyr Asp Ser Asp Gly Glu Lys Leu Ser Tyr Ile Ser
            420                 425                 430

Gln Gly Ser Val Val Trp Leu Asp Lys Asp Arg Lys Ser Asp Asp Lys
        435                 440                 445

Arg Leu Ala Ile Thr Ile Ser Gly Leu Ser Gly Tyr Met Lys Thr Glu
    450                 455                 460

Asp Leu Gln Ala Leu Asp Ala Ser Lys Asp Phe Ile Pro Tyr Tyr Glu
465                 470                 475                 480

Ser Asp Gly His Arg Phe Tyr His Tyr Val Ala Gln Asn Ala Ser Ile
                485                 490                 495

Pro Val Ala Ser His Leu Ser Asp Met Glu Val Gly Lys Lys Tyr Tyr
            500                 505                 510

Ser Ala Asp Gly Leu His Phe Asp Gly Phe Lys Leu Glu Asn Pro Phe
        515                 520                 525

Leu Phe Lys Asp Leu Thr Glu Ala Thr Asn Tyr Ser Ala Glu Glu Leu
    530                 535                 540

Asp Lys Val Phe Ser Leu Leu Asn Ile Asn Asn Ser Leu Leu Glu Asn

545                 550                 555                 560

Lys Gly Ala Thr Phe Lys Glu Ala Glu Glu His Tyr His Ile Asn Ala
                565                 570                 575

Leu Tyr Leu Leu Ala His Ser Ala Leu Glu Ser Asn Trp Gly Arg Ser
            580                 585                 590

Lys Ile Ala Lys Asp Lys Asn Asn Phe Phe Gly Ile Thr Ala Tyr Asp
        595                 600                 605

Thr Thr Pro Tyr Leu Ser Ala Lys Thr Phe Asp Asp Val Asp Lys Gly
    610                 615                 620

Ile Leu Gly Ala Thr Lys Trp Ile Lys Glu Asn Tyr Ile Asp Arg Gly
625                 630                 635                 640

Arg Thr Phe Leu Gly Asn Lys Ala Ser Gly Met Asn Val Glu Tyr Ala
                645                 650                 655

Ser Asp Pro Tyr Trp Gly Glu Lys Ile Ala Ser Val Met Met Lys Ile
            660                 665                 670

Asn Glu Lys Leu Gly Gly Lys Asp
        675                 680

<210>   43
<211>   469
<212>   PRT
<213>   Streptococcus pneumoniae

<400>   43
Ala Asn Glu Thr Glu Val Ala Lys Thr Ser Gln Asp Thr Thr Thr Ala
1               5                   10                  15

Ser Ser Ser Ser Glu Gln Asn Gln Ser Ser Asn Lys Thr Gln Thr Ser
            20                  25                  30

Ala Glu Val Gln Thr Asn Ala Ala Ala Tyr Trp Asp Gly Asp Tyr Tyr
        35                  40                  45

Val Lys Asp Asp Gly Ser Lys Ala Gln Ser Glu Trp Ile Phe Asp Asn
    50                  55                  60

Tyr Tyr Lys Ala Trp Phe Tyr Ile Asn Ser Asp Gly Arg Tyr Ser Gln
65                  70                  75                  80

Asn Glu Trp His Gly Asn Tyr Tyr Leu Lys Ser Gly Gly Tyr Met Ala
                85                  90                  95

Gln Asn Glu Trp Ile Tyr Asp Ser Asn Tyr Lys Ser Trp Phe Tyr Leu
            100                 105                 110

Lys Ser Asp Gly Ala Tyr Ala His Gln Glu Trp Gln Leu Ile Gly Asn
        115                 120                 125

Lys Trp Tyr Tyr Phe Lys Lys Trp Gly Tyr Met Ala Lys Ser Gln Trp
    130                 135                 140

Gln Gly Ser Tyr Phe Leu Asn Gly Gln Gly Ala Met Ile Gln Asn Glu
145                 150                 155                 160

Trp Leu Tyr Asp Pro Ala Tyr Ser Ala Tyr Phe Tyr Leu Lys Ser Asp

165                 170                 175

Gly Thr Tyr Ala Asn Gln Glu Trp Gln Lys Val Gly Gly Lys Trp Tyr
            180                 185                 190

Tyr Phe Lys Lys Trp Gly Tyr Met Ala Arg Asn Glu Trp Gln Gly Asn
        195                 200                 205

Tyr Tyr Leu Thr Gly Ser Gly Ala Met Ala Thr Asp Glu Val Ile Met
    210                 215                 220

Asp Gly Ala Arg Tyr Ile Phe Ala Ala Ser Gly Glu Leu Lys Glu Lys
225                 230                 235                 240

Lys Asp Leu Asn Val Gly Trp Val His Arg Asp Gly Lys Arg Tyr Phe
                245                 250                 255

Phe Asn Asn Arg Glu Glu Gln Val Gly Thr Glu His Ala Lys Lys Ile
            260                 265                 270

Ile Asp Ile Ser Glu His Asn Gly Arg Ile Asn Asp Trp Lys Lys Val
        275                 280                 285

Ile Asp Glu Asn Lys Val Asp Gly Val Ile Val Arg Leu Gly Tyr Ser
    290                 295                 300

Gly Lys Glu Asp Lys Glu Leu Ala His Asn Ile Lys Glu Leu Asn Arg
305                 310                 315                 320

Leu Gly Ile Pro Tyr Gly Val Tyr Leu Tyr Thr Tyr Ala Glu Asn Glu
                325                 330                 335

Thr Asp Ala Glu Asn Asp Ala Lys Gln Thr Ile Glu Leu Ile Lys Lys
            340                 345                 350

Tyr Asn Met Asn Leu Ser Tyr Pro Ile Tyr Tyr Asp Val Glu Asn Trp
        355                 360                 365

Glu Tyr Val Asn Lys Ser Lys Arg Ala Pro Ser Asp Thr Asp Thr Trp
    370                 375                 380

Val Lys Ile Ile Asn Lys Tyr Met Asp Thr Met Lys Gln Ala Gly Tyr
385                 390                 395                 400

Gln Asn Val Tyr Val Tyr Ser Tyr Arg Ser Leu Leu Gln Thr Arg Leu
                405                 410                 415

Lys His Pro Asp Ile Leu Lys His Val Asn Trp Val Ala Ala Tyr Thr
            420                 425                 430

Asn Ala Leu Glu Trp Glu Asn Pro Tyr Tyr Ser Gly Glu Lys Gly Trp
        435                 440                 445

Gln Tyr Thr Ser Ser Glu Tyr Met Lys Gly Ile Gln Gly Arg Val Asp
    450                 455                 460

Val Ser Val Trp Tyr
465

<210>   44
<211>   471
<212>   PRT
<213>   Streptococcus pneumoniae

<400>   44
Met Ala Asn Lys Ala Val Asn Asp Phe Ile Leu Ala Met Asn Tyr Asp
1               5                   10                  15

Lys Lys Lys Leu Leu Thr His Gln Gly Glu Ser Ile Glu Asn Arg Phe
            20                  25                  30

Ile Lys Glu Gly Asn Gln Leu Pro Asp Glu Phe Val Val Ile Glu Arg
        35                  40                  45

Lys Lys Arg Ser Leu Ser Thr Asn Thr Ser Asp Ile Ser Val Thr Ala
    50                  55                  60

Thr Asn Asp Ser Arg Leu Tyr Pro Gly Ala Leu Leu Val Val Asp Glu
65                  70                  75                  80

Thr Leu Leu Glu Asn Asn Pro Thr Leu Leu Ala Val Asp Arg Ala Pro
                85                  90                  95

Met Thr Tyr Ser Ile Asp Leu Pro Gly Leu Ala Ser Ser Asp Ser Phe
            100                 105                 110

Leu Gln Val Glu Asp Pro Ser Asn Ser Ser Val Arg Gly Ala Val Asn
        115                 120                 125

Asp Leu Leu Ala Lys Trp His Gln Asp Tyr Gly Gln Val Asn Asn Val
    130                 135                 140

Pro Ala Arg Met Gln Tyr Glu Lys Ile Thr Ala His Ser Met Glu Gln
145                 150                 155                 160

Leu Lys Val Lys Phe Gly Ser Asp Phe Glu Lys Thr Gly Asn Ser Leu
                165                 170                 175

Asp Ile Asp Phe Asn Ser Val His Ser Gly Glu Lys Gln Ile Gln Ile
            180                 185                 190

Val Asn Phe Lys Gln Ile Tyr Tyr Thr Val Ser Val Asp Ala Val Lys
        195                 200                 205

Asn Pro Gly Asp Val Phe Gln Asp Thr Val Thr Val Glu Asp Leu Lys
    210                 215                 220

Gln Arg Gly Ile Ser Ala Glu Arg Pro Leu Val Tyr Ile Ser Ser Val
225                 230                 235                 240

Ala Tyr Gly Arg Gln Val Tyr Leu Lys Leu Glu Thr Thr Ser Lys Ser
                245                 250                 255

Asp Glu Val Glu Ala Ala Phe Glu Ala Leu Ile Lys Gly Val Lys Val
            260                 265                 270

Ala Pro Gln Thr Glu Trp Lys Gln Ile Leu Asp Asn Thr Glu Val Lys
        275                 280                 285

Ala Val Ile Leu Gly Gly Asp Pro Ser Ser Gly Ala Arg Val Val Thr
    290                 295                 300

Gly Lys Val Asp Met Val Glu Asp Leu Ile Gln Glu Gly Ser Arg Phe
305                 310                 315                 320

Thr Ala Asp His Leu Gly Leu Pro Ile Ser Tyr Thr Thr Ser Phe Leu

325                 330                 335

Arg Asp Asn Val Val Ala Thr Phe Gln Asn Ser Thr Asp Tyr Val Glu
            340                 345                 350

Thr Lys Val Thr Ala Tyr Arg Asn Gly Asp Leu Leu Leu Asp His Ser
        355                 360                 365

Gly Ala Tyr Val Ala Gln Tyr Tyr Ile Thr Trp Asn Glu Leu Ser Tyr
    370                 375                 380

Asp His Gln Gly Lys Glu Val Leu Thr Pro Lys Ala Trp Asp Arg Asn
385                 390                 395                 400

Gly Gln Asp Leu Thr Ala His Phe Thr Thr Ser Ile Pro Leu Lys Gly
                405                 410                 415

Asn Val Arg Asn Leu Ser Val Lys Ile Arg Glu Cys Thr Gly Leu Ala
            420                 425                 430

Trp Glu Trp Trp Arg Thr Val Tyr Glu Lys Thr Asp Leu Pro Leu Val
        435                 440                 445

Arg Lys Arg Thr Ile Ser Ile Trp Gly Thr Thr Leu Tyr Pro Gln Val
    450                 455                 460

Glu Asp Lys Val Glu Asn Asp
465                 470

<210>   45
<211>   471
<212>   PRT
<213>   Streptococcus pneumoniae

<400>   45
Met Ala Asn Lys Ala Val Asn Asp Phe Ile Leu Ala Met Asn Tyr Asp
1               5                   10                  15

Lys Lys Lys Leu Leu Thr His Gln Gly Glu Ser Ile Glu Asn Arg Phe
            20                  25                  30

Ile Lys Glu Gly Asn Gln Leu Pro Asp Glu Phe Val Val Ile Glu Arg
        35                  40                  45

Lys Lys Arg Ser Leu Ser Thr Asn Thr Ser Asp Ile Ser Val Thr Ala
    50                  55                  60

Thr Asn Asp Ser Arg Leu Tyr Pro Gly Ala Leu Leu Val Val Asp Glu
65                  70                  75                  80

Thr Leu Leu Glu Asn Asn Pro Thr Leu Leu Ala Val Asp Arg Ala Pro
                85                  90                  95

Met Thr Tyr Ser Ile Asp Leu Pro Gly Leu Ala Ser Ser Asp Ser Phe
            100                 105                 110

Leu Gln Val Glu Asp Pro Ser Asn Ser Ser Val Arg Gly Ala Val Asn
        115                 120                 125

Asp Leu Leu Ala Lys Trp His Gln Asp Tyr Gly Gln Val Asn Asn Val
    130                 135                 140

Pro Ala Arg Met Gln Tyr Glu Lys Ile Thr Ala His Ser Met Glu Gln

145                 150                 155                 160

Leu Lys Val Lys Phe Gly Ser Asp Phe Glu Lys Thr Gly Asn Ser Leu
                165                 170                 175

Asp Ile Asp Phe Asn Ser Val His Ser Gly Glu Lys Gln Ile Gln Ile
            180                 185                 190

Val Asn Phe Lys Gln Ile Tyr Tyr Thr Val Ser Val Asp Ala Val Lys
        195                 200                 205

Asn Pro Gly Asp Val Phe Gln Asp Thr Val Thr Val Glu Asp Leu Lys
    210                 215                 220

Gln Arg Gly Ile Ser Ala Glu Arg Pro Leu Val Tyr Ile Ser Ser Val
225                 230                 235                 240

Ala Tyr Gly Arg Gln Val Tyr Leu Lys Leu Glu Thr Thr Ser Lys Ser
                245                 250                 255

Asp Glu Val Glu Ala Ala Phe Glu Ala Leu Ile Lys Gly Val Lys Val
            260                 265                 270

Ala Pro Gln Thr Glu Trp Lys Gln Ile Leu Asp Asn Thr Glu Val Lys
        275                 280                 285

Ala Val Ile Leu Gly Gly Asp Pro Ser Ser Gly Ala Arg Val Val Thr
    290                 295                 300

Gly Lys Val Asp Met Val Glu Asp Leu Ile Gln Glu Gly Ser Arg Phe
305                 310                 315                 320

Thr Ala Asp His Leu Gly Leu Pro Ile Ser Tyr Thr Thr Ser Phe Leu
                325                 330                 335

Arg Asp Asn Val Val Ala Thr Phe Gln Asn Ser Thr Asp Tyr Val Glu
            340                 345                 350

Thr Lys Val Thr Ala Tyr Arg Asn Gly Asp Leu Leu Leu Asp His Ser
        355                 360                 365

Gly Ala Tyr Val Ala Gln Tyr Tyr Ile Thr Trp Asn Glu Leu Ser Tyr
    370                 375                 380

Asp His Gln Gly Lys Glu Val Leu Thr Pro Lys Ala Trp Asp Arg Asn
385                 390                 395                 400

Gly Gln Asp Leu Thr Ala His Phe Thr Thr Ser Ile Pro Leu Lys Gly
                405                 410                 415

Asn Val Arg Asn Leu Ser Val Lys Ile Arg Glu Cys Thr Gly Leu Ala
            420                 425                 430

Phe Glu Trp Trp Arg Thr Val Tyr Glu Lys Thr Asp Leu Pro Leu Val
        435                 440                 445

Arg Lys Arg Thr Ile Ser Ile Trp Gly Thr Thr Leu Tyr Pro Gln Val
    450                 455                 460

Glu Asp Lys Val Glu Asn Asp
465                 470

<210>  46

<211>   464
<212>   PRT
<213>   Streptococcus pneumoniae

<400>   46
Met Ala Asn Lys Ala Val Asn Asp Phe Ile Leu Ala Met Asn Tyr Asp
1               5                   10                  15

Lys Lys Lys Leu Leu Thr His Gln Gly Glu Ser Ile Glu Asn Arg Phe
            20                  25                  30

Ile Lys Glu Gly Asn Gln Leu Pro Asp Glu Phe Val Val Ile Glu Arg
        35                  40                  45

Lys Lys Arg Ser Leu Ser Thr Asn Thr Ser Asp Ile Ser Val Thr Ala
    50                  55                  60

Thr Asn Asp Ser Arg Leu Tyr Pro Gly Ala Leu Leu Val Val Asp Glu
65                  70                  75                  80

Thr Leu Leu Glu Asn Asn Pro Thr Leu Leu Ala Val Asp Arg Ala Pro
                85                  90                  95

Met Thr Tyr Ser Ile Asp Leu Pro Gly Leu Ala Ser Ser Asp Ser Phe
            100                 105                 110

Leu Gln Val Glu Asp Pro Ser Asn Ser Ser Val Arg Gly Ala Val Asn
        115                 120                 125

Asp Leu Leu Ala Lys Trp His Gln Asp Tyr Gly Gln Val Asn Asn Val
    130                 135                 140

Pro Ala Arg Met Gln Tyr Glu Lys Ile Thr Ala His Ser Met Glu Gln
145                 150                 155                 160

Leu Lys Val Lys Phe Gly Ser Asp Phe Glu Lys Thr Gly Asn Ser Leu
                165                 170                 175

Asp Ile Asp Phe Asn Ser Val His Ser Gly Glu Lys Gln Ile Gln Ile
            180                 185                 190

Val Asn Phe Lys Gln Ile Tyr Tyr Thr Val Ser Val Asp Ala Val Lys
        195                 200                 205

Asn Pro Gly Asp Val Phe Gln Asp Thr Val Thr Val Glu Asp Leu Lys
    210                 215                 220

Gln Arg Gly Ile Ser Ala Glu Arg Pro Leu Val Tyr Ile Ser Ser Val
225                 230                 235                 240

Ala Tyr Gly Arg Gln Val Tyr Leu Lys Leu Glu Thr Thr Ser Lys Ser
                245                 250                 255

Asp Glu Val Glu Ala Ala Phe Glu Ala Leu Ile Lys Gly Val Lys Val
            260                 265                 270

Ala Pro Gln Thr Glu Trp Lys Gln Ile Leu Asp Asn Thr Glu Val Lys
        275                 280                 285

Ala Val Ile Leu Gly Gly Asp Pro Ser Ser Gly Ala Arg Val Val Thr
    290                 295                 300

Gly Lys Val Asp Met Val Glu Asp Leu Ile Gln Glu Gly Ser Arg Phe

305                 310                 315                 320

Thr Ala Asp His Leu Gly Leu Pro Ile Ser Tyr Thr Thr Ser Phe Leu
                325                 330                 335

Arg Asp Asn Val Val Ala Thr Phe Gln Asn Ser Thr Asp Tyr Val Glu
            340                 345                 350

Thr Lys Val Thr Ala Tyr Arg Asn Gly Asp Leu Leu Leu Asp His Ser
        355                 360                 365

Gly Ala Tyr Val Ala Gln Tyr Tyr Ile Thr Trp Asn Glu Leu Ser Tyr
    370                 375                 380

Asp His Gln Gly Lys Glu Val Leu Thr Pro Lys Ala Trp Asp Arg Asn
385                 390                 395                 400

Gly Gln Asp Leu Thr Ala His Phe Thr Thr Ser Ile Pro Leu Lys Gly
                405                 410                 415

Asn Val Arg Asn Leu Ser Val Lys Ile Arg Glu Cys Thr Gly Leu Ala
            420                 425                 430

Trp Glu Trp Trp Arg Thr Val Tyr Glu Lys Thr Asp Leu Pro Leu Val
        435                 440                 445

Arg Lys Arg Thr Ile Ser Ile Trp Gly Thr Thr Leu Tyr Pro Gln Val
    450                 455                 460

<210>   47
<211>   366
<212>   PRT
<213>   Streptococcus pneumoniae

<400>   47
Ala Glu Thr Thr Asp Asp Lys Ile Ala Ala Gln Asp Asn Lys Ile Ser
1               5                   10                  15

Asn Leu Thr Ala Gln Gln Gln Glu Ala Gln Lys Gln Val Asp Gln Ile
            20                  25                  30

Gln Glu Gln Val Ser Ala Ile Gln Ala Glu Gln Ser Asn Leu Gln Ala
        35                  40                  45

Glu Asn Asp Arg Leu Gln Ala Glu Ser Lys Lys Leu Glu Gly Glu Ile
    50                  55                  60

Thr Glu Leu Ser Lys Asn Ile Val Ser Arg Asn Gln Ser Leu Glu Lys
65                  70                  75                  80

Gln Ala Arg Ser Ala Gln Thr Asn Gly Ala Val Thr Ser Tyr Ile Asn
                85                  90                  95

Thr Ile Val Asn Ser Lys Ser Ile Thr Glu Ala Ile Ser Arg Val Ala
            100                 105                 110

Ala Met Ser Glu Ile Val Ser Ala Asn Asn Lys Met Leu Glu Gln Gln
        115                 120                 125

Lys Ala Asp Lys Lys Ala Ile Ser Glu Lys Gln Val Ala Asn Asn Asp
    130                 135                 140

Ala Ile Asn Thr Val Ile Ala Asn Gln Gln Lys Leu Ala Asp Asp Ala

145                 150                 155                 160

Gln Ala Leu Thr Thr Lys Gln Ala Glu Leu Lys Ala Ala Glu Leu Ser
                165                 170                 175

Leu Ala Ala Glu Lys Ala Thr Ala Glu Gly Glu Lys Ala Ser Leu Leu
            180                 185                 190

Glu Gln Lys Ala Ala Ala Glu Ala Glu Ala Arg Ala Ala Ala Val Ala
        195                 200                 205

Glu Ala Ala Tyr Lys Glu Lys Arg Ala Ser Gln Gln Gln Ser Val Leu
    210                 215                 220

Ala Ser Ala Asn Thr Asn Leu Thr Ala Gln Val Gln Ala Val Ser Glu
225                 230                 235                 240

Ser Ala Ala Ala Pro Val Arg Ala Lys Val Arg Pro Thr Tyr Ser Thr
                245                 250                 255

Asn Ala Ser Ser Tyr Pro Ile Gly Glu Cys Thr Trp Gly Val Lys Thr
            260                 265                 270

Leu Ala Pro Trp Ala Gly Asp Tyr Trp Gly Asn Gly Ala Gln Trp Ala
        275                 280                 285

Thr Ser Ala Ala Ala Ala Gly Phe Arg Thr Gly Ser Thr Pro Gln Val
    290                 295                 300

Gly Ala Ile Ala Cys Trp Asn Asp Gly Gly Tyr Gly His Val Ala Val
305                 310                 315                 320

Val Thr Ala Val Glu Ser Thr Thr Arg Ile Gln Val Ser Glu Ser Asn
                325                 330                 335

Tyr Ala Gly Asn Arg Thr Ile Gly Asn His Arg Gly Trp Phe Asn Pro
            340                 345                 350

Thr Thr Thr Ser Glu Gly Phe Val Thr Tyr Ile Tyr Ala Asp
        355                 360                 365

<210>   48
<211>   195
<212>   PRT
<213>   Streptococcus pneumoniae

<400>   48
Met Lys Ser Ile Thr Lys Lys Ile Lys Ala Thr Leu Ala Gly Val Ala
1               5                   10                  15

Ala Leu Phe Ala Val Phe Ala Pro Ser Phe Val Ser Ala Gln Glu Ser
            20                  25                  30

Ser Thr Tyr Thr Val Lys Glu Gly Asp Thr Leu Ser Glu Ile Ala Glu
        35                  40                  45

Thr His Asn Thr Thr Val Glu Lys Leu Ala Glu Asn Asn His Ile Asp
    50                  55                  60

Asn Ile His Leu Ile Tyr Val Asp Gln Glu Leu Val Ile Asp Gly Pro
65                  70                  75                  80

Val Ala Pro Val Ala Thr Pro Ala Pro Ala Thr Tyr Ala Ala Pro Ala

85                  90                  95

Ala Gln Asp Glu Thr Val Ser Ala Pro Val Ala Glu Thr Pro Val Val
            100                 105                 110

Ser Glu Thr Val Val Ser Thr Val Ser Gly Ser Glu Ala Glu Ala Lys
        115                 120                 125

Glu Trp Ile Ala Gln Lys Glu Ser Gly Gly Ser Tyr Thr Ala Thr Asn
    130                 135                 140

Gly Arg Tyr Ile Gly Arg Tyr Gln Leu Thr Asp Ser Tyr Leu Asn Gly
145                 150                 155                 160

Asp Tyr Ser Ala Glu Asn Gln Glu Arg Val Ala Asp Ala Tyr Val Ala
                165                 170                 175

Gly Arg Tyr Gly Ser Trp Thr Ala Ala Lys Asn Phe Trp Leu Asn Asn
            180                 185                 190

Gly Trp Tyr
        195

<210>   49
<211>   659
<212>   PRT
<213>   Streptococcus pneumoniae

<400>   49
Met Lys Lys Asn Arg Val Phe Ala Thr Ala Gly Leu Val Leu Leu Ala
1               5                   10                  15

Ala Gly Val Leu Ala Ala Cys Ser Ser Ser Lys Ser Ser Asp Ser Ser
            20                  25                  30

Ala Pro Lys Ala Tyr Gly Tyr Val Tyr Thr Ala Asp Pro Glu Thr Leu
        35                  40                  45

Asp Tyr Leu Ile Ser Ser Lys Asn Ser Thr Thr Val Val Thr Ser Asn
    50                  55                  60

Gly Ile Asp Gly Leu Phe Thr Asn Asp Asn Tyr Gly Asn Leu Ala Pro
65                  70                  75                  80

Ala Val Ala Glu Asp Trp Glu Val Ser Lys Asp Gly Leu Thr Tyr Thr
                85                  90                  95

Tyr Lys Ile Arg Lys Gly Val Lys Trp Phe Thr Ser Asp Gly Glu Glu
            100                 105                 110

Tyr Ala Glu Val Thr Ala Lys Asp Phe Val Asn Gly Leu Lys His Ala
        115                 120                 125

Ala Asp Lys Lys Ser Glu Ala Met Tyr Leu Ala Glu Asn Ser Val Lys
    130                 135                 140

Gly Leu Ala Asp Tyr Leu Ser Gly Thr Ser Thr Asp Phe Ser Thr Val
145                 150                 155                 160

Gly Val Lys Ala Val Asp Asp Tyr Thr Leu Gln Tyr Thr Leu Asn Gln
                165                 170                 175

Pro Glu Pro Phe Trp Asn Ser Lys Leu Thr Tyr Ser Ile Phe Trp Pro

180                 185                 190

Leu Asn Glu Glu Phe Glu Thr Ser Lys Gly Ser Asp Phe Ala Lys Pro
        195                 200                 205

Thr Asp Pro Thr Ser Leu Leu Tyr Asn Gly Pro Phe Leu Leu Lys Gly
    210                 215                 220

Leu Thr Ala Lys Ser Ser Val Glu Phe Val Lys Asn Glu Gln Tyr Trp
225                 230                 235                 240

Asp Lys Glu Asn Val His Leu Asp Thr Ile Asn Leu Ala Tyr Tyr Asp
                245                 250                 255

Gly Ser Asp Gln Glu Ser Leu Glu Arg Asn Phe Thr Ser Gly Ala Tyr
            260                 265                 270

Ser Tyr Ala Arg Leu Tyr Pro Thr Ser Ser Asn Tyr Ser Lys Val Ala
        275                 280                 285

Glu Glu Tyr Lys Asp Asn Ile Tyr Tyr Thr Gln Ser Gly Ser Gly Ile
    290                 295                 300

Ala Gly Leu Gly Val Asn Ile Asp Arg Gln Ser Tyr Asn Tyr Thr Ser
305                 310                 315                 320

Lys Thr Thr Asp Ser Glu Lys Val Ala Thr Lys Lys Ala Leu Leu Asn
                325                 330                 335

Lys Asp Phe Arg Gln Ala Leu Asn Phe Ala Leu Asp Arg Ser Ala Tyr
            340                 345                 350

Ser Ala Gln Ile Asn Gly Lys Asp Gly Ala Ala Leu Ala Val Arg Asn
        355                 360                 365

Leu Phe Val Lys Pro Asp Phe Val Ser Ala Gly Glu Lys Thr Phe Gly
    370                 375                 380

Asp Leu Val Ala Ala Gln Leu Pro Ala Tyr Gly Asp Glu Trp Lys Gly
385                 390                 395                 400

Val Asn Leu Ala Asp Gly Gln Asp Gly Leu Phe Asn Ala Asp Lys Ala
                405                 410                 415

Lys Ala Glu Phe Ala Lys Ala Lys Lys Ala Leu Glu Ala Asp Gly Val
            420                 425                 430

Gln Phe Pro Ile His Leu Asp Val Pro Val Asp Gln Ala Ser Lys Asn
        435                 440                 445

Tyr Ile Ser Arg Ile Gln Ser Phe Lys Gln Ser Val Glu Thr Val Leu
    450                 455                 460

Gly Val Glu Asn Val Val Val Asp Ile Gln Gln Met Thr Ser Asp Glu
465                 470                 475                 480

Phe Leu Asn Ile Thr Tyr Tyr Ala Ala Asn Ala Ser Ser Glu Asp Trp
                485                 490                 495

Asp Val Ser Gly Gly Val Ser Trp Gly Pro Asp Tyr Gln Asp Pro Ser
            500                 505                 510

Thr Tyr Leu Asp Ile Leu Lys Thr Thr Ser Ser Glu Thr Thr Lys Thr

515                 520                 525

Tyr Leu Gly Phe Asp Asn Pro Asn Ser Pro Ser Val Val Gln Val Gly
    530                 535                 540

Leu Lys Glu Tyr Asp Lys Leu Val Asp Glu Ala Ala Arg Glu Thr Ser
545                 550                 555                 560

Asp Leu Asn Val Arg Tyr Glu Lys Tyr Ala Ala Ala Gln Ala Trp Leu
                565                 570                 575

Thr Asp Ser Ser Leu Phe Ile Pro Ala Met Ala Ser Ser Gly Ala Ala
            580                 585                 590

Pro Val Leu Ser Arg Ile Val Pro Phe Thr Gly Ala Ser Ala Gln Thr
        595                 600                 605

Gly Ser Lys Gly Ser Asp Val Tyr Phe Lys Tyr Leu Lys Ser Gln Asp
    610                 615                 620

Lys Val Val Thr Lys Glu Glu Tyr Glu Lys Ala Arg Glu Lys Trp Leu
625                 630                 635                 640

Lys Glu Lys Ala Glu Ser Asn Glu Lys Ala Gln Lys Glu Leu Ala Ser
                645                 650                 655

His Val Lys


<210>   50
<211>   619
<212>   PRT
<213>   Streptococcus pneumoniae

<400>   50
Met Asn Lys Lys Lys Met Ile Leu Thr Ser Leu Ala Ser Val Ala Ile
1               5                   10                  15

Leu Gly Ala Gly Phe Val Ala Ser Gln Pro Thr Val Val Arg Ala Glu
            20                  25                  30

Glu Ser Pro Val Ala Ser Gln Ser Lys Ala Glu Lys Asp Tyr Asp Ala
        35                  40                  45

Ala Lys Lys Asp Ala Lys Asn Ala Lys Lys Ala Val Glu Asp Ala Gln
    50                  55                  60

Lys Ala Leu Asp Asp Ala Lys Ala Ala Gln Lys Lys Tyr Asp Glu Asp
65                  70                  75                  80

Gln Lys Lys Thr Glu Glu Lys Ala Ala Leu Glu Lys Ala Ala Ser Glu
                85                  90                  95

Glu Met Asp Lys Ala Val Ala Ala Val Gln Gln Ala Tyr Leu Ala Tyr
            100                 105                 110

Gln Gln Ala Thr Asp Lys Ala Ala Lys Asp Ala Ala Asp Lys Met Ile
        115                 120                 125

Asp Glu Ala Lys Lys Arg Glu Glu Glu Ala Lys Thr Lys Phe Asn Thr
    130                 135                 140

Val Arg Ala Met Val Val Pro Glu Pro Glu Gln Leu Ala Glu Thr Lys

145                 150                 155                 160

Lys Lys Ser Glu Glu Ala Lys Gln Lys Ala Pro Glu Leu Thr Lys Lys
                165                 170                 175

Leu Glu Glu Ala Lys Ala Lys Leu Glu Glu Ala Glu Lys Lys Ala Thr
            180                 185                 190

Glu Ala Lys Gln Lys Val Asp Ala Glu Glu Val Ala Pro Gln Ala Lys
        195                 200                 205

Ile Ala Glu Leu Glu Asn Gln Val His Arg Leu Glu Gln Glu Leu Lys
    210                 215                 220

Glu Ile Asp Glu Ser Glu Ser Glu Asp Tyr Ala Lys Glu Gly Phe Arg
225                 230                 235                 240

Ala Pro Leu Gln Ser Lys Leu Asp Ala Lys Lys Ala Lys Leu Ser Lys
                245                 250                 255

Leu Glu Glu Leu Ser Asp Lys Ile Asp Glu Leu Asp Ala Glu Ile Ala
            260                 265                 270

Lys Leu Glu Asp Gln Leu Lys Ala Ala Glu Glu Asn Asn Asn Val Glu
        275                 280                 285

Asp Tyr Phe Lys Glu Gly Leu Glu Lys Thr Ile Ala Ala Lys Lys Ala
    290                 295                 300

Glu Leu Glu Lys Thr Glu Ala Asp Leu Lys Lys Ala Val Asn Glu Pro
305                 310                 315                 320

Glu Lys Pro Ala Pro Ala Pro Glu Thr Pro Ala Pro Glu Ala Pro Ala
                325                 330                 335

Glu Gln Pro Lys Pro Ala Pro Ala Pro Gln Pro Ala Pro Ala Pro Lys
            340                 345                 350

Pro Glu Lys Pro Ala Glu Gln Pro Lys Pro Glu Lys Thr Asp Asp Gln
        355                 360                 365

Gln Ala Glu Glu Asp Tyr Ala Arg Arg Ser Glu Glu Glu Tyr Asn Arg
    370                 375                 380

Leu Thr Gln Gln Gln Pro Pro Lys Ala Glu Lys Pro Ala Pro Ala Pro
385                 390                 395                 400

Lys Thr Gly Trp Lys Gln Glu Asn Gly Met Trp Tyr Phe Tyr Asn Thr
                405                 410                 415

Asp Gly Ser Met Ala Thr Gly Trp Leu Gln Asn Asn Gly Ser Trp Tyr
            420                 425                 430

Tyr Leu Asn Ser Asn Gly Ala Met Ala Thr Gly Trp Leu Gln Tyr Asn
        435                 440                 445

Gly Ser Trp Tyr Tyr Leu Asn Ala Asn Gly Ala Met Ala Thr Gly Trp
    450                 455                 460

Ala Lys Val Asn Gly Ser Trp Tyr Tyr Leu Asn Ala Asn Gly Ala Met
465                 470                 475                 480

Ala Thr Gly Trp Leu Gln Tyr Asn Gly Ser Trp Tyr Tyr Leu Asn Ala

485                 490                 495

Asn Gly Ala Met Ala Thr Gly Trp Ala Lys Val Asn Gly Ser Trp Tyr
            500                 505                 510

Tyr Leu Asn Ala Asn Gly Ala Met Ala Thr Gly Trp Leu Gln Tyr Asn
        515                 520                 525

Gly Ser Trp Tyr Tyr Leu Asn Ala Asn Gly Ala Met Ala Thr Gly Trp
    530                 535                 540

Ala Lys Val Asn Gly Ser Trp Tyr Tyr Leu Asn Ala Asn Gly Ala Met
545                 550                 555                 560

Ala Thr Gly Trp Val Lys Asp Gly Asp Thr Trp Tyr Tyr Leu Glu Ala
                565                 570                 575

Ser Gly Ala Met Lys Ala Ser Gln Trp Phe Lys Val Ser Asp Lys Trp
            580                 585                 590

Tyr Tyr Val Asn Gly Leu Gly Ala Leu Ala Val Asn Thr Thr Val Asp
        595                 600                 605

Gly Tyr Lys Val Asn Ala Asn Gly Glu Trp Val
    610                 615

<210>   51
<211>   309
<212>   PRT
<213>   Streptococcus pneumoniae

<400>   51
Met Lys Lys Leu Gly Thr Leu Leu Val Leu Phe Leu Ser Ala Ile Ile
1               5                   10                  15

Leu Val Ala Cys Ala Ser Gly Lys Lys Asp Thr Thr Ser Gly Gln Lys
            20                  25                  30

Leu Lys Val Val Ala Thr Asn Ser Ile Ile Ala Asp Ile Thr Lys Asn
        35                  40                  45

Ile Ala Gly Asp Lys Ile Asp Leu His Ser Ile Val Pro Ile Gly Gln
    50                  55                  60

Asp Pro His Glu Tyr Glu Pro Leu Pro Glu Asp Val Lys Lys Thr Ser
65                  70                  75                  80

Glu Ala Asp Leu Ile Phe Tyr Asn Gly Ile Asn Leu Glu Thr Gly Gly
                85                  90                  95

Asn Ala Trp Phe Thr Lys Leu Val Glu Asn Ala Lys Lys Thr Glu Asn
            100                 105                 110

Lys Asp Tyr Phe Ala Val Ser Asp Gly Val Asp Val Ile Tyr Leu Glu
        115                 120                 125

Gly Gln Asn Glu Lys Gly Lys Glu Asp Pro His Ala Trp Leu Asn Leu
    130                 135                 140

Glu Asn Gly Ile Ile Phe Ala Lys Asn Ile Ala Lys Gln Leu Ser Ala
145                 150                 155                 160

Lys Asp Pro Asn Asn Lys Glu Phe Tyr Glu Lys Asn Leu Lys Glu Tyr

165                 170                 175

Thr Asp Lys Leu Asp Lys Leu Asp Lys Glu Ser Lys Asp Lys Phe Asn
            180                 185                 190

Lys Ile Pro Ala Glu Lys Lys Leu Ile Val Thr Ser Glu Gly Ala Phe
        195                 200                 205

Lys Tyr Phe Ser Lys Ala Tyr Gly Val Pro Ser Ala Tyr Ile Trp Glu
    210                 215                 220

Ile Asn Thr Glu Glu Glu Gly Thr Pro Glu Gln Ile Lys Thr Leu Val
225                 230                 235                 240

Glu Lys Leu Arg Gln Thr Lys Val Pro Ser Leu Phe Val Glu Ser Ser
                245                 250                 255

Val Asp Asp Arg Pro Met Lys Thr Val Ser Gln Asp Thr Asn Ile Pro
            260                 265                 270

Ile Tyr Ala Gln Ile Phe Thr Asp Ser Ile Ala Glu Gln Gly Lys Glu
        275                 280                 285

Gly Asp Ser Tyr Tyr Ser Met Met Lys Tyr Asn Leu Asp Lys Ile Ala
    290                 295                 300

Glu Gly Leu Ala Lys
305

<210>   52
<211>   701
<212>   PRT
<213>   Streptococcus pneumoniae

<400>   52
Met Phe Ala Ser Lys Ser Glu Arg Lys Val His Tyr Ser Ile Arg Lys
1               5                   10                  15

Phe Ser Ile Gly Val Ala Ser Val Ala Val Ala Ser Leu Val Met Gly
            20                  25                  30

Ser Val Val His Ala Thr Glu Asn Glu Gly Ser Thr Gln Ala Ala Thr
        35                  40                  45

Ser Ser Asn Met Ala Lys Thr Glu His Arg Lys Ala Ala Lys Gln Val
    50                  55                  60

Val Asp Glu Tyr Ile Glu Lys Met Leu Arg Glu Ile Gln Leu Asp Arg
65                  70                  75                  80

Arg Lys His Thr Gln Asn Val Ala Leu Asn Ile Lys Leu Ser Ala Ile
                85                  90                  95

Lys Thr Lys Tyr Leu Arg Glu Leu Asn Val Leu Glu Glu Lys Ser Lys
            100                 105                 110

Asp Glu Leu Pro Ser Glu Ile Lys Ala Lys Leu Asp Ala Ala Phe Glu
        115                 120                 125

Lys Phe Lys Lys Asp Thr Leu Lys Pro Gly Glu Lys Val Ala Glu Ala
    130                 135                 140

Lys Lys Lys Val Glu Glu Ala Lys Lys Lys Ala Glu Asp Gln Lys Glu

145                 150                 155                 160

Glu Asp Arg Arg Asn Tyr Pro Thr Asn Thr Tyr Lys Thr Leu Glu Leu
                165                 170                 175

Glu Ile Ala Glu Phe Asp Val Lys Val Lys Glu Ala Glu Leu Glu Leu
            180                 185                 190

Val Lys Glu Glu Ala Lys Glu Ser Arg Asn Glu Gly Thr Ile Lys Gln
        195                 200                 205

Ala Lys Glu Lys Val Glu Ser Lys Lys Ala Glu Ala Thr Arg Leu Glu
    210                 215                 220

Asn Ile Lys Thr Asp Arg Lys Lys Ala Glu Glu Glu Ala Lys Arg Lys
225                 230                 235                 240

Ala Asp Ala Lys Leu Lys Glu Ala Asn Val Ala Thr Ser Asp Gln Gly
                245                 250                 255

Lys Pro Lys Gly Arg Ala Lys Arg Gly Val Pro Gly Glu Leu Ala Thr
            260                 265                 270

Pro Asp Lys Lys Glu Asn Asp Ala Lys Ser Ser Asp Ser Ser Val Gly
        275                 280                 285

Glu Glu Thr Leu Pro Ser Ser Ser Leu Lys Ser Gly Lys Lys Val Ala
    290                 295                 300

Glu Ala Glu Lys Lys Val Glu Glu Ala Glu Lys Lys Ala Lys Asp Gln
305                 310                 315                 320

Lys Glu Glu Asp Arg Arg Asn Tyr Pro Thr Asn Thr Tyr Lys Thr Leu
                325                 330                 335

Asp Leu Glu Ile Ala Glu Ser Asp Val Lys Val Lys Glu Ala Glu Leu
            340                 345                 350

Glu Leu Val Lys Glu Glu Ala Lys Glu Pro Arg Asp Glu Glu Lys Ile
        355                 360                 365

Lys Gln Ala Lys Ala Lys Val Glu Ser Lys Lys Ala Glu Ala Thr Arg
    370                 375                 380

Leu Glu Asn Ile Lys Thr Asp Arg Lys Lys Ala Glu Glu Glu Ala Lys
385                 390                 395                 400

Arg Lys Ala Ala Glu Glu Asp Lys Val Lys Glu Lys Pro Ala Glu Gln
                405                 410                 415

Pro Gln Pro Ala Pro Ala Thr Gln Pro Glu Lys Pro Ala Pro Lys Pro
            420                 425                 430

Glu Lys Pro Ala Glu Gln Pro Lys Ala Glu Lys Thr Asp Asp Gln Gln
        435                 440                 445

Ala Glu Glu Asp Tyr Ala Arg Arg Ser Glu Glu Glu Tyr Asn Arg Leu
    450                 455                 460

Thr Gln Gln Gln Pro Pro Lys Thr Glu Lys Pro Ala Gln Pro Ser Thr
465                 470                 475                 480

Pro Lys Thr Gly Trp Lys Gln Glu Asn Gly Met Trp Tyr Phe Tyr Asn

485                 490                 495

Thr Asp Gly Ser Met Ala Thr Gly Trp Leu Gln Asn Asn Gly Ser Trp
            500                 505                 510

Tyr Tyr Leu Asn Ala Asn Gly Ala Met Ala Thr Gly Trp Leu Gln Asn
        515                 520                 525

Asn Gly Ser Trp Tyr Tyr Leu Asn Ala Asn Gly Ser Met Ala Thr Gly
    530                 535                 540

Trp Leu Gln Asn Asn Gly Ser Trp Tyr Tyr Leu Asn Ala Asn Gly Ala
545                 550                 555                 560

Met Ala Thr Gly Trp Leu Gln Tyr Asn Gly Ser Trp Tyr Tyr Leu Asn
                565                 570                 575

Ser Asn Gly Ala Met Ala Thr Gly Trp Leu Gln Tyr Asn Gly Ser Trp
            580                 585                 590

Tyr Tyr Leu Asn Ala Asn Gly Asp Met Ala Thr Gly Trp Leu Gln Asn
        595                 600                 605

Asn Gly Ser Trp Tyr Tyr Leu Asn Ala Asn Gly Asp Met Ala Thr Gly
    610                 615                 620

Trp Leu Gln Tyr Asn Gly Ser Trp Tyr Tyr Leu Asn Ala Asn Gly Asp
625                 630                 635                 640

Met Ala Thr Gly Trp Val Lys Asp Gly Asp Thr Trp Tyr Tyr Leu Glu
                645                 650                 655

Ala Ser Gly Ala Met Lys Ala Ser Gln Trp Phe Lys Val Ser Asp Lys
            660                 665                 670

Trp Tyr Tyr Val Asn Gly Ser Gly Ala Leu Ala Val Asn Thr Thr Val
        675                 680                 685

Asp Gly Tyr Gly Val Asn Ala Asn Gly Glu Trp Val Asn
    690                 695                 700

<210>   53
<211>   612
<212>   PRT
<213>   Streptococcus pneumoniae

<400>   53
Met Phe Ala Phe Lys Lys Arg Arg Lys Val His Tyr Ser Ile Arg Lys
1               5                   10                  15

Phe Ser Ile Gly Val Ala Ser Val Ala Val Ala Ser Leu Phe Met Gly
            20                  25                  30

Ser Val Val His Ala Thr Glu Lys Glu Val Thr Thr Gln Val Ala Thr
        35                  40                  45

Ser Ser Asn Lys Ala Asn Lys Ser Gln Thr Glu His Met Lys Ala Ala
    50                  55                  60

Lys Gln Val Asp Glu Tyr Ile Glu Lys Met Leu Ser Glu Ile Gln Leu
65                  70                  75                  80

Asp Arg Arg Lys His Thr Gln Asn Val Gly Leu Leu Thr Lys Leu Gly

85                  90                  95

Ala Ile Lys Thr Glu Tyr Leu Arg Gly Leu Ser Val Ser Lys Glu Lys
            100                 105                 110

Ser Thr Ala Glu Leu Pro Ser Glu Ile Lys Glu Lys Leu Thr Ala Ala
        115                 120                 125

Phe Glu Gln Phe Lys Lys Asp Thr Leu Lys Ser Gly Lys Lys Val Ala
    130                 135                 140

Glu Ala Gln Lys Lys Ala Lys Asp Gln Lys Glu Ala Lys Gln Glu Ile
145                 150                 155                 160

Glu Ala Leu Ile Val Lys His Lys Gly Arg Glu Ile Asp Leu Asp Arg
                165                 170                 175

Lys Lys Ala Lys Ala Ala Val Thr Glu His Leu Lys Lys Leu Leu Asn
            180                 185                 190

Asp Ile Glu Lys Asn Leu Lys Lys Glu Gln His Thr His Thr Val Glu
        195                 200                 205

Leu Ile Lys Asn Leu Lys Asp Ile Glu Lys Thr Tyr Leu His Lys Leu
    210                 215                 220

Asp Glu Ser Thr Gln Lys Ala Gln Leu Gln Lys Leu Ile Ala Glu Ser
225                 230                 235                 240

Gln Ser Lys Leu Asp Glu Ala Phe Ser Lys Phe Lys Asn Gly Leu Ser
                245                 250                 255

Ser Ser Ser Asn Ser Gly Ser Ser Thr Lys Pro Glu Thr Pro Gln Pro
            260                 265                 270

Glu Thr Pro Lys Pro Glu Val Lys Pro Glu Leu Glu Thr Pro Lys Pro
        275                 280                 285

Glu Val Lys Pro Glu Pro Glu Thr Pro Lys Pro Glu Val Lys Pro Glu
    290                 295                 300

Pro Glu Thr Pro Lys Pro Glu Val Lys Pro Glu Leu Glu Thr Pro Lys
305                 310                 315                 320

Pro Glu Val Lys Pro Glu Pro Glu Thr Pro Lys Pro Glu Val Lys Pro
                325                 330                 335

Glu Pro Glu Thr Pro Lys Pro Glu Val Lys Pro Glu Pro Glu Thr Pro
            340                 345                 350

Lys Pro Glu Val Lys Pro Glu Leu Glu Thr Pro Lys Pro Glu Val Lys
        355                 360                 365

Pro Glu Leu Glu Thr Pro Lys Pro Glu Val Lys Pro Glu Pro Glu Thr
    370                 375                 380

Pro Lys Pro Glu Val Lys Pro Glu Leu Glu Thr Pro Lys Pro Glu Val
385                 390                 395                 400

Lys Pro Glu Pro Glu Thr Pro Lys Pro Glu Val Lys Pro Glu Leu Glu
                405                 410                 415

Thr Pro Lys Pro Glu Val Lys Pro Glu Pro Glu Thr Pro Lys Pro Glu

420                 425                 430

Val Lys Pro Glu Leu Glu Thr Pro Lys Pro Glu Val Lys Pro Glu Pro
        435                 440                 445

Glu Thr Pro Lys Pro Glu Val Lys Pro Glu Pro Glu Thr Pro Lys Pro
    450                 455                 460

Glu Val Lys Pro Glu Pro Glu Thr Pro Lys Pro Glu Val Lys Pro Glu
465                 470                 475                 480

Leu Glu Thr Pro Lys Gln Lys Val Lys Pro Glu Pro Glu Thr Pro Lys
                485                 490                 495

Pro Glu Val Lys Pro Glu Pro Glu Thr Pro Lys Pro Glu Val Lys Pro
            500                 505                 510

Glu Leu Glu Thr Pro Lys Pro Glu Val Lys Pro Glu Leu Glu Ile Pro
        515                 520                 525

Lys Pro Glu Val Lys Pro Asp Asn Ser Lys Pro Gln Ala Asp Asp Lys
    530                 535                 540

Lys Pro Ser Thr Pro Asn Asn Leu Ser Lys Asp Lys Gln Ser Ser Asn
545                 550                 555                 560

Gln Ala Ser Thr Asn Glu Asn Lys Lys Gln Gly Pro Ala Thr Asn Lys
                565                 570                 575

Pro Lys Lys Ser Leu Pro Ser Thr Gly Ser Ile Ser Asn Leu Ala Leu
            580                 585                 590

Glu Ile Ala Gly Leu Leu Thr Leu Ala Gly Ala Thr Ile Leu Ala Lys
        595                 600                 605

Lys Arg Met Lys
    610

<210>   54
<211>   318
<212>   PRT
<213>   Streptococcus pneumoniae

<400>   54
Met Glu Ile Asn Val Ser Lys Leu Arg Thr Asp Leu Pro Gln Val Gly
1               5                   10                  15

Val Gln Pro Tyr Arg Gln Val His Ala His Ser Thr Gly Asn Pro His
            20                  25                  30

Ser Thr Val Gln Asn Glu Ala Asp Tyr His Trp Arg Lys Asp Pro Glu
        35                  40                  45

Leu Gly Phe Phe Ser His Ile Val Gly Asn Gly Cys Ile Met Gln Val
    50                  55                  60

Gly Pro Val Asp Asn Gly Ala Trp Asp Val Gly Gly Gly Trp Asn Ala
65                  70                  75                  80

Glu Thr Tyr Ala Ala Val Glu Leu Ile Glu Ser His Ser Thr Lys Glu
                85                  90                  95

Glu Phe Met Thr Asp Tyr Arg Leu Tyr Ile Glu Leu Leu Arg Asn Leu

            100                 105                 110

Ala Asp Glu Ala Gly Leu Pro Lys Thr Leu Asp Thr Gly Ser Leu Ala
        115                 120                 125

Gly Ile Lys Thr His Glu Tyr Cys Thr Asn Asn Gln Pro Asn Asn His
    130                 135                 140

Ser Asp His Val Asp Pro Tyr Pro Tyr Leu Ala Lys Trp Gly Ile Ser
145                 150                 155                 160

Arg Glu Gln Phe Lys His Asp Ile Glu Asn Gly Leu Thr Ile Glu Thr
                165                 170                 175

Gly Trp Gln Lys Asn Asp Thr Gly Tyr Trp Tyr Val His Ser Asp Gly
            180                 185                 190

Ser Tyr Pro Lys Asp Lys Phe Glu Lys Ile Asn Gly Thr Trp Tyr Tyr
        195                 200                 205

Phe Asp Ser Ser Gly Tyr Met Leu Ala Asp Arg Trp Arg Lys His Thr
    210                 215                 220

Asp Gly Asn Trp Tyr Trp Phe Asp Asn Ser Gly Glu Met Ala Thr Gly
225                 230                 235                 240

Trp Lys Lys Ile Ala Asp Lys Trp Tyr Tyr Phe Asn Glu Glu Gly Ala
                245                 250                 255

Met Lys Thr Gly Trp Val Lys Tyr Lys Asp Thr Trp Tyr Tyr Leu Asp
            260                 265                 270

Ala Lys Glu Gly Ala Met Val Ser Asn Ala Phe Ile Gln Ser Ala Asp
        275                 280                 285

Gly Thr Gly Trp Tyr Tyr Leu Lys Pro Asp Gly Thr Leu Ala Asp Arg
    290                 295                 300

Pro Glu Phe Thr Val Glu Pro Asp Gly Leu Ile Thr Val Lys
305                 310                 315

<210> 55
<211> 828
<212> PRT
<213> Streptococcus pneumoniae

<400> 55
Met Gln Leu Glu Ile Ser Asn Arg Lys Arg Val Ser Met Lys Ile Asn
1               5                   10                  15

Lys Lys Tyr Leu Val Gly Ser Ala Ala Ala Leu Ile Leu Ser Val Cys
            20                  25                  30

Ser Tyr Glu Leu Gly Leu Tyr Gln Ala Arg Thr Val Lys Glu Asn Asn
        35                  40                  45

Arg Val Ser Tyr Ile Asp Gly Lys Gln Ala Thr Gln Lys Thr Glu Asn
    50                  55                  60

Leu Thr Pro Asp Glu Val Ser Lys Arg Glu Gly Ile Asn Ala Glu Gln
65                  70                  75                  80

Ile Val Ile Lys Ile Thr Asp Gln Gly Tyr Val Thr Ser His Gly Asp

85                  90                  95

His Tyr His Tyr Tyr Asn Gly Lys Val Pro Tyr Asp Ala Ile Phe Ser
            100                 105                 110

Glu Glu Leu Leu Met Lys Asp Pro Asn Tyr Lys Leu Lys Asp Glu Asp
        115                 120                 125

Ile Val Asn Glu Val Lys Gly Gly Tyr Val Ile Lys Val Asp Gly Lys
    130                 135                 140

Tyr Tyr Val Tyr Leu Lys Asp Ala Ala His Ala Asp Asn Val Arg Thr
145                 150                 155                 160

Lys Glu Glu Ile Asn Arg Gln Lys Gln Glu His Ser Gln His Arg Glu
                165                 170                 175

Gly Gly Thr Pro Arg Asn Asp Gly Ala Val Ala Leu Ala Arg Ser Gln
            180                 185                 190

Gly Arg Tyr Thr Thr Asp Asp Gly Tyr Ile Phe Asn Ala Ser Asp Ile
        195                 200                 205

Ile Glu Asp Thr Gly Asp Ala Tyr Ile Val Pro His Gly Asp His Tyr
    210                 215                 220

His Tyr Ile Pro Lys Asn Glu Leu Ser Ala Ser Glu Leu Ala Ala Ala
225                 230                 235                 240

Glu Ala Phe Leu Ser Gly Arg Gly Asn Leu Ser Asn Ser Arg Thr Tyr
                245                 250                 255

Arg Arg Gln Asn Ser Asp Asn Thr Ser Arg Thr Asn Trp Val Pro Ser
            260                 265                 270

Val Ser Asn Pro Gly Thr Thr Asn Thr Asn Thr Ser Asn Asn Ser Asn
        275                 280                 285

Thr Asn Ser Gln Ala Ser Gln Ser Asn Asp Ile Asp Ser Leu Leu Lys
    290                 295                 300

Gln Leu Tyr Lys Leu Pro Leu Ser Gln Arg His Val Glu Ser Asp Gly
305                 310                 315                 320

Leu Val Phe Asp Pro Ala Gln Ile Thr Ser Arg Thr Ala Arg Gly Val
                325                 330                 335

Ala Val Pro His Gly Asp His Tyr His Phe Ile Pro Tyr Ser Gln Met
            340                 345                 350

Ser Glu Leu Glu Glu Arg Ile Ala Arg Ile Ile Pro Leu Arg Tyr Arg
        355                 360                 365

Ser Asn His Trp Val Pro Asp Ser Arg Pro Glu Gln Pro Ser Pro Gln
    370                 375                 380

Pro Thr Pro Glu Pro Ser Pro Gly Pro Gln Pro Ala Pro Asn Leu Lys
385                 390                 395                 400

Ile Asp Ser Asn Ser Ser Leu Val Ser Gln Leu Val Arg Lys Val Gly
                405                 410                 415

Glu Gly Tyr Val Phe Glu Glu Lys Gly Ile Ser Arg Tyr Val Phe Ala

            420                 425                 430

Lys Asp Leu Pro Ser Glu Thr Val Lys Asn Leu Glu Ser Lys Leu Ser
        435                 440                 445

Lys Gln Glu Ser Val Ser His Thr Leu Thr Ala Lys Lys Glu Asn Val
    450                 455                 460

Ala Pro Arg Asp Gln Glu Phe Tyr Asp Lys Ala Tyr Asn Leu Leu Thr
465                 470                 475                 480

Glu Ala His Lys Ala Leu Phe Glu Asn Lys Gly Arg Asn Ser Asp Phe
                485                 490                 495

Gln Ala Leu Asp Lys Leu Leu Glu Arg Leu Asn Asp Glu Ser Thr Asn
            500                 505                 510

Lys Glu Lys Leu Val Asp Asp Leu Leu Ala Phe Leu Ala Pro Ile Thr
        515                 520                 525

His Pro Glu Arg Leu Gly Lys Pro Asn Ser Gln Ile Glu Tyr Thr Glu
    530                 535                 540

Asp Glu Val Arg Ile Ala Gln Leu Ala Asp Lys Tyr Thr Thr Ser Asp
545                 550                 555                 560

Gly Tyr Ile Phe Asp Glu His Asp Ile Ile Ser Asp Glu Gly Asp Ala
                565                 570                 575

Tyr Val Thr Pro His Met Gly His Ser His Trp Ile Gly Lys Asp Ser
            580                 585                 590

Leu Ser Asp Lys Glu Lys Val Ala Ala Gln Ala Tyr Thr Lys Glu Lys
        595                 600                 605

Gly Ile Leu Pro Pro Ser Pro Asp Ala Asp Val Lys Ala Asn Pro Thr
    610                 615                 620

Gly Asp Ser Ala Ala Ala Ile Tyr Asn Arg Val Lys Gly Glu Lys Arg
625                 630                 635                 640

Ile Pro Leu Val Arg Leu Pro Tyr Met Val Glu His Thr Val Glu Val
                645                 650                 655

Lys Asn Gly Asn Leu Ile Ile Pro His Lys Asp His Tyr His Asn Ile
            660                 665                 670

Lys Phe Ala Trp Phe Asp Asp His Thr Tyr Lys Ala Pro Asn Gly Tyr
        675                 680                 685

Thr Leu Glu Asp Leu Phe Ala Thr Ile Lys Tyr Tyr Val Glu His Pro
    690                 695                 700

Asp Glu Arg Pro His Ser Asn Asp Gly Trp Gly Asn Ala Ser Glu His
705                 710                 715                 720

Val Leu Gly Lys Lys Asp His Ser Glu Asp Pro Asn Lys Asn Phe Lys
                725                 730                 735

Ala Asp Glu Glu Pro Val Glu Glu Thr Pro Ala Glu Pro Glu Val Pro
            740                 745                 750

Gln Val Glu Thr Glu Lys Val Glu Ala Gln Leu Lys Glu Ala Glu Val

755                 760                 765

Leu Leu Ala Lys Val Thr Asp Ser Ser Leu Lys Ala Asn Ala Thr Glu
    770                 775                 780

Thr Leu Ala Gly Leu Arg Asn Asn Leu Thr Leu Gln Ile Met Asp Asn
785                 790                 795                 800

Asn Ser Ile Met Ala Glu Ala Glu Lys Leu Leu Ala Leu Leu Lys Gly
                805                 810                 815

Ser Asn Pro Ser Ser Val Ser Lys Glu Lys Ile Asn
            820                 825

<210>   56
<211>   819
<212>   PRT
<213>   Streptococcus pneumoniae

<400>   56
Met Lys Ile Asn Lys Lys Tyr Leu Ala Gly Ser Val Ala Val Leu Ala
1               5                   10                  15

Leu Ser Val Cys Ser Tyr Glu Leu Gly Arg Tyr Gln Ala Gly Gln Asp
            20                  25                  30

Lys Lys Glu Ser Asn Arg Val Ala Tyr Ile Asp Gly Asp Gln Ala Gly
        35                  40                  45

Gln Lys Ala Glu Asn Leu Thr Pro Asp Glu Val Ser Lys Arg Glu Gly
    50                  55                  60

Ile Asn Ala Glu Gln Ile Val Ile Lys Ile Thr Asp Gln Gly Tyr Val
65                  70                  75                  80

Thr Ser His Gly Asp His Tyr His Tyr Tyr Asn Gly Lys Val Pro Tyr
                85                  90                  95

Asp Ala Ile Ile Ser Glu Glu Leu Leu Met Lys Asp Pro Asn Tyr Gln
            100                 105                 110

Leu Lys Asp Ser Asp Ile Val Asn Glu Ile Lys Gly Gly Tyr Val Ile
        115                 120                 125

Lys Val Asn Gly Lys Tyr Tyr Val Tyr Leu Lys Asp Ala Ala His Ala
    130                 135                 140

Asp Asn Ile Arg Thr Lys Glu Glu Ile Lys Arg Gln Lys Gln Glu Arg
145                 150                 155                 160

Ser His Asn His Asn Ser Arg Ala Asp Asn Ala Val Ala Ala Ala Arg
                165                 170                 175

Ala Gln Gly Arg Tyr Thr Thr Asp Asp Gly Tyr Ile Phe Asn Ala Ser
            180                 185                 190

Asp Ile Ile Glu Asp Thr Gly Asp Ala Tyr Ile Val Pro His Gly Asp
        195                 200                 205

His Tyr His Tyr Ile Pro Lys Asn Glu Leu Ser Ala Ser Glu Leu Ala
    210                 215                 220

Ala Ala Glu Ala Tyr Trp Asn Gly Lys Gln Gly Ser Arg Pro Ser Ser

225                 230                 235                 240

Ser Ser Ser Tyr Asn Ala Asn Pro Ala Gln Pro Arg Leu Ser Glu Asn
                245                 250                 255

His Asn Leu Thr Val Thr Pro Thr Tyr His Gln Asn Gln Gly Glu Asn
            260                 265                 270

Ile Ser Ser Leu Leu Arg Glu Leu Tyr Ala Lys Pro Leu Ser Glu Arg
        275                 280                 285

His Val Glu Ser Asp Gly Leu Ile Phe Asp Pro Ala Gln Ile Thr Ser
    290                 295                 300

Arg Thr Ala Arg Gly Val Ala Val Pro His Gly Asn His Tyr His Phe
305                 310                 315                 320

Ile Pro Tyr Glu Gln Met Ser Glu Leu Glu Lys Arg Ile Ala Arg Ile
                325                 330                 335

Ile Pro Leu Arg Tyr Arg Ser Asn His Trp Val Pro Asp Ser Arg Pro
            340                 345                 350

Glu Glu Pro Ser Pro Gln Pro Thr Pro Glu Pro Ser Pro Ser Pro Gln
        355                 360                 365

Pro Ala Pro Ser Asn Pro Ile Asp Gly Lys Leu Val Lys Glu Ala Val
    370                 375                 380

Arg Lys Val Gly Asp Gly Tyr Val Phe Glu Glu Asn Gly Val Ser Arg
385                 390                 395                 400

Tyr Ile Pro Ala Lys Asp Leu Ser Ala Glu Thr Ala Ala Gly Ile Asp
                405                 410                 415

Ser Lys Leu Ala Lys Gln Glu Ser Leu Ser His Lys Leu Gly Thr Lys
            420                 425                 430

Lys Thr Asp Leu Pro Ser Ser Asp Arg Glu Phe Tyr Asn Lys Ala Tyr
        435                 440                 445

Asp Leu Leu Ala Arg Ile His Gln Asp Leu Leu Asp Asn Lys Gly Arg
    450                 455                 460

Gln Val Asp Phe Glu Ala Leu Asp Asn Leu Leu Glu Arg Leu Lys Asp
465                 470                 475                 480

Val Ser Ser Asp Lys Val Lys Leu Val Glu Asp Ile Leu Ala Phe Leu
                485                 490                 495

Ala Pro Ile Arg His Pro Glu Arg Leu Gly Lys Pro Asn Ala Gln Ile
            500                 505                 510

Thr Tyr Thr Asp Asp Glu Ile Gln Val Ala Lys Leu Ala Gly Lys Tyr
        515                 520                 525

Thr Ala Glu Asp Gly Tyr Ile Phe Asp Pro Arg Asp Ile Thr Ser Asp
    530                 535                 540

Glu Gly Asp Ala Tyr Val Thr Pro His Met Thr His Ser His Trp Ile
545                 550                 555                 560

Lys Lys Asp Ser Leu Ser Glu Ala Glu Arg Ala Ala Ala Gln Ala Tyr

565                 570                 575

Ala Xaa Glu Lys Gly Leu Thr Pro Pro Ser Thr Asp His Gln Asp Ser
            580                 585                 590

Gly Asn Thr Glu Ala Lys Gly Ala Glu Ala Ile Tyr Asn Arg Val Lys
        595                 600                 605

Ala Ala Lys Lys Val Pro Leu Asp Arg Met Pro Tyr Asn Leu Gln Tyr
    610                 615                 620

Thr Val Glu Val Lys Asn Gly Ser Leu Ile Ile Pro His Tyr Asp His
625                 630                 635                 640

Tyr His Asn Ile Lys Phe Glu Trp Phe Asp Glu Gly Leu Tyr Glu Ala
                645                 650                 655

Pro Lys Gly Tyr Thr Leu Glu Asp Leu Leu Ala Thr Val Lys Tyr Tyr
            660                 665                 670

Val Glu His Pro Asn Glu Arg Pro His Ser Asp Asn Gly Phe Gly Asn
        675                 680                 685

Ala Ser Asp His Val Gln Arg Asn Lys Asn Gly Gln Ala Asp Thr Asn
    690                 695                 700

Gln Thr Glu Lys Pro Ser Glu Glu Lys Pro Gln Thr Glu Lys Pro Glu
705                 710                 715                 720

Glu Glu Thr Pro Arg Glu Glu Lys Pro Gln Ser Glu Lys Pro Glu Ser
                725                 730                 735

Pro Lys Pro Thr Glu Glu Pro Glu Glu Ser Pro Glu Glu Ser Glu Glu
            740                 745                 750

Pro Gln Val Glu Thr Glu Lys Val Glu Glu Lys Leu Arg Glu Ala Glu
        755                 760                 765

Asp Leu Leu Gly Lys Ile Gln Asp Pro Ile Ile Lys Ser Asn Ala Lys
    770                 775                 780

Glu Thr Leu Thr Gly Leu Lys Asn Asn Leu Leu Phe Gly Thr Gln Asp
785                 790                 795                 800

Asn Asn Thr Ile Met Ala Glu Ala Glu Lys Leu Leu Ala Leu Leu Lys
                805                 810                 815

Glu Ser Lys

<210>   57
<211>   853
<212>   PRT
<213>   Streptococcus pneumoniae

<400>   57
Met Lys Ile Asn Lys Lys Tyr Leu Ala Gly Ser Val Ala Val Leu Ala
1               5                   10                  15

Leu Ser Val Cys Ser Tyr Glu Leu Gly Arg His Gln Ala Gly Gln Val
            20                  25                  30

Lys Lys Glu Ser Asn Arg Val Ser Tyr Ile Asp Gly Asp Gln Ala Gly

35                  40                  45

Gln Lys Ala Glu Asn Leu Thr Pro Asp Glu Val Ser Lys Arg Glu Gly
    50                  55                  60

Ile Asn Ala Glu Gln Ile Val Ile Lys Ile Thr Asp Gln Gly Tyr Val
65                  70                  75                  80

Thr Ser His Gly Asp His Tyr His Tyr Tyr Asn Gly Lys Val Pro Tyr
                85                  90                  95

Asp Ala Ile Ile Ser Glu Glu Leu Leu Met Lys Asp Pro Asn Tyr Gln
            100                 105                 110

Leu Lys Asp Ser Asp Ile Val Asn Glu Ile Lys Gly Gly Tyr Val Ile
        115                 120                 125

Lys Val Asp Gly Lys Tyr Tyr Val Tyr Leu Lys Asp Ala Ala His Ala
    130                 135                 140

Asp Asn Ile Arg Thr Lys Glu Glu Ile Lys Arg Gln Lys Gln Glu Arg
145                 150                 155                 160

Ser His Asn His Asn Ser Arg Ala Asp Asn Ala Val Ala Ala Ala Arg
                165                 170                 175

Ala Gln Gly Arg Tyr Thr Thr Asp Asp Gly Tyr Ile Phe Asn Ala Ser
            180                 185                 190

Asp Ile Ile Glu Asp Thr Gly Asp Ala Tyr Ile Val Pro His Gly Asp
        195                 200                 205

His Tyr His Tyr Ile Pro Lys Ser Asp Leu Ser Ala Ser Glu Leu Ala
    210                 215                 220

Ala Ala Gln Ala Tyr Trp Asn Gly Lys Gln Gly Ser Arg Pro Ser Ser
225                 230                 235                 240

Ser Ser Ser His Asn Ala Asn Pro Ala Gln Pro Arg Leu Ser Glu Asn
                245                 250                 255

His Asn Leu Thr Val Thr Pro Thr Tyr His Gln Asn Gln Gly Glu Asn
            260                 265                 270

Ile Ser Ser Leu Leu Arg Glu Leu Tyr Ala Lys Pro Leu Ser Glu Arg
        275                 280                 285

His Val Glu Ser Asp Gly Leu Ile Phe Asp Pro Ala Gln Ile Thr Ser
    290                 295                 300

Arg Thr Ala Asn Gly Val Ala Val Pro His Gly Asp His Tyr His Phe
305                 310                 315                 320

Ile Pro Tyr Ser Gln Leu Ser Pro Leu Glu Glu Lys Leu Ala Arg Ile
                325                 330                 335

Ile Pro Leu Arg Tyr Arg Ser Asn His Trp Val Pro Asp Ser Arg Pro
            340                 345                 350

Glu Gln Pro Ser Pro Gln Ser Thr Pro Glu Pro Ser Pro Ser Pro Gln
        355                 360                 365

Pro Ala Pro Asn Pro Gln Pro Ala Pro Ser Asn Pro Ile Asp Glu Lys

370                 375                 380

Leu Val Lys Glu Ala Val Arg Lys Val Gly Asp Gly Tyr Val Phe Glu
385                 390                 395                 400

Glu Asn Gly Val Pro Arg Tyr Ile Pro Ala Lys Asp Leu Ser Ala Glu
                405                 410                 415

Thr Ala Ala Gly Ile Asp Ser Lys Leu Ala Lys Gln Glu Ser Leu Ser
            420                 425                 430

His Lys Leu Gly Ala Lys Lys Thr Asp Leu Pro Ser Ser Asp Arg Glu
        435                 440                 445

Phe Tyr Asn Lys Ala Tyr Asp Leu Leu Ala Arg Ile His Gln Asp Leu
    450                 455                 460

Leu Asp Asn Lys Gly Arg Gln Val Asp Phe Glu Ala Leu Asp Asn Leu
465                 470                 475                 480

Leu Glu Arg Leu Lys Asp Val Ser Ser Asp Lys Val Lys Leu Val Asp
                485                 490                 495

Asp Ile Leu Ala Phe Leu Ala Pro Ile Arg His Pro Glu Arg Leu Gly
            500                 505                 510

Lys Pro Asn Ala Gln Ile Thr Tyr Thr Asp Asp Glu Ile Gln Val Ala
        515                 520                 525

Lys Leu Ala Gly Lys Tyr Thr Thr Glu Asp Gly Tyr Ile Phe Asp Pro
    530                 535                 540

Arg Asp Ile Thr Ser Asp Glu Gly Asp Ala Tyr Val Thr Pro His Met
545                 550                 555                 560

Thr His Ser His Trp Ile Lys Lys Asp Ser Leu Ser Glu Ala Glu Arg
                565                 570                 575

Ala Ala Ala Gln Ala Tyr Ala Lys Glu Lys Gly Leu Thr Pro Pro Ser
            580                 585                 590

Thr Asp His Gln Asp Ser Gly Asn Thr Glu Ala Lys Gly Ala Glu Ala
        595                 600                 605

Ile Tyr Asn Arg Val Lys Ala Ala Lys Lys Val Pro Leu Asp Arg Met
    610                 615                 620

Pro Tyr Asn Leu Gln Tyr Thr Val Glu Val Lys Asn Gly Ser Leu Ile
625                 630                 635                 640

Ile Pro His Tyr Asp His Tyr His Asn Ile Lys Phe Glu Trp Phe Asp
                645                 650                 655

Glu Gly Leu Tyr Glu Ala Pro Lys Gly Tyr Ser Leu Glu Asp Leu Leu
            660                 665                 670

Ala Thr Val Lys Tyr Tyr Val Glu His Pro Asn Glu Arg Pro His Ser
        675                 680                 685

Asp Asn Gly Phe Gly Asn Ala Ser Asp His Val Gln Arg Asn Lys Asn
    690                 695                 700

Gly Gln Ala Asp Thr Asn Gln Thr Glu Lys Pro Asn Glu Glu Lys Pro

705                 710                 715                 720

Gln Thr Glu Lys Pro Glu Glu Asp Lys Glu His Asp Glu Val Ser Glu
                725                 730                 735

Pro Thr His Pro Glu Ser Asp Glu Lys Glu Asn His Val Gly Leu Asn
            740                 745                 750

Pro Ser Ala Asp Asn Leu Tyr Lys Pro Ser Thr Asp Thr Glu Glu Thr
        755                 760                 765

Glu Glu Glu Ala Glu Asp Thr Thr Asp Glu Ala Glu Ile Pro Gln Val
    770                 775                 780

Glu His Ser Val Ile Asn Ala Lys Ile Ala Glu Ala Glu Ala Leu Leu
785                 790                 795                 800

Glu Lys Val Thr Asp Ser Ser Ile Arg Gln Asn Ala Val Glu Thr Leu
                805                 810                 815

Thr Gly Leu Lys Ser Ser Leu Leu Leu Gly Thr Lys Asp Asn Asn Thr
            820                 825                 830

Ile Ser Ala Glu Val Asp Ser Leu Leu Ala Leu Leu Lys Glu Ser Gln
        835                 840                 845

Pro Thr Pro Ile Gln
    850

<210>   58
<211>   1039
<212>   PRT
<213>   Streptococcus pneumoniae

<400>   58
Met Lys Phe Ser Lys Lys Tyr Ile Ala Ala Gly Ser Ala Val Ile Val
1               5                   10                  15

Ser Leu Ser Leu Cys Ala Tyr Ala Leu Asn Gln His Arg Ser Gln Glu
            20                  25                  30

Asn Lys Asp Asn Asn Arg Val Ser Tyr Val Asp Gly Ser Gln Ser Ser
        35                  40                  45

Gln Lys Ser Glu Asn Leu Thr Pro Asp Gln Val Ser Gln Lys Glu Gly
    50                  55                  60

Ile Gln Ala Glu Gln Ile Val Ile Lys Ile Thr Asp Gln Gly Tyr Val
65                  70                  75                  80

Thr Ser His Gly Asp His Tyr His Tyr Tyr Asn Gly Lys Val Pro Tyr
                85                  90                  95

Asp Ala Leu Phe Ser Glu Glu Leu Leu Met Lys Asp Pro Asn Tyr Gln
            100                 105                 110

Leu Lys Asp Ala Asp Ile Val Asn Glu Val Lys Gly Gly Tyr Ile Ile
        115                 120                 125

Lys Val Asp Gly Lys Tyr Tyr Val Tyr Leu Lys Asp Ala Ala His Ala
    130                 135                 140

Asp Asn Val Arg Thr Lys Asp Glu Ile Asn Arg Gln Lys Gln Glu His

145                 150                 155                 160

Val Lys Asp Asn Glu Lys Val Asn Ser Asn Val Ala Val Ala Arg Ser
                165                 170                 175

Gln Gly Arg Tyr Thr Thr Asn Asp Gly Tyr Val Phe Asn Pro Ala Asp
            180                 185                 190

Ile Ile Glu Asp Thr Gly Asn Ala Tyr Ile Val Pro His Gly Gly His
        195                 200                 205

Tyr His Tyr Ile Pro Lys Ser Asp Leu Ser Ala Ser Glu Leu Ala Ala
    210                 215                 220

Ala Lys Ala His Leu Ala Gly Lys Asn Met Gln Pro Ser Gln Leu Ser
225                 230                 235                 240

Tyr Ser Ser Thr Ala Ser Asp Asn Asn Thr Gln Ser Val Ala Lys Gly
                245                 250                 255

Ser Thr Ser Lys Pro Ala Asn Lys Ser Glu Asn Leu Gln Ser Leu Leu
            260                 265                 270

Lys Glu Leu Tyr Asp Ser Pro Ser Ala Gln Arg Tyr Ser Glu Ser Asp
        275                 280                 285

Gly Leu Val Phe Asp Pro Ala Lys Ile Ile Ser Arg Thr Pro Asn Gly
    290                 295                 300

Val Ala Ile Pro His Gly Asp His Tyr His Phe Ile Pro Tyr Ser Lys
305                 310                 315                 320

Leu Ser Ala Leu Glu Glu Lys Ile Ala Arg Arg Val Pro Ile Ser Gly
                325                 330                 335

Thr Gly Ser Thr Val Ser Thr Asn Ala Lys Pro Asn Glu Val Val Ser
            340                 345                 350

Ser Leu Gly Ser Leu Ser Ser Asn Pro Ser Ser Leu Thr Thr Ser Lys
        355                 360                 365

Glu Leu Ser Ser Ala Ser Asp Gly Tyr Ile Phe Asn Pro Lys Asp Ile
    370                 375                 380

Val Glu Glu Thr Ala Thr Ala Tyr Ile Val Arg His Gly Asp His Phe
385                 390                 395                 400

His Tyr Ile Pro Lys Ser Asn Gln Ile Gly Gln Pro Thr Leu Pro Asn
                405                 410                 415

Asn Ser Leu Ala Thr Pro Ser Pro Ser Leu Pro Ile Asn Pro Gly Ile
            420                 425                 430

Ser His Glu Lys His Glu Glu Asp Gly Tyr Gly Phe Asp Ala Asn Arg
        435                 440                 445

Ile Ile Ala Glu Asp Glu Ser Gly Phe Ile Met Ser His Gly Asn His
    450                 455                 460

Asn His Tyr Phe Phe Lys Lys Asp Leu Thr Glu Glu Gln Ile Lys Ala
465                 470                 475                 480

Ala Gln Lys His Leu Glu Glu Val Lys Thr Ser His Asn Gly Leu Asp

485                 490                 495

Ser Leu Ser Ser His Glu Gln Asp Tyr Pro Gly Asn Ala Lys Glu Met
            500                 505                 510

Lys Asp Leu Asp Lys Lys Ile Glu Glu Lys Ile Ala Gly Ile Met Lys
        515                 520                 525

Gln Tyr Gly Val Lys Arg Glu Ser Ile Val Val Asn Lys Glu Lys Asn
    530                 535                 540

Ala Ile Ile Tyr Pro His Gly Asp His His His Ala Asp Pro Ile Asp
545                 550                 555                 560

Glu His Lys Pro Val Gly Ile Gly His Ser His Ser Asn Tyr Glu Leu
                565                 570                 575

Phe Lys Pro Glu Glu Gly Val Ala Lys Lys Glu Gly Asn Lys Val Tyr
            580                 585                 590

Thr Gly Glu Glu Leu Thr Asn Val Val Asn Leu Leu Lys Asn Ser Thr
        595                 600                 605

Phe Asn Asn Gln Asn Phe Thr Leu Ala Asn Gly Gln Lys Arg Val Ser
    610                 615                 620

Phe Ser Phe Pro Pro Glu Leu Glu Lys Lys Leu Gly Ile Asn Met Leu
625                 630                 635                 640

Val Lys Leu Ile Thr Pro Asp Gly Lys Val Leu Glu Lys Val Ser Gly
                645                 650                 655

Lys Val Phe Gly Glu Gly Val Gly Asn Ile Ala Asn Phe Glu Leu Asp
            660                 665                 670

Gln Pro Tyr Leu Pro Gly Gln Thr Phe Lys Tyr Thr Ile Ala Ser Lys
        675                 680                 685

Asp Tyr Pro Glu Val Ser Tyr Asp Gly Thr Phe Thr Val Pro Thr Ser
    690                 695                 700

Leu Ala Tyr Lys Met Ala Ser Gln Thr Ile Phe Tyr Pro Phe His Ala
705                 710                 715                 720

Gly Asp Thr Tyr Leu Arg Val Asn Pro Gln Phe Ala Val Pro Lys Gly
                725                 730                 735

Thr Asp Ala Leu Val Arg Val Phe Asp Glu Phe His Gly Asn Ala Tyr
            740                 745                 750

Leu Glu Asn Asn Tyr Lys Val Gly Glu Ile Lys Leu Pro Ile Pro Lys
        755                 760                 765

Leu Asn Gln Gly Thr Thr Arg Thr Ala Gly Asn Lys Ile Pro Val Thr
    770                 775                 780

Phe Met Ala Asn Ala Tyr Leu Asp Asn Gln Ser Thr Tyr Ile Val Glu
785                 790                 795                 800

Val Pro Ile Leu Glu Lys Glu Asn Gln Thr Asp Lys Pro Ser Ile Leu
                805                 810                 815

Pro Gln Phe Lys Arg Asn Lys Ala Gln Glu Asn Ser Lys Leu Asp Glu

820                 825                 830

Lys Val Glu Glu Pro Lys Thr Ser Glu Lys Val Glu Lys Glu Lys Leu
        835                 840                 845

Ser Glu Thr Gly Asn Ser Thr Ser Asn Ser Thr Leu Glu Glu Val Pro
    850                 855                 860

Thr Val Asp Pro Val Gln Glu Lys Val Ala Lys Phe Ala Glu Ser Tyr
865                 870                 875                 880

Gly Met Lys Leu Glu Asn Val Leu Phe Asn Met Asp Gly Thr Ile Glu
                885                 890                 895

Leu Tyr Leu Pro Ser Gly Glu Val Ile Lys Lys Asn Met Ala Asp Phe
            900                 905                 910

Thr Gly Glu Ala Pro Gln Gly Asn Gly Glu Asn Lys Pro Ser Glu Asn
        915                 920                 925

Gly Lys Val Ser Thr Gly Thr Val Glu Asn Gln Pro Thr Glu Asn Lys
    930                 935                 940

Pro Ala Asp Ser Leu Pro Glu Ala Pro Asn Glu Lys Pro Val Lys Pro
945                 950                 955                 960

Glu Asn Ser Thr Asp Asn Gly Met Leu Asn Pro Glu Gly Asn Val Gly
                965                 970                 975

Ser Asp Pro Met Leu Asp Pro Ala Leu Glu Glu Ala Pro Ala Val Asp
            980                 985                 990

Pro Val Gln Glu Lys Leu Glu Lys Phe Thr Ala Ser Tyr Gly Leu Gly
        995                 1000                1005

Leu Asp Ser Val Ile Phe Asn Met Asp Gly Thr Ile Glu Leu Arg Leu
    1010                1015                1020

Pro Ser Gly Glu Val Ile Lys Lys Asn Leu Ser Asp Leu Ile Ala
1025                1030                1035

<210>   59
<211>   5
<212>   PRT
<213>   Artificial Sequence

<220>
<223>   Linker

<400>   59
Ala Ala Ala Leu Glu
1               5

<210>   60
<211>   274
<212>   PRT
<213>   Neisseria meningitidis

<400>   60
Met Asn Arg Thr Ala Phe Cys Cys Leu Ser Leu Thr Thr Ala Leu Ile
1               5                   10                  15

Leu Thr Ala Cys Ser Ser Gly Gly Gly Gly Val Ala Ala Asp Ile Gly

20                  25                  30

Ala Gly Leu Ala Asp Ala Leu Thr Ala Pro Leu Asp His Lys Asp Lys
        35                  40                  45

Gly Leu Gln Ser Leu Thr Leu Asp Gln Ser Val Arg Lys Asn Glu Lys
    50                  55                  60

Leu Lys Leu Ala Ala Gln Gly Ala Glu Lys Thr Tyr Gly Asn Gly Asp
65                  70                  75                  80

Ser Leu Asn Thr Gly Lys Leu Lys Asn Asp Lys Val Ser Arg Phe Asp
                85                  90                  95

Phe Ile Arg Gln Ile Glu Val Asp Gly Gln Leu Ile Thr Leu Glu Ser
            100                 105                 110

Gly Glu Phe Gln Val Tyr Lys Gln Ser His Ser Ala Leu Thr Ala Phe
        115                 120                 125

Gln Thr Glu Gln Ile Gln Asp Ser Glu His Ser Gly Lys Met Val Ala
    130                 135                 140

Lys Arg Gln Phe Arg Ile Gly Asp Ile Ala Gly Glu His Thr Ser Phe
145                 150                 155                 160

Asp Lys Leu Pro Glu Gly Gly Arg Ala Thr Tyr Arg Gly Thr Ala Phe
                165                 170                 175

Gly Ser Asp Asp Ala Gly Gly Lys Leu Thr Tyr Thr Ile Asp Phe Ala
            180                 185                 190

Ala Lys Gln Gly Asn Gly Lys Ile Glu His Leu Lys Ser Pro Glu Leu
        195                 200                 205

Asn Val Asp Leu Ala Ala Ala Asp Ile Lys Pro Asp Gly Lys Arg His
    210                 215                 220

Ala Val Ile Ser Gly Ser Val Leu Tyr Asn Gln Ala Glu Lys Gly Ser
225                 230                 235                 240

Tyr Ser Leu Gly Ile Phe Gly Gly Lys Ala Gln Glu Val Ala Gly Ser
                245                 250                 255

Ala Glu Val Lys Thr Val Asn Gly Ile Arg His Ile Gly Leu Ala Ala
            260                 265                 270

Lys Gln

<210>  61
<211>  273
<212>  PRT
<213>  Neisseria meningitidis

<400>  61
Met Asn Arg Thr Ala Phe Cys Cys Leu Ser Leu Thr Ala Ala Leu Ile
1               5                   10                  15

Leu Thr Ala Cys Ser Ser Gly Gly Gly Gly Val Ala Ala Asp Ile Gly
            20                  25                  30

Ala Gly Leu Ala Asp Ala Leu Thr Ala Pro Leu Asp His Lys Asp Lys

35                  40                  45

Ser Leu Gln Ser Leu Thr Leu Asp Gln Ser Val Arg Lys Asn Glu Lys
    50                  55                  60

Leu Lys Leu Ala Ala Gln Gly Ala Glu Lys Thr Tyr Gly Asn Gly Asp
65                  70                  75                  80

Ser Leu Asn Thr Gly Lys Leu Lys Asn Asp Lys Val Ser Arg Phe Asp
                85                  90                  95

Phe Ile Arg Gln Ile Glu Val Asp Gly Gln Leu Ile Thr Leu Glu Ser
            100                 105                 110

Gly Glu Phe Gln Ile Tyr Lys Gln Asp His Ser Ala Val Val Ala Leu
        115                 120                 125

Gln Ile Glu Lys Ile Asn Asn Pro Asp Lys Ile Asp Ser Leu Ile Asn
    130                 135                 140

Gln Arg Ser Phe Leu Val Ser Gly Leu Gly Gly Glu His Thr Ala Phe
145                 150                 155                 160

Asn Gln Leu Pro Asp Gly Lys Ala Glu Tyr His Gly Lys Ala Phe Ser
                165                 170                 175

Ser Asp Asp Ala Gly Gly Lys Leu Thr Tyr Thr Ile Asp Phe Ala Ala
            180                 185                 190

Lys Gln Gly His Gly Lys Ile Glu His Leu Lys Thr Pro Glu Gln Asn
        195                 200                 205

Val Glu Leu Ala Ala Ala Glu Leu Lys Ala Asp Glu Lys Ser His Ala
    210                 215                 220

Val Ile Leu Gly Asp Thr Arg Tyr Gly Ser Glu Glu Lys Gly Thr Tyr
225                 230                 235                 240

His Leu Ala Leu Phe Gly Asp Arg Ala Gln Glu Ile Ala Gly Ser Ala
                245                 250                 255

Thr Val Lys Ile Gly Glu Lys Val His Glu Ile Gly Ile Ala Gly Lys
            260                 265                 270

Gln

<210>  62
<211>  281
<212>  PRT
<213>  Neisseria meningitidis

<400>  62
Met Asn Arg Thr Ala Phe Cys Cys Leu Ser Leu Thr Thr Ala Leu Ile
1               5                   10                  15

Leu Thr Ala Cys Ser Ser Gly Gly Gly Gly Ser Gly Gly Gly Gly Val
            20                  25                  30

Ala Ala Asp Ile Gly Thr Gly Leu Ala Asp Ala Leu Thr Ala Pro Leu
        35                  40                  45

Asp His Lys Asp Lys Gly Leu Lys Ser Leu Thr Leu Glu Asp Ser Ile

50                  55                  60

Pro Gln Asn Gly Thr Leu Thr Leu Ser Ala Gln Gly Ala Glu Lys Thr
65                  70                  75                  80

Phe Lys Ala Gly Asp Lys Asp Asn Ser Leu Asn Thr Gly Lys Leu Lys
                85                  90                  95

Asn Asp Lys Ile Ser Arg Phe Asp Phe Val Gln Lys Ile Glu Val Asp
            100                 105                 110

Gly Gln Thr Ile Thr Leu Ala Ser Gly Glu Phe Gln Ile Tyr Lys Gln
        115                 120                 125

Asn His Ser Ala Val Val Ala Leu Gln Ile Glu Lys Ile Asn Asn Pro
    130                 135                 140

Asp Lys Thr Asp Ser Leu Ile Asn Gln Arg Ser Phe Leu Val Ser Gly
145                 150                 155                 160

Leu Gly Gly Glu His Thr Ala Phe Asn Gln Leu Pro Gly Gly Lys Ala
                165                 170                 175

Glu Tyr His Gly Lys Ala Phe Ser Ser Asp Asp Pro Asn Gly Arg Leu
            180                 185                 190

His Tyr Ser Ile Asp Phe Thr Lys Lys Gln Gly Tyr Gly Arg Ile Glu
        195                 200                 205

His Leu Lys Thr Leu Glu Gln Asn Val Glu Leu Ala Ala Ala Glu Leu
    210                 215                 220

Lys Ala Asp Glu Lys Ser His Ala Val Ile Leu Gly Asp Thr Arg Tyr
225                 230                 235                 240

Gly Ser Glu Glu Lys Gly Thr Tyr His Leu Ala Leu Phe Gly Asp Arg
                245                 250                 255

Ala Gln Glu Ile Ala Gly Ser Ala Thr Val Lys Ile Gly Glu Lys Val
            260                 265                 270

His Glu Ile Gly Ile Ala Gly Lys Gln
        275                 280

<210>   63
<211>   260
<212>   PRT
<213>   Neisseria meningitidis

<400>   63
Cys Ser Ser Gly Gly Gly Gly Ser Gly Gly Gly Gly Val Thr Ala Asp
1               5                   10                  15

Ile Gly Thr Gly Leu Ala Asp Ala Leu Thr Ala Pro Leu Asp His Lys
            20                  25                  30

Asp Lys Gly Leu Lys Ser Leu Thr Leu Glu Asp Ser Ile Ser Gln Asn
        35                  40                  45

Gly Thr Leu Thr Leu Ser Ala Gln Gly Ala Glu Lys Thr Tyr Gly Asn
    50                  55                  60

Gly Asp Ser Leu Asn Thr Gly Lys Leu Lys Asn Asp Lys Val Ser Arg

65                  70                  75                  80

Phe Asp Phe Ile Arg Gln Ile Glu Val Asp Gly Gln Leu Ile Thr Leu
                85                  90                  95

Glu Ser Gly Glu Phe Gln Val Tyr Lys Gln Ser His Ser Ala Leu Thr
            100                 105                 110

Ala Leu Gln Thr Glu Gln Glu Gln Asp Pro Glu His Ser Glu Lys Met
        115                 120                 125

Val Ala Lys Arg Arg Phe Arg Ile Gly Asp Ile Ala Gly Glu His Thr
    130                 135                 140

Ser Phe Asp Lys Leu Pro Lys Asp Val Met Ala Thr Tyr Arg Gly Thr
145                 150                 155                 160

Ala Phe Gly Ser Asp Asp Ala Gly Gly Lys Leu Thr Tyr Thr Ile Asp
                165                 170                 175

Phe Ala Ala Lys Gln Gly His Gly Lys Ile Glu His Leu Lys Ser Pro
            180                 185                 190

Glu Leu Asn Val Asp Leu Ala Val Ala Tyr Ile Lys Pro Asp Glu Lys
        195                 200                 205

His His Ala Val Ile Ser Gly Ser Val Leu Tyr Asn Gln Asp Glu Lys
    210                 215                 220

Gly Ser Tyr Ser Leu Gly Ile Phe Gly Glu Lys Ala Gln Glu Val Ala
225                 230                 235                 240

Gly Ser Ala Glu Val Glu Thr Ala Asn Gly Ile His His Ile Gly Leu
                245                 250                 255

Ala Ala Lys Gln
            260

<210>   64
<211>   262
<212>   PRT
<213>   Neisseria meningitidis

<400>   64
Cys Ser Ser Gly Gly Gly Gly Ser Gly Gly Gly Gly Val Ala Ala Asp
1               5                   10                  15

Ile Gly Ala Gly Leu Ala Asp Ala Leu Thr Ala Pro Leu Asp His Lys
            20                  25                  30

Asp Lys Gly Leu Lys Ser Leu Thr Leu Glu Asp Ser Ile Ser Gln Asn
        35                  40                  45

Gly Thr Leu Thr Leu Ser Ala Gln Gly Ala Glu Arg Thr Phe Lys Ala
    50                  55                  60

Gly Asp Lys Asp Asn Ser Leu Asn Thr Gly Lys Leu Lys Asn Asp Lys
65                  70                  75                  80

Ile Ser Arg Phe Asp Phe Ile Arg Gln Ile Glu Val Asp Gly Gln Leu
                85                  90                  95

Ile Thr Leu Glu Ser Gly Glu Phe Gln Val Tyr Lys Gln Ser His Ser

            100                 105                 110

Ala Leu Thr Ala Leu Gln Thr Glu Gln Val Gln Asp Ser Glu His Ser
        115                 120                 125

Gly Lys Met Val Ala Lys Arg Gln Phe Arg Ile Gly Asp Ile Val Gly
    130                 135                 140

Glu His Thr Ser Phe Asp Lys Leu Pro Lys Asp Val Met Ala Thr Tyr
145                 150                 155                 160

Arg Gly Thr Ala Phe Gly Ser Asp Asp Ala Gly Gly Lys Leu Thr Tyr
                165                 170                 175

Thr Ile Asp Ala Ala Lys Gln Gly His Gly Lys Ile Glu His Leu Lys
            180                 185                 190

Ser Pro Glu Leu Asn Val Asp Leu Ala Ala Ala Asp Ile Lys Pro Asp
        195                 200                 205

Glu Lys His His Ala Val Ile Ser Gly Ser Val Leu Tyr Asn Gln Ala
    210                 215                 220

Glu Lys Gly Ser Tyr Ser Leu Gly Ile Phe Gly Gly Gln Ala Gln Glu
225                 230                 235                 240

Val Ala Gly Ser Ala Glu Val Glu Thr Ala Asn Gly Ile Arg His Ile
                245                 250                 255

Gly Leu Ala Ala Lys Gln
            260

<210> 65
<211> 261
<212> PRT
<213> Neisseria meningitidis

<400> 65
Cys Ser Ser Gly Ser Gly Ser Gly Gly Gly Gly Val Ala Ala Asp Ile
1               5                   10                  15

Gly Thr Gly Leu Ala Asp Ala Leu Thr Ala Pro Leu Asp His Lys Asp
            20                  25                  30

Lys Gly Leu Lys Ser Leu Thr Leu Glu Asp Ser Ile Ser Gln Asn Gly
        35                  40                  45

Thr Leu Thr Leu Ser Ala Gln Gly Ala Glu Lys Thr Phe Lys Val Gly
    50                  55                  60

Asp Lys Asp Asn Ser Leu Asn Thr Gly Lys Leu Lys Asn Asp Lys Ile
65                  70                  75                  80

Ser Arg Phe Asp Phe Val Gln Lys Ile Glu Val Asp Gly Gln Thr Ile
                85                  90                  95

Thr Leu Ala Ser Gly Glu Phe Gln Ile Tyr Lys Gln Asp His Ser Ala
            100                 105                 110

Val Val Ala Leu Gln Ile Glu Lys Ile Asn Asn Pro Asp Lys Ile Asp
        115                 120                 125

Ser Leu Ile Asn Gln Arg Ser Phe Leu Val Ser Gly Leu Gly Gly Glu

130                 135                 140

His Thr Ala Phe Asn Gln Leu Pro Ser Gly Lys Ala Glu Tyr His Gly
145                 150                 155                 160

Lys Ala Phe Ser Ser Asp Asp Ala Gly Gly Lys Leu Thr Tyr Thr Ile
                165                 170                 175

Asp Phe Ala Ala Lys Gln Gly His Gly Lys Ile Glu His Leu Lys Thr
            180                 185                 190

Pro Glu Gln Asn Val Glu Leu Ala Ser Ala Glu Leu Lys Ala Asp Glu
        195                 200                 205

Lys Ser His Ala Val Ile Leu Gly Asp Thr Arg Tyr Gly Ser Glu Glu
    210                 215                 220

Lys Gly Thr Tyr His Leu Ala Leu Phe Gly Asp Arg Ala Gln Glu Ile
225                 230                 235                 240

Ala Gly Ser Ala Thr Val Lys Ile Arg Glu Lys Val His Glu Ile Gly
                245                 250                 255

Ile Ala Gly Lys Gln
            260

<210>   66
<211>   248
<212>   PRT
<213>   Neisseria meningitidis

<400>   66
Val Thr Ala Asp Ile Gly Thr Gly Leu Ala Asp Ala Leu Thr Ala Pro
1               5                   10                  15

Leu Asp His Lys Asp Lys Gly Leu Lys Ser Leu Thr Leu Glu Asp Ser
            20                  25                  30

Ile Ser Gln Asn Gly Thr Leu Thr Leu Ser Ala Gln Gly Ala Glu Lys
        35                  40                  45

Thr Tyr Gly Asn Gly Asp Ser Leu Asn Thr Gly Lys Leu Lys Asn Asp
    50                  55                  60

Lys Val Ser Arg Phe Asp Phe Ile Arg Gln Ile Glu Val Asp Gly Gln
65                  70                  75                  80

Leu Ile Thr Leu Glu Ser Gly Glu Phe Gln Val Tyr Lys Gln Ser His
                85                  90                  95

Ser Ala Leu Thr Ala Leu Gln Thr Glu Gln Glu Gln Asp Pro Glu His
            100                 105                 110

Ser Glu Lys Met Val Ala Lys Arg Arg Phe Arg Ile Gly Asp Ile Ala
        115                 120                 125

Gly Glu His Thr Ser Phe Asp Lys Leu Pro Lys Asp Val Met Ala Thr
    130                 135                 140

Tyr Arg Gly Thr Ala Phe Gly Ser Asp Asp Ala Gly Gly Lys Leu Thr
145                 150                 155                 160

Tyr Thr Ile Asp Phe Ala Ala Lys Gln Gly His Gly Lys Ile Glu His

165                 170                 175

Leu Lys Ser Pro Glu Leu Asn Val Asp Leu Ala Val Ala Tyr Ile Lys
            180                 185                 190

Pro Asp Glu Lys His His Ala Val Ile Ser Gly Ser Val Leu Tyr Asn
        195                 200                 205

Gln Asp Glu Lys Gly Ser Tyr Ser Leu Gly Ile Phe Gly Glu Lys Ala
    210                 215                 220

Gln Glu Val Ala Gly Ser Ala Glu Val Glu Thr Ala Asn Gly Ile His
225                 230                 235                 240

His Ile Gly Leu Ala Ala Lys Gln
                245

<210>   67
<211>   250
<212>   PRT
<213>   Neisseria meningitidis

<400>   67
Val Ala Ala Asp Ile Gly Ala Gly Leu Ala Asp Ala Leu Thr Ala Pro
1               5                   10                  15

Leu Asp His Lys Asp Lys Gly Leu Lys Ser Leu Thr Leu Glu Asp Ser
            20                  25                  30

Ile Ser Gln Asn Gly Thr Leu Thr Leu Ser Ala Gln Gly Ala Glu Arg
        35                  40                  45

Thr Phe Lys Ala Gly Asp Lys Asp Asn Ser Leu Asn Thr Gly Lys Leu
    50                  55                  60

Lys Asn Asp Lys Ile Ser Arg Phe Asp Phe Ile Arg Gln Ile Glu Val
65                  70                  75                  80

Asp Gly Gln Leu Ile Thr Leu Glu Ser Gly Glu Phe Gln Val Tyr Lys
                85                  90                  95

Gln Ser His Ser Ala Leu Thr Ala Leu Gln Thr Glu Gln Val Gln Asp
            100                 105                 110

Ser Glu His Ser Gly Lys Met Val Ala Lys Arg Gln Phe Arg Ile Gly
        115                 120                 125

Asp Ile Val Gly Glu His Thr Ser Phe Asp Lys Leu Pro Lys Asp Val
    130                 135                 140

Met Ala Thr Tyr Arg Gly Thr Ala Phe Gly Ser Asp Asp Ala Gly Gly
145                 150                 155                 160

Lys Leu Thr Tyr Thr Ile Asp Ala Ala Lys Gln Gly His Gly Lys Ile
                165                 170                 175

Glu His Leu Lys Ser Pro Glu Leu Asn Val Asp Leu Ala Ala Ala Asp
            180                 185                 190

Ile Lys Pro Asp Glu Lys His His Ala Val Ile Ser Gly Ser Val Leu
        195                 200                 205

Tyr Asn Gln Ala Glu Lys Gly Ser Tyr Ser Leu Gly Ile Phe Gly Gly

210                 215                 220

Gln Ala Gln Glu Val Ala Gly Ser Ala Glu Val Glu Thr Ala Asn Gly
225                 230                 235                 240

Ile Arg His Ile Gly Leu Ala Ala Lys Gln
                245                 250

<210>   68
<211>   250
<212>   PRT
<213>   Neisseria meningitidis

<400>   68
Val Ala Ala Asp Ile Gly Thr Gly Leu Ala Asp Ala Leu Thr Ala Pro
1               5                   10                  15

Leu Asp His Lys Asp Lys Gly Leu Lys Ser Leu Thr Leu Glu Asp Ser
            20                  25                  30

Ile Ser Gln Asn Gly Thr Leu Thr Leu Ser Ala Gln Gly Ala Glu Lys
        35                  40                  45

Thr Phe Lys Val Gly Asp Lys Asp Asn Ser Leu Asn Thr Gly Lys Leu
    50                  55                  60

Lys Asn Asp Lys Ile Ser Arg Phe Asp Phe Val Gln Lys Ile Glu Val
65                  70                  75                  80

Asp Gly Gln Thr Ile Thr Leu Ala Ser Gly Glu Phe Gln Ile Tyr Lys
                85                  90                  95

Gln Asp His Ser Ala Val Val Ala Leu Gln Ile Glu Lys Ile Asn Asn
            100                 105                 110

Pro Asp Lys Ile Asp Ser Leu Ile Asn Gln Arg Ser Phe Leu Val Ser
        115                 120                 125

Gly Leu Gly Gly Glu His Thr Ala Phe Asn Gln Leu Pro Ser Gly Lys
    130                 135                 140

Ala Glu Tyr His Gly Lys Ala Phe Ser Ser Asp Asp Ala Gly Gly Lys
145                 150                 155                 160

Leu Thr Tyr Thr Ile Asp Phe Ala Ala Lys Gln Gly His Gly Lys Ile
                165                 170                 175

Glu His Leu Lys Thr Pro Glu Gln Asn Val Glu Leu Ala Ser Ala Glu
            180                 185                 190

Leu Lys Ala Asp Glu Lys Ser His Ala Val Ile Leu Gly Asp Thr Arg
        195                 200                 205

Tyr Gly Ser Glu Glu Lys Gly Thr Tyr His Leu Ala Leu Phe Gly Asp
    210                 215                 220

Arg Ala Gln Glu Ile Ala Gly Ser Ala Thr Val Lys Ile Arg Glu Lys
225                 230                 235                 240

Val His Glu Ile Gly Ile Ala Gly Lys Gln
                245                 250